(19) Europäisches Patentamt
European Patent Office
Office européen des brevets

(11) **EP 3 760 992 B1**

(12) **EUROPÄISCHE PATENTSCHRIFT**

(45) Veröffentlichungstag und Bekanntmachung des Hinweises auf die Patenterteilung:
**20.04.2022 Patentblatt 2022/16**

(21) Anmeldenummer: **20167242.5**

(22) Anmeldetag: **31.03.2020**

(51) Internationale Patentklassifikation (IPC):
**G01J 3/453** (2006.01)   **G01J 3/28** (2006.01)
**G01J 3/02** (2006.01)

(52) Gemeinsame Patentklassifikation (CPC):
**G01J 3/453; G01J 3/0229; G01J 3/2823**

(54) **VERFAHREN UND FOURIER-TRANSFORMATIONS-SPEKTROMETER MIT ZWEISTRAHL-INTERFEROMETER ZUR SINGLE-SHOT-IMAGING-FOURIER-SPEKTROSKOPIE**

METHOD AND FOURIER TRANSFORM SPECTROMETER WITH DUAL BEAM INTERFEROMETER FOR SINGLE-SHOT-IMAGING FOURIER SPECTROSCOPY

PROCÉDÉ ET SPECTROMÈTRE À TRANSFORMÉE DE FOURIER POURVU D'INTERFÉROMÈTRE À DEUX RAYONS DESTINÉS À LA SPECTROSCOPIE PAR TRANSFORMÉE DE FOURIER À L'IMAGERIE À PRISE UNIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.01.2021 Patentblatt 2021/01**

(73) Patentinhaber: **Universität Stuttgart**
**70174 Stuttgart (DE)**

(72) Erfinder:
• **KÖRNER, Klaus**
**10367 Berlin (DE)**
• **HERKOMMER, Alois M.**
**73431 Aalen (DE)**

(74) Vertreter: **Müller-Boré & Partner**
**Patentanwälte PartG mbB**
**Friedenheimer Brücke 21**
**80639 München (DE)**

(56) Entgegenhaltungen:
**WO-A1-2013/140396    US-A1- 2019 162 520**

• **PENG GAO ET AL: "Parallel two-step phase-shifting point-diffraction interferometry for microscopy based on a pair of cube beamsplitters", OPTICS EXPRESS, Bd. 19, Nr. 3, 31. Januar 2011 (2011-01-31), Seite 1930, XP055219411, US ISSN: 2161-2072, DOI: 10.1364/OE.19.001930**

EP 3 760 992 B1

**Beschreibung**

Die Erfindung betrifft ein Fourier-Transformations-(FT-)Spektrometer mit Zweistrahl-Interferometer und ein Verfahren zur interferometrischen Messung, jeweils insbesondere für die Single-Shot-Imaging-Fourier-Spektroskopie zur Gewinnung hyperspektraler Teilbilder auf Basis optimierter Anordnungen und optimierter Lagen von Bildfeld-Diskriminatoren, die stets innerhalb des Zweistrahl-Interferometers angeordnet sind.

Insbesondere kann die Single-Shot-Fourier-Transformations-Spektroskopie mit einer vergleichsweise niedrigen bis zu einer moderaten spektralen Auflösung verwendet werden. Bevorzugt liegt die spektrale Auflösung bei dieser Spektroskopie in einem Bereich von etwa 4cm⁻¹ bis etwa 1000cm⁻¹ (1cm⁻¹ = 1/cm = reciprocal centimeters, Einheit der Wellenzahl), wobei der Spektralbereich bevorzugt vom Ultravioletten bis zum Terahertz-Bereich der Wellenlänge elektromagnetischer Strahlung adressiert wird.

Ein Spektralbild kann insbesondere ein hyperspektrales Bild sein, welches in der Regel in Form eines Datenkubus (x, y, sigma) mit der Wellenzahl sigma oder (x, y, lambda) mit der Wellenlänge lambda vorliegen kann.

Ein Anwendungsgebiet der Erfindung kann die Untersuchung der Haut am Menschen durch einen Arzt, beispielsweise beim Hautkrebs-Sreening sein. Ein weiteres Anwendungsgebiet kann bei einer chirurgischen Operation am lebenden Menschen die Untersuchung von zumindest teilweise freigelegten Organgeweben darstellen. Ein weiteres Anwendungsgebiet kann die Inspektion des Augeninneren, beispielsweise die Untersuchung der Netzhaut darstellen.

Ein anderes Anwendungsgebiet kann beispielsweise die Analyse von Lebensmitteln sein, wenn diese insbesondere auf einem unruhig und/oder ruckelnd laufenden Transportband bewegt werden. Dazu zählt auch die Prüfung landwirtschaftlich erzeugter Lebensmittel wie beispielsweise Schüttgut-Produkte wie Getreide und Hülsenfrüchte, wo eine 100%-Prüfung am laufenden Band erforderlich ist und/oder zumindest eine hohe Stichprobenzahl genommen werden muss.

Ferner kann die Erfindung bei der Fluoreszenzlicht-Analyse von Objekten und Szenen in UV-Auflicht mit Auswertung des Fluoreszenzlichts als Informationsträger angewandt werden.

Darüber hinaus kann das Messen in sich bewegter Objekte, wie ausbrechende Vulkane und/oder Feuerstürme bei Waldbränden, mit einer relativ gleichmäßigen Bewegung des Spektrometers beim Überflug mittels eines Helikopters und/oder eines Flugzeugs für die Applikation der Erfindung zugänglich sein.

Auch wirbelnde Partikel, beispielsweise in einer Strömung, sind mit dem erfindungsgemäßen Ansatz spektral und zumindest mit Einschränkungen ortsaufgelöst, also zumindest teilweise bildlich, erfassbar.

Es ist auch möglich, verschiedenartige Kunststoffe im Hausmüll und/oder in Industrieabfällen anhand des Spektrums selbst in der Bewegung zu identifizieren und der Sortierung zuzuführen. Es ist auch möglich, bei der Müllsortierung an Land und/oder auch im Wasser eines Ozeans bewegte Objekte aus verschiedenartigen Kunststoffen anhand des Spektrums zu identifizieren und gegebenenfalls die Sortierung vor Ort zu ermöglichen.

Bei Messungen mit dem erfindungsgemäßen Spektrometer aus der Hand durch einen manuellen Scan über das Objekt können Objekte aus der Spurensicherung in der Kriminalistik, aus der Medizin, Hygiene, Archäologie, Botanik, Mineralogie, Landwirtschaft hyperspektral, typischerweise mit deutlich mehr als 10 Spektralkanälen pro Messpunkt und mit Ortsauflösung analysiert werden. Dabei steht in der Regel die spektrale Information im Vordergrund und nicht das perfekte hyperspektrale Bild vom Messobjekt. Dieses hyperspektrale Bild kann trotz einer aufwendigen Bildnachbearbeitung gewisse Rest-Artefakte aufweisen, was in vielen Fällen jedoch als tolerierbar gilt, da bei einer erheblichen Anzahl von Messaufgaben das Hauptinteresse der spektralen Information gilt, die weitestgehend ohne Durchschlupf, also möglichst vollumfänglich erfasst werden soll.

Des Weiteren kann die Erfindung zur Wärmebildgebung im Nah- und Fernbereich eingesetzt werden. Es ist aber auch möglich, die Erfindung insbesondere im Terahertz-Bereich für Flughafen-Scanner in Sicherheitsschleusen für Personen und Transportgüter im Durchlichtverfahren mit Spektralauflösung einzusetzen.

Der Single-Shot-Ansatz ermöglicht auch den Einsatz von vergleichsweise kostengünstigen Lichtquellen, die hinsichtlich der Ausgangsleistung zeitlich nicht sehr stabil sind, also Schwankungen der Ausgangsleistung von durchaus 10% und mehr aufweisen können. Schwankungen der Ausgangsleistung stellen bei seriellen Verfahren zur Aufnahme von Interferogrammen schon ein erhebliches Problem dar.

Stand der Technik

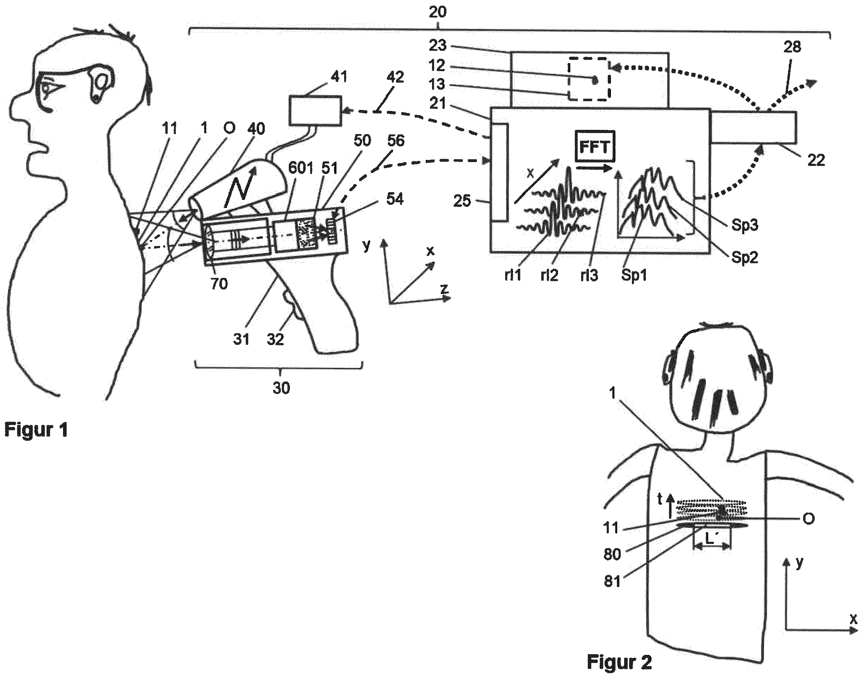

Das Patent US 4976542 von Smith mit einem zyklischen Interferometer in Form des Sagnac-Interferometers stellt eine Common-path-Anordnung dar. Dort ist in Figur 1 eine Sagnac-Interferometer-Anordnung mit einer Zylinderoptik 38 in Kombination mit einer Fourier-Optik 36 beschrieben. Eine Spaltöffnung 24 wird von der Zylinderoptik 38 in Spaltlängsrichtung scharf auf einem gerasterten Detektor, hier als CCD-Chip abgebildet. Die Zylinderoptik 38 in Kombination mit der Fourier-Optik 36 stellt eine anamorpotische Abbildungsstufe dar. In Spaltquerrichtung entsteht durch die Wirkung der Fourier-Optik 36 in Kombination mit dem Lateral-Shear erzeugenden Sagnac-Interferometer eine Vielzahl von räumlichen Interferogrammen auf dem CCD-Chip. So besteht die Möglichkeit, im Single-Shot-Modus räumliche Interferogramme zu detektieren. Die Spaltöffnung, die hier also als Bildfeldblende oder als Bildfeld-Diskriminator wirkt, ist jedoch vor dem Sagnac-Interferometers angeordnet. Das reduziert den Öffnungswinkel des Strahlenbündels im Sagnac-Interferometer aufgrund der hier vergleichsweise langen optische Wege ganz erheblich.

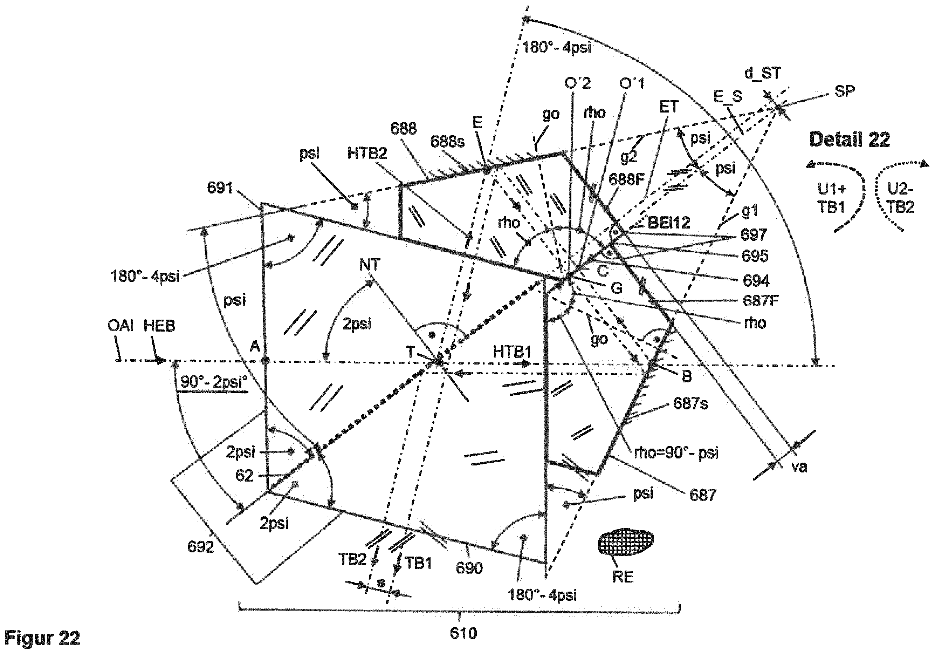

Im Patent US 5777736 von Horton ist ein Interferometer vom Mach-Zehnder-Typ beschrieben, welches vergleichsweise lange optische Wege im Interferometer im Vergleich zu einem Michelson-Interferometer aufweist. Als Interferometer vom Mach-Zehnder-Typ ist es grundsätzlich schwerer zu justieren - im Vergleich zu einem Michelsontyp-Interferometer, welches nur einen einzigen Strahlteiler aufweist. Diese Schwierigkeit besteht auch aufgrund der Anordnung von zwei Strahlteilern im Mach-Zehnder-Interferometer. Demzufolge weist ein Interferometer vom Mach-Zehnder-Typ eher keine besonders hohe Langzeitstabilität ohne erheblichen technischen Aufwand auf und ist nur bedingt kompakt aufzubauen. In Figur 22 dieses Patents besteht ein starker Astigmatismus, bedingt durch den jeweils einmaligen Durchgang durch eine geneigte Strahlteilerplatte, der im interferometrischen Strahlengang nicht kompensiert wird. Das kann Nichtlinearitäten im räumlichen Interferogramm mit sich bringen, welche die Spektrenberechnung erheblich erschweren, bzw. zu sehr unerwünschten Artefakten im berechneten Spektrum führen. Es entsteht das Vollbild des Messobjekts, der Szene oder der Lichtquelle auf dem Detektor, überlagert von einem einzigen räumlichen Interferogramm, da als Relay-Optik ein Sammelobjektiv, dort als "exit lens" bezeichnet, angeordnet ist. So ist nur eine serielle Interferogramm-Gewinnung mittels einer Relativbewegung zwischen Interferometer und Messobjekt und kein Single-Shot-Betrieb möglich, da eine gleichmäßige Bewegung der Szene bei der Interferogramm-Gewinnung erforderlich ist und das Interferogramm stets seriell entsteht und erst aus einem Bilderstapel für jeden angemessenen Objektpunkt extrahiert werden muss. Weiterhin sind in der Schrift US 5777736 keine Bildfeld-Diskriminatoren im Interferometer in der Bildposition angeordnet.

Im Patent US3684379A ist von Girard ein kompaktes Michelsontyp-Interferometer mit Keilinterferenzen für den Feldeinsatz beschrieben. Das Bild entsteht auf den beiden Planspiegeln eines Michelson-Interferometers. Es gibt jedoch keinen Bildfeld-Diskriminator im Interferometer. Da es sich um geöffnete Bündel handelt, kann der hier eingeführte Keil unerwünschte Wellenfront-Aberrationen erzeugen, welche ein räumliches Interferogramm erheblich stören können. Dies kann bei der Auswertung durch eine Fourier-Transformation erhebliche Probleme bereiteten und zu nicht akzeptablen Fehlern im Spektrum führen.

Im Patent US 4523846 von Breckingridge ist ebenfalls ein sehr kompaktes Michelsontyp-Interferometer in monolithischer Bauweise mit einer nichtrechtwinkligen Anordnung eines Planspiegels als Interferometer-Endspiegel beschrieben. So entsteht die Interferenz zueinander verkippter Wellenfronten. Auch hier kann der in das Interferometer eingeführte Keil aus refraktivem Material Wellenfront-Aberrationen erzeugen, welche ein räumliches Interferogramm erheblich stören können. Eine Spaltöffnung zur Diskriminierung des Bildfeldes, die als effektive Quelle wirkt, befindet sich hierbei am Eingang des Interferometers, also außerhalb desselben. In einer Position des Instruments wird offensichtlich nur das räumliche Interferogramm des gesamten Schlitzes gewonnen, welches in der Pupillenebene detektiert wird. Innerhalb des länglichen Schlitzes gibt es also offenbar keine Ortsauflösung. Die konvexe Fläche mit Spaltblende am Interferometereingang stellt eine Feldlinse dar, steht also am Bildort oder näherungsweise in einer Zwischenbildebene. So kann bei einer Relativbewegung zwischen Interferometer und Messobjekt, hier beispielsweise im Satellitenüberflug einer Landschaft, aus den sequenziell detektierten räumlichen Interferogrammen ein eindimensionales Hyperspektralbild nach dem Pushbroom-Prinzip erstellt werden. Dabei liefert zu einem Zeitpunkt offensichtlich stets nur ein einziger, hier streifenförmiger Bildausschnitt, ein räumliches Interferogramm. Aufgrund der Anordnung der Spaltöffnung am Eingang des Interferometers ist der Öffnungswinkel für das Bündel aufgrund der optischen Weglänge vom Eingang bis zum Ausgang des Interferometers etwas begrenzt. Jedoch kann noch ein halber Öffnungswinkel von gut 10° erreicht werden. Demzufolge kann die Lichtausbeute durch den limitierten Öffnungswinkel jedoch etwas begrenzt sein. Dies kann zu einem nichtoptimalen Signal-Rausch-Verhältnis im gemessenen Interferogramm und somit auch im errechneten Spektrum führen.

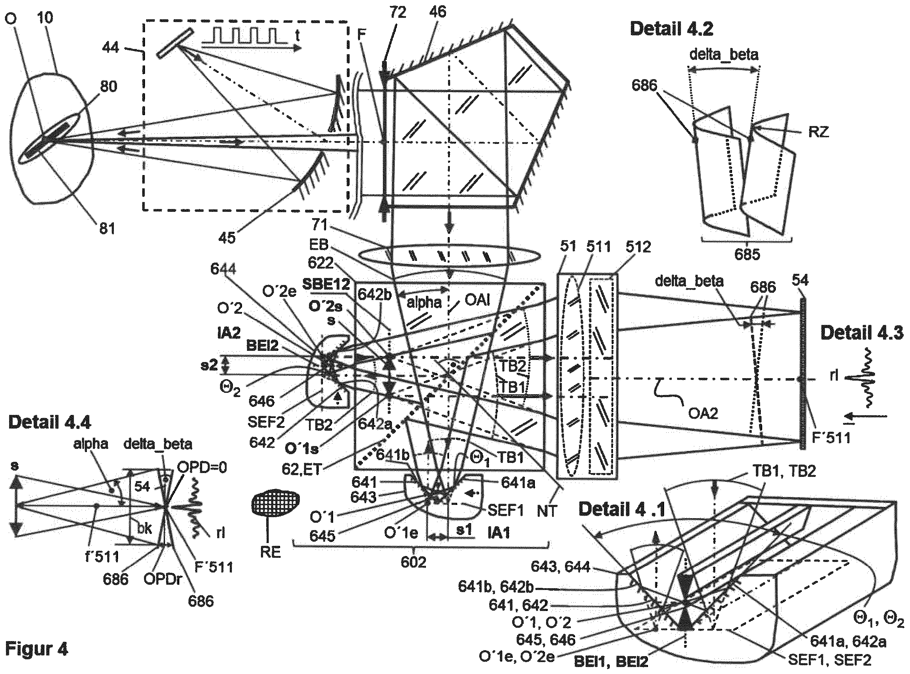

Die Lateral-Shear kann in einer optischen Anordnung als Grundlage zur Generierung von Interferenzen zueinander geneigter Wellenfronten genutzt werden. Einen ganz klassischen Ansatz stellt dafür eine Michelson-Interferometer-Anordnung mit zwei Dachkant-Reflektoren dar, um die erforderliche Lateral-Shear zu erzeugen. Dieser Ansatz mit zwei Dachkant-Reflektoren wird in der Regel auch zur Wellenfrontanalyse eingesetzt und ist den Fachleuten gut bekannt, siehe dazu auch Malacara, Optical Shop Testing, John Wiley & Sons, Inc., 1992, S. 140 -141, Figur 4. 16 [1] und auch Steel, Interferometry, Cambridge University Press, 1967, S. 83 letzter Absatz bis S. 84 oben [2].

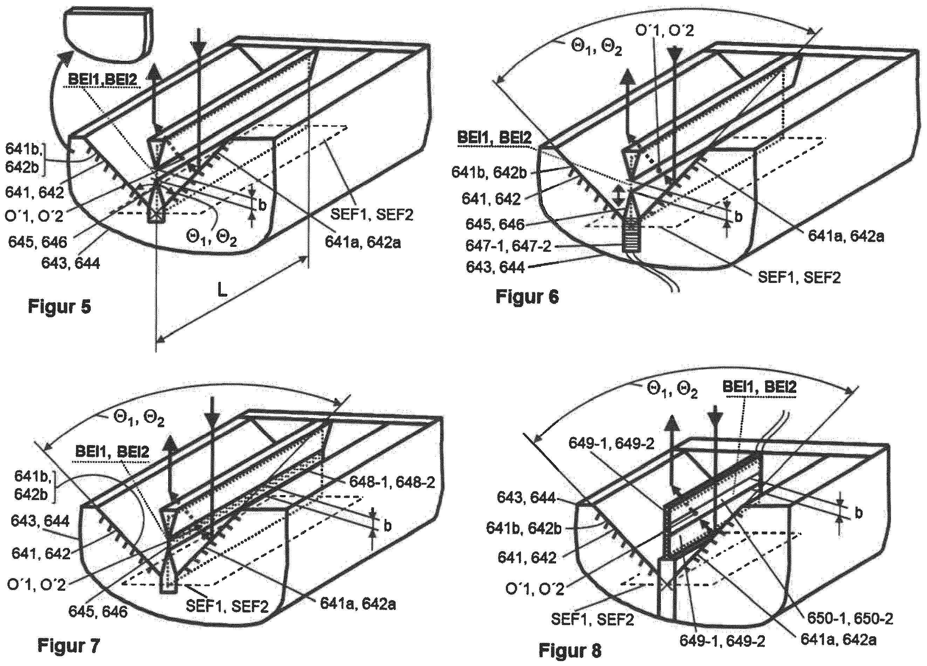

Bekannt ist auch der von Kelsall im Jahr 1959 in Proc. Phys. Society, 73, S. 470, Fig. 1 [3] veröffentlichte Ansatz mit zwei Tripelreflektoren als Endreflektoren eines Michelson-Interferometers. Die laterale Verschiebung eines Tripelreflektors erzeugt ebenfalls eine Lateral-Shear zwischen Objekt- und Referenzwellenfronten am Ausgang eines Michelson-Interferometers. Der Einsatz eines Tripelreflektors im Referenzstrahlengang eines Michelson-Interferometers geht nach bestem Wissen jedoch bereits auf Twyman und Green zurück, s. dazu auch das US-Patent 1 565 533 und dort Figur 6.

In der Schrift US 5131747 wird von Cerutti-Maori ein hyperspektrales Verfahren mit einem in sich starren Michelson-Interferometer mit einer Doppel-Dachkant-Anordnung beschrieben. Dabei erfährt das Michelson-Interferometer beim Überflug mittels eines Fluggerätes über eine Bodenoberfläche in der Regel eine sehr konstante Bewegung. Dabei entsteht das Bild von der Bodenoberfläche auf dem gerasterten Detektor. Das Interferogramm mit Bildinformation ist durch zueinander verkippte Wellenfronten auf dem gerasterten Detektor gebildet. Durch die konstante Bewegung kann in jedem Pixel des gerasterten Detektors das Signal eines Zweistrahl-Interferogramms im Vorbeiziehen desselben durch eine Synchronisierung der Systemkomponenten zeitseriell aufgezeichnet werden. Mit diesem Verfahren ist jedoch die Single-Shot-Erfassung eines Interferogramms überhaupt nicht möglich. Bei einem unruhig über das Messobjekt bewegtem Interferometer oder bei Messobjekten mit chaotischer Relativbewegung oder bei turbulenten Szenen wie blubberndes Magma oder ein Feuerwerk kann mit diesem Ansatz kein ungestörtes Interferogramm gewonnen werden.

In der Schrift CN 106338342 A von Dou Jianyun und anderen wird ein hyperspektrales Verfahren mit einer Doppel-Dachkant-Anordnung im Michelson-Interferometer für Objekte in Abtastung mit einem rotierenden Scanner-Spiegel beschrieben. Es entsteht das volle Bild einer statischen Szene auf dem gerasterten flächigen Detektor in der Fourier-Ebene einer Optik. Das Bild ist aufgrund der mittels eines Interferometers eingeführten Lateral-Shear von einem räumlichen Interferogramm überlagert. Jedoch wird dieses Interferogramm, welches einem Objektpunkt zuzuordnen ist, auch erst zeitseriell im Überflug der Szene gewonnen. Voraussetzung ist also, dass dieser Überflug wie auch die Rotation des Scannerspiegels sehr gleichmäßig sein müssen, da sonst gestörte Interferogramm-Signale entstehen. Somit ist dieses hyperspektrale Verfahren überhaupt nicht für turbulente Szenen, oder gar Objekte mit chaotischer Eigenbewegung oder für Hand-held-Geräte beim Betrieb aus einer eher unruhigen oder etwas zitternden Hand geeignet.

Im Fachartikel "Large field-of-view Fourier transform imaging spectrometer using dualchannel stitching" von Chengmiao Liu und anderen in OPTICS EXPRESS, Vol. 24, No. 25 vom 12. Dezember 2016, S. 28473- 28490, http://dx.doi.org/10.1364/OE.24.028473, [4] ist in Figur 1 ein Lateral-Shear-Interferometer mit Dachkantreflektoren dargestellt. Das räumliche Interferogramm entsteht in der Fourier-Ebene eines nachgeordneten Objektivs mit Bildinformation. Dieses System arbeitet für die Gewinnung des Interferogramm-Signals jedoch zeitseriell und ist somit für Single-Shot-Applikationen nicht geeignet.

Mit anderen Worten: Ein spektrales Messverfahren, basierend auf den Schriften US 5131747, CN 106338342 A oder auch [4], liefert zwar stets ein volles Bild des Messobjekts oder der Szene, aber die Gewinnung eines räumlichen Interferogramms erfolgt zeitseriell. Dies setzt also eine nahezu konstante Relativbewegung zwischen Interferometer und Messobjekt voraus, wie es bei einem ruhigen Überflug einer Landschaft durch einen Satelliten oder ein Flugzeug in der Regel gegeben ist, um den Aufbau eines ungestörten Hyperspektralbildes zu ermöglichen. Dieser Ansatz ist somit eher nicht für turbulente Szenen geeignet, da die Interferogramm-Signale durch chaotische Bewegungen in der Szene - wie bei einem Auto-Crashtest - oder bei ungleichmäßigen oder gar chaotischen Bewegungen des Interferometers beim Halten des Instruments mittels einer freien Hand sehr wahrscheinlich gestört und für die übliche Auswertung mittels einer Fourier-Transformation völlig ungeeignet sind.

Im Fachartikel "Fourier transform spectrometer with a self-scanning photodiode array von T. Okamoto, S. Kawata und S. Minami in Applied Optics, Vol. 23, No. 2, S. 269-273 vom 15. Januar 1984 [5] wird ein Sagnac-Interferometer mit einer dem Interferometer vorgeordneten Lichtquelle und einem diesem Interferometer nachgeordneten Sammel-Objektiv beschrieben. Mittel zur Bildfeld-Diskriminierung sind nicht angegeben, da es sich um eine Ein-KanalAnordnung handelt, bei welcher ein räumliches Interferogramm in der Fourier-Ebene des Sammel-Objektivs mittels einer Fotodioden-Zeile detektiert wird.

In der Publikation "Global estimates of lunar iron and titanium contents from the Chang' E-1 IIM Data" von Yunzhao Wu und anderen in der Zeitschrift JOURNAL OF GEOPHYSICAL RESEARCH, VOL. 117, E02 von 2012 (doi: 10.1029/2011JE003879) [6] wird eine Anordnung mit einem Sagnac-Interferometer beschrieben, welchem eine anamorphotische Abbildungsstufe mit einem Fourier-Objektiv und mit einer Zylinderoptik nachgeordnet ist. Die Spaltöffnung, also der Bildfeld-Diskriminator, ist jedoch außerhalb des Sagnac-Interferometers angeordnet. Das reduziert den erreichbaren Aperturwinkel erheblich.

Mittels eines Sagnac-Interferometers kann in einem Single-Shot-Verfahren zwar auch ein volles Bild des Messobjekts gewonnen werden, jedoch nicht ein Interferogramm für jeden Bildpunkt desselben, da nur in Teilen des Bildes eine Modulation auftritt. Denn die Vollbild-Verfahren arbeiten für die Gewinnung eines Interferogramms für jeden Bildpunkt zeitseriell mittels eines Scanners, sodass das Interferogramm im Scan durch das Bild läuft. Dies ist auch im Patent US 5539517 von Cabib und anderen dargestellt. Diese Vollbild-Verfahren sind somit nicht für chaotisch bewegte Messobjekte, nicht für ein unruhig bewegtes Spektrometer und auch nicht für turbulente Szenen geeignet, da die zeitseriell im Scan aufgenommenen Interferogramme bei chaotischer Bewegungen des Messobjekts, bei unruhiger Bewegungen des Spektrometers oder bei Turbulenzen innerhalb der Szene in der Regel so gestört sind, dass eine Berechnung des Spektrums zu starken Messfehlern führt oder selbst ein extrem hoher Rechenaufwand zu eher nicht befriedigenden Ergebnissen bei der Berechnung von Spektren führt.

Im Patent US 6930781 B2 von Agladze wird in Fig. 7 ein Sagnac-Interferometer für den THz-Bereich beschrieben, bei dem ein Fokus im Sagnac-Interferometer gebildet ist. Es gibt jedoch keinen Bildfeld-Diskriminator im Interferometer. Auch gibt es keine Erzeugung von mehreren Bildpunkten bei der Detektion, da es sich um ein Ein-Kanal-Spektrometer ohne Single-Shot-Bildgebung handelt.

Im Patent US 5541728 von Dierking wird ein Fourier-Transform-Spektrometer mit einem kompakten zyklischen Interferometer mit vier Spiegeln und einem Fourier-Objektiv am Ausgang des Interferometers beschrieben. Die optischen Wege sind vergleichsweise lang, so dass der Öffnungswinkel in dieser Anordnung recht begrenzt ist. Eine Bildfeld-Diskriminierung ist nicht gegeben, da, es sich um ein Ein-Kanal-Spektrometer ohne Single-Shot-Bildgebung handelt. US 2019/162520 A1 offenbart ein neues interferometrisches Modul, das als holographische / interferometrische tragbare optische Aufbauten implementiert werden kann, basierend auf der Interferometrie mit mehreren gleichzeitig erfassten Wellenlängen, ohne die Abbildungsparameter wie die Vergrößerung und die Auflösung zu ändern. WO 2013/140396 A1 offenbart einen neuen einfachen, tragbaren, kompakten und kostengünstigen Ansatz für interferometrische optische Dickenmessungen, der mit vorhandenen Kameras leicht in ein vorhandenes Mikroskop (oder andere Bildgebungssysteme) integriert werden kann. Peng Gao, Baoli Yao, Junwei Min, Rongli Guo, Juanjuan Zheng, Tong Ye, Irina Harder, Vanusch Nercissian, and Klaus Mantel, "Parallel two-step phase-shifting point-diffraction interferometry for microscopy based on a pair of cube beamsplitters," Opt. Express 19, 1930-1935 (2011) offenbart eine parallele zweistufige phasenverschiebende Punktbeugungsinterferometrie für die Mikroskopie, die auf einem Paar von Würfelstrahlteilern basiert.

Im genannten Stand der Technik ist es in der Regel vergleichsweise einfach, ein verwackeltes Bild aus nicht äquidistanten Bildpunkten zusammenzusetzen verglichen mit der Gewinnung eines fehlerarmen Spektrums aus einem gestörten Interferogramm, insbesondere für den Fall dass die Art und die Stärke der Störung im Interferometer weitgehend unbekannt sind, was häufig der Fall ist.

Erfindung

Die der Erfindung zugrundeliegende Aufgabe besteht gemäß einem Aspekt darin, ein robustes effizientes Fourier-Transformations-Spektrometer bereitzustellen. Insbesondere soll eine zumindest teilweise hyperspektrale Bildgebung von Teilen eines bewegten Messobjekts, und/oder einer turbulenten Szene und/oder bei der Messung mit einem spektroskopischen Instrument, beispielsweise aus der freien Hand in vergleichsweise kurzer Messzeit, bereitgestellt werden.

Die vorstehende Aufgabe wird gelöst durch ein Fourier-Transformations-Spektrometer mit den Merkmalen von Anspruch 1.

Insbesondere stellt das räumlich ausgedehnte Objekt üblicherweise eine Weißlichtquelle im Sinne einer spektral breitbandigen Lichtquelle dar, und streut oder reflektiert einen Teil des darauf fallenden Lichtes zumindest teilweise als einfallendes Lichtbündel in das FT-Spektrometer. In speziellen Fällen können aber auch Objekte untersucht werden, die von einer Laserlichtquelle, beispielsweise im Infraroten beleuchtet sind, ohne dass Fluoreszenz auftritt. In diesem Fall ist das in das FT-Spektrometer einfallende Licht spektral schmalbandig, also, wenn es im Wesentlichen von der Laserlichtquelle stammt.

Insbesondere entspricht hier das FT-Spektrometer einem Single-Shot-Imaging-FT-Spektrometer auf Basis eines Michelsontyp-Interferometers, eines Mach-Zehnder Interferometers oder eines zyklischen Zweistrahl-Interferometers.

In anderen Worten entspricht hier ein FT-Spektrometer einem FT-Spektrometer mit zumindest teilweiser hyperspektraler Single-Shot-Bildgebung von einem Messobjekt bzw. Objekt als Ergebnis einer Berechnung mit einem Rechnersystem bzw. Recheneinheit mit einem Rechenprogramm zur Gewinnung von Spektren mittels einer Fourier-Transformation, bevorzugt einer schnellen Fourier-Transformation (FFT = fast Fourier transform) und mit einem vorgeordneten Objektiv als Abbildungssystem für das Messobjekt und mit einem Zweistrahl-Interferometer, dem das vorgeordnete Objektiv vorangestellt ist und welches aufweist:

- entweder einerseits eine Strahlteilereinheit mit einem Strahlteiler mit ebener Strahlteilerfläche wobei der Strahlteiler sowohl zur Strahlenbündelteilung bzw. zum Aufspalten des einfallenden Lichtbündels in der Strahlteilerebene genutzt wird, wodurch zwei Teilstrahlenbündel gebildet werden, und zwar ein erstes Teilstrahlenbündel, das in einen ersten Interferometer-Arm gesendet wird, und ein zweites Teilstrahlenbündel, das in einen zweiten Interferometer-Arm gesendet wird, als auch zur zumindest teilweisen Strahlenbündelvereinigung bzw. zur teilweisen räumlichen Überlagerung der Teilstrahlenbündel in der Strahlteilerebene mit einer Lateral-Shear s zwischen den beiden Teilstrahlenbündeln genutzt wird

- oder andererseits eine Strahlteilereinheit mit zwei Strahlteilern mit jeweils ebener Strahlteilerfläche, ein erster zur Strahlenbündelteilung in der Strahlteilerebene, wodurch zwei Teilstrahlenbündel gebildet werden, und ein zweiter Strahlteiler zur zumindest teilweisen Strahlenbündelvereinigung mit einer Lateral-Shear s zwischen den beiden Teilstrahlenbündeln und am Zweistrahl-Interferometer eine Referenzebene besteht, welche durch die Normale der ebenen Strahlteilerfläche und durch die optische Achse des vorgeordneten Objektivs am Eingang des Zweistrahl-Interferometers aufgespannt ist, und einen gerasterten Detektor am Ausgang des Zweistrahl-Interferometers und das vorgeordnete Objektiv vor dem Zweistrahl-Interferometer - unter Berücksichtigung der Position des Messobjekts - ein Bild oder mindestens ein Teilbild des Messobjekts in Lichtrichtung bzw. Ausbreitungsrichtung des Lichts in der Regel nach dem Strahlteiler, jedoch in der Regel vor dem gerasterten Detektor, erzeugt und unter Mitwirkung einer dem Zweistrahl-Interferometer nachgeordneten anamorphotischen Abbildungsstufe eine Vielzahl, mindestens jedoch zwei, von räumlichen Interferogrammen gebildet wird und diese räumlichen Interferogramme auf dem gerasterten flächigen Detektor ausgebildet sind und jedem räumlichen Interferogramm einen Objektpunkt auf dem Messobjekt zugeordnet ist,

wobei jeweils ein Bildfeld-Diskriminator in einem Interferometer-Arm zwischen der Strahlteilereinheit und dem Ausgang des Zweistrahl-Interferometers angeordnet ist, sodass beide Bildfeld-Diskriminatoren zumindest näherungsweise zueinander optisch konjugiert sind. Die Erfindung ermöglicht insbesondere den Einsatz von Strahlenbündeln mit einem vergleichsweise großen Öffnungswinkel im Zweistrahl-Interferometer aufgrund vergleichsweise kurzer Wege in den beiden Interferometerarmen. Es ist die Möglichkeit einer erheblichen Miniaturisierung des Interferometers aufgrund des optischen Setups gegeben.

Gemäß einem Aspekt weist ein FT-Spektrometer Folgendes auf:

- ein Zweistrahl-Interferometer umfassend

  • zumindest eine Strahlteilereinheit zum Aufspalten eines einfallenden Lichtbündels bzw. eines Eingangsstrahlenbündels eines räumlich ausgedehnten Objekts in ein erstes Teilstrahlenbündel und ein zweites Teilstrahlenbündel;
  • zumindest eine erste Strahlumlenkungseinheit, die dazu ausgelegt ist, das erste Teilstrahlenbündel zumindest ein erstes und ein zweites Mal abzulenken, wobei

    ◦ die erste Strahlumlenkungseinheit dazu ausgelegt ist, auch das zweite Teilstrahlenbündel zumindest ein erstes und ein zweites Mal abzulenken entsprechend eines Strahlenverlaufs in einem zyklischen Zweistrahlinterferometer; oder
    ◦ das Zweistrahl-Interferometer eine zweite Strahlumlenkungseinheit umfasst, die dazu ausgelegt ist, das zweite Teilstrahlenbündel zumindest ein erstes und ein zweites Mal abzulenken,
    wobei die Strahlteilereinheit auch dazu ausgelegt ist, das erste Teilstrahlenbündel und das zweite Teilstrahlenbündel mit einer Lateral Shear räumlich zumindest teilweise zu überlagern und das jeweils erste und zweite Ablenken des ersten Teilstrahlenbündel und des zweiten Teilstrahlenbündel die Lateral Shear erzeugt;

  • zumindest eine erste Bildfeld-Diskriminatoreinheit, die im Zweistrahl-Interferometer so angeordnet ist, dass das erste Teilstrahlenbündel nach dem Aufspalten und vor dem zweiten Ablenken räumlich selektiert wird;
  • zumindest eine zweite Bildfeld-Diskriminatoreinheit, die im Zweistrahl-Interferometer so angeordnet ist, dass das zweite Teilstrahlenbündel nach dem Aufspalten und vor dem zweiten Ablenken räumlich selektiert wird;

wobei das FT-Spektrometer ferner umfasst:

- zumindest ein Objektiv, das so gegenüber der Strahlteilereinheit angeordnet ist, dass das einfallende Lichtbündel das Objektiv zumindest teilweise vor dessen Aufspaltung an der Strahlteilereinheit passiert und das erste Teilstrahlenbündel und das zweite Teilstrahlenbündel jeweils eine Vielzahl von kohärenten Bildpunkten des räumlich ausgedehnten Objekts in einer Bildebene zwischen der Strahlteilereinheit und einem Detektor erzeugt;
- den Detektor mit mindestens einem Detektorfeld zur Erfassung einer Vielzahl von räumlichen Interferogrammen auf Basis der räumlichen Überlagerung des ersten Teilstrahlenbündels und des zweiten Teilstrahlenbündels, die der zumindest teilweisen Abbildung der Vielzahl von kohärenten Bildpunkten entspricht; und

zumindest eine Recheneinheit zur Fourier-Transformation der Vielzahl von räumlichen Interferogrammen, zur Erzeugung einer Vielzahl von Spektren und darauf basierend zur Erzeugung eines hyperspektralen Bildes des räumlich ausgedehnten Objekts.

In dieser kombinierten Ausführungsform liegen zwei alternative Ausführungsformen vor: einerseits ein zyklisches und andererseits ein nicht-zyklisches Zweistrahlinterferometer, wie beispielsweise ein Michelson-Typ oder ein Mach-Zehnder-Interferometer.

In einem zyklischen Zweistrahlinterferometer liegt nur eine Strahlumlenkungseinheit vor. Diese Strahlumlenkungseinheit, die eine "erste Strahlumlenkungseinheit" genannt wird, weist im Wesentlichen zwei Reflektoren, insbesondere zwei Spiegel oder zwei Spiegelprismen auf, welche jeweils das erste und das zweite Teilstrahlenbündel einmal umlenken bzw. spiegeln. Das heißt, die erste Strahlumlenkungseinheit umfasst insbesondere zwei umlenkende bzw. reflektierende Flächen, wie beispielsweise zwei Spiegelflächen. Jede der beiden reflektierenden Flächen ist dazu ausgelegt jeweils das erste Teilstrahlenbündel und das zweite Teilstrahlenbündel einmal zu reflektieren, sodass beide Teilstrahlenbündel lediglich eine einzige Kombination aus zwei Spiegeln nutzt.

In einem nicht-zyklischen Interferometer, wie dem Michelson-Typ- oder dem Mach-Zehnder-Interferometer gibt es jedoch neben einer ersten Strahlumlenkungseinheit auch noch eine zweite Strahlumlenkungseinheit. Beide Strahlumlenkungseinheiten weisen insbesondere jeweils zwei reflektierende Flächen auf. Die Strahlumlenkungseinheiten werden außerdem nicht von dem ersten und dem zweiten Teilstrahlenbündel geteilt. Vielmehr ist die erste Strahlumlenkungseinheit dazu ausgelegt das erste Teilstrahlenbündel zweimal zu reflektieren und die zweite Strahlumlenkungseinheit ist dazu ausgelegt das zweite Teilstrahlenbündel zweimal zu reflektieren. Die Strahlumlenkungseinheiten können jeweils eine Doppelspiegelanordnung aufweisen, wie beispielsweise einen Dachkantreflektor oder ein Doppelspiegelprisma.

Es gibt insbesondere während der Signalaufnahme auch im Wesentlichen keine bewegten und sondern im Wesentlichen (in sich) starre Komponenten im gesamten Spektrometer. Dies hat den Vorteil, dass das FT-Spektrometer ggf. eine relativ hohe Robustheit aufweisen kann.

Es besteht die Möglichkeit, ein vorgeordnetes Objektiv einzusetzen, welche das Bild des Messobjekts direkt in ein Zweistrahl-Interferometer abbildet.

Insbesondere ergeben sich für die Anwendung des Verfahrens und des Zweistrahl-Interferometers Vorteile bei lichtschwachen Objekten in besonders rauer Umgebung hinsichtlich des erreichbaren Signal-Rauschverhältnisses und der Robustheit gegenüber Verwackeln und/oder Erschütterungen. Insbesondere ist dies auf die Invarianz des Winkels bei einer Strahlablenkung im Interferometer zurückzuführen. Diese ist bevorzugt durch weitgehend miniaturisierte Optikkomponenten gegeben, welche bevorzugt als tilt-invariante, monolithische Komponenten mit jeweils zwei Spiegelflächen mit einer hohen mechanischen Stabilität ausgebildet sind.

Durch Mitlaufen einer vergleichsweise simplen Monitor-Kamera ist das Zusammensetzen auch etwas verwackelter Bildserien vergleichsweise einfach - im Vergleich zur Gewinnung eines fehlerarmen Spektrums aus einem gestörten Interferogramm. Es ist für den Fachmann ein Monitoring des Messobjekts und/oder der Szene vergleichsweise einfach beispielsweise mit einer Bewegungs- und Beschleunigungs-Sensorik machbar, um nützliche Zusatzinformationen zu gewinnen, die für den Aufbau eines hyperspektralen Bildes hilfreich sind.

Es besteht ein Vorteil darin, dass von einzelnen diskriminierten Bereichen des Bildes vom zu detektierenden Objekt zeitgleich mindestens zwei räumliche Interferogramme erzeugt werden können. Bevorzugt kann eine größere Anzahl von räumlichen Interferogrammen erzeugt werden, welche die Information über das gesuchte Spektrum zumindest teilweise enthalten, um so die Erfassung von zwei und/oder mehreren vollständigen räumlichen Interferogrammen in einer einzigen Aufnahme durchzuführen zu können. Damit kann insbesondere ein Single-Shot-Betrieb ermöglicht werden.

Bei einem Single-Shot-Verfahren führt der Detektor - hier in der Regel ein gerasterter Matrix-Detektor im FT-Spektrometer - nach einem externen oder internen digitalen Startbefehl für den Matrix-Detektor jeweils eine einzelne Bildaufnahme durch. Mittels Zweistrahl-Interferometer besteht auf dem Matrix-Detektor eine Anzahl nebeneinander angeordneter räumlicher Interferogramme. Die Anzahl derselben liegt beispielsweise bei üblichen Matrix-Detektoren in der Größenordnung von Einhundert bis Eintausend. Die räumlichen Interferogramme auf einem Kamerabild gehören in der Regel jeweils zu einem linienhaften Teilbereich des Messobjekts. Zumindest handelt es sich um einen selektierten Teilbereich des Messobjekts mittels räumlicher Diskriminierung. Die räumlichen Interferogramme werden mittels schneller Fourier-Transformation (FFT) numerisch zu Spektren verrechnet, gegebenenfalls auch erst nach einer Zwischenspeicherung. Die Aufnahmezeit, also das Zeitfenster für den Single-Shot, ergibt sich dabei in der Regel aus der Integrationszeit (allgemeiner: Bildaufnahmezeit) des gerasterten Matrix-Detektors, die sich je nach Detektortyp und Lichtverhältnissen vom einstelligen Mikrosekundenbereich bis in den dreistelligen Millisekundenbereich erstrecken kann. Im Extremfall kann sich bei extrem geringer Lichtenergie und vergleichsweise sehr geringer Dynamik im Messobjekt die Integrationszeit auch bis in den einstelligen Sekundenbereich erstrecken. Bei Blitzbeleuchtung oder einer gepulsten Beleuchtung des Messobjekts, Synchronisation vorausgesetzt, definiert jedoch die Blitz- oder Pulsdauer das Zeitfenster für den Single-Shot wie auch analog die Offenzeit einer gesteuerten Blende, vorausgesetzt, dass diese jeweils kürzer als die o. g. Integrationszeit des Matrix-Detektors sind.

Mehrere Aufnahmen des gerasterten Matrix-Detektors in möglichst schneller Folge, beispielhaft mit einer Aufnahme-Frequenz von 60Hz, führen dann zu vielen benachbarten, in der Regel linienhaften Teilbereichen eines Messobjekts, so dass mittels Relativbewegung nach und nach zu jedem auflösbaren Flächeninkrement eines flächenhaften Messobjekts ein Spektrum vorliegen kann und so ein hyperspektrales Bild gegeben ist. Die erreichbare laterale Auflösung im hyperspektralen Bild ergibt sich dabei bekannterweise aus den Parametern der optischen Komponenten im abbildenden System und ggf. auch der Rasterkonstante des (gerasterten) Matrix-Detektors des FT-Spektrometers. Moderne Hochgeschwindigkeits-Matrix-Detektoren ermöglichen auch Integrationszeiten (Bildaufnahmezeit) im Bereich von 10 Mikrosekunden, ggf. auch noch darunter.

Insbesondere kann außerdem Licht mit einem vergleichsweisen großen Raumwinkel von einem Ort des Messobjekts und/oder der Szene erfasst und mittels eines Zweistrahl-Interferometers als Interferenzlicht detektiert werden.

Dies soll insbesondere ermöglichen, am Ausgang ein nachgeordnetes Objektiv zur Erfassung dieses Interferenzlichts mit einem möglichst großen Aperturwinkel alpha einsetzen zu können, um einen möglichst großen Anteil der erfassten Lichtenergie für die Detektion nutzen zu können. Dies kann bevorzugt auch vergleichsweise kurze Integrationszeiten von gerasterten Detektoren ermöglichen, um Single-Shot-Messungen auch an bewegten Messobjekten und bei turbulenten Szenen durchführen zu können.

Bei aktiver Beleuchtung mit einer Lichtquelle kann die Energie derselben ggf. optimal genutzt werden. Das gesamte Licht derselben soll beispielsweise auf ein schmales Objektfeld gebracht werden. Es kann unter dem Gesichtspunkt der Energienutzung ein Nachteil vermieden werden, wenn nur ein kleines, schmales Feld und/oder ein Objektausschnitt bei einer Single-Shot-Messung erfasst wird.

Um eine hohe Robustheit zu bewirken, insbesondere durch die Reduzierung des Problems einer sehr unerwünschten und/oder unkontrollierten und/oder fehlerhaften Justierung, insbesondere einer Dejustierung des Interferometers, kann das Interferometer insbesondere in miniaturisierter Form vorliegen. Insbesondere können der Strahlteiler und/oder die Endreflektoren des Interferometers miniaturisiert werden, da z.B. auch Folienstrahlteiler und/oder Pellikel-Strahlteiler eingesetzt werden können, welche bei großer Ausdehnung vibrationsempfindlich sein können. Weiterhin können in den Armen des Interferometers besonders kurze Wege verwirklicht werden. Darüber hinaus können die beiden Teilstrahlenbündel, welche das Interferometer verlassen, ggf. frei von einem Astigmatismus sein oder auch einen zumindest näherungsweise gleichen Astigmatismus aufweisen.

Weiterhin kann bei Bedarf auch eine Adaptierbarkeit an das Messobjekt sowie auch eine Durchmusterung des Messobjekts ermöglicht werden. Dabei kann bevorzugt auch eine Flexibilität bei Wahl der lateralen Auflösung im Bild des Messobjekts in weiten Grenzen möglich sein.

Die nach einer zweifacher Spiegelung mittels der Strahlumlenkungseinheit erzeugte laterale Verschiebung ("Lateral-Shear") des Teilstrahlbündels im Sinne eines Querversatzes parallel zur Referenzebene ist dabei in der Regel und im Wesentlichen bei mechanisch robuster Bauart des Zweistrahl-Interferometers konstant. Der seitliche Versatz, die Lateral-Shear, zwischen den Teilstrahlbündeln hat Einfluss auf das gebildete Interferogramm. Dadurch, dass diese Lateral-Shear in der Regel und im Wesentlichen konstant ist, ist das Interferometer an sich im Wesentlichen unempfindlich gegenüber Schwingungen. Dies gilt, wenn die Strahlumlenkungseinheit selbst durch eine robuste Bauweise des Zweistrahl-Interferometers im Wesentlichen keine Bewegung mit lateraler Komponente aufweist beziehungsweise Spiegel der Strahlumlenkungseinheit keine Eigenbewegung mit lateraler Komponente aufweisen.

Nach der Spiegelung mittels der Strahlumlenkungseinheit entsteht auch eine Richtungsänderung des Teilstrahlbündels. Diese ist hinsichtlich des Winkels dieser Richtungsänderung des Teilstrahlbündels auch bei Änderungen der Winkellage der Strahlumlenkungseinheit, wenn diese hinreichend starr in sich ausgebildet ist, unveränderlich - zumindest bei Kippung derselben um eine Kippachse, die senkrecht zu einer Referenzebene steht. Das Ergebnis ist ein "tilt-invariantes Zweistrahlinterferometer" das auch durch Erschütterungen und bei Schwingungen im Wesentlichen hinsichtlich der Winkel der Teilstrahlbündel am Ausgang des Zweistrahlinterferometer im Idealfall nicht dejustiert wird. Der Idealfall kann insbesondere für ein nichtzyklisches Zweistrahl-Interferometer angenähert werden, wenn also die Strahlumlenkungseinheit jeweils in sich mechanisch sehr starr aufgebaut ist und somit eine kompakte Bauweise aufweist, beispielhaft in der Form eines möglichst kleinen Monolithen.

Gegenüber Störungen, die langsam sind im Vergleich zur Bildentstehung, ist das Zweistrahl-Interferometer durch die Single-Shot Aufnahmen ebenfalls relativ unempfindlich. So können damit insbesondere auch chaotisch bewegte Objekte vermessen werden. Alternativ können auch unbewegte Objekte gemessen werden, während das Spektrometer sich chaotisch bewegt.

Zusätzlich kann das so aufgebaute Zweistrahl-Interferometer in unterschiedlichsten Ausführungsformen folgende Vorteile gegenüber herkömmlichen Zweistrahlinterferometern aufweisen:

- Das erfindungsgemäße Spektrometer ist vergleichsweise robust, auch für größere Messflecken. Im Spektrometer sind ausschließlich starre Komponenten verbaut. Dadurch weist das Spektrometer zusätzlich eine relativ hohe Langzeitstabilität auf.
- Außerdem lässt sich der Aufbau gut miniaturisieren. So können auch Geräte, die mit einer Hand über die Objektfläche geführt werden, realisiert werden.
- Durch den Lichtleitwert-Vorteil der Fourier-Spektroskopie im Vergleich zur dispersiven Spektroskopie können auch insbesondere weniger hellstrahlende Objekte erfasst werden oder es kann schneller gemessen werden.

Der beschriebene Aufbau hat insbesondere den Vorteil, bei lichtschwachen Objekten, unter unruhigen Bedingungen zu einem besseren Signal-Rauschverhältnis zu führen.

Insbesondere weist die Strahlteilereinheit einen einzelnen Strahlteiler auf, um

- das einfallende Lichtbündel eines räumlich ausgedehnten Objekts, in das erste Teilstrahlenbündel und das zweite Teilstrahlenbündel aufzuspalten; und

- das erste Teilstrahlenbündel und das zweite Teilstrahlenbündel zumindest teilweise räumlich zu überlagern.

Insbesondere sind die erste Bildfeld-Diskriminatoreinheit und die zweite Bildfeld-Diskriminatoreinheit so zueinander angeordnet, dass zumindest näherungsweise eine optische Konjugation zwischen der ersten Bildfeld-Diskriminatoreinheit und der zweiten Bildfeld-Diskriminatoreinheit besteht.

Insbesondere entspricht das Zweistrahl-Interferometer einem Michelsontyp-Interferometer, umfassend die zweite Strahlumlenkungseinheit, wobei

die erste Strahlumlenkungseinheit einen Zweispiegel-Winkelreflektor aufweist, der im Folgenden umfasst

  ◦ einen ersten Spiegel, der dazu ausgelegt ist, das erste Teilstrahlenbündel zumindest ein erstes Mal zu reflektieren; und
  ◦ einen zweiten Spiegel, der in einen Winkel $\Theta_1$ gegenüber dem ersten Spiegel angeordnet ist und dazu ausgelegt ist, das erste Teilstrahlenbündel zumindest ein zweites Mal zu reflektieren; und/oder

die zweite Strahlumlenkungseinheit einen weiteren Zweispiegel-Winkelreflektor aufweist, der im Folgenden umfasst

  ◦ einen ersten Spiegel, der dazu ausgelegt ist, das zweite Teilstrahlenbündel zumindest ein erstes Mal zu reflektieren; und
  ◦ einen zweiten Spiegel, der in einem Winkel $\Theta_2$ gegenüber dem ersten Spiegel angeordnet ist und dazu ausgelegt ist, das zweite Teilstrahlenbündel zumindest ein zweites Mal zu reflektieren.

Zumindest näherungsweise sind die Winkel $\Theta_1$ und $\Theta_2$ der beiden Zweispiegel-Winkelreflektoren gleichgemacht.

Alternativ entspricht das Zweistrahl-Interferometer einem Michelsontyp-Interferometer, umfassend die zweite Strahlumlenkungseinheit, wobei

die erste Strahlumlenkungseinheit einen Tripelspiegel-Winkelreflektor, bevorzugt auch als Corner-cube-Anordnung bekannt, aufweist, der im Folgenden umfasst

  ◦ einen ersten Spiegel, der dazu ausgelegt ist, das erste Teilstrahlenbündel zumindest ein erstes Mal zu reflektieren; und
  ◦ einen zweiten Spiegel, der in einen Winkel $\Theta_1$ gegenüber dem ersten Spiegel angeordnet ist und dazu ausgelegt ist, das erste Teilstrahlenbündel zumindest ein zweites Mal zu reflektieren; und
  ◦ einen dritten Spiegel, der in einem Winkel $\Theta_{a1}$ gegenüber dem ersten Spiegel und in einem Winkel $\Theta_{b1}$ gegenüber dem zweiten Spiegel angeordnet ist und dazu ausgelegt ist, das erste Teilstrahlenbündel zumindest ein drittes Mal zu reflektieren; und/oder

die zweite Strahlumlenkungseinheit einen weiteren zum ersten Tripelspiegel-Winkelreflektor im Wesentlichen baugleichen Tripelspiegel-Winkelreflektor, bevorzugt auch als Corner-cube-Anordnung bekannt, aufweist, der im Folgenden umfasst

  ◦ einen ersten Spiegel, der dazu ausgelegt ist, das zweite Teilstrahlenbündel zumindest ein erstes Mal zu reflektieren; und
  ◦ einen zweiten Spiegel, der in einem Winkel $\Theta_2$ gegenüber dem ersten Spiegel angeordnet ist und dazu ausgelegt ist, das zweite Teilstrahlenbündel zumindest ein zweites Mal zu reflektieren; und
  ◦ einen dritten Spiegel, der in einem Winkel $\Theta_{a2}$ gegenüber dem ersten Spiegel und in einem Winkel $\Theta_{b2}$ gegenüber dem zweiten Spiegel angeordnet ist und dazu ausgelegt ist, das zweite Teilstrahlenbündel zumindest ein drittes Mal zu reflektieren.

Insbesondere handelt es sich in dem FT-Spektrometer um ein Zweistrahl-Interferometer, insbesondere um ein Michelsontyp-Interferometer, wobei

- die erste Bildfeld-Diskriminatoreinheit eine Öffnung aufweist, die dazu ausgelegt ist, das erste Teilstrahlenbündel nach dem ersten Reflektieren mittels des ersten Spiegels der ersten Strahlumlenkungseinheit und vor dem zweiten Reflektieren mittels des zweiten Spiegels der ersten Strahlumlenkungseinheit räumlich zu selektieren; und/oder
- die zweite Bildfeld-Diskriminatoreinheit eine weitere Öffnung aufweist, die dazu ausgelegt ist, das zweite Teilstrahlenbündel nach dem ersten Reflektieren mittels des ersten Spiegels der zweiten Strahlumlenkungseinheit und vor

**EP 3 760 992 B1**

dem zweiten Reflektieren mittels des zweiten Spiegels der zweiten Strahlumlenkungseinheit räumlich zu selektieren.

Alternativ entspricht das Zweistrahl-Interferometer in dem FT-Spektrometer einem Michelsontyp-Interferometer, umfassend die zweite Strahlumlenkungseinheit, wobei

- die erste Strahlumlenkungseinheit ferner umfasst
  ○ ein erstes Prisma mit

    ▪ einer ersten Reflexionsfläche, die dazu ausgelegt ist, das erste Teilstrahlenbündel zumindest ein erstes Mal zu reflektieren; und
    ▪ einer zweiten Reflexionsfläche, die dazu ausgelegt ist, das erste Teilstrahlenbündel zumindest ein zweites Mal zu reflektieren; und/oder
    ▪ die zweite Strahlumlenkungseinheit ferner umfasst
    ○ ein zweites Prisma mit

    ▪ einer ersten Reflexionsfläche, die dazu ausgelegt ist, das zweite Teilstrahlenbündel zumindest ein erstes Mal zu reflektieren; und
    ▪ einer zweiten Reflexionsfläche, die dazu ausgelegt ist, das zweite Teilstrahlenbündel zumindest ein zweites Mal zu reflektieren.

Insbesondere handelt es sich in dem FT-Spektrometer um ein Zweistrahl-Interferometer, insbesondere um ein Michelsontyp-Interferometer, wobei

- die erste Bildfeld-Diskriminatoreinheit eine Öffnung aufweist, die dazu ausgelegt ist, das erste Teilstrahlenbündel nach dem Aufspalten des einfallenden Lichtbündel mittels der Strahlteilereinheit und vor dem ersten Reflektieren des ersten Teilstrahlenbündels mittels der ersten Reflexionsfläche des ersten Prismas räumlich zu selektieren; und/oder
- die zweite Bildfeld-Diskriminatoreinheit eine Öffnung aufweist, die dazu ausgelegt ist, das zweite Teilstrahlenbündel nach dem Aufspalten des einfallenden Lichtbündels mittels der Strahlteilereinheit und vor dem ersten Reflektieren des zweiten Teilstrahlenbündels mittels der ersten Reflexionsfläche des zweiten Prismas räumlich zu selektieren.

Insbesondere handelt es sich um Fourier-Transformations-Spektrometer, welche eine vergleichsweise niedrige bis moderate spektrale Auflösung delta sigma aufweisen, bevorzugt im Bereich für delta_sigma zwischen etwa 1 cm$^{-1}$ und etwa 5000 cm$^{-1}$, insbesondere zwischen etwa 3 cm$^{-1}$ und etwa 3000 cm$^{-1}$ und bevorzugt zwischen etwa 4 cm$^{-1}$ und etwa 1000 cm$^{-1}$. Die Erfindung ist insbesondere anwendbar im visuellen Spektralbereich (VIS), insbesondere zwischen etwa 380nm und 750nm, im nahen Infrarot (NIR), insbesondere zwischen etwa 0,78 $\mu$m und 3 $\mu$m, im mittleren Infrarot (MIR) insbesondere zwischen etwa 3 $\mu$m und 50 $\mu$m, im fernen Infrarot-Bereich (FIR) insbesondere zwischen etwa 50 $\mu$m und 1mm, im Terahertz-Bereich und/oder auch in kombinierten Spektralbereichen angewendet werden. Die Erfindung kann zusätzlich oder alternativ auch im UV-Bereich anwendbar sein, insbesondere im nahen UV-A, insbesondere zwischen etwa 315nm und 380nm, im UV-B, insbesondere zwischen etwa 280nm und 315nm und im UV-C, insbesondere zwischen etwa 100nm und 280nm.
Von bewegten Objekten und/oder auch bei Vibrationen und/oder Turbulenzen in einer zu vermessenden Szene, also z.B. im Feldeinsatz, sollen in der Regel weitgehend unverfälschte Spektren gewonnen werden, jedoch gewisse Fehler bei der Abtastung des Messobjektes, also im Aufbau eines hyperspektralen Bildes in Form des bekannten Datenkubus (x, y, sigma) mit sigma als Wellenzahl oder (x, y, lambda) mit lambda als Wellenlänge zugelassen, beziehungsweise in Kauf genommen werden. Die spektralen Informationen werden bevorzugt im Single-Shot-Modus gewonnenen. Dadurch sind diese in der Tendenz weniger fehlerbehaftet als die Ortsinformationen vom Messobjekt, die seriell gewonnen werden.
Erfindungsgemäß befindet sich das Messobjekt im Wesentlichen bzw. zumindest teilweise außerhalb des Zweistrahl-Interferometers und ist diesem in der Regel vorgeordnet bzw. örtlich davor angeordnet. Grundsätzlich kann das Messobjekt aber auch dem Interferometer nachgeordnet bzw. örtlich dahinter angeordnet sein.
Es soll zunächst festgestellt werden, dass hier insbesondere auch die hyperspektrale Single-Shot-Bildgebung gemeint ist, wenn im Wesentlichen nur zwei oder relativ wenige räumliche Interferogramme von einem Messobjekt im Single-Shot-Modus gewonnen werden können und zu einem hyperspektralen Teilbild verrechnet werden sollen. In der Regel sollen jedoch typischerweise mindestens etwa einhundert räumliche Interferogramme mit der Erfindung im Single-Shot-Modus, also im Wesentlichen gleichzeitig, gewonnen werden. Es können auch etwa 20 bis etwa 5000, insbesondere etwa 30 bis etwa 1000, bevorzugt jedoch etwa 50 bis etwa 150 Interferogramme erfasst werden. Diese räumlichen Interferogramme können beispielsweise im Wesentlichen von einer Linie, einem schmalen Bereich und/oder von einem Raster jeweils auf dem Messobjekt stammen.

Räumliche Interferogramme für die Fourier-Spektroskopie können mittels einer Lateral-Shear zwischen Objekt- und Referenzwellenfronten am Ausgang eines Michelsontyp-Interferometers unter Nutzung eines dem Michelsontyp-Interferometer nachgeordneten Objektivs mit positiver Brechkraft erzeugt bzw. gewonnen werden und mittels nachfolgendem gerastertem Detektor erfasst bzw. aufgenommen werden. Mittels einer numerisch ausgeführten schnellen Fourier-Transformation (FFT) kann das Spektrum aus dem räumlichen Interferogramm errechnet werden.

Bei der vorliegenden Erfindung kann es im Vergleich zu den seriell mit einem räumlichen Interferogramm im Vollbild arbeitenden Verfahren entweder ein eindimensional begrenztes Messfeld geben und/oder auch ein flächiges Messfeld in einem relativ groben Raster von Messstellen geben. Diese mögliche Eigenschaft relativiert sich jedoch, wenn bei einer aktiven Beleuchtung des Messobjekts mittels einer künstlichen Lichtquelle die verfügbare Lichtenergie P_total dieser Lichtquelle anstelle auf eine z.B. Kreisfläche auf eine beispielsweise im Wesentlichen streifenförmige Fläche konzentriert wird, wodurch sich eine wesentlich höhere Beleuchtungsstärke ergibt. In dem Fall ist die Beleuchtungsstärke insbesondere auf der kleineren streifenförmigen Fläche, also dem Messfeld, entsprechend höher und die Integrationszeit eines gerasterten Detektors kann entsprechend verkleinert werden. Dementsprechend besteht ein Vorteil der Erfindung darin, dass insbesondere bei der Beleuchtung mit einem an das Messfeld des Messobjekts geometrisch angepassten Lichtfeldes die verfügbare Lichtenergie effektiv genutzt wird. Ein besonderer Vorteil ist gegeben für den Fall, wenn ein im Wesentlichen nichtbiologisches Messobjekt bei der Beleuchtung zumindest kurzzeitig einer sehr hohen Beleuchtungsstärke, beispielsweise in der Form eines schmalen Lichtstreifens auf dem Messobjekt, ausgesetzt ist. Die Beleuchtung des Messobjekts kann insbesondere mit einem Bündel-Querschnittswandler durchgeführt werden, der zu einem im Wesentlichen langgezogenen schmalen Bereich geformt ist.

Bevorzugt weist die erste Bildfeld-Diskriminatoreinheit eine Öffnung auf, die dazu ausgelegt ist, das erste Teilstrahlenbündel nach dem Aufspalten des einfallenden Lichtbündels mittels der Strahlteilereinheit und nach dem zweiten Reflektieren des ersten Teilstrahlenbündels mittels der zweiten Reflexionsfläche des ersten Prismas räumlich zu selektieren; und/oder die zweite Bildfeld-Diskriminatoreinheit weist eine Öffnung auf, die dazu ausgelegt ist, das zweite Teilstrahlenbündel nach dem Aufspalten des einfallenden Lichtbündels mittels der Strahlteilereinheit und nach dem zweiten Reflektieren des zweiten Teilstrahlenbündels mittels der ersten Reflexionsfläche des zweiten Prismas räumlich zu selektieren.

Bevorzugt weist die erste Bildfeld-Diskriminatoreinheit eine Öffnung auf, die dazu ausgelegt ist, das erste Teilstrahlenbündel nach dem Aufspalten des einfallenden Lichtbündels mittels der Strahlteilereinheit und vor dem zweiten Reflektieren des ersten Teilstrahlenbündels mittels der zweiten Reflexionsfläche des ersten Prismas räumlich zu selektieren; und/oder die zweite Bildfeld-Diskriminatoreinheit weist eine Öffnung auf, die dazu ausgelegt ist, das zweite Teilstrahlenbündel nach dem Aufspalten des einfallenden Lichtbündels mittels der Strahlteilereinheit und vor dem zweiten Reflektieren des zweiten Teilstrahlenbündels mittels der ersten Reflexionsfläche des zweiten Prismas räumlich zu selektieren.

Insbesondere entspricht das Zweistrahl-Interferometer in dem FT-Spektrometer einem Mach-Zehnder-Interferometer, umfassend die zweite Strahlumlenkungseinheit und wobei die Strahlteilereinheit ferner umfasst:

- einen ersten Einzel-Strahlteiler zum Aufspalten des einfallenden Lichtbündels in das erste Teilstrahlenbündel und das zweite Teilstrahlenbündel; und
- einen zweiten Einzel-Strahlteiler zum räumlichen Überlagern des ersten Teilstrahlenbündels und des zweiten Teilstrahlenbündels.

Insbesondere handelt es sich in dem FT-Spektrometer um ein Zweistrahl-Interferometer, insbesondere um ein Mach-Zehnder-Interferometer, wobei

- die erste Strahlumlenkungseinheit ferner umfasst

   ∘ einen ersten Spiegel, der dazu ausgelegt ist, das erste Teilstrahlenbündel zumindest ein erstes Mal zu reflektieren; und
   ∘ einen zweiten Spiegel, der in einem Winkel $\Theta_3$ gegenüber dem ersten Spiegel angeordnet ist und dazu ausgelegt ist, das erste Teilstrahlenbündel zumindest ein zweites Mal zu reflektieren; und/oder

- die zweite Strahlumlenkungseinheit ferner umfasst

   ∘ einen ersten Spiegel, der dazu ausgelegt ist, das zweite Teilstrahlenbündel zumindest ein erstes Mal zu reflektieren; und
   ∘ einen zweiten Spiegel, der in einem Winkel $\Theta_4$ gegenüber dem ersten Spiegel angeordnet ist und dazu ausgelegt ist, das zweite Teilstrahlenbündel zumindest ein zweites Mal zu reflektieren.

Insbesondere handelt es sich in dem FT-Spektrometer um ein Zweistrahl-Interferometer, insbesondere um ein Mach-Zehnder-Interferometer, wobei

- die erste Bildfeld-Diskriminatoreinheit eine Öffnung aufweist, die dazu ausgelegt ist, das erste Teilstrahlenbündel nach dem ersten Reflektieren mittels des ersten Spiegels der ersten Strahlumlenkungseinheit und vor dem zweiten Reflektieren mittels des zweiten Spiegels der ersten Strahlumlenkungseinheit räumlich zu selektieren; und/oder
- die zweite Bildfeld-Diskriminatoreinheit eine weitere Öffnung aufweist, die dazu ausgelegt ist, das zweite Teilstrahlenbündel nach dem ersten Reflektieren mittels des ersten Spiegels der zweiten Strahlumlenkungseinheit und vor dem zweiten Reflektieren mittels des zweiten Spiegels der zweiten Strahlumlenkungseinheit räumlich zu selektieren.

In anderen Worten kann das Zweistrahl-Interferometer mit zwei Interferometerarmen als nichtzyklisches Interferometer, beispielsweise als Michelsontyp-Interferometer oder als Mach-Zehnder-Interferometer ausgebildet sein und in jedem Interferometerarm kann jeweils ein Zweispiegel-Winkelreflektor oder in jedem Interferometerarm jeweils ein Tripelspiegel-Reflektor oder jeweils ein Tripelspiegelprisma zumindest näherungsweise in Raumeckenform angeordnet sein, wobei die Differenz der Spiegel oder Spiegelflächen in den beiden Interferometerarmen insbesondere null ist und in einer Position zwischen einem Drittel und zwei Dritteln der optischen Weglänge OPD im Strahlverlauf zwischen Strahlenbündelteilung und Strahlenbündelwiedervereinigung angeordnet sind.

Der Vorteil bei einem nichtzyklischen Zweistrahl-Interferometer, also einem Michelsontyp-Interferometer und/oder einem Mach-Zehnder-Interferometer, mit vier Spiegeln, besteht darin, dass die optische Weglängendifferenz und die Lateral-Shear unabhängig voneinander eingestellt werden können. Oder, es ist/sind jeweils ein Tripelspiegel-Reflektor und/oder jeweils ein Tripelspiegelprisma zumindest näherungsweise in Raumeckenform in jedem Interferometerarm angeordnet. Bevorzugt sind diese Reflektoren im nichtzyklischen Zweistrahl-Interferometer baugleich ausgeführt. Alternativ können Reflektoren auch nicht baugleich ausgestaltet sein. So können beispielsweise ein Spiegelbasierter Reflektor in einem Arm und ein Prismen-basierter Reflektor in dem anderen Arm angeordnet sein.

Insbesondere handelt es sich in dem FT-Spektrometer um ein Zweistrahl-Interferometer, insbesondere um ein zyklisches Zweistrahl-Interferometer, wobei

- die erste Strahlumlenkungseinheit dazu ausgelegt ist, auch das zweite Teilstrahlenbündel zumindest ein erstes und ein zweites Mal abzulenken,
- die erste Bildfeld-Diskriminatoreinheit eine Öffnung aufweist, die dazu ausgelegt ist, das erste Teilstrahlenbündel nach dem ersten Ablenken und vor dem zweiten Ablenken räumlich zu selektieren; und
- die zweite Bildfeld-Diskriminatoreinheit eine weitere Öffnung aufweist, die dazu ausgelegt ist, das zweite Teilstrahlenbündel nach dem ersten Ablenken und vor dem zweiten Ablenken räumlich zu selektieren.

Insbesondere handelt es sich um ein Zweistrahl-Interferometer, insbesondere um ein zyklisches Zweistrahl-Interferometer, wobei die Strahlumlenkungseinheit eine Winkelspiegelanordnung aufweist, die im Folgenden umfasst

- einen ersten Spiegel, der dazu ausgelegt ist, das erste Teilstrahlenbündel zumindest ein erstes Mal zu reflektieren und das zweite Teilstrahlenbündel zumindest ein zweites Mal zu reflektieren; und
- einen zweiten Spiegel, der in einem Winkel epsilon gegenüber dem ersten Spiegel angeordnet ist und dazu ausgelegt ist, das zweite Teilstrahlenbündel zumindest ein erstes Mal zu reflektieren und das erste Teilstrahlenbündel zumindest ein zweites Mal zu reflektieren.

In anderen Worten kann das Zweistrahl-Interferometer als zyklisches Zweistrahl-Interferometer ausgestaltet sein und zwei Planspiegel aufweisen, die eine Winkelspiegelanordnung bilden, bei welcher die beiden Planspiegel von der Strahlteilerebene, welche die ebene Strahlteilerfläche enthält, ungleich beabstandet und zumindest näherungsweise senkrecht zur Referenzebene ausgerichtet sind.

Insbesondere handelt es sich um ein Zweistrahl-Interferometer, insbesondere um ein zyklisches Zweistrahl-Interferometer, wobei die Strahlumlenkungseinheit eine Doppelprismen-Anordnung aufweist, die im Folgenden umfasst

- ein erstes Prisma mit zumindest einer Reflexionsfläche, die dazu ausgelegt ist, das erste Teilstrahlenbündel zumindest ein erstes Mal zu reflektieren und das zweite Teilstrahlenbündel zumindest ein zweites Mal zu reflektieren; und
- ein zweites Prisma mit zumindest einer Reflexionsfläche, die dazu ausgelegt ist, das zweite Teilstrahlenbündel zumindest ein erstes Mal zu reflektieren und das erste Teilstrahlenbündel zumindest ein zweites Mal zu reflektieren.

In anderen Worten ist das Zweistrahl-Interferometer als zyklisches Zweistrahl-Interferometer ausgebildet, und zwar mit zwei planen Spiegelflächen, von denen jeweils genau eine an je einem Spiegelprisma, insbesondere aus refraktivem Material ausgebildet ist und so eine Doppel-Spiegelprismen-Anordnung besteht, die mit jeweils genau zwei Spiegelprismen ausgebildet ist, und entweder die beiden Planspiegel, welche als Winkelspiegelanordnung ausgestaltet sind oder die beiden planen Spiegelflächen der Doppel-Spiegelprismen-Anordnung von der Strahlteilerebene ET, welche die Strahlteilerfläche enthält, vorbestimmt ungleich beabstandet und zumindest näherungsweise senkrecht zur Referenzebene ausgerichtet sind und zwischen den beiden Planspiegeln oder planen Spiegelflächen ein Winkel epsilon mit dem doppelten Betrag des Halbwinkels psi besteht und dieser Winkel epsilon durch zwei verlängerte Geraden aus den Planspiegeln oder aus den planen Spiegelflächen und in der Referenzebene dargestellt ist.und der Halbwinkel psi mit einem Betrag größer als 20 Altgrad und mit einem Betrag kleiner als 30 Altgrad ausgebildet ist und im Zweistrahl-Interferometer eine erste Bildebene sowie eine zweite Bildebene bestehen, welche jeweils durch das vorgeordnete Objektiv, die Lage des Messobjekts und das Zweistrahl-Interferometer gegeben sind, und der ersten Bildebeneein erster Bildfeld-Diskriminator sowie der zweiten Bildebene ein zweiter Bildfeld-Diskriminator im Zweistrahl-Interferometer zugeordnet ist und diese Bildfeld-Diskriminatoren mit jeweils mindestens einem nichtdiskriminierenden Bereich in Transmission oder Reflexion ausgebildet sind und diese beiden Bildfeld-Diskriminatoren so im Zweistrahl-Interferometer angeordnet sind, dass zumindest näherungsweise zwischen diesen Bildfeld-Diskriminatoren eine optische Konjugation besteht.

Insbesondere handelt es sich um ein Zweistrahl-Interferometer, insbesondere um ein zyklisches Zweistrahl-Interferometer, wobei

- die erste Bildfeld-Diskriminatoreinheit eine Öffnung aufweist, die dazu ausgelegt ist, das erste Teilstrahlenbündel nach dem ersten Reflektieren mittels des ersten Spiegels oder der Reflexionsfläche des erstes Prismas und vor dem zweiten Reflektieren mittels des zweiten Spiegels oder der Reflexionsfläche des zweiten Prismas räumlich zu selektieren; und
- die zweite Bildfeld-Diskriminatoreinheit eine weitere Öffnung aufweist, die dazu ausgelegt ist, das zweite Teilstrahlenbündel nach dem ersten Reflektieren mittels des zweiten Spiegels oder der Reflexionsfläche des zweiten Prismas und vor dem zweiten Reflektieren mittels des ersten Spiegels oder der Reflexionsfläche des erstes Prismas räumlich zu selektieren.

In anderen Worten kann der Aufbau eines FT-Spektrometers aus einem Zweistrahlinterferometer bestehen, das zyklisch oder nichtzyklisch sein kann und zusätzlich zwei der folgenden Bestandteile aufweisen kann: Zweispiegel-Winkelreflektor, Planspiegel, Spiegelprisma und Spiegelfläche.

Die Kombination der oben genannten Bestandteile ist dabei in der Regel so gewählt, dass bei einem von zwei Teilstrahlbündel eine "Lateral-Shear" erzeugt wird. Die "Lateral-Shear" ist insbesondere der Betrag, um den das Teilstrahlbündel nach der Spiegelung seitlich versetzt ist.

Beide Teilstrahlbündel werden daraufhin im Wesentlichen parallel im Zweistrahlinterferometer geführt. Schließlich können beide Lichtstrahlbündel mittels eines anamorphotischen Objektivs zueinander hin gebeugt werden und auf dem Detektor-Array zur Interferenz gebracht werden.

Für jeden der Messpunkte auf der betrachteten Linie kann auf dem Detektor-Array ein dazugehöriges Interferogramm bestehen aus dem mittels Fourier-Transformation das dazugehörige Spektrum berechnet werden kann.

Die im inneren des Zweistrahlinterferometers verwendeten Bildfeld-Diskriminatoren sorgen insbesondere dafür, dass das Licht von den einzelnen Messpunkten voneinander getrennt bleibt.

Bei der Anwendung kommt insbesondere das Prinzip der Weißlichtinterferometrie (WLI) zum Einsatz. Dieses Verfahren wird verwendet um das Höhenprofil eines Objektes mit einer Auflösung von bis zu etwa 1 nm zu bestimmen. Hierzu wird insbesondere das Intensitätsmaximum bestimmt und daraus die Höhe des dazugehörigen Objektpunktes berechnet.

Durch Abtasten des Objektes in x- und y-Richtung kann derart ein vollständiges Höhenprofil des Objektes erstellt werden.

Bei der erfindungsgemäßen Anwendung werden insbesondere aus dem gemessenen Interferogramm die spektralen Eigenschaften des Objektpunktes bestimmt. Durch die Verwendung von Weißlicht (also Licht mit einer kurzen Kohärenzlänge, also mit hohen Weglängen- und/oder Laufzeitunterschieden) ist die Ausdehnung des Interferogramms auf dem Detektor beschränkt. Das führt insbesondere zu einem größeren Signal zu Rausch Verhältnis. Für die Auflösung des erfindungsgemäßen Spektrometers ist insbesondere die Anzahl der Pixel auf dem Detektor-Array ausschlaggebend. Die daraus berechneten Spektren haben nur eine niedrige bis moderate spektrale Auflösung, die für viele Messaufgaben ausreichend ist. Durch eine einzige Aufnahme lassen sich so die spektralen Eigenschaften eines Objektpunktes bestimmen, weshalb auch von 'Single-Shot'-Spektroskopie gesprochen wird.

In allen Zweistrahl-Interferometertypen kann jeder Bildfeld-Diskriminator einem Zweispiegel-Winkelreflektor oder einem Tripelspiegel-Reflektor in Form einer Raumecke zugeordnet sein.

Insbesondere kann mindestens eine Bildebene bei der Abbildung des Messobjekts in einem Zweistrahl-Interferometer zumindest näherungsweise jeweils mit einem Bildfeld-Diskriminator zumindest teilweise zur Koinzidenz gebracht sein.

Zumindest einer der beiden Bildfeld-Diskriminatoren in einem Zweistrahl-Interferometer ist spaltförmig ausgebildet.

Insbesondere kann zumindest einer der beiden Bildfeld-Diskriminatoren in einem Zweistrahl-Interferometer als rechnersteuerbarer, räumlicher Lichtmodulator ausgebildet sein.

Optional kann zumindest einer der beiden Bildfeld-Diskriminatoren, insbesondere in einem Michelsontyp-Interferometer als Spaltblende ausgebildet und angeordnet sein und, wobei bevorzugt jeweils genau eine Spaltblende in jedem Arm des Michelsontyp-Interferometers positioniert ist.

Optional kann insbesondere in einem Michelsontyp-Interferometer jede Spaltblende zur scheinbaren Endspiegelfläche des jeweiligen Interferometerarms in optischer Konjugation angeordnet sein.

Optional können insbesondere in einem Michelsontyp-Interferometer die Bildfeld-Diskriminatoren jeweils in einer Position zumindest näherungsweise bei der Hälfte der optischen Weglänge im Strahlverlauf zwischen Strahlenbündelteilung und Strahlenbündelwiedervereinigung angeordnet sein.

Optional kann insbesondere in einem Michelsontyp-Interferometer jeder Zweispiegel-Winkelreflektor mit einer Strahlablenkung von zumindest näherungsweise 180 Altgrad ausgebildet sein.

Optional kann insbesondere in einem Michelsontyp-Interferometer ein Bildfeld-Diskriminator mit einem Flüssigkristall-Display ausgebildet sein.

Optional können insbesondere in einem Mach-Zehnder-Interferometer die Bildfeld-Diskriminatoren angeordnet sein und dabei jeweils genau eine Spaltblende als Bildfeld-Diskriminator in jedem Arm des Mach-Zehnder-Interferometers (604) positioniert sein.

Optional können insbesondere in einem Zweistrahl-Interferometer die Bildfeld-Diskriminatoren in jeweils einer Position zumindest näherungsweise bei der Hälfte der optischen Weglänge im Strahlverlauf zwischen Strahlenbündelteilung und Strahlenbündelwiedervereinigung angeordnet sein.

Optional können insbesondere in einem Zweistrahl-Interferometer die Zweispiegel-Winkelreflektoren mit einer Strahlablenkung zwischen 45 Altgrad und 135 Altgrad ausgebildet sein.

Optional kann insbesondere in einem Zweistrahl-Interferometer mindestens ein Bildfeld-Diskriminator als ein digitales Mikrospiegel-Array ausgebildet sein.

Optional kann insbesondere in einem zyklischen Zweistrahl-Interferometer mit zwei Planspiegeln oder zwei planen Spiegelflächen in dessen beiden entgegengesetzt umlaufenden Teilstrahlenbündeln je eine Spaltöffnung in jeweils einer Spaltblende als Bildfeld-Diskriminator in einer Bildebene des Messobjekts angeordnet sein.

Optional kann insbesondere in einem zyklischen Zweistrahl-Interferometer mit zwei Planspiegeln oder zwei planen Spiegelflächen in dessen beiden entgegengesetzt umlaufenden Teilstrahlenbündeln eine Doppelspaltblende mit jeweils zwei Spaltöffnungen in einer Ebene senkrecht zu den Hauptstrahlen der Teilstrahlenbündel angeordnet sein, welche die gemeinsame Bildebene darstellt.

Optional kann insbesondere in einem zyklischen Zweistrahl-Interferometer mit zwei Planspiegeln ein rechnersteuerbares, transmissives Flüssigkristall-Display zwischen diesen Planspiegeln oder planen Spiegelflächen mit mindestens zwei Durchlassbereichen angeordnet sein.

Optional können insbesondere im transmissiven Flüssigkristall-Display zwei spaltförmige Durchlassbereiche eingeschrieben sein.

Optional können insbesondere in einem zyklischen Zweistrahl-Interferometer die Strahlteilerebene und der Schnittpunkt SP der Geraden g1 und g2 um den Abstand d_ST voneinander separiert sein und dieser Abstand d_ST kann mindestens etwa zehn Wellenlängen der größten Wellenlänge im Spektrum des zur Detektion kommenden Lichts betragen.

Optional kann insbesondere im Bereich Z, wo es keine räumliche Überdeckung der beiden Teilstrahlenbündel gibt, mindestens eine Spiegeltreppe mit je zwei Spiegeln angeordnet sein.

Optional können insbesondere in einem zyklischen Zweistrahl-Interferometer im Bereich Z, wo es keine räumliche Überdeckung der beiden Teilstrahlenbündel gibt, zwei Spiegeltreppen mit je zwei Spiegeln angeordnet sein.

Optional können insbesondere in einem zyklischen Zweistrahl-Interferometer die beiden Spiegel einer ersten Spiegeltreppe als Planspiegel und die beiden Spiegel einer zweiten Spiegeltreppe als Planspiegel ausgebildet sein.

Optional können insbesondere in einem zyklischen Zweistrahl-Interferometer im Bereich Z, wo es keine räumliche Überdeckung der beiden Teilstrahlenbündel gibt, sowohl bei der ersten miniaturisierten Spiegeltreppe die beiden Planspiegel als auch bei der zweiten miniaturisierten Spiegeltreppe die beiden Planspiegel jeweils parallel zueinander angeordnet sein.

Optional können die beiden parallel gestellten Planspiegel einer ersten Spiegeltreppe sowie die parallel gestellten Planspiegel einer zweiten Spiegeltreppe in einem zyklischen Zweistrahl-Interferometer bevorzugt einen etwas unterschiedlichen Abstand dm_1 und dm_2 aufweisen.

Optional können insbesondere in einem zyklischen Zweistrahl-Interferometer im Bereich Z, wo es keine räumliche Überdeckung der beiden Teilstrahlenbündel, die erste Spiegeltreppe und die zweite Spiegeltreppe zu einer Doppelspiegeltreppe mit einem Mittelsteg kombiniert sind und die beiden inneren Planspiegel jeweils auf einer Seite des Mittelsteges der Doppelspiegeltreppe parallel zueinander angeordnet sein.

Optional kann insbesondere in einem zyklischen Zweistrahl-Interferometer mindestens einer Spiegeltreppe ein Bildfeld-Diskriminator zugeordnet sein.

Optional kann der Bildfeld-Diskriminator entweder als eine Spaltblende, als ein schmales Pinhole-Array oder als ein gerastertes, rechnersteuerbares mikromechanisches Pinhole-Array ausgebildet sein.

Optional kann ein Bildfeld-Diskriminator als ein schmaler Planspiegel ausgebildet sein, welcher einen der beiden Planspiegel einer Spiegeltreppe bildet.

Optional kann ein Bildfeld-Diskriminator als ein rechnersteuerbares Digitales Mikrospiegel-Array in mindestens einer Spiegeltreppe ausgebildet sein.

Optional können insbesondere in einem zyklischen Zweistrahl-Interferometer sowohl ein vergleichsweise kleiner Winkel tau_1 von bis zu 10 Altgrad zwischen den beiden Planspiegeln einer ersten Spiegeltreppe als auch ein vergleichsweise kleiner Winkel von bis zu 10 Altgrad zwischen den beiden Planspiegeln einer zweiten Spiegeltreppe bestehen und die Beträge der Winkel tau_1, tau_2 in beiden Spiegeltreppen zumindest näherungsweise gleich gemacht sein und der Winkel kappa zwischen dem Hauptstrahl HTB1a, der von der ersten Spiegeltreppe abgeht und dem Hauptstrahl HTB2a, der von der zweiten Spiegeltreppe (83) abgeht, auf der dem Interferometer zugekehrten Seite kleiner als 180 Altgrad, jedoch nicht kleiner als 140 Altgrad ausgebildet sein.

Optional kann insbesondere in einem zyklischen Zweistrahl-Interferometer mindestens eine miniaturisierte Spiegeltreppe im Bereich Z angeordnet sein, bei welcher mindestens ein Spiegel mit einer schwachen Krümmung ausgebildet ist.

Optional kann insbesondere in einem zyklischen Zweistrahl-Interferometer für die Fourier-Transformations-Spektroskopie im umlaufenden Strahlengang zwei baugleiche Spiegelprismen aus jeweils gleichem refraktiven Material angeordnet sein und die Spiegelprismen können jeweils mit einer einzigen Spiegelfläche ausgebildet sein und diese Spiegelprismen können jeweils den gleichen spitzen Winkel psi zwischen der Spiegelfläche und jeweils einer nichtverspiegelten Fläche aufweisen und zwischen diesen beiden baugleichen Spiegelprismen können zwei Bildfeld-Diskriminatoren positioniert sein.

Optional kann insbesondere in einem zyklischen Zweistrahl-Interferometer für die Fourier-Transformations-Spektroskopie im umlaufenden Strahlengang zwei winkelgleiche Spiegelprismen aus jeweils gleichem refraktiven Material angeordnet sein und die Spiegelprismen können jeweils mit einer einzigen Spiegelfläche ausgebildet sein und diese Spiegelprismen können jeweils den gleichen spitzen Winkel psi zwischen der Spiegelfläche und jeweils einer nichtverspiegelten Fläche aufweisen und zwischen diesen beiden winkelgleichen Spiegelprismen können zwei Bildfeld-Diskriminatoren positioniert sein.

Optional können insbesondere in einem zyklischen Zweistrahl-Interferometer zwei Spiegelprismen aus gleichem refraktiven Material, welche nur jeweils eine einzige Spiegelfläche aufweisen, angeordnet sein und die beiden Spiegelprismen können jeweils einen ersten spitzen Winkel psi, einen zweiten spitzen Winkel 2psi und einen dritten Winkel mit dem Betrag 180Altgrad minus 3psi aufweisen und so mindestens drei Winkel an den beiden Spiegelprismen jeweils übereinstimmen und außerdem kann eine Planparallelplatte aus refraktivem Material zwischen diesen beiden Spiegelprismen angeordnet sein und die Planparallelplatte kann durch zwei optisch transparente Kittschichten zwischen diesen beiden Spiegelprismen fixiert sein und entweder einerseits kann eine Strahlteilerschicht auf der Seite der Planparallelplatte aufgebracht sein, welche dem Spiegelprisma zugekehrt ist, oder andererseits kann die Strahlteilerschicht auf dem Spiegelprisma auf der Seite der Planparallelplatte aufgebracht sein, welche der Planparallelplatte zugekehrt ist.

Insbesondere kann es sich um ein Fourier-Transformations-Spektrometer handeln, und zwar mit zumindest teilweiser hyperspektraler Single-Shot-Bildgebung von einem Messobjekt als Ergebnis einer Berechnung mit einem Rechnersystem zur Gewinnung von Spektren mittels einer Fourier-Transformation und mit einem vorgeordneten Objektiv als Abbildungssystem für das Messobjekt

und mit einem zyklischen Zweistrahl-Interferometer, dem das vorgeordnete Objektiv vorangestellt ist und welches aufweist:
einen Strahlteiler mit ebener Strahlteilerfläche und der Strahlteiler sowohl zur Strahlenbündelteilung in der Strahlteilerebene genutzt wird, wodurch zwei Teilstrahlenbündel gebildet werden, als auch zur zumindest teilweisen Strahlenbündelvereinigung in der Strahlteilerebene mit einer Lateral-Shear s zwischen den beiden Teilstrahlenbündeln genutzt wird
und am zyklischen Zweistrahl-Interferometer eine Referenzebene besteht, welche durch die Normale der ebenen Strahlteilerfläche und durch die optische Achse des vorgeordneten Objektivs am Eingang des Interferometers aufgespannt ist,
und einen gerasterten Detektor am Ausgang des zyklischen Zweistrahl-Interferometers und das vorgeordnete Objektiv vor dem zyklischen Zweistrahl-Interferometer - unter Berücksichtigung der Position des Messobjekts - ein Bild oder mindestens ein Teilbild des Messobjekts in Lichtrichtung in der Regel nach dem Strahlteiler, jedoch in der Regel vor dem gerasterten Detektor, erzeugt

und unter Mitwirkung einer dem zyklischen Zweistrahl-Interferometer nachgeordneten anamorphotischen Abbildungsstufe eine Vielzahl, mindestens jedoch zwei, von räumlichen Interferogrammen gebildet wird und diese räumlichen Interferogramme auf dem gerasterten flächigen Empfänger ausgebildet sind und jedem räumlichen Interferogramm ein Objektpunkt auf dem Messobjekt zugeordnet ist,

wobei das zyklische Zweistrahl-Interferometer

mit zwei Planspiegeln ausgebildet ist, die eine Winkelspiegelanordnung bilden, bei welcher die beiden Planspiegel von der Strahlteilerebene ET, welche die ebene Strahlteilerfläche enthält, gleich im Abstand sind und zumindest näherungsweise senkrecht zur Referenzebene ausgerichtet sind

und zwischen den beiden Planspiegeln, welche die Winkelspiegelanordnung bilden, ein Winkel epsilon mit dem doppelten Betrag des Halbwinkels psi besteht

und dieser Winkel epsilon durch zwei verlängerte Geraden aus den Planspiegeln oder aus den planen Spiegelflächen in der Referenzebene dargestellt ist und die Winkelhalbierende des Winkels epsilon in der Strahlteilerebene ET liegt

und der Halbwinkel psi mit einem Betrag größer als 20 Altgrad und mit einem Betrag kleiner als 30 Altgrad ausgebildet ist

und im symmetrisch aufgebauten zyklischen Zweistrahl-Interferometer eine erste Bildebene sowie eine zweite Bildebene bestehen, welche jeweils durch das vorgeordnete Objektiv, die Lage des Messobjekts und das Zweistrahl-Interferometer gegeben sind,

und der ersten Bildebene ein erster Bildfeld-Diskriminator sowie der zweiten Bildebene ein zweiter Bildfeld-Diskriminator im Zweistrahl-Interferometer zugeordnet ist.

und diese beiden Bildfeld-Diskriminatoren im symmetrisch aufgebauten zyklischen Zweistrahl-Interferometer so angeordnet sind, dass zumindest näherungsweise zwischen diesen Bildfeld-Diskriminatoren eine optische Konjugation besteht,

und im zyklischen Zweistrahl-Interferometer zwischen den Planspiegeln im umlaufenden Strahlengang, die eine Winkelspiegelanordnung bilden, je eine optische Baugruppe zur Erzeugung eines Strahlversatzes je einem Bildfeld-Diskriminator zugeordnet ist und die optische Baugruppe einen Strahlversatz in jeweils entgegengesetzter Richtung erzeugen und so am Ausgang des zyklischen Zweistrahl-Interferometers eine Lateral-Shear s besteht.

Insbesondere können die optischen Baugruppen zur Erzeugung eines Strahlversatzes zyklischen Zweistrahl-Interferometer als zwei Spiegeltreppen in Luft oder als zwei zumindest Rhomboid-ähnliche Spiegelprismen ausgebildet sein.

Insbesondere können die Bildfeld-Diskriminatoren als Spaltblenden ausgebildet sein.

Gemäß einem weiteren Aspekt der Erfindung umfasst ein Verfahren zur interferometrischen Messung mittels eines Zweistrahl-Interferometers die Schritte von Anspruch 14.

Im Folgenden werden weitere Ausführungsformen, Merkmale und Beispiele dargestellt, die einerseits die Erfindung nicht einschränken und andererseits miteinander kombinierbar sind, sofern sie sich nicht ausschließen.

Es handelt sich um ein Fourier-Transformations-Spektrometer mit zumindest teilweiser hyperspektraler Single-Shot-Bildgebung von einem Messobjekt als Ergebnis einer Berechnung mit einem Rechnersystem mit einem Rechenprogramm zur Gewinnung von Spektren mittels Fourier-Transformation. Das Fourier-Transformations-Spektrometer weist ein Zweistrahl-Interferometer auf, dem ein vorgeordnetes Objektiv als Abbildungssystem für das Messobjekt vorangestellt ist. Dieses vorgeordnete Objektiv mit einer dem Zweistrahl-Interferometer zugewandten optischen Achse OAI kann dabei einstufig, zweistufig oder auch mehrstufig ausgebildet sein. In das Zweistrahl-Interferometer treten mittels eines vorgeordneten Objektivs gebildete Eingangsstrahlenbündel ein.

Diese Eingangsstrahlenbündel werden hier in der Regel als ein Ensemble von Eingangsstrahlenbündeln verstanden, weil eine Bildinformation transportiert und zu jedem Bildpunkt ein eigenes Eingangsstrahlenbündel gehört, sodass für viele Bildpunkte auch viele Eingangsstrahlenbündel zugehörig sind. So besteht in der Regel ein Ensemble von Eingangsstrahlenbündeln. In der weiteren Beschreibung in den Figuren geht es bei einem Strahlenbündel letztlich um das Ensemble von Strahlenbündel und repräsentativ ist ein Strahlenbündel genannt oder figürlich dargestellt. Dies gilt im Weiteren auch für Teilstrahlenbündel. Jedes Teilstrahlenbündel ist im Sinne der Erfindung hier für ein Ensemble von Teilstrahlenbündeln repräsentativ.

Das Zweistrahl-Interferometer kann aufweisen

Entweder einerseits einen Strahlteiler mit ebener Strahlteilerfläche und der Strahlteiler sowohl zur Strahlenbündelteilung in der Strahlteilerebene ET genutzt wird, wodurch zwei Teilstrahlenbündel TB1 und TB2 gebildet werden, als auch zur zumindest teilweisen Strahlenbündelvereinigung in der Strahlteilerebene ET mit einer Lateral-Shear s zwischen den beiden Teilstrahlenbündeln TB1 und TB2 genutzt wird. In diesem Fall handelt es sich um ein Michelsontyp-Interferometer und/oder um ein zyklisches Zweistrahl-Interferometer.

Oder andererseits zwei Strahlteiler mit jeweils ebener Strahlteilerfläche, ein erster zur Strahlenbündelteilung in der Strahlteilerebene ET, wodurch zwei Teilstrahlenbündel TB1 und TB2 mit Lateral-Shear s gebildet werden, und ein zweiter Strahlteiler zur zumindest teilweisen Strahlenbündelvereinigung mit einer Lateral-Shear s zwischen diesen beiden Teilstrahlenbündeln. In diesem Fall handelt es sich um ein Mach-Zehnder-Interferometer.

Die Strahlteilerfläche kann entweder durch eine Strahlteilerschicht, eine Strahlteilerfolie und/oder durch ein Gitter gebildet sein.

Die dem Zweistrahl-Interferometer zugewandte optische Achse OAI des vorgeordneten Objektivs, gegebenenfalls auch die optische Achse OAI der letzten Optikkomponente desselben, also die optische Achse OAI am Eingang des Zweistrahl-Interferometers und die Normale NT der Strahlteilerebene ET spannen in der Regel am Zweistrahl-Interferometer eine Referenzebene RE auf. Diese Referenzebene RE ist üblicherweise senkrecht zur Strahlteilerebene ET. Im Falle eines Mach-Zehnder-Interferometers ist es üblicherweise der erste Strahlteiler, dessen Strahlteilerebene ET die Referenzebene RE festlegt.

Dem Zweistrahl-Interferometer ist ein bevorzugt flächiger, gerasterter Detektor zur Aufnahme räumlicher Interferogramme nachgeordnet.

Der gerasterte Detektor kann eine UV-Kamera und im sichtbaren Spektralbereich (VIS) eine VIS-CCD- und/oder eine VIS-CMOS-Kamera sein. Im nah-infraroten Spektralbereich kann eine InGaAs-Kamera mit Vorteil eingesetzt werden. Im mittleren Infrarot kommt als gerasterter Detektor ein Focal Plane Array (FPA), auch als IRFPA bekannt, zur Anwendung, das gegebenenfalls auch gekühlt ist. In der hybriden CMOS FPA-Technik wird mit Vorteil eine Quecksilber-Cadmium Telluride-Verbindung (MCT) eingesetzt. Im gesamten Infrarotbereich können Bolometer-Matrix-Detektoren, insbesondere Mikrobolometer, eingesetzt werden. Bevorzugt werden für diese Erfindung Matrix-Detektoren eingesetzt.

Es ist bevorzugt auch möglich, dem Zweistrahl-Interferometers zwei und/oder mehrere schnelle Zeilen-Detektoren in einer der vorab bereits genannten Technologien und Spektralbereiche nachzuordnen. Dabei sind die einzelnen Zeilen-Detektoren bei der primären Datenerfassung bevorzugt in einem gemeinsamen Detektorfeld in der Regel parallel zueinander angeordnet. Digital-elektronisch sind diese Zeilen-Detektoren bei der Datenerfassung jedoch unabhängig voneinander. Diese Zeilen-Detektoren werden insbesondere getrennt betrieben, um ein Maximum an Auslesegeschwindigkeit erreichen zu können. Die Anzahl der Zeilen-Detektoren entspricht vorzugsweise der Anzahl der Messpunkte auf dem Objekt. Dabei können die einzelnen Pixel einer Zeile auch mit einem besonders hohen Aspektverhältnis ausgebildet sein, um zwecks Gewinnung eines hohen Signal-Rauschverhältnisses einen möglichst großen Betrag an Lichtenergie zu detektieren. Derartige Anwendungen können insbesondere für die Analyse turbulenter Prozesse einsetzbar sein.

Das vorgeordnete Objektiv und/oder Objektivsystem vor dem Zweistrahl-Interferometer erzeugt insbesondere - unter Berücksichtigung der Position des Messobjekts - ein Bild oder mindestens ein Teilbild des Messobjekts in Lichtrichtung mit Bildort in der Regel nach dem Strahlteiler, jedoch üblicherweise auch vor dem gerasterten Detektor. Das vorgeordnete Objektiv und/oder Objektivsystem kann sowohl aus refraktiven als auch aus Spiegelkomponenten bestehen, aber auch aus einer Kombination von Spiegelkomponenten und refraktiven Komponenten.

Unter Mitwirkung einer dem Zweistrahl-Interferometer nachgeordneten anamorphotischen Abbildungsstufe kann eine Vielzahl von räumlichen Interferogrammen gebildet werden. Diese anamorphotische Abbildungsstufe kann auch sowohl aus refraktiven Komponenten als auch aus Spiegelkomponenten bestehen. Insbesondere die Zylinderoptik kann mittels einer Spiegelkomponente ausgebildet sein. Die räumlichen Interferogramme sind auf dem gerasterten flächigen Empfänger ausgebildet und es besteht eine Zuordnung zwischen jedem erfassten Objektpunkt und/oder Objektfleck auf dem Messobjekt und einem räumlichen Interferogramm. Der angemessene Objektfleck kann dabei einerseits sehr fein sein, also nahezu dem beugungsbegrenzten Auflösungsinkrement entsprechen, wenn es um eine hohe laterale Auflösung geht. Andererseits kann der erfasste Objektfleck aber auch vergleichsweise groß sein, wenn eine hohe laterale Auflösung des Messobjekts nicht im Vordergrund steht, jedoch eine hohe spektrale Auflösung und ein besonders schnelles Messen gefordert sind. Bei einem vergleichsweise großen erfassten Objektfleck als Messfleck, ist die hier beschriebene Fourier-Spektroskopie den dispersiven spektroskopischen Verfahren überlegen, da hierbei eine hohe spektrale Auflösung nicht physikalisch an eine feine Spaltöffnung gebunden ist wie bei den dispersiven spektroskopischen Verfahren. Bei diesen letztgenannten ist dadurch das Signal-Rauschverhältnis bei gleicher spektraler Auflösung in der Regel deutlich schlechter als bei der Fourier-Spektroskopie. Jedoch sinkt üblicherweise bei großen Messflecken die Bildauflösung. Insbesondere kommt der Lichtleitwertvorteil der Fourier-spektroskopischen Verfahren nach Jacquinot somit zur Geltung, jedoch üblicherweise zu Lasten der lateralen Auflösung. Es kann in der Regel ein deutlich besseres Signal-Rauschverhältnis erreicht werden und/oder schneller gemessen werden als mit dispersiven spektroskopischen Verfahren, welche Beugungsgitter und/oder Prismen einsetzen.

Weiterhin kann dem Zweistrahl-Interferometer zur Beleuchtung des Messobjekts einerseits entweder mindestens eine Lichtquelle beigeordnet sein, welche bevorzugt auch steuerbar ist und Lichtmuster ausbilden kann. Andererseits kann das Messobjekt auch ein Selbststrahler wie eine heiße Abgaswolke mit großer Strahlungsintensität im infraroten Spektralbereich sein.

Das Zweistrahl-Interferometer mit zwei Interferometerarmen kann einerseits als nichtzyklisches Interferometer ausgebildet sein und in jedem Interferometerarm jeweils ein Zweispiegel-Winkelreflektor aufweisen. So besteht der Vorteil bei einem nichtzyklischen Zweistrahl-Interferometer, also einem Michelsontyp-Interferometer und/oder einem Mach-Zehnder-Interferometer, mit vier Spiegeln, dass die optische Weglängendifferenz und die Lateral-Shear unabhängig voneinander eingestellt werden können. Oder, es ist jeweils ein Tripelspiegel-Reflektor und/oder jeweils ein Tripelspiegelprisma zumindest näherungsweise in Raumeckenform in jedem Interferometerarm angeordnet. Bevorzugt sind diese Reflektoren im nichtzyklischen Zweistrahl-Interferometer baugleich ausgeführt.

Die Differenz der Spiegel bzw. Spiegelflächen in den beiden Interferometerarmen ist somit in der Regel null. In einer Position vorzugsweise zwischen einem Drittel und zwei Dritteln der optischen Weglänge im Strahlverlauf zwischen Strahlenbündelteilung und Strahlenbündelwiedervereinigung kann in jedem Interferometerarm eines Zweistrahl-Interferometers ein Bildfeld-Diskriminator angeordnet sein. Diese beiden Bildfeld-Diskriminatoren sind in der Regel zumindest näherungsweise zueinander optisch konjugiert. Wenn der Bildfeld-Diskriminator nicht strukturiert ist, kann dieser auch als eine klassische Bildfeldblende, beispielsweise in Form einer dünnen Abschattblende, ausgebildet sein.

Andererseits kann das Zweistrahl-Interferometer als zyklisches Zweistrahl-Interferometer ausgestaltet sein, und zwar insbesondere mit zwei Planspiegeln, die eine Winkelspiegelanordnung bilden, bei welcher die beiden Planspiegel von der Strahlteilerebene ET, welche die Strahlteilerfläche enthält, ungleich beabstandet sind. Außerdem sind die beiden Planspiegel zumindest näherungsweise senkrecht in der Regel zur Referenzebene RE ausgerichtet. So besteht ein Zweispiegel-Winkelreflektor im zyklischen Zweistrahl-Interferometer.

Alternativ kann das Zweistrahl-Interferometer als zyklisches Zweistrahl-Interferometer mit zwei planen Spiegelflächen ausgebildet, von denen jeweils genau eine Spiegelfläche an je einem Spiegelprisma aus refraktivem Material ausgebildet ist. So besteht eine Doppel-Spiegelprismen-Anordnung, die mit jeweils zwei Spiegelprismen ausgebildet ist. Bei dieser Anordnung sind die beiden planen Spiegelflächen eines Spiegelprismas von der Strahlteilerebene ET, welche die Strahlteilerfläche enthält, ungleich beabstandet und zumindest näherungsweise senkrecht zur Referenzebene RE ausgerichtet.

Einerseits können zwei Planspiegel angeordnet werden, die eine Doppel-Spiegelprismen-Anordnung bilden, und somit also einerseits paarweise in einem zyklischen Zweistrahl-Interferometer eine Winkelspiegelanordnung bilden, die insbesondere vorbestimmt versetzt zur Strahlteilerebene ET angeordnet sein kann.

Andererseits können die zwei Spiegelprismen der Doppel-Spiegelprismen-Anordnung vorbestimmt versetzt zur Strahlteilerebene ET angeordnet sein. Die Doppel-Spiegelprismen-Anordnung stellt ein Spiegelprismen-Paar dar. Jedem Spiegelprisma ist in der Regel dabei genau eine plane Spiegelfläche zugeordnet. Eine Anordnung mit zwei Spiegelprismen, die als Doppel-Spiegelprismen-Anordnung mit je einer planen Spiegelfläche gestaltet ist, kann also auch als eine Winkelspiegelanordnung betrachtet werden und stellt auch einen Zweispiegel-Winkelreflektor dar.

An jedem Planspiegel bzw. an jeder Spiegelfläche im Zweistrahl-Interferometer erfahren die Strahlen eines Teilstrahlenbündels üblicherweise nur jeweils eine einmalige Reflexion.

Die Winkelspiegelanordnung als Luftanordnung ist also zur Strahlteilerebene ET vorbestimmt etwas quer versetzt beziehungsweise die Doppel-Spiegelprismen-Anordnung mit den zwei planen Spiegelflächen ist vorbestimmt etwas quer zur Strahlteilerebene ET versetzt.

Es kann in einem zyklischen Zweistrahl-Interferometer in der Referenzebene RE sowohl zwischen den beiden Planspiegel, welche als Winkelspiegelanordnung, also speziell als Zweispiegel-Winkelreflektor, ausgestaltet sind, als auch zwischen den beiden planen Spiegelflächen der Doppel-Spiegelprismen-Anordnung die ja letztlich auch einen Zweispiegel-Winkelreflektor darstellen, in der Regel ein Winkel epsilon bestehen. Dieser Winkel epsilon ist insbesondere durch zwei verlängerte Geraden g1 und g2 aus den Planspiegeln bzw.aus den planen Spiegelflächen in der Referenzebene RE dargestellt. Der Halbwinkel psi beträgt die Hälfte des Winkels epsilon und ist in der Regel ein im Wesentlichen spitzer Winkel mit einem Betrag größer als 20 Altgrad und einem Betrag kleiner als 30 Altgrad.

Weiterhin ist der Halbwinkel psi bevorzugt mit einem Betrag größer oder etwa gleich 22 Altgrad und einem Betrag kleiner als etwa 29 Altgrad ausgebildet.

Weiterhin ist der Halbwinkel psi bevorzugt mit einem Betrag größer als etwa 24 Altgrad und einem Betrag kleiner als etwa 28 Altgrad ausgebildet, um im zyklischen Zweistrahl-Interferometer einen vergleichsweise großen Aperturwinkel realisieren zu können, der bis zu etwa zehn Altgrad betragen kann.

Von beleuchteten und/oder selbstleuchtenden und/oder List-streuenden Objektpunkten des Messobjekts werden durch das vorgeordnete Objektiv Bildpunkte in einer Bildebene im Zweistrahl-Interferometer erzeugt. Jeweils eine Bildebene vom abgebildeten Messobjekt, welche durch das vorgeordnete Objektiv und die Lage des Messobjekts gegeben ist, ist einem Bildfeld-Diskriminator in jedem Arm des Zweistrahl-Interferometers zumindest näherungsweise zugeordnet.

Im Zweistrahl-Interferometer können eine erste Bildebene sowie eine zweite Bildebene bestehen, die optisch zueinander konjugiert sind, welche jeweils durch das vorgeordnete Objektiv, die Lage des Messobjekts und das Zweistrahl-Interferometer gegeben sind. Der ersten Bildebene ist ein erster Bildfeld-Diskriminator sowie der zweiten Bildebene ein zweiter Bildfeld-Diskriminator im Zweistrahl-Interferometer zugeordnet. Diese Bildfeld-Diskriminatoren sind mit jeweils mindestens einem nichtdiskriminierenden Bereich in Transmission, also einem Durchlassbereich, oder mindestens einem nichtdiskriminierenden Bereich in Reflexion, also einem reflektierenden Bereich, ausgebildet. Dieser reflektierende Bereich ist vorzugsweise vergleichsweise schmal ausgebildet.

Dabei können diese beiden Bildfeld-Diskriminatoren so im Zweistrahl-Interferometer angeordnet sein, dass zumindest näherungsweise zwischen diesen Bildfeld-Diskriminatoren eine optische Konjugation besteht, diese Bildfeld-Diskriminatoren also optisch konjugiert sind. Das heißt, der eine Bildfeld-Diskriminator stellt das Bild des anderen dar.

Das Bild vom Messobjekt im Zweistrahl-Interferometer ist dabei in der Regel bevorzugt zumindest etwas größer als der nichtdiskriminierende Bereich eines Bildfeld-Diskriminators, welcher beispielsweise durch eine Öffnung und/oder einen bevorzugt schmalen reflektierenden Bereich dargestellt ist.

Vorzugsweise besteht beim Michelsontyp-Interferometer eine zumindest näherungsweise Parallelität zwischen den Hauptstrahlen des hin- und des rücklaufenden Bündels in einem Arm des Michelsontyp-Interferometers.

Vorzugsweise gibt es zu jedem Hauptstrahl in einem Arm des Mach-Zehnder-Interferometers einen parallelen Hauptstrahl im anderen Arm des Mach-Zehnder-Interferometers.

Bevorzugt kann das Zweistrahl-Interferometer mit vorgeordnetem Objektiv, bevorzugt mit einer zum gerasterten Detektor synchronisierten Blitzlichtquelle, auch als mobiler Messkopf ausgebildet sein.

Das vorgeordnete Objektiv zur Abbildung des Messobjekts ist bevorzugt auf der dem Zweistrahl-Interferometer zugewandten Seite zumindest näherungsweise telezentrisch ausgebildet. Das vorgeordnete Objektiv kann bevorzugt auch beidseitig telezentrisch ausgebildet sein. Eine Telezentrie auf der dem Interferometer zugewandten Seite verringert die Anforderungen an die nachfolgende Optik in der Regel erheblich.

Die Anordnung der beiden Bildfeld-Diskriminatoren im Zweistrahl-Interferometer, wo gemäß der Erfindung zwei kohärente Bilder des Messobjekts entstehen, kann eine Abbildungsstufe zur Abbildung des Messobjekts vor dem Zweistrahl-Interferometer einsparen. Dabei können die Bildfeld-Diskriminatoren bevorzugt auch eine konfokale Diskriminierung bewirken, wenn eine konfokale Anordnung besteht. Insbesondere in einem Grenzfall können die Bildfeld-Diskriminatoren, wenn diese als Pinhole und/oder als Mikrospiegel ausgebildet sind, die Größe eines Airy-Scheibchens und/oder eine Spaltöffnung und/oder Spiegelstreifen mit der Breite eines Durchmessers eines Airy-Scheibchens aufweisen. Derartig kleine Bildfeld-Diskriminatoren stehen jedoch hier eher nicht im Fokus der Erfindung, da bei sehr kleinen Bildfeld-Diskriminatoren der Vorteil der Fourier-Spektroskopie gegenüber der dispersiven Spektroskopie schwinden würde. Die dispersive Spektroskopie ist ja bei höherer spektraler Auflösung auf einen schmalen Spalt angewiesen, dagegen die Fourier-Spektroskopie aufgrund des dem Fachmann bekannten Jacquinot-Vorteils nicht.

Durch die hier typischerweise relativ gröbere Bildfeld-Diskriminierung mit im Vergleich zur konfokalen Mikroskopie vergleichsweise großflächigen Bildfeld-Diskriminatoren im Zweistrahl-Interferometer entsteht hier in der Regel auch kein geometrisch-optisches Schärfentiefeproblem, auch wenn das Objekt eine etwas größere Tiefe aufweist. Bei einem vergleichsweise kurzbrennweitigen vorgeordnetem Objektiv und entfernten Objekten und/oder relativ flachen Objekten ist die Tiefenausdehnung des Bildes relativ gering, sodass das hier verwendete Modell der Bildebene in der Regel recht gut zutrifft. Es ist von Vorteil, wenn das vorgeordnete Objektiv vorzugsweise mit einem Autofokus-System ausgebildet ist, um zumindest die relevanten Bereiche eines Messobjekts vergleichsweise scharf in die Ebene der Bildfeld-Diskriminatoren abzubilden, sodass zumindest ein hyperspektrales Teilbild gewonnen werden kann.

Bei Messobjekten mit einer größeren Tiefe und/oder bei sehr kleinen Messobjekten ist es in der Regel notwendig, ein Verfahren mit einem mit Tiefen-Scan durchzuführen und es ist in der Regel notwendig, dass in jeder Tiefenposition, also schichtenweise, räumliche Interferogramme aufgenommen werden.

Darüber hinaus ist in einem Zweistrahl-Interferometer vorzugsweise jeder Bildfeld-Diskriminator einem Zweispiegel-Winkelreflektor oder einem Tripelspiegel-Reflektor in Form einer Raumecke (engl. corner cube reflector) zugeordnet. Der Bildfeld-Diskriminator kann dabei zwischen zwei Planspiegeln des Zweispiegel-Winkelreflektors oder im Tripelspiegel-Reflektor selbst angeordnet oder diesen Reflektoren zugeordnet sein. Ein Bildfeld-Diskriminator kann vorzugsweise auch dem Zweispiegel-Winkelreflektor oder dem Tripelspiegel-Reflektor im Strahlverlauf vor- oder nachgeordnet sein. Der Tripelspiegel-Reflektor kann dabei als Tripelprisma oder als Luftspiegelgruppe (engl. hollow cube) ausgebildet sein. Dabei sind in einem Michelsontyp-Interferometer und in einem Mach-Zehnder-Interferometer jeweils in der Regel zwei Zweispiegel-Winkelreflektoren angeordnet. Dagegen ist in einem zyklischen Zweistrahl-Interferometer jeweils ein Zweispiegel-Winkelreflektor angeordnet und diesem zyklischen Zweistrahl-Interferometer sind dann zwei Bildfeld-Diskriminatoren zugeordnet.

Bevorzugt ist weiterhin die Bildebene bei der Abbildung des Messobjekts in einem Zweistrahl-Interferometer zumindest näherungsweise jeweils mit einem Bildfeld-Diskriminator zumindest teilweise zur Koinzidenz gebracht. Nur so ist eine präzise Diskriminierung des Bildfeldes gegeben.

Weiterhin sind vorzugsweise die zwei Bildfeld-Diskriminatoren in einem Zweistrahl-Interferometer spaltförmig ausgebildet. Dies ermöglicht eine einfache Zuordnung der Messpunkte zu den Zeilen eines gerasterten Detektors.

Weiterhin ist vorzugsweise mindestens ein Bildfeld-Diskriminator als rechnersteuerbarer, räumlicher Lichtmodulator in einem Zweistrahl-Interferometer ausgebildet. Damit kann der Bildfeld-Diskriminator mit variabler Größe und Form der Messaufgabe optimal angepasst werden. Zur Anwendung können Flüssigkristall-Displays (LCD) in Transmission oder Reflexion und/oder auch digitale Mikrospiegel-Arrays (DMD) kommen. Letztere finden insbesondere im infraroten Spektralbereich Anwendung.

Weiterhin sind vorzugsweise die beiden Bildfeld-Diskriminatoren in einem Michelsontyp-Interferometer als Spaltblende ausgebildet und angeordnet und jeweils genau eine Spaltblende ist in jedem Arm des Michelsontyp-Interferometers positioniert.

Weiterhin ist vorzugsweise in einem Michelsontyp-Interferometer jede Spaltblende zur scheinbaren Endspiegelfläche des jeweiligen Interferometerarms in optischer Konjugation angeordnet.

Weiterhin sind vorzugsweise die Bildfeld-Diskriminatoren in einem Michelsontyp-Interferometer, welches ein Beispiel für ein Zweistrahl-Interferometer darstellt, jeweils in einer Position zumindest näherungsweise bei der Hälfte der optischen Weglänge im Strahlverlauf zwischen Strahlenbündelteilung und Strahlenbündelwiedervereinigung angeordnet.

Weiterhin ist vorzugsweise jeder Zweispiegel-Winkelreflektor in einem Michelsontyp-Interferometer mit einer Strahlablenkung von zumindest näherungsweise 180 Altgrad ausgebildet. Der Zweispiegel-Winkelreflektor stellt also einen 90-Altgrad-Dachkantreflektor dar.

Weiterhin ist vorzugsweise mindestens ein Bildfeld-Diskriminator in einem Michelsontyp-Interferometer mit einem rechnersteuerbaren Flüssigkristall-Display (LCD) gebildet. Dieses ist vorzugsweise zwischen den beiden Spiegeln eines Zweispiegel-Winkelreflektors angeordnet und wird in Transmission benutzt. Bevorzugt werden hierbei zwei baugleiche Flüssigkristall-Displays eingesetzt.

Weiterhin sind vorzugsweise die Bildfeld-Diskriminatoren in einem Mach-Zehnder-Interferometer angeordnet und dabei ist jeweils genau ein Bildfeld-Diskriminator in jedem Arm des Mach-Zehnder-Interferometers positioniert. Da die Aberrationen in den beiden Armen eines Mach-Zehnder-Interferometers unterschiedlich sein können, insbesondere bei der Nutzung einer im Strahlengang geneigten Strahlteilerplatte anstelle eines Strahlteilerwürfels, können die Bildfeld-Diskriminator in Spaltform auch mit etwas unterschiedlichen Spaltbreiten ausgebildet werden. Beispielsweise, wenn in einem Arm des Mach-Zehnder-Interferometers der Öffnungsfehler nicht korrigiert ist, entsteht ein etwas unscharfes Bild, auch von einem schmalen Bereich des Messobjekts. Hier kann die Spaltöffnung dann etwas größer gewählt werden im Vergleich zum Interferometerarm weitgehend ohne Aberrationen bei der Abbildung von einem schmalen Bereich des Messobjekts.

Weiterhin sind vorzugsweise die Bildfeld-Diskriminatoren in einem Zweistrahl-Interferometer jeweils in einer Position zumindest näherungsweise bei der Hälfte der optischen Weglänge im Strahlverlauf zwischen Strahlenbündelteilung und Strahlenbündelwiedervereinigung angeordnet. Dies ermöglicht einen vergleichsweise großen Aperturwinkel im Interferometer.

Weiterhin sind vorzugsweise die Zweispiegel-Winkelreflektoren in einem Mach-Zehnder-Interferometer mit einer Strahlablenkung zwischen 45 Altgrad und 135 Altgrad ausgebildet. Insbesondere ist der Bereich der Strahlablenkung zwischen 75 Altgrad und 105 Altgrad bevorzugt.

Weiterhin ist vorzugsweise mindestens ein Bildfeld-Diskriminator in einem Mach-Zehnder-Interferometer als ein digitales Mikrospiegel-Array ausgebildet. Dabei kann vorzugsweise ein Mikrospiegel-Array anstelle eines Spiegels eines Zweispiegel-Winkelreflektors im Mach-Zehnder-Interferometer eingesetzt werden. Dabei wird vorzugsweise nur ein schmaler Bereich auf dem Mikrospiegel-Array in Reflexion adressiert, wo auch ein zumindest teilweise scharfes Bild auf dem Mikrospiegel-Array gebildet ist, da der Lichteinfall hierbei ja nicht senkrecht erfolgt. Dabei besteht in diesem Fall ein schmaler Bildausschnitt vom Messobjekt in scharfer Abbildung auf dem Mikrospiegel-Array im adressierten Bereich desselben. Das Mikrospiegel-Array ist somit anstelle eines üblichen Planspiegels im Zweispiegel-Winkelreflektor eingesetzt. In jedem Fall stellt der adressierte Bereich eines räumlichen Lichtmodulators auch den diskriminierenden Bereich eines Bildfeld-Diskriminators dar. Im zweiten Zweispiegel-Winkelreflektor des Interferometers kann zwischen den beiden Planspiegeln bevorzugt eine Spaltblende eingesetzt sein, insbesondere dann, wenn in diesem Interferometerarm eine aberrationsfreie Abbildung des Messobjekts besteht. Vorzugsweise kann aber auch in jedem der beiden Interferometerarme des Mach-Zehnder-Interferometers im Zweispiegel-Winkelreflektor ein Planspiegel bevorzugt durch ein digitales Mikrospiegel-Array ersetzt sein. Dies kann eine rechnergestützte Justierung der Anordnung ermöglichen und somit die Justierung vereinfachen.

Weiterhin ist vorzugsweise in einem zyklischen Zweistrahl-Interferometer mit zwei Planspiegeln bzw. zwei planen Spiegelflächen in dessen beiden entgegengesetzt umlaufenden Teilstrahlenbündeln je eine Spaltöffnung in jeweils einer Spaltblende oder einer Doppelspaltblende als Bildfeld-Diskriminator in einer Bildebene des Messobjekts angeordnet. Die erste Spaltöffnung befindet sich dabei in der ersten Bildebene des Messobjekts im zyklischen Zweistrahl-Interferometer und die zweite Spaltöffnung befindet sich dabei in der zweiten Bildebene des Messobjekts im zyklischen Zweistrahl-Interferometer, die ja optisch konjugiert sind. Dabei können die beiden Spaltöffnungen der einzelnen Spaltblenden - je nach Lage der Bildebenen - auch in der Tiefe versetzt sein. Jedoch befinden sich die beiden Spaltblenden in der Regel zwischen den beiden Planspiegeln bzw. den beiden planen Spiegelflächen des zyklischen Interferometers.

Das zyklische Zweistrahl-Interferometer ist hier also entweder mit zwei Planspiegeln in einer von refraktiven Materialien freien Anordnung im umlaufenden Strahlengang aufgebaut. Oder, das zyklische Zweistrahl-Interferometer ist bei Nutzung von zwei Spiegelprismen mit je einer Spiegelfläche an diesen Spiegelprismen ausgebildet.

Diese Bildfeld-Diskriminatoren BFD1 und BFD2 im zyklischen Zweistrahl-Interferometer sind dabei vorzugsweise zumindest näherungsweise bei der Hälfte der optischen Weglänge im Strahlverlauf zwischen Strahlenbündelteilung und Strahlenbündelwiedervereinigung in jedem der beiden Strahlengänge angeordnet. Jedoch sind die Bildfeld-Diskriminatoren BFD1 und BFD2 stets dort angeordnet, wo jeweils eine Bildebene bestehen. Das ist vorzugsweise der Bereich im zyklischen Zweistrahl-Interferometer, wo sich die Teilstrahlenbündel TB1 und TB2 nicht überdecken.

Weiterhin ist vorzugsweise in einem zyklischen Zweistrahl-Interferometer mit zwei Planspiegeln und/oder zwei planen Spiegelflächen in dessen beiden entgegengesetzt umlaufenden Teilstrahlenbündeln eine Doppelspaltblende mit jeweils zwei Spaltöffnungen in einer Ebene senkrecht zu den Hauptstrahlen der Teilstrahlenbündel TB1 und TB2 angeordnet. In dieser Ebene fallen die beiden optisch konjugierten Bildebenen von der Abbildung des Messobjekts zusammen und bilden so eine gemeinsame Bildebene. Dazu sind vorzugsweise die Bildverschiebungen in der Tiefe in refraktivem, transparenten Material gleicher Art von der Strahlteilung bis zu je einem Bildfeld-Diskriminator in den beiden Armen des zyklischen Zweistrahl-Interferometers gleichgemacht. In der Regel handelt es sich vorzugsweise um feste refraktive Materialien im Zweistrahl-Interferometer, aber auch ein flüssiges, transparentes, refraktives Material ist vorzugsweise einsetzbar.

Weiterhin ist vorzugsweise in einem zyklischen Zweistrahl-Interferometer für die Fourier-Transformations-Spektroskopie mit zwei Planspiegeln und/oder zwei planen Spiegelflächen ein rechnersteuerbares, transmissives Flüssigkristall-Display zwischen diesen Planspiegeln und/oder planen Spiegelflächen mit mindestens zwei Durchlassbereichen angeordnet.

Weiterhin sind vorzugsweise im transmissives Flüssigkristall-Display in einem zyklischen Zweistrahl-Interferometer für die Fourier-Transformations-Spektroskopie zwei spaltförmige Durchlassbereiche eingeschriebenen. Dies erfolgt im Zusammenwirken mit Polarisationsoptik und polarisiertem Licht in dem Fachmann bekannter Art und Weise.

Weiterhin sind vorzugsweise in einem zyklischen Zweistrahl-Interferometer für die Fourier-Transformations-Spektroskopie die Strahlteilerebene ET und der Schnittpunkt SP der Geraden g1 und g2 um den Abstand d_ST voneinander separiert und dieser Abstand d_ST beträgt mindestens zehn Wellenlängen der größten Wellenlänge im Spektrum des zur Detektion kommenden Lichts. So ist ein Minimum an optischer Weglängendifferenz in der Detektionsebene der Interferenz DE gegeben, um wenigstens ein Minimum an spektraler Auflösung zu ermöglichen. In der Regel ist der Abstand d_ST jedoch deutlich größer als zehn Wellenlängen der größten Wellenlänge im Spektrum ausgebildet und kann in einem typischen Fall durchaus einige einhundert Wellenlängen der größten Wellenlänge im Spektrum betragen. Die Geraden g1 und g2 stellen im zyklischen Zweistrahl-Interferometer verlängerte Geraden in der Referenzebene RE dar, welche in der Fläche des ersten Planspiegels beziehungsweise des zweiten Planspiegels und/oder in der ersten und beziehungsweise der zweiten planen Spiegelfläche liegen und jede plane Spiegelfläche ist dabei Bestandteil eines Spiegelprismas.

Weiterhin ist vorzugsweise in einem zyklischen Zweistrahl-Interferometer für die Fourier-Transformations-Spektroskopie in jedem der beiden Strahlengänge im zyklischen Zweistrahl-Interferometer mit umgekehrtem Richtungssinn mit den Teilstrahlenbündeln TB1 und TB2 jeweils ein Bildfeld-Diskriminator BFD1 und BFD2 mit jeweils mindestens einem nichtdiskriminierenden Bereich in Transmission oder Reflexion angeordnet. Die Breite b des nichtdiskriminierenden Bereichs überschreitet vorzugsweise jeweils nicht den Abstand 1,2*d_ST. Somit wird ein Übersprechen zwischen den Teilstrahlenbündeln mit großer Sicherheit vermieden, da sich die zum Hauptstrahl zentrierten Bildfeld-Diskriminator BFD1 und BFD2 näherungsweise um den Abstand 1,4*d_ST voneinander entfernt befinden.

Weiterhin ist vorzugsweise in einem zyklischen Zweistrahl-Interferometer für die Fourier-Transformations-Spektroskopie in jedem der beiden Strahlengänge im zyklischen Zweistrahl-Interferometer mit umgekehrtem Richtungssinn die Breite b_S" des geometrisch-optischen Bildes der Lichtquelle kleiner als der Abstand d_ST oder gleich dem Abstand d_ST am Ort der Bildfeld-Diskriminatoren ausgebildet. Somit wird ein in der Regel schädliches Übersprechen zwischen den beiden Teilstrahlenbündeln sicher verhindert.

Weiterhin ist in einem zyklischen Zweistrahl-Interferometer im Bereich Z, wo es keine räumliche Überdeckung der beiden Teilstrahlenbündel TB1 und TB2 gibt, bevorzugt mindestens eine Spiegeltreppe mit je zwei Spiegeln angeordnet.

Bevorzugt sind in einem zyklischen Zweistrahl-Interferometer im Bereich Z, wo es keine räumliche Überdeckung der beiden Teilstrahlenbündel TB1 und TB2 gibt, zwei Spiegeltreppen mit je zwei Spiegeln angeordnet, welche bevorzugt miniaturisiert ausgebildet sind. Die Anordnung von einer oder von zwei Spiegeltreppen ermöglicht eine gezielte Beeinflussung sowohl der Lateral-Shear s als auch der optischen Weglängendifferenz in einem zyklischen Zweistrahl-Interferometer. Bei einer Anordnung von zwei Spiegeltreppen ist eine Kompensation der optischen Wege der Spiegeltreppen im zyklischen Zweistrahl-Interferometer gegeben, sodass in Differenz der optischen Weglängen der beiden Spiegeltreppen sehr kleine optische Weglängendifferenzen vorbestimmt erzeugt werden können.

Bevorzugt sind in einem zyklischen Zweistrahl-Interferometer im Bereich Z, wo es keine räumliche Überdeckung der beiden Teilstrahlenbündel TB1 und TB2 gibt, die beiden Spiegel einer ersten Spiegeltreppe als Planspiegel und die beiden Spiegel einer zweiten Spiegeltreppe als Planspiegel ausgebildet. Dies ermöglicht eine vergleichsweise kostengünstige Herstellung einer Spiegeltreppe und eine vergleichsweise einfache Justierung des zyklischen Zweistrahl-Interferometers.

Bevorzugt sind in einem zyklischen Zweistrahl-Interferometer im Bereich Z, wo es keine räumliche Überdeckung der beiden Teilstrahlenbündel TB 1 und TB2 gibt, sowohl bei der ersten miniaturisierten Spiegeltreppe die beiden Planspiegel als auch bei der zweiten miniaturisierten Spiegeltreppe die beiden Planspiegel jeweils parallel zueinander angeordnet. Diese Parallelanordnung ermöglicht einen Strahlversatz mit zueinander parallelen optischen Achsen der Teilstrahlenbündel. Auch für die Herstellung einer Spiegeltreppe, beispielsweise durch eine Single-point-Diamantbearbeitung, stellt dies einen Vorteil dar, da so die Abweichung von der Parallelität der beiden Planspiegel sehr klein gehalten werden kann.

Die beiden parallel gestellten Planspiegel der ersten Spiegeltreppe und die beiden parallel gestellten Planspiegel der zweiten Spiegeltreppe in einem zyklischen Zweistrahl-Interferometer weisen bevorzugt einen etwas unterschiedlichen Abstand auf. Die parallel gestellten Planspiegel der ersten Spiegeltreppe und die beiden parallel gestellten Planspiegel der zweiten Spiegeltreppe sind also unterschiedlich beabstandet. Dies ermöglicht die Einführung einer zusätzlichen optischen Weglängendifferenz zwischen den beiden Teilstrahlenbündeln mit umgekehrtem Richtungssinn im zyklischen Zweistrahl-Interferometer, da jedes Teilstrahlenbündel TB1 und TB2 einer anderen Spiegeltreppe zugeordnet ist. Die Spiegeltreppen sind im Vergleich zum ersten und zum zweiten Planspiegel im umlaufenden Strahlengang des zyklischen Zweistrahl-Interferometers bevorzugt miniaturisiert ausgebildet. Bevorzugt ist entweder je einer Spiegeltreppe ein Bildfeld-Diskriminator zugeordnet, bevorzugt in Form einer Spaltblende, und/oder jeweils ein Planspiegel einer Spiegeltreppe ist bevorzugt hinreichend schmal, im Extremfall in Schneidenform, ausgebildet, um dann selbst als Bildfeld-Diskriminator in der Bildebene zu wirken. Bevorzugt kann der Bildfeld-Diskriminator in der Bildebene auch als gerasterte Abschattblende ausgebildet sein, bevorzugt auch als ein Pinhole-Array, optimiert hinsichtlich der Pinhole-Größe für den jeweiligen Spektralbereich. Auch kann bevorzugt als Bildfeld-Diskriminator in der Bildebene ein mikromechanisches, rechnersteuerbares Pinhole-Array eingesetzt werden, bei welchem die Pinholes einzeln adressierbar sind. Wird dagegen ein schmaler Spiegel als Bildfeld-Diskriminator in der Bildebene angeordnet, kann dieser auch als ein rechnersteuerbares digitales Mikrospiegel-Array ausgebildet sein. In Verbindung mit einem Scanner können diese Anordnungen auch im Terahertz-Bereich für bildgebende Flughafenscanner für Personen und Transportgüter im Durchlichtverfahren mit Spektralauflösung eingesetzt werden. Dabei befindet sich die Person und/oder das Transportgut zwischen einer langgestreckten Terahertz-Strahlungsquelle und dem Spektrometer.

Die Nutzung von Spiegeltreppen in einem zyklischen Zweistrahl-Interferometer ist also besonders für den mittel- und ferninfraroten Spektralbereich sowie für den Terahertz-Bereich von Vorteil, wo die wellenoptische Schärfentiefe aufgrund der großen Wellenlänge der elektromagnetischen Strahlung vergleichsweise groß ist und die durch die unterschiedlichen Abstände der beiden Planspiegel einer Spiegeltreppe zusätzlich eingeführte optischen Weglängendifferenz im zyklischen Zweistrahl-Interferometer bevorzugt die wellenoptische Schärfentiefe nicht überschreitet. Dies minimiert Nichtlinearitäten in den räumlichen Interferogrammen. Andererseits sollte die Differenz der Abstände der beiden Planspiegel bevorzugt mindestens zwei Wellenlängen der größten Wellenlänge im genutzten Spektralbereich betragen, um räumliche Interferogramme asymmetrisch auf dem gerasterten Detektor auszubilden.

Bevorzugt kann bei zwei Spiegeltreppen in einem zyklischen Zweistrahl-Interferometer mit einem etwas unterschiedlichen Abstand der Planspiegel derselben, wodurch sich unterschiedliche optische Weglängen ergeben, jeder Spiegeltreppe noch eine Planparallelplatte aus refraktivem Material zugeordnet sein, um Bildverschiebungen zu kompensieren. Die Planparallelplatten sind dabei mit unterschiedlichen Dicken und/oder mit unterschiedlichen Brechungsindizes ausgebildet. So lässt sich bei Bedarf eine vollständige Kompensation der Bildverschiebungen bei unterschiedlichen optischen Weglängen von Spiegeltreppen erreichen. Dies stellt auch für den nah- und mittel-infraroten Spektralbereich einen interessanten Ansatz dar, um ungestörte räumliche Interferogramme zu erhalten, wenn die gegebene wellenoptische Schärfentiefe nicht sehr groß ist.

Weiterhin ist es auch möglich, dass die Spiegeltreppen in Prismenform mit refraktiven Materialien ausgebildet sind.

Weiterhin sind in einem zyklischen Zweistrahl-Interferometer im Bereich Z, wo es keine räumliche Überdeckung der beiden Teilstrahlenbündel TB1 und TB2 gibt, die erste Spiegeltreppe und die zweite Spiegeltreppe zu einer Doppelspiegeltreppe mit einem Mittelsteg kombiniert und die beiden inneren Planspiegel sind jeweils auf einer Seite des Mittelsteges der Doppelspiegeltreppe parallel zueinander angeordnet. Die Ausbildung und Anordnung einer kompakten Doppelspiegeltreppe lässt einen besonders großen Aperturwinkel alpha zu, da die inneren Planspiegel durch die Nutzung eines bevorzugt vergleichsweise dünnen Mittelsteges mit je einer Spiegelfläche vergleichsweise räumlich dicht beieinander angeordnet sind. So ist also bevorzugt ein dünner Mittelsteg mit je einer Spiegelfläche in der Mitte einer Doppelspiegeltreppe angeordnet. Außerdem ermöglicht die Ausbildung einer Doppelspiegeltreppe, welche bevorzugt in einem Arbeitsgang durch Single-point-Diamantbearbeitung auf einer Ultrapräzisionsmaschine hergestellt wird, dass der Pyramidalfehler der vier Planspiegel sehr klein gehalten werden kann. Durch die bevorzugte Herstellung der Doppelspiegeltreppe als kompakter Monolith aus Metall mittels einer Single-point-Diamantbearbeitung weist das zyklische Interferometer eine hohe Robustheit auf.

Weiterhin ist bevorzugt in einem zyklischen Zweistrahl-Interferometer mindestens einer Spiegeltreppe ein Bildfeld-Diskriminator zugeordnet. In der Regel ist vorzugsweise sowohl der ersten Spiegeltreppe als auch der zweiten Spiegeltreppe jeweils ein Bildfeld-Diskriminator zugeordnet.

Weiterhin ist bevorzugt in einem zyklischen Zweistrahl-Interferometer der Bildfeld-Diskriminator entweder als eine Spaltblende, als ein schmales Pinhole-Array und/oder als ein gerastertes, mikromechanisches, rechnersteuerbares Pinhole-Array ausgebildet.

Bei bevorzugter Anordnung von zwei Bildfeld-Diskriminatoren - in Zuordnung eines ersten Bildfeld-Diskriminators zur ersten Spiegeltreppe und eines zweiten Bildfeld-Diskriminators zu einer zweiten Spiegeltreppe - sind diese beiden Bildfeld-Diskriminatoren vorzugsweise baugleich ausgebildet. Diese Bildfeld-Diskriminatoren sind entweder jeweils vorzugsweise als eine Spaltblende und/oder vorzugsweise als jeweils ein schmales Pinhole-Array und/oder vorzugsweise jeweils als ein gerastertes, mikromechanisches, rechnersteuerbares Pinhole-Array ausgebildet. Das mikromechanische, rechnersteuerbare Pinhole-Array kann also vorzugsweise auch als ein mikromechanisches Bauelement gestaltet sein, bei welchem die Pinholes einzeln rechnersteuert adressierbar sind. Das Letztgenannte kann die Flexibilität des Spektrometers erhöhen und kann zur Reduzierung des optischen Übersprechens zwischen den beiden Teilstrahlengängen bei wenig kooperativen Messobjekten und/oder Messfeldern beitragen, welche beispielsweise hinsichtlich der Strahlungsenergie in sich stark strukturiert sind.

Weiterhin ist bevorzugt in einem zyklischen Zweistrahl-Interferometer ein Bildfeld-Diskriminator als ein schmaler Planspiegel ausgebildet, welcher einen der beiden Planspiegel einer Spiegeltreppe bildet. Andere Bildfeld-Diskriminatoren wie beispielsweise eine Spaltblende sind somit überflüssig.

Weiterhin ist bevorzugt in einem zyklischen Zweistrahl-Interferometer ein Bildfeld-Diskriminator als ein rechnersteuerbares Digitales Mikrospiegel-Array (DMD) in mindestens einer Spiegeltreppe ausgebildet. Ein Planspiegel einer Spiegeltreppe ist also durch ein rechnersteuerbares Digitales Mikrospiegel-Array (DMD) ersetzt.

Im langwelligen Spektralbereich ist die in der Regel funktionsbedingt gegebene Neigung des Digitales Mikrospiegel-Arrays aufgrund der bei großen Wellenlängen vergleichsweise großen Schärfentiefe eher kein Problem, wenn der adressierte Bereich auf dem geneigten Mikrospiegel-Array vergleichsweise schmal ausgebildet ist.

Weiterhin besteht bevorzugt in einem zyklischen Zweistrahl-Interferometer mit zwei Spiegeltreppen im umlaufenden Strahlengang sowohl ein vergleichsweise kleiner Keilwinkel tau_1 von bis zu 10 Altgrad zwischen den beiden Planspiegeln der ersten Spiegeltreppe als auch ein vergleichsweise kleiner Keilwinkel tau_2 von bis zu 10 Altgrad zwischen den beiden Planspiegeln der zweiten Spiegeltreppe und die Beträge dieser beiden Keilwinkel tau_1 und tau_2 sind in beiden Spiegeltreppen zumindest näherungsweise gleichgemacht. Die zumindest näherungsweise Gleichheit der Beträge der Keilwinkel tau_1 und tau_2 ist eine Voraussetzung, dass kontrastreiche räumliche Interferogramme in der Detektionsebene erzeugt werden können. Durch die Einführung der Keilwinkel tau_1 und tau_2 zwischen den beiden Planspiegeln der ersten und der zweiten Spiegeltreppe erfolgt im zyklischen Zweistrahl-Interferometer ein Abknicken der beiden Strahlengänge. Dieses Abknicken erfolgt vorzugsweise zum Strahlteiler im zyklischen Zweistrahl-Interferometer hin und schließt somit den Strahlengang etwas. Dies führt zu einer gewissen Minimierung der optischen Weglänge im zyklischen Zweistrahl-Interferometer, welche die Voraussetzung ist, um einen möglichst großen Öffnungswinkel für das Eingangsstrahlenbündel und somit für die beiden Teilstrahlenbündel TB1 und TB2 im Interferometer ausbilden zu können. So kann bei einer Optimierung der Lage der Komponenten des zyklischen Zweistrahl-Interferometers eine numerische Apertur von NA=0,2 für das Eingangsstrahlenbündel und auch für die beiden Teilstrahlenbündel bei einem zyklischen Zweistrahl-Interferometer erreicht werden. Durch das Abknicken zum Strahlteiler hin schließen die beiden Hauptstrahlen der von einer Spiegeltreppe nach oben und nach unten jeweils abgehenden Teilstrahlenbündel auf der Seite des Strahlteilers einen Winkel von weniger als 180 Altgrad ein, beispielsweise einen Winkel kappa von 168 Altgrad. Der Winkel kappa gemäß der Gleichung (1)

$$\text{kappa} = 180\ \text{Altgrad} - 2(\text{tau\_1} + \text{tau\_2}) \qquad\qquad \text{Gleichung (1)}$$

besteht also zwischen dem Hauptstrahl HTB1a, der von der ersten Spiegeltreppe abgeht, und dem Hauptstrahl HTB2a, der von der zweiten Spiegeltreppe abgeht, auf der dem Interferometer zugekehrten Seite. Der Winkel kappa ist vorzugsweise kleiner als 180 Altgrad, jedoch bevorzugt nicht kleiner als 140 Altgrad ausgebildet.

Weiterhin ist in einem zyklischen Zweistrahl-Interferometer bevorzugt mindestens eine miniaturisierte Spiegeltreppe im Bereich Z angeordnet, bei welcher mindestens ein Spiegel mit einer schwachen Krümmung ausgebildet ist. Besser ist es jedoch bevorzugt die beiden Spiegel einer Spiegeltreppe mit einer schwachen Krümmung auszubilden. So kann innerhalb des zyklischen Zweistrahl-Interferometers eine schwache Wellenfrontformung in einem Teilstrahlenbündel durchgeführt werden. Damit kann der Ort des Bildes bei mindestens einem Teilstrahlenbündel in der Tiefe so beeinflusst werden, dass auch bei unterschiedlichen optischen Weglängen für die Hauptstrahlen in den beiden Teilstrahlenbündeln im zyklischen Zweistrahl-Interferometer, welche durch etwas unterschiedliche Spiegelabstände der beiden Spiegeltreppen bestehen, die beiden kohärenten Bilder dennoch in einer gemeinsamen Bildebene liegen. So ist es möglich, Nichtlinearitäten in den räumlichen Interferogrammen durch eine gemeinsame Bildebene der kohärenten Teilbilder weitgehend zu vermeiden.

So sind bei einer bevorzugten Anordnung von zwei Spiegeltreppen im zyklischen Zweistrahl-Interferometer in der Regel insgesamt sechs Spiegel angeordnet, von denen vier Spiegel jedoch im Wesentlichen miniaturisiert sind.

Weiterhin sind in einem zyklischen Zweistrahl-Interferometer für die Fourier-Transformations-Spektroskopie im umlaufenden Strahlengang vorzugsweise zwei baugleiche Spiegelprismen aus jeweils gleichem refraktiven Material, also ein erstes und ein zweites Spiegelprisma, angeordnet und die Spiegelprismen sind jeweils mit einer einzigen Spiegelfläche ausgebildet. Diese beiden Spiegelprismen weisen jeweils den gleichen spitzen Winkel psi zwischen der Spiegelfläche und jeweils einer nichtverspiegelten Fläche auf. Dabei sind die unverspiegelten Flächen der Spiegelprismen jeweils sowohl zum Strahl-Eintritt als auch zum Strahl-Austritt genutzt. Zwischen diesen beiden baugleichen Spiegelprismen sind zwei Bildfeld-Diskriminatoren positioniert. Diese können sich zwischen den baugleichen Spiegelprismen in einem parallelen Luftspalt befinden und/oder auch in einer Kittschicht eingebettet sein, welche die beiden Spiegelprismen mechanisch und auch optisch verbindet. Vorzugsweise sind die beiden Bildfeld-Diskriminatoren durch zwei einzelne Spaltblenden und/oder auch durch eine Doppelspaltblende dargestellt, deren Spalten jeweils die Bildfeld-Diskriminatoren darstellen. Es ist aber auch bevorzugt der Einsatz eines Flüssigkristall-Displays möglich, welches in Verbindung mit Polarisationsoptik zwei Durchlassbereiche, vorzugsweise in Spaltenform aufweist. Bevorzugt sind die baugleichen Spiegelprismen auch baugleich.

In diesem zyklischen Interferometer sind zwei weitere, spitzwinklige und gleichschenklige Dreieckprismen, also ein drittes und ein viertes Prisma, angeordnet, die auch baugleich sind. Diese beiden Dreieckprismen weisen jeweils zwei Mal einen Winkel von 2psi auf. Die Ausbildung der vier Prismen mit den hier angegeben Winkeln, basierend auf dem Halbwinkel psi, ermöglichen für die Hauptstrahlen der beiden Teilstrahlenbündel TB 1 und TB2 in der Regel einen senkrechten Strahl-Ein- und Strahl-Austritt.

Zwischen diesen beiden Dreieckprismen, die miteinander verkittet sind, befindet sich eine Strahlteilerschicht. Diese beiden Dreieckprismen bilden somit einen Strahlteiler. Die Strahlteilerschicht wird sowohl zur Strahlteilung als auch zur Strahlwiedervereinigung genutzt, wobei bei der Strahlwiedervereinigung eine erhebliche, jedoch erwünschte Lateral-Shear s zwischen den Teilstrahlenbündel besteht. Dennoch wird hier nicht ganz korrekt von Strahlwiedervereinigung der Teilstrahlenbündel gesprochen, da diese ja mit einer Lateral-Shear s vereinigt werden, also die Vereinigung ja somit nur teilweise besteht. Die Interferenz wird ja sowieso erst auf dem Detektor sichtbar und kommt dort erst zur Erfassung.

Der spitze Winkel psi bei den beiden baugleichen Spiegelprismen ist größer/gleich 15 Altgrad und kleiner/gleich 30 Altgrad. Ein kleinerer Winkel für psi als 15 Altgrad vergrößert in der Tendenz zunehmend die optische Weglänge im Interferometer und führt zunehmend zu einem kleineren nutzbaren Aperturwinkel alpha. Ein größerer Winkel für psi als 30 Altgrad begrenzt jedoch auch wieder den verfügbaren Raum für die Teilstrahlenbündel und führt ebenfalls zunehmend zu einem kleineren nutzbaren Aperturwinkel alpha.

Als besonders geeignet erweist sich für den spitzen Halbwinkel psi im zyklischen Zweistrahl-Interferometer ein Bereich bevorzugt von 20 Altgrad bis 28 Altgrad, um einen großen nutzbaren Aperturwinkel alpha refraktiven Material zu erreichen. Optimal ist in vielen Fällen vorzugsweise ein spitzer Halbwinkel psi von 23 Altgrad bis 26 Altgrad mit einem Optimum bei bevorzugt 24 Altgrad. Dieser Winkelbereich ermöglicht einen Aperturwinkel im refraktiven Material von 9 Altgrad zyklischen Interferometer.

Durch die beiden spitzen Winkel psi in jedem Spiegelprisma besteht dort jeweils auch ein stumpfer Winkel 2rho, dessen Betrag rho sich aus der Differenz von 90 Altgrad und dem Betrag des spitzen Winkels psi ergibt. Durch Einhaltung dieser Bedingung für den Winkel rho kann ein achssenkrechter Strahldurchgang durch jedes Spiegelprisma erreicht werden, was die Aberrationen im Strahlverlauf ganz wesentlich minimiert und so die Gewinnung weitgehend ungestörter räumlicher Interferogramme erst ermöglicht.

Es ist aber auch möglich, in einem zyklischen Zweistrahl-Interferometer für die Fourier-Transformations-Spektroskopie vorzugsweise zwei baugleiche Spiegelprismen mit einem Winkel psi angeordnet, welche jeweils eine einzige Spiegelfläche aufweisen. Dabei ist im zyklischen Zweistrahl-Interferometer hier ein Strahlteiler angeordnet, der diesen Spiegelprismen zugeordnet ist. Der Strahlteiler ist hier entweder in Folienform und/oder als ein feines, ebenflächiges Drahtgitter in Parallelanordnung der Drähte ausgebildet. Der spitze Halbwinkel psi im zyklischen Zweistrahl-Interferometer ist vorzugsweise mit einem Betrag größer gleich 20 Altgrad und kleiner/gleich 30 Altgrad ausgebildet. Dieser Winkelbereich erweist sich als besonders geeignet, um einen großen nutzbaren Aperturwinkel alpha zu erreichen. Optimal ist in vielen Fällen vorzugsweise ein spitzer Halbwinkel psi mit einem Betrag von 24 Altgrad bis 27 Altgrad mit einem Optimum bei bevorzugt 26 Altgrad. Letzteres ermöglicht einen Aperturwinkel alpha im refraktiven Material von mindestens 9 Altgrad.

Weiterhin sind in einem zyklischen Zweistrahl-Interferometer für die Fourier-Transformations-Spektroskopie vorzugsweise zwei winkelgleiche Spiegelprismen und bevorzugt ein weiteres spitzwinkliges und gleichschenkliges Dreieckprisma angeordnet, welches an eines der beiden Spiegelprismen gekittet ist. Der Kittschicht ist eine Strahlteilerschicht zugeordnet. Die beiden winkelgleichen Spiegelprismen weisen jeweils einen spitzen Winkel psi zwischen der verspiegelten und mindestens einer nichtverspiegelten Fläche auf. Das spitzwinklige und gleichschenklige Dreieckprisma weist zweimal den gleichen Winkel von 2psi auf.

Zwischen dem ersten Spiegelprisma und dem zweiten Spiegelprisma sind jeweils die beiden Bildfeld-Diskriminatoren entweder in einem parallelen Luftspalt angeordnet und/oder in einer Kittschicht eingebettet.

Dabei ist eine dieser unverspiegelten Flächen des ersten Spiegelprismas sowohl jeweils zum Strahl-Eintritt als auch jeweils zum Strahl-Austritt zum Strahl-Eintritt genutzt. Eine unverspiegelte Fläche des zweiten Spiegelprismas ist ebenfalls sowohl jeweils zum Strahl-Eintritt als auch jeweils zum Strahl-Austritt genutzt. Die Ausbildung der drei Prismen mit den hier angegeben Winkeln, basierend auf dem Halbwinkel psi, ermöglichen für die Hauptstrahlen der beiden Teilstrahlenbündel TB1 und TB2 in der Regel einen senkrechten Strahl-Ein- und Strahl-Austritt.

Der spitze Halbwinkel psi im zyklischen Zweistrahl-Interferometer ist im Betrag größer gleich 15 Altgrad und kleiner/gleich 30 Altgrad. Auch hier begrenzt ein größerer Winkel als 30 Altgrad für den spitzen Winkel den Strahlraum und führt zunehmend zu einem kleineren nutzbaren Aperturwinkel alpha. Ein kleinerer Winkel als 15 Altgrad für den spitzen Winkel führt im Strahlraum ebenfalls zunehmend zu einem kleineren nutzbaren Aperturwinkel alpha im zyklischen Zweistrahl-Interferometer, was nicht angestrebt wird.

Als besonders geeignet erweist sich hier im zyklischen Zweistrahl-Interferometer für den spitzen Halbwinkel psi auch ein Bereich von bevorzugt 20 Altgrad bis 28 Altgrad, um einen großen nutzbaren Aperturwinkel alpha zu erreichen. Optimal ist in vielen Fällen vorzugsweise ein Halbwinkel psi mit einem Betrag von 22 Altgrad bis 26 Altgrad mit einem Optimum bei bevorzugt 24 Altgrad. Letzteres ermöglicht einen Aperturwinkel alpha im refraktiven Material von mindestens 9 Altgrad, ohne dass Strahlenbündelkegel zu beschneiden.

Auch hier besteht durch die beiden spitzen Winkel psi in jedem Spiegelprisma in jedem Spiegelprisma auch ein stumpfer Winkel 2rho und der Betrag rho ergibt sich aus der Differenz von 90 Altgrad und dem Betrag des spitzen Winkels psi. Durch Einhaltung dieser Bedingung kann ebenfalls ein achssenkrechter Strahldurchgang der Hauptstrahlen durch jedes Spiegelprisma erreicht werden, was die Aberrationen im optischen System erheblich minimiert.

Weiterhin sind in einem zyklischen Zweistrahl-Interferometer für die Fourier-Transformations-Spektroskopie vorzugsweise zwei Spiegelprismen aus gleichem refraktiven Material, welche nur jeweils eine einzige Spiegelfläche aufweisen, angeordnet. Die beiden Spiegelprismen weisen jeweils einen ersten spitzen Winkel psi, einen zweiten spitzen Winkel 2psi und einen dritten Winkel mit dem Betrag 180Altgrad minus 3psi auf. Damit stimmen mindestens drei Winkel an den beiden Spiegelprismen jeweils überein. Außerdem ist eine Planparallelplatte aus refraktivem Material zwischen diesen beiden Spiegelprismen angeordnet und die Planparallelplatte ist durch zwei optisch transparente Kittschichten zwischen diesen beiden Spiegelprismen fixiert.

Bevorzugt sind die beiden Spiegelprismen zumindest näherungsweise geometrisch baugleich ausgebildet. Jedoch sind diese dann etwas gegeneinander verschoben, damit die Glasweglängen in den beiden umlaufenden Strahlengängen gleich sind.

Dabei ist entweder einerseits eine Strahlteilerschicht bevorzugt auf der Seite der Planparallelplatte aufgebracht, welche dem Spiegelprismas zugekehrt ist, oder andererseits ist die Strahlteilerschicht auf dem Spiegelprisma bevorzugt auf der Seite der Planparallelplatte aufgebracht, welche der Planparallelplatte zugekehrt ist.

Diese Anordnung ist gut zu justieren und bietet optimale Bedingungen, um Aberrationen bei der Ausbreitung der Strahlenbündel zu minimieren. Die Frontflächen der Spiegelprismen können zu Justierzwecken, beispielsweise bei einer Kittung unter optischer Beobachtung auspoliert sein, um dies optisch anzutasten, beispielsweise mit einem Autokollimationsfernrohr und Fokussensoren. Bei einem Winkel von 26 Altgrad für den spitzen Halbwinkel psi ist im zyklischen Zweistrahl-Interferometer ein Aperturwinkel im refraktiven Material von 9 Altgrad sicher zu erreichen, was ein Optimum darstellt. Dies gilt auch für den spitzen Halbwinkel psi von 24 Altgrad. Die beiden baugleichen Spiegelprismen sind vorzugsweise in einem Arbeitsgang gefertigt, um somit zumindest näherungsweise eine Baugleichheit zu erreichen.

Bei einem Fourier-Transformations-Spektrometer mit zumindest teilweiser hyperspektraler Single-Shot-Bildgebung von einem Messobjekt als Ergebnis einer Berechnung mit einem Rechnersystem zur Gewinnung von Spektren mittels einer Fourier-Transformation mit einem vorgeordneten Objektiv als Abbildungssystem für das Messobjekt gibt es ein zyklisches Zweistrahl-Interferometer, dem das vorgeordnete Objektiv vorangestellt ist, welches ein Eingangsstrahlenbündel erzeugt.

Dieses Eingangsstrahlenbündel wird hier in der Regel als ein Ensemble von Eingangsstrahlenbündeln verstanden, weil es ja eine Bildinformation transportiert und zu jedem Bildpunkt ein eigenes Eingangsstrahlenbündel gehört, sodass für viele Bildpunkte auch viele Eingangsstrahlenbündel zugehörig sind. So besteht ein Ensemble von Eingangsstrahlenbündeln. In der weiteren Beschreibung in den Figuren geht es bei einem Strahlenbündel letztlich immer um das Ensemble von Strahlenbündel und repräsentativ für viele Strahlenbündel ist ein Strahlenbündel genannt oder figürlich dargestellt.

Das zyklische Zweistrahl-Interferometer weist auf:

einen Strahlteiler mit ebener Strahlteilerfläche
und einen gerasterten Detektor am Ausgang des Zweistrahl-Interferometers.

Der Strahlteiler ist sowohl zur Strahlenbündelteilung in der Strahlteilerebene genutzt, wodurch aus dem Eingangsstrahlenbündel zwei Teilstrahlenbündel (TB1, TB2) gebildet werden, als auch zur zumindest teilweisen Strahlenbündelvereinigung in der Strahlteilerebene mit einer Lateral-Shear s zwischen den beiden Teilstrahlenbündeln genutzt. Präzise formuliert entsteht nach der Strahlteilung jeweils ein erstes Ensemble und ein zweites Ensemble von Teilstrahlenbündeln, daja jedes Teilstrahlenbündeln nur zu einem Bildpunkt gehört, hier jedoch Bilder im Interferometer erzeugt werden, wozu viele Strahlenbündel benötigt werden, da es zu jedem Bildpunkt ein zu diesem zugehöriges Strahlenbündel gibt.

Am zyklischen Zweistrahl-Interferometer besteht eine Referenzebene, welche durch die Normale der ebenen Strahlteilerfläche und durch die optische Achse des vorgeordneten Objektivs am Eingang des Interferometers aufgespannt ist. Das vorgeordnete Objektiv vor dem zyklischen Zweistrahl-Interferometer erzeugt - unter Berücksichtigung der Position des Messobjekts - ein Bild oder mindestens ein Teilbild des Messobjekts in Lichtrichtung in der Regel nach dem Strahlteiler, jedoch in der Regel vor dem gerasterten Detektor. Unter Mitwirkung einer dem zyklischen Zweistrahl-Interferometer nachgeordneten anamorphotischen Abbildungsstufe ist eine Vielzahl von räumlichen Interferogrammen gebildet und diese räumlichen Interferogramme sind auf dem gerasterten flächigen Empfänger ausgebildet und jedem räumlichen Interferogramm ist ein Objektpunkt auf dem Messobjekt zugeordnet. Es werden im Minimum mindestens zwei räumliche Interferogramme generiert.

Das zyklische Zweistrahl-Interferometer ist einerseits wobei es mit zwei Planspiegeln ausgebildet ist, die eine Winkelspiegelanordnung bilden, bei welcher die beiden Planspiegel von der Strahlteilerebene ET, welche die ebene Strahlteilerfläche enthält, gleich im Abstand sind und zumindest näherungsweise senkrecht zur Referenzebene (RE) ausgerichtet sind. Durch die Gleichheit der beiden Planspiegel im Abstand ergibt sich ein symmetrisch aufgebautes zyklisches Zweistrahl-Interferometer.

Das zyklische Zweistrahl-Interferometer ist andererseits wobei es mit zwei Spiegelflächen ausgebildet ist, von denen jeweils genau eine an je einem Spiegelprisma aus refraktivem Material ausgebildet ist und so eine Doppel-Spiegelprismen-Anordnung besteht. Die Doppel-Spiegelprismen-Anordnung ist mit jeweils genau zwei Spiegelprismen ausgebildet. Bei dieser Doppel-Spiegelprismen-Anordnung sind die beiden planen Spiegelflächen von der Strahlteilerebene ET, welche die Strahlteilerfläche enthält, im gleichen Abstand angeordnet und zumindest näherungsweise senkrecht zur Referenzebene (RE) ausgerichtet. Auch hier ergibt sich so durch die Gleichheit der beiden Spiegelflächen im Abstand ein symmetrisch aufgebautes zyklisches Zweistrahl-Interferometer.

Zwischen den beiden Planspiegeln, welche die Winkelspiegelanordnung bilden, und/oder den planen Spiegelflächen der Doppel-Spiegelprismen-Anordnung besteht jeweils ein Winkel epsilon mit dem doppelten Betrag des Halbwinkels psi. Dieser Winkel epsilon ist durch zwei verlängerte Geraden g1 und g2 aus den Planspiegeln und/oder aus den planen Spiegelflächen in der Referenzebene (RE) dargestellt und die Winkelhalbierende des Winkels epsilon liegt in der Strahlteilerebene ET. Der halbe Winkel von epsilon, der Halbwinkel psi, ist mit einem Betrag größer als 20 Altgrad und mit einem Betrag kleiner als 30 Altgrad ausgebildet.

Im symmetrisch aufgebauten zyklischen Zweistrahl-Interferometer bestehen eine erste Bildebene sowie eine zweite Bildebene, welche jeweils durch das vorgeordnete Objektiv, die Lage des Messobjekts und das Zweistrahl-Interferometer gegeben sind. Der ersten Bildebene BEI1 ist ein erster Bildfeld-Diskriminator sowie der zweiten Bildebene ein zweiter Bildfeld-Diskriminator im Zweistrahl-Interferometer zugeordnet und diese beiden Bildfeld-Diskriminatoren im symmetrisch aufgebauten zyklischen Zweistrahl-Interferometer so angeordnet sind, dass zumindest näherungsweise zwischen diesen Bildfeld-Diskriminatoren eine optische Konjugation besteht, und im zyklischen Zweistrahl-Interferometer zwischen den Planspiegeln oder Spiegelprismen im umlaufenden Strahlengang, welche dem Strahlteiler direkt zugeordnet sind, je eine optische Baugruppe zur Erzeugung eines Strahlversatzes je einem Bildfeld-Diskriminator zugeordnet ist und die optische Baugruppe einen Strahlversatz in bevorzugt jeweils entgegengesetzter Richtung erzeugen und so am Ausgang des zyklischen Zweistrahl-Interferometers eine Lateral-Shear s besteht.

Weiterhin sind die optischen Baugruppen zur Erzeugung eines Strahlversatzes im zyklischen Zweistrahl-Interferometer als zwei Spiegeltreppen in Luft und/oder als zwei Rhomboid-Spiegelprismen und/oder als zwei Rhomboid-ähnliche Spiegelprismen ausgebildet. Die Hauptstrahlen der Teilstrahlenbündel nach den Planspiegeln, welche im zyklischen Zweistrahl-Interferometer im umlaufenden Strahlengang eine Winkelspiegelanordnung bilden, sind im gemeinsamen Raum zwischen diesen Planspiegeln kollinear bis diese Hauptstrahlen auf die Spiegeltreppen und/oder Rhomboid-Spiegelprismen auftreffen. Diese Spiegeltreppen und/oder Spiegelprismen bringen die Hauptstrahlen zum Versatz, der vorzugsweise entgegengesetzt erfolgt, um eine möglichst große Lateral-Shear s am Ausgang des zyklischen Zweistrahl-Interferometers entstehen zu lassen.

Weiterhin ist im zyklischen Zweistrahl-Interferometer bevorzugt ein Bildfeld-Diskriminator BFD1, BFD2 als Spaltblende und/oder als ein schmaler Spiegel ausgebildet. Dabei können beide Bildfeld-Diskriminatoren jeweils als Spaltblende und/oder als schmaler Spiegel ausgebildet sein. Es kann aber auch ein Bildfeld-Diskriminator als eine Spaltblende und ein Bildfeld-Diskriminator als ein schmaler Spiegel ausgebildet sein. Bevorzugt kann aber auch mindestens ein Bildfeld-Diskriminator als ein rechnersteuerbares Digitales Mikrospiegel-Array (DMD) ausgebildet sein.

Zusammengefasst gilt: Es ist also auch möglich, bei Einsatz von zwei unterschiedlich ausgebildeten Spiegeltreppen in einem zyklischen Zweistrahl-Interferometer den Abstand d_ST, also den Abstand des Schnittpunkts SP von der Strahlteilerebene ET, auch zu null werden zu lassen. Dann besteht bezüglich der Lage der beiden Planspiegel und/oder Spiegelflächen an Winkelprismen im zyklischen Zweistrahl-Interferometer eine strenge Symmetrie. Die bereits beschriebenen Spiegeltreppen sind im Vergleich zu den beiden Planspiegeln und/oder Spiegelflächen vergleichsweise klein ausgebildet. Diese Spiegeltreppen sind vom Typ entweder als Hohlkörper oder vom Typ als Spiegelprisma mit zwei Spiegelflächen ausgebildet. Das Spiegelprisma ist dabei bevorzugt in Form eines Rhomboid-Spiegelprismas gestaltet. Bevorzugt wird durch die Spiegeltreppen ein Parallelversatz der Teilstrahlenbündel erzeugt, was jedoch nicht zwingend ist. Es genügt, wenn ein Versatz mit dem gleichen Winkelbetrag gegeben ist.

Mittels zwei dieser Spiegeltreppen und jeweils vom gleichen Typ kann eine gewünschte Lateral-Shear s zwischen den Teilstrahlenbündeln eingestellt sein. Diese Lateral-Shear s besteht durch die Anordnung der Spiegeltreppen in bevorzugt entgegengesetzten Richtungen. Auch die optischen Weglängen in den beiden umlaufenden Strahlengängen können durch die Ausbildung der beiden Spiegeltreppen so eingestellt sein, sodass sich auch etwas unterschiedliche Weglängen für die Hauptstrahlen der Teilstrahlenbündel in den beiden umlaufenden Strahlengängen ergeben. Dies dient dem Ziel, die räumlichen Interferogramme etwas verschoben auf dem gerasterten Detektor zu erhalten, um die spektrale Auflösung etwas zu erhöhen zu können.

Weiterhin ist in einem zyklischen Zweistrahl-Interferometer für die Fourier-Transformations-Spektroskopie bevorzugt ein Zweispiegel-Winkelreflektor mit den beiden Planspiegeln um den Symmetriepunkt S mit einem Betrag um bis zu 6 Altgrad gedreht. Somit ist der Zweispiegel-Winkelreflektor aus der Symmetrielage in einem zyklischen Zweistrahl-Interferometer herausgedreht. Es kann aber vorzugsweise auch eine Doppel-Spiegelprismen-Anordnung mit den beiden planen Spiegelflächen um den Symmetriepunkt S mit einem Betrag um bis zu 6 Altgrad gedreht sein. Die Flächen der beiden Spiegelprismen sind dabei in der Winkellage so angepasst, dass die Hauptstrahlen der Teilstrahlenbündel TB1 und TB2 die Flächen der Spiegelprismen jeweils senkrecht passieren. Diese Asymmetrie kann bei schwierigen konstruktiven Randbedingungen zu einem Vorteil hinsichtlich der Nutzung eines großen Aperturwinkels alpha führen.

Weiterhin sind in einem zyklischen Zweistrahl-Interferometer für die Fourier-Transformations-Spektroskopie im Bereich Z, wo es keine räumliche Überdeckung der beiden Teilstrahlenbündel TB1 und TB2 gibt, vorzugsweise sowohl im Teilstrahlenbündel TB1 eine erste zumindest näherungsweise achssenkrecht stehende Planparallelplatte der geometrischen Dicke h1 und aus optischem Material mit dem Brechungsindex n1 als auch im Teilstrahlenbündel TB2 eine zweite zumindest näherungsweise achssenkrecht stehende Planparallelplatte der geometrischen Dicke h2 und aus optischem Material mit dem Brechungsindex n2 angeordnet. Dabei ist jeder Platte eine Spaltblende zugeordnet, die einen Bildfeld-Diskriminator darstellt.

Bei der Dimensionierung der beiden Planparallelplatten ist die Gleichung (2) für deren geometrische Dicken h1 und h2 und die Brechungsindizes n1 und n2 zumindest näherungsweise einzuhalten:

$$h1 * (n1 - 1) / n1 = h2 * (n2 - 1) / n2. \hspace{3cm} \text{Gleichung (2)}$$

Dadurch ist die durch die beiden Planparallelplatten eingeführte axiale Bildverschiebung v1 und v2 gleich. Es besteht also eine vollständige Kompensation der axialen Bildverschiebung am Ausgang des zyklischen Zweistrahl-Interferometers zwischen den beiden Teilstrahlenbündeln TB1 und TB2.

Dabei übersteigt die optische Weglängendifferenz OPD_zykaP am Ausgang des zyklischen Zweistrahl-Interferometers mit der Gleichung (3)

$$OPD\_zykaP = Betrag[(n1 * h1) - (n2 * h2)] \hspace{2cm} \text{Gleichung (3)}$$

vorzugsweise den Betrag von 1mm nicht. In vielen Fällen beträgt diese optische Weglängendifferenz OPD zykaP vorzugsweise nur 0,03mm bis 0,2mm. Dies ist vor allem auch der Verfügbarkeit von gerasterten Detektoren, wie Mikrobolometer-Array, im NIR- und MIR-Spektralbereich beim Stand der Technik geschuldet, die selten mehr als 1.000 Pixel in der Breite bk der Sensorfläche, in der Regel ein Chip, aufweisen. Im VIS-Spektralbereich können dagegen CMOS-Chips mit bis zu 10.000 Pixel in der Breite bk des Chips aufweisen, wodurch für die optische Weglängendifferenz OPD_zykaP entsprechend größere Werte ergeben, die hier vorzugsweise 2mm nicht überschreiten.

Ein sinnvoller Wert ist in vielen Fällen, wenn die optische Weglängendifferenz OPD zykaP etwa 50% der optischen Weglängendifferenz OPDr beträgt, welche wiederum bevorzugt in vielen Fällen um 0,15mm für den NIR- und MIR-Bereich beträgt. So ergibt sich eine maximale optische Weglängendifferenz OPD_rechts bei der Detektion eines räumlichen Interferogramms von 0,225mm. Daraus ergibt sich eine spektrale Auflösung delta_sigma als Kehrwert der maximalen optischen Weglängendifferenz OPD rechts zu delta_sigma zu 44,4cm$^{-1}$. Für die Berechnung der Phasenkorrektur bei der Berechnung des Spektrums mittels einer schnellen Fourier-Transformation verbleibt hier auch ein Stück des linksseitigen Interferogramms mit einer optischen Weglängendifferenz OPD links von 0,075mm. Diese Zusammenhänge sind den Fachleuten in der Fourier-Transformations-Spektroskopie jedoch bestens bekannt.

Die optische Weglängendifferenz OPDr ergibt sich aus der Lateral-Shear s, der verfügbaren Länge bk des gerasterten Detektors und der numerischen Apertur des rotationssymmetrischen Objektivs im nachgeordneten anamorphotischen Objektiv.

So ist am Ausgang des zyklischen Zweistrahl-Interferometers die Bildverschiebung v1 in der Tiefe für das Teilstrahlenbündel TB 1 gleich der Bildverschiebung v2 für das Teilstrahlenbündel TB2 und somit besteht eine Kompensation der Bildverschiebungen in der Tiefe. Dies ist die Voraussetzung für das Entstehen von Zylinderwellen mit jeweils geradliniger Scheitellinie auf dem gerasterten Detektor, die dort zur Interferenz kommen. Dies ist eine Voraussetzung für das Entstehen räumlicher Interferogramme, die weitgehend frei von Nichtlinearitäten sind.

Die Bildverschiebung v1 sich zu

$$v1 = h1(n1-1)/n1 \qquad \text{Gleichung (4)}$$

und die Bildverschiebung v2 ist

$$v2 = h2(n2-1)/n2. \qquad \text{Gleichung (5)}$$

Die sich ergebende optische Weglängendifferenz OPD_zykaP ist dann ungleich null und ergibt sich betragsmäßig mit der bereits genannten Gleichung (3).

Die unterschiedliche Dispersion in den Materialien der beiden Planparallelplatten kann algorithmisch durch eine Phasenkorrektur bei der Berechnung des Spektrums hinsichtlich verfälschender Einflüsse unschädlich gemacht werden. Hierbei ist zumindest im VIS- und im NIR-Spektralbereich durch die Auswahl der refraktiven Materialien sowohl hinsichtlich deren Brechungsindizes als auch deren Dispersion auch eine Minimierung der unterschiedlichen Dispersionen möglich. Nichtkompensierte Dispersionen im Zweistrahl-Interferometer erzeugen letztlich im räumlichen Interferogramm das gut bekannte Chirping. Dieses Chirping ist jedoch unter Nutzung der Algorithmen zur Phasenkorrektur beim Stand der Technik weitgehend ohne Einfluss auf das berechnete Spektrum.

Weiterhin ist in einem zyklischen Zweistrahl-Interferometer für die Fourier-Transfonnations-Spektroskopie im Bereich Z, wo es keine räumliche Überdeckung der beiden Teilstrahlenbündel TB1 und TB2 gibt, vorzugsweise eine optische Trägerplatte der Dicke h1 und dem Brechungsindex n1 oder eine Trägerplatte der Dicke h2 und dem Brechungsindex n2 sowohl der ersten als auch der zweiten Planparallelplatte zugeordnet. Zwischen der Trägerplatte und sowohl der ersten als auch der zweiten Planparallelplatte befindet sich dabei jeweils eine Doppelspaltblende mit zwei Spaltöffnungen. Wird die optische Trägerplatte mit der Dicke h2 ausgebildet, was hier das optische Material mit dem höheren Brechungsindex darstellt, kann diese etwas dünner ausgebildet sein, was konstruktive Vorteile bring. In Standardfällen wird die Trägerplatte aus dem niederbrechenden optischen Material gefertigt. Dann kann als optisches Material das kostengünstige Borkron-Standardglas BK7 eingesetzt werden. Anstelle der Doppelspaltblende können vorzugsweise aber auch zwei einzelne Spaltöffnungen angeordnet sein.

Weiterhin ist in einem Zweistrahl-Interferometer für die Fourier-Transformations-Spektroskopie vorzugsweise der Strahlteiler mit einer polarisierenden Strahlteilerschicht ausgebildet. So verlässt den Strahlteiler senkrecht und parallel zur Einfallsrichtung polarisiertes Licht und gelangt beidseitig auf das transmissive Flüssigkristall-Display. Nach Transmission desselben ist in den adressierten Bereichen desselben die Polarisationsrichtung um 90 Altgrad gedreht. Zunächst beim Einfall ins zyklische Interferometer am polarisationsoptischen Strahlteiler: reflektiertes Licht wird nun transmittiert. Diese Zusammenhänge sind der Fachwelt bestens vertraut. Für den ferninfraroten Spektralbereich kann der Strahlteiler vorzugsweise auch als eine Anordnung mit einem feinen, ebenflächigen Drahtgitter ausgebildet sein.

Weiterhin ist vorzugsweise dem Zweistrahl-Interferometer für die Fourier-Transformations-Spektroskopie ein Polarisationsanalysator nachgeordnet. Dabei ist das Zweistrahl-Interferometer bevorzugt als ein zyklisches Zweistrahl-Interferometer ausgebildet. Das Zweistrahl-Interferometer kann aber vorzugsweise auch als ein Michelsontyp-Interferometer oder als ein Mach-Zehnder-Interferometer dargestellt sein. Die Anordnung eines Polarisationsanalysators erfolgt dabei in der Regel in Kombination mit wenigstens einer anderen polarisationsoptischen Komponente wie einem Strahlteiler mit einer polarisierenden Strahlteilerschicht. Diese Zusammenhänge sind der Fachwelt bestens vertraut.

Weiterhin ist vorzugsweise dem Messobjekt für die Fourier-Transformations-Spektroskopie eine Lichtquelle zur Beleuchtung des Messobjekts zugeordnet und diese ist mit mindestens einem leuchtenden Feld in Form mindestens eines einzelnen, bevorzugt schmalen Lichtstreifens ausgebildet. Diese Beleuchtung mittels Streifen kommt bevorzugt bei einem zyklischen Interferometer zur Anwendung, da gesichert werden muss, dass jeder Durchlassbereich eines Bildfeld-Diskriminators nur von Licht in einer Umlaufrichtung beleuchtet wird. Wenn ein Übersprechen von Licht in einer Umlaufrichtung auf den zweiten, in der Regel unmittelbar benachbarten Bildfeld-Diskriminator im zyklischen Interferometer erfolgt, ist die Entstehung von Zweistrahlinterferenz nicht mehr gegeben, was die Signalauswertung erheblich erschweren oder ganz unmöglich machen würde. Beim Michelsontyp-Interferometer und beim Mach-Zehnder-Interferometer besteht dagegen die Gefahr eines Übersprechens nicht, da die Bildfeld-Diskriminatoren in den beiden separierten Armen des Interferometers angeordnet und somit deutlich getrennt sind. Beim Michelsontyp-Interferometer und beim Mach-Zehnder-Interferometer dient eine Beleuchtung mittels eines Lichtstreifens vor allem dem effektiven Nutzen von Lichtenergie, aber auch der Vermeidung von unerwünschten Lichtreflexen und von Streulicht. Um definierte Verhältnisse bei der lateralen Auflösung des Messobjekts zu bekommen, macht es Sinn, wenn der Lichtstreifen dabei den Durchlassbereich eines Bildfeld-Diskriminators voll überdeckt. Der Durchlassbereich eines Bildfeld-Diskriminators kann eine Öffnung in Spaltform und/oder auch ein schmaler, hochreflektierender Bereich sein. Die Lichtquelle kann auch als Blitzlichtquelle ausgebildet sein, was insbesondere bei bewegten Szenen von Vorteil ist.

Weiterhin ist vorzugsweise dem Messobjekt für die Fourier-Transformations-Spektroskopie eine Lichtquelle zur strukturierten Beleuchtung des Messobjekts zugeordnet. Die strukturierte Beleuchtung des Messobjekts kann in Abstimmung mit der Ausbildung der Bildfeld-Diskriminatoren ein beliebiges Muster annehmen, vorzugsweise auch in Form eines Musters mit feinen Lichtflecken auf dem Messobjekt. Diese strukturierte Art der Objektbeleuchtung mit nachfolgender Diskriminierung ist von der konfokalen Technik bekannt. So wirken in einem Zweistrahl-Interferometer vorzugsweise die Bildfeld-Diskriminatoren bei entsprechender lateraler Strukturierung derselben hinsichtlich des Musters der Beleuchtung und bei entsprechender Tiefenlage des Schärfebereiches des abgebildeten Musters der Beleuchtung im Raum des Messobjekts als konfokale Diskriminatoren. Dies erfolgt unter Berücksichtigung des Abbildungsmaßstabes der dem Zweistrahl-Interferometer vorgeordneten Optik. Weiterhin sind bevorzugt Mittel zur Verschiebung des Messobjekts und/oder des Messkopfes angeordnet, sodass das Messobjekt durch eine relative Bewegung zum Messkopf in der Tiefe ortsaufgelöst durchmustert und dabei spektral analysiert werden kann.

Weiterhin ist vorzugsweise die Lichtquelle hierbei bevorzugt als eine rechnersteuerbare, gerasterte Lichtquelle ausgebildet. Diese weist also rechnersteuerbare leuchtende Pixel auf. Dabei ist diese Lichtquelle vorzugsweise durch ein OLED dargestellt. Dies ermöglicht eine sehr hohe Flexibilität bei der Ausleuchtung des Durchlassbereiches eines Bildfeld-Diskriminators. Damit können beispielsweise auch Fehllagen der Bildfeld-Diskriminatoren ausgeglichen werden. Dabei kann die rechnersteuerbare, gerasterte Lichtquelle vorzugsweise auch im Zusammenwirken mit einem räumlichen Lichtmodulator ausgebildet, vorzugsweise in Form eines rechnersteuerbaren Mikrospiegel-Arrays und/oder eines rechnersteuerbaren Flüssigkristall-Displays. Es kann das Messobjekt vorzugsweise mit einem feinen Muster von Lichtflecken beleuchtet werden. Bei entsprechender lateraler Strukturierung der Bildfeld-Diskriminatoren als räumliche Lichtmodulatoren hinsichtlich des Musters der Beleuchtung mittels der rechnersteuerbaren, gerasterten Lichtquelle und bei entsprechender Tiefenlage des Schärfebereiches des abgebildeten Musters der Beleuchtung im Raum des Messobjekts wirken diese im Zweistrahl-Interferometer als konfokale Diskriminatoren. Durch eine relative Bewegung des Messobjekts zum Messkopf in der Tiefe kann hier der konfokale Ansatz zur Anwendung kommen.

Weiterhin ist vorzugsweise dem Zweistrahl-Interferometer für die Fourier-Transformations-Spektroskopie eine zur Strahlteilerfläche eines Plattenstrahlteilers, der mit mindestens einer planparallelen Platte ausgebildet ist, geneigte, zumindest näherungsweise planparallele Kompensationsplatte vorgeordnet. In der Regel ist der Plattenstrahlteiler mit zwei planparallelen Platten aufgebaut. Die zum Plattenstrahlteiler geneigte Kompensationsplatte dient der Vermeidung von Abbildungsfehlern wie Astigmatismus und Koma bei der Bildentstehung im Zweistrahl-Interferometer. Dabei ist die der Orientierung der Kompensationsplatte so, dass ein im Betrag gleicher, jedoch entgegengesetzter Winkel besteht. Die Plattendicke dieser dem Zweistrahl-Interferometer vorgeordneten Kompensationsplatte ist der zumindest näherungsweise planparallelen Strahlteilerplatte gleich oder gleich der Summe der Dicken der beiden Platten bei Verwendung eines Plattenstrahlteilers mit Kompensationsplatte. Es ist für die vorgeordnete Kompensationsplatte auch der gleiche Substratwerkstoff wie bei der Strahlteilerplatte und/oder dem Strahlteilerplatten-Paar einzusetzen. So verbleibt aufgrund der sich ergebenden effektiven Plattendicke im Strahlengang nur ein Öffnungsfehler der in das vorgeordnete Objektiv einkorrigiert werden kann.

Weiterhin ist vorzugsweise dem Zweistrahl-Interferometer für die Fourier-Transformations-Spektroskopie eine zur Strahlteilerfläche eines Plattenstrahlteilers, der mit mindestens einer planparallelen Platte ausgebildet ist, geneigte, zumindest näherungsweise planparallele Kompensationsplatte nachgeordnet. In der Regel ist der Plattenstrahlteiler jedoch mit zwei planparallelen Platten aufgebaut. Die zum Plattenstrahlteiler geneigte Kompensationsplatte dient der Vermeidung von Wellenfrontdeformationen bei der Detektion der interferierenden Wellenfronten. Analog gilt auch hier: Die Plattendicke der dem Zweistrahl-Interferometer nachgeordneten Kompensationsplatte ist der Strahlteilerplatte gleich oder gleich der Summe der Dicken der beiden Platten bei Verwendung eines Plattenstrahlteilers mit Kompensationsplatte. Es ist für die nachgeordnete Kompensationsplatte auch der gleiche Substratwerkstoff wie bei der Strahlteilerplatte und/oder dem Strahlteilerplatten-Paar einzusetzen. So verbleibt aufgrund der sich ergebenden effektiven Plattendicke im Strahlengang nur ein Öffnungsfehler der in das nachgeordnete Objektiv einkorrigiert werden kann. Das Zweistrahl-Interferometer kann dabei als ein Michelsontyp-Interferometer, als ein Mach-Zehnder-Interferometer oder auch als ein zyklisches Interferometer mit zwei Planspiegeln und/oder planen Spiegelflächen im umlaufenden Strahlengang ausgebildet sein. Beim Mach-Zehnder-Interferometer sind die planparallelen Platten eines Plattenstrahlteilers bevorzugt nebeneinander in zueinander parallelen Ebenen angeordnet, wobei es im Mach-Zehnder-Interferometer also zwei Teilerschichten gibt, eine zur Strahlteilung und eine zur Strahlwiedervereinigung.

Weiterhin ist vorzugsweise in einem Zweistrahl-Interferometer für die Fourier-Transformations-Spektroskopie in jedem Interferometerarm je eine zur Strahlteilerfläche eines Plattenstrahlteilers, der mit mindestens einer planparallelen Platte ausgebildet ist, geneigte, zumindest näherungsweise planparallele Kompensationsplatte angeordnet. Wenn funktionell eine Plattenstrahlteiler zur Vereinigung von Teilstrahlenbündeln genutzt wird, wie beispielsweise in einem Mach-Zehnder-Interferometer wird hier dennoch von einem Plattenstrahlteiler gesprochen.

Weiterhin sind vorzugsweise in einem Zweistrahl-Interferometer für die Fourier-Transformations-Spektroskopie die beiden geneigten, zumindest näherungsweise planparallelen Kompensationsplatten in zueinander parallelen Ebenen angeordnet. Dies ermöglicht, dass Dicke aller zumindest näherungsweise planparallelen Kompensationsplatten gleich ist. Dabei ist es auch möglich, dass in einem Mach-Zehnder-Interferometer die beiden planparallelen Kompensationsplatten durch eine einzige Kompensationsplatte entsprechender Länge mit zwei Bereichen des Strahlendurchgangs ersetzt sind. Dies kann die Justierung des Mach-Zehnder-Interferometers vereinfachen.

Weiterhin sind in einem Zweistrahl-Interferometer für die Fourier-Transformations-Spektroskopie vorzugsweise die optischen Weglängen in refraktivem, transparenten Material gleicher Art von der Strahlteilung bis zu den Bildfeld-Diskriminatoren BFD1 und BFD2 in den beiden Armen des Zweistrahl-Interferometers für die Hauptstrahlen HB der Teilstrahlenbündel TB1 und TB2 zumindest näherungsweise gleichgemacht. In der Regel handelt es sich um ein festes, zumindest teiltransparentes optisches Material im Zweistrahl-Interferometer, aber auch ein flüssiges und zumindest teiltransparentes optischen Material kann eingesetzt werden.

Weiterhin genügen in einem zyklischen Zweistrahl-Interferometer für die Fourier-Transformations-Spektroskopie vorzugsweise die optischen Weglängen (OPLB_1, OPLB_2) in refraktivem, transparenten Material gleicher Art von der Strahlteilung bis zu den Bildfeld-Diskriminatoren (BFD1, BFD2) in den beiden Armen des Zweistrahl-Interferometers für die Hauptstrahlen der Teilstrahlenbündel (TB1, TB2) zumindest näherungsweise einer der Gleichungen:

$$AB + BC = AT + TE + EG, \qquad \text{Gleichung (6)}$$

$$TB + BC = TE + EG, \qquad \text{Gleichung (7)}$$

$$HB + BC = JE + EG. \qquad \text{Gleichung (8)}$$

Dabei stellen die Strecken AB, BC, AT, TE, TB, BC, EG sowie HB und JE jeweils optische Weglängen in refraktivem, transparentem Material gleicher Art dar. Bei Spiegelprismen mit gleichen Winkeln sind die Höhen h1 und h2 gleichgemacht. So kann gesichert werden, dass achssenkrecht zu den Hauptstrahlen der beiden Teilstrahlenbündel in einem zyklischen Zweistrahl-Interferometer zumindest näherungsweise auf dem halben optischen Weg im Umlauf eine Doppelspaltblende und/oder ein Flüssigkristall-Display eingesetzt werden kann. Die Aberrationen im Strahlengang stellen hier einen chromatischen Öffnungsfehler dar, der durch den Einsatz von Winkelprismen mit achssenkrechtem Strahl-Eintritt und Strahl-Austritt mit der Wirkung einer dicken planparallelen Platte entsteht. Diese Aberrationen sind dann in den beiden Strahlengängen mit entgegengerichtetem Umlauf gleich und können somit mittels vor- und nachgeordneten Objektiven korrigiert werden, die auch chromatische Öffnungsfehler kompensieren. Die Kompensation der chromatischen Öffnungsfehler ermöglicht die Interferenz von Wellen mit nahezu idealer Zylinderwellenform, was die sehr unerwünschten Nichtlinearitäten in den räumlichen Interferogrammen auf dem gerasterten Detektor minimiert.

Weiterhin ist in einem Michelsontyp-Interferometer und/oder in einem Mach-Zehnder-Interferometer durch refraktive optische Mittel vorzugsweise die Differenz der optischen Bildverschiebungen in der Tiefe v1 und v2 in den beiden Armen IA1 und IA2 des jeweiligen Interferometers kleiner als die wellenoptische Schärfentiefe für die kürzeste Wellenlänge gemacht, welche sich aus den Quotienten der kürzesten Wellenlänge und dem Quadrat der numerischen Apertur errechnet, die sich aus dem Aperturwinkel alpha_yz des dem Michelsontyp-Interferometer und/oder dem Mach-Zehnder-Interferometer nachgeordneten, anamorphotischen Objektivs ergibt. Dabei beträgt die optische Weglängendifferenz OPD_zykaP zwischen den beiden Teilstrahlenbündeln TB1 und TB2 am Ausgang des Interferometers vorzugsweise bis zur Größenordnung von einem Millimeter, wenn sehr hochpixlige gerasterte Detektoren im VIS-Spektralbereich in der Größenordnung von 100 Millionen Pixeln eingesetzt werden, wie in der astronomischen Forschung bereits üblich.

Dies dient dazu, auf dem gerasterten Detektor ein zweiseitiges räumliches Interferogramm mit einem kurzen, in der üblichen Darstellung linken, Teil zu erzeugen, um auf der rechten Seite des räumlichen Interferogramms mit einer möglichst großen optischen Weglängendifferenz zu erfassen. Der Ort der optischen Weglängendifferenz null liegt also in der Nähe des Randes des gerasterten Detektors. Diese Einseitigkeit verbessert die spektrale Auflösung, wobei ein kurzes linkes Stück des räumlichen Interferogramms für die Phasenkorrektur üblicherweise notwendig ist.

Weiterhin ist in einem zyklischen Zweistrahl-Interferometer durch eine Planplattengruppe im Bereich Z, wo sich die beiden Teilstrahlenbündel geometrisch nicht überdecken, vorzugsweise die Differenz der optischen Bildverschiebungen v1 und v2 in der Tiefe in den beiden Umlaufrichtungen am Ausgang des Interferometers kleiner als die wellenoptische Schärfentiefe für die kürzeste Wellenlänge gemacht, welche sich aus den Quotienten der kürzesten Wellenlänge und dem Quadrat der numerischen Apertur, die sich aus dem Aperturwinkel alpha des dem zyklischen Zweistrahl-Interferometer nachgeordneten, anamorphotischen Objektivs ergibt, und die optische Weglängendifferenz OPD_zykaP dabei in den beiden Teilstrahlenbündeln TB1 und TB2 bis zu einem Millimeter beträgt. Dabei handelt es sich in der Regel um den Aperturwinkel alpha in Luft. Die Einführung einer optischen Weglängendifferenz OPD_zykaP dient dazu, auf dem gerasterten Detektor ein zweiseitiges räumliches Interferogramm mit einem kurzen, in der üblichen Darstellung linken, Stück zu erzeugen, um auf der rechten Seite das Interferogramm mit einer möglichst großen optischen Weglängendifferenz zu erfassen. Der Ort der optischen Weglängendifferenz null liegt also in der Nähe des Randes des gerasterten Detektors.

Weiterhin sind in einem zyklischen Zweistrahl-Interferometer mit einem Folienstrahlteiler und/oder einem Drahtgitter-Strahlteiler für den mittleren und/oder ferneren Infrarotbereich für die Fourier-Transformations-Spektroskopie im umlaufenden Strahlengang vorzugsweise zwei baugleiche Spiegelprismen eingesetzt. Diese sind beide aus dem gleichen refraktivem Material ausgebildet, welches für diesen Spektralbereich zumindest teilweise transparent ist, beispielsweise CaF2, KBr und/oder ZnSe. Diese Spiegelprismen weisen jeweils zweimal den spitzen Halbwinkel psi auf, jeweils zwischen der Spiegelfläche und einer unverspiegelten Fläche, welche an einem Winkelprisma jeweils die Eintritts- und die Austrittsfläche darstellen. Somit ergeben sich für den Umlauf im zyklischen Zweistrahl-Interferometer in jeder Richtung die gleichen optischen Weglängen bis zu einem Bildfeld-Diskriminator. Dieser befindet sich bei der halben optischen Weglänge im Umlauf des Lichts im zyklischen Zweistrahl-Interferometer. So kann an jedem der beiden Bildfeld-Diskriminator ein scharfes Bild des Messobjekts entstehen und ein Teilbereich des Bildes diskriminiert werden.

Weiterhin ist in einem zyklischen Zweistrahl-Interferometer im Bereich Z, wo es keine räumliche Überdeckung der beiden Teilstrahlenbündel TB1 und TB2 gibt, bevorzugt sowohl im Teilstrahlenbündel TB 1 eine Planparallelplatte der Dicke h1 und aus optischem Material mit dem Brechungsindex n1 als auch im Teilstrahlenbündel TB2 eine Planparallelplatte der Dicke h2 und aus optischem Material mit dem Brechungsindex n2 angeordnet. Dabei werden die Dicken h1 und h2 nach der bereits genannten Gleichung (2) bestimmt. Dadurch ist so die jeweilige Bildverschiebung in der Tiefe in den beiden Teilstrahlenbündeln TB1 und TB2 am Ausgang des zyklischen Zweistrahl-Interferometers gleich und die optische Weglängendifferenz OPD_zykaP am Ausgang des zyklischen Zweistrahl-Interferometers ergibt sich mit der bereits genannten Gleichung (3). Die optische Weglängendifferenz OPD zykaP soll so gewählt sein, dass dabei die Ungleichung

$0{,}1*OPDr < OPD\_zykaP < 0{,}9\ OPDr$ eingehalten ist.

Die optische Weglängendifferenz OPDr ergibt sich aus der Lateral-Shear s, der genutzten Länge bk des gerasterten Detektors und der numerischen Apertur des rotationssymmetrischen Objektivs im nachgeordneten anamorphotischen Objektiv.

Die optische Weglängendifferenz OPDr ist hier die maximal erreichbare optische Weglängendifferenz im räumlichen Interferogramm am Rand des gerasterten Detektors bei einer symmetrischen Lage des räumlichen Interferogramms auf dem gerasterten Detektor.

So ist am Ausgang des zyklischen Zweistrahl-Interferometers die Bildverschiebung v1 in der Tiefe für das Teilstrahlenbündel TB1 gleich der Bildverschiebung v2 für das Teilstrahlenbündel TB2 und somit besteht eine Kompensation der Bildverschiebungen in der Tiefe. Dies ist die Voraussetzung für das Entstehen von Zylinderwellen mit geradliniger Scheitellinie auf dem gerasterten Detektor und somit räumlicher Interferogramme rI ohne Nichtlinearitäten und die optische Weglängendifferenz OPD_zykaP am Ausgang des zyklischen Zweistrahl-Interferometers ergibt sich mit der bereits genannten Gleichung (3).

Weiterhin ist vorzugsweise in einem Zweistrahl-Interferometer für die Fourier-Transformations-Spektroskopie für den fern-infraroten Bereich und/oder den Terahertz-Bereich der Strahlteiler als ein feines, ebenflächiges Drahtgitter in Parallelanordnung der Drähte ausgebildet. Diese Anordnung stellt einen polarisierenden Strahlteiler dar. Das Zweistrahl-Interferometer kann aber auch vorzugsweise als ein Michelsontyp-Interferometer und/oder als ein zyklisches Zweistrahl-Interferometer dargestellt sein. Wird ein Mach-Zehnder-Interferometer eingesetzt, werden bevorzugt zwei feine Drahtgitter als Strahlteiler eingesetzt, eines bei der Strahlteilung und eines bei der Strahlwiedervereinigung.

Beschreibung der Figuren

Im Folgenden werden einige Ausführungsbeispiele näher beschrieben, wobei die Erfindung nicht auf die beschriebenen Ausführungsbeispiele beschränkt sein soll. Einzelne Merkmale, die in einer bestimmten Ausführungsform beschrieben werden, können beliebig kombiniert werden, vorausgesetzt, sie schließen einander nicht aus. Darüber hinaus sind verschiedene Merkmale, die in den beispielhaften Ausführungsformen zusammen bereitgestellt werden, sind nicht als die Erfindung einschränkend anzusehen.

Es folgt eine Beschreibung der Zeichnungen, die beispielhafte Ausführungsformen zeigen:

Fig. 1 ist eine schematische seitliche Darstellung einer beispielhaften Anwendung eines beispielhaften FT-Spektrometers an einem Patienten;

Fig. 2 ist eine schematische Rückdarstellung einer beispielhaften Anwendung eines beispielhaften FT-Spektrometers an einem Patienten;

Fig. 3 ist eine schematische Darstellung eines beispielhaften FT-Spektrometers, insbesondere eines Single-Shot-Linien-Spektrometers;

Fig. 4 ist eine schematische Darstellung eines beispielhaften Michelsontyp-Interferometers;

Fig. 5 ist eine schematische Darstellung eines beispielhaften Dachkantreflektors;

Fig. 6 ist eine schematische Darstellung eines beispielhaften Dachkantreflektors;

Fig. 7 ist eine schematische Darstellung eines beispielhaften Dachkantreflektors;

Fig. 8 ist eine schematische Darstellung eines beispielhaften Dachkantreflektors;

Fig. 9 ist eine schematische Darstellung von Abbildungseigenschaften eines dem Michelsontyp-Interferometer nachgeordneten, anamorphotischen und weitgehend achromatischen beispielhaften Objektivs zur Detektion räumlicher Interferogramme;

Fig. 10 ist eine weitere schematische Darstellung von Abbildungseigenschaften eines dem Michelsontyp-Interferometer nachgeordneten, anamorphotischen und weitgehend achromatischen beispielhaften Objektivs zur Detektion räumlicher Interferogramme;

Fig. 11 ist eine schematische Darstellung eines Ausschnitts eines beispielhaften Michelsontyp-Interferometers;

Fig. 12 ist eine schematische Darstellung eines beispielhaften Mach-Zehnder-Interferometers;

Fig. 13 ist eine schematische Darstellung eines beispielhaften zyklischen Zweistrahl-Interferometers;

Fig. 14 ist eine schematische Darstellung eines beispielhaften zyklischen Zweistrahl-Interferometers;

Fig. 15 ist eine schematische Darstellung eines beispielhaften zyklischen Zweistrahl-Interferometers;

Fig. 16 ist eine schematische Darstellung eines beispielhaften zyklischen Zweistrahl-Interferometers;

Fig. 17 ist eine schematische Darstellung eines beispielhaften zyklischen Zweistrahl-Interferometers;

Fig. 18a ist eine schematische Darstellung der optischen Wirkung einer beispielhaften Planplattengruppe;

Fig. 18b ist eine schematische Darstellung einer Bildlagendifferenz in der Tiefe delta_v;

Fig. 19a ist eine schematische Darstellung einer beispielhaften Planplattengruppe;

Fig. 19b ist eine schematische Darstellung einer Bildlagendifferenz in der Tiefe delta_v=0;

Fig. 20 ist eine schematische Darstellung eines beispielhaften zyklischen Zweistrahl-Interferometers;

Fig. 21 ist eine schematische Darstellung eines beispielhaften zyklischen Zweistrahl-Interferometers;

Fig. 22 ist eine schematische Darstellung eines beispielhaften zyklischen Zweistrahl-Interferometers;

Fig. 23 ist eine schematische Darstellung eines beispielhaften zyklischen Zweistrahl-Interferometers;

Fig. 24 ist eine schematische Darstellung eines beispielhaften zyklischen Zweistrahl-Interferometers;

Fig. 25 ist eine schematische Darstellung eines beispielhaften zyklischen Zweistrahl-Interferometers;

Fig. 26 ist eine schematische Darstellung eines beispielhaften zyklischen Zweistrahl-Interferometers;

Fig. 27 ist eine schematische Darstellung eines beispielhaften zyklischen Zweistrahl-Interferometers;

Fig. 28a ist eine schematische Darstellung einer beispielhaften Planplattengruppe;

Fig. 28b ist eine schematische Darstellung einer Bildlagendifferenz in der Tiefe delta_v=0;

Fig. 29 ist eine schematische Darstellung einer beispielhaften Planplattengruppe;

Fig. 30 ist eine schematische Darstellung eines beispielhaften zyklischen Interferometers;

Fig. 31 ist eine schematische Darstellung eines beispielhaften zyklischen Interferometers;

Fig. 32 ist eine schematische Darstellung einer beispielhaften Doppelspiegeltreppe;

Fig. 33 ist eine schematische Darstellung eines beispielhaften zyklischen Zweistrahl-Interferometers;

Fig. 34 ist eine schematische Darstellung einer beispielhaften Doppelspiegeltreppe;

Fig. 35 ist eine schematische Darstellung einer beispielhaften Anordnung mit zwei separierten Spiegeltreppen;

Fig. 36 ist eine schematische Darstellung eines zyklischen Zweistrahl-Interferometers; und

Fig. 37 ist eine schematische Darstellung eines beispielhaften Spektrometersystems im Einsatz.

Es erfolgt eine Beschreibung mittels der Fig. 1 bis Fig. 37 sowie von elf Ausführungsbeispielen jeweils ohne Figur (nicht schematisch anhand einer Zeichnung dargestellt).

Der Begriff Licht wird in der folgenden Beschreibung im Wesentlichen als Synonym für elektromagnetische Strahlung insbesondere vom UV- bis zum Terahertz-Bereich, einschließlich von Infrarotstrahlung insbesondere von Wärmestrahlung, verwendet. Mit dem Begriff "Spektrometersystem" ist vorliegend insbesondere ein Single-Shot-Fourier-Transformations-Spektrometer mit einem Zweistrahl-Interferometer gemeint, welches dazu ausgelegt ist, eine Lateral-Shear s zu erzeugen. Dabei kann entweder eine aktive Beleuchtung des Messobjekts erfolgen, welche vorzugsweise auch eine strukturierte Beleuchtung darstellen kann und/oder das Messobjekt kann selbstleuchtend sein, insbesondere kann externes Licht von der Oberfläche des Messobjekts gestreut werden. Der Begriff "Single-Shot" bezieht sich im Wesentlichen auf die Gewinnung bzw. die Erzeugung bzw. Erfassung von räumlichen Interferogrammen mittels der Zweistrahl-Interferometrie durch eine einzige Aufnahme eines gerasterten Detektors, in der Regel eine Bildaufnahme in einer vergleichsweise kurzen Zeit. Dies ist in der Regel die Integrationszeit einer Kamera im UV-, im VIS-, im NIR- oder im MIR-Bereich. Diese Integrationszeit kann je nach Kameratyp und je nach Aufnahmebedingungen einen Bereich vom einstelligen Mikrosekundenbereich bis in den einstelligen Sekundenbereich umfassen.

Fig. 1 stellt eine Anwendung eines beispielhaften FT-Spektrometers dar. Fig. 1 stellt schematisch insbesondere eine Anordnung eines Patienten mit Schutzbrille dar, wobei ein aus medizinischer Sicht auffälligen Hautmerkmal 2 auf dem Rücken 1 des Patienten untersucht werden soll. Insbesondere soll mittels Diagnostik die Frage geklärt werden, ob es sich bei dem Hautmerkmal 2 um ein Muttermal oder ein Melanom handelt. Die komplizierte Diagnostizierung soll bevorzugt mittels einer spektralen Messung und insbesondere mittels eines Spektrometersystems 20 möglichst objektiv abgesichert werden.

Zur Untersuchung kann den Dermatologen zum Haut-Screening ein im Wesentlichen mobiler Messkopf 30 im Single-Shot-Betrieb dienen, welcher als Hand-held-Gerät für einen Scan über ein Messobjekt, hier der Bereich eines Rückens 1, ausgeführt ist. Diesem Gerät kann eine gepulste NIR-Lichtquelle 40 zur aktiven Beleuchtung in Streifenform und/oder zur feinen strukturierten Beleuchtung des Rückens 1 und eine Optik-Einheit 50 mit einem integrierten Zweistrahl-Interferometer 601 beigeordnet sein.

Dieser im Wesentlichen mobile Messkopf 30 wird langsam, z.B. mit einer Geschwindigkeit von etwa v=lcm/s von Hand über den Rücken 1 des Patienten, hier von unten nach oben also im Wesentlichen in +y-Richtung, geführt.

Die Integration einer Kamera, insbesondere einer VIS-Monitorkamera in den mobilen Messkopf 30 für die Hautoberfläche unter Diagnose auf dem Rücken 1 ist hier gegeben, jedoch nicht dargestellt. Die mittels dieser Monitorkamera aufgenommenen Bilddaten dienen ggf. zusätzlich der Unterstützung beim Aufbau eines hyperspektralen Bildes, insbesondere, wenn die Scan-Bewegung des mobilen Messkopfes 30 aus der Hand nicht mit einer im Wesentlichen konstanten Geschwindigkeit erfolgt. Dazu sind auf dem Rücken 1 hier zwei nicht dargestellte Positionsmarken aufgebracht. Die im Scan bei einer moderaten Bewegung des mobilen Messkopfes 30 nach und nach anfallenden räumlichen Interferogramme rI, die in der Regel im Single-Shot-Modus mittels einer InGaAs-Kamera 54 für den nahinfraroten Spektralbereich hier entlang einer Linie in waagerechter Richtung gewonnen werden können, können in Spektren SP umgerechnet und zu einem hyperspektralen Bilddatensatz zusammengesetzt werden. Die Kamera, die insbesondere eine InGaAs-Kamera 54 sein kann, kann bevorzugt in diesem Ausführungsbeispiel wie auch die gepulste Lichtquelle, die insbesondere eine NIR-Lichtquelle 40 darstellt oder aufweist, durch den Rechner 21 im Wesentlichen steuerbar ausgebildet sein. Es kann auch bevorzugt eine Synchronisation dieser beiden Komponenten erfolgen.

Die mittels eines Zweistrahl-Interferometers 601 in der Regel in einem Schuss aufgenommenen räumlichen Interferogramme rI können also mittels eines Rechenprogrammes zur Ausführung der schnellen Fourier-Transformation (FFT) zu Spektren entlang der Linie in waagerechter Richtung verrechnet werden. Dies kann zeilenweise für den ausgewählten Hautbereich auf dem Rücken 1 für die räumlichen Interferogramme rI erfolgen, die von der Optik-Einheit 50 des mobilen Messkopfes 30 erfasst werden. Nach Beendigung der Aufnahmen von räumlichen Interferogrammen rI in einer Aufwärtsbewegung, die das Hautgebiet von medizinischem Interesse abdeckt, können die errechneten Spektren punktweise einer Bildkarte des untersuchten Hautgebiets zugeordnet werden. Dabei wird es als zulässig angesehen, wenn das Bildpunktraster aufgrund der möglicherweise nicht perfekt bzw. nicht optimal gleichmäßigen und im Wesentlichen nicht perfekt lateral geführten Handbewegung des Dermatologen bei Benutzung des mobilen Messkopfes 30 gewisse Dehnungen und Stauchungen aufweist, jedoch im Wesentlichen ohne Lücken ist. Mittels eines Auswerteprogrammes 22 zur Bewertung der Spektren Sp, ggf. ausgeführt auf einem leistungsfähigen Rechner 21 und/oder einem Rechnersystem, können aufgrund der spektralen Signaturen der Spektren SP insbesondere Risiko- und Hochrisikobereiche ermittelt werden. Dabei können Algorithmen zur Spektrenbewertung, beispielsweise nach dem Principal Component Analysis-Ansatz, zur Anwendung und/oder Ansätze mit künstlicher Intelligenz in Betracht kommen. Die Algorithmik zur Bewertung und Analyse der Spektren Sp hinsichtlich der Abschätzung eines Tumor-Risikos steht im Rahmen dieser Erfindung nicht im Fokus, da es hier insbesondere um die schnelle Bereitstellung von optischen Primärdaten, also räumlichen Interferogrammen rI, geht. Das Ergebnis der Bewertung und Analyse der Spektren Sp kann auf einem Monitor 23 dargestellt werden.

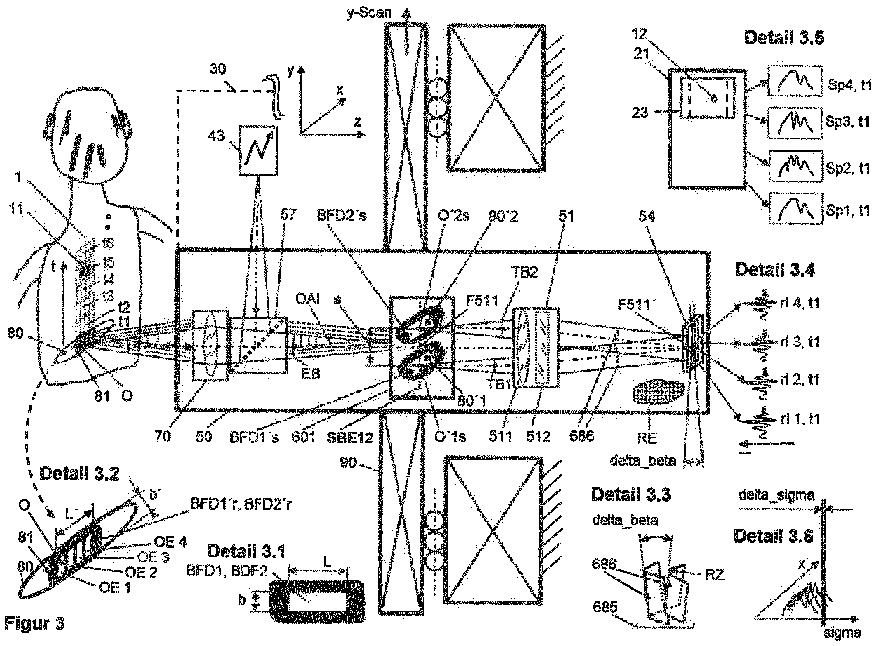

Anders als in Fig. 1 dargestellt, kann in einem weiteren Ausführungsbeispiel 1 (nicht schematisch anhand einer Zeichnung dargestellt) die Beleuchtung koaxial durch die Einkopplung des Lichts mittels eines Strahlteilers erfolgen. Insbesondere in diesem Fall ist eine stete Überdeckung eines projizierten Lichtstreifens und des Messfeldes in beliebiger Positionierung desselben gegeben. In dem Fall kann auch problemlos eine Zoom-Funktion in die Optik-Einheit 50 des mobilen Messkopfes 30 integriert werden. Dieser Ansatz mit koaxialer Einkopplung des Lichts zur Beleuchtung des Rückens 1 kommt beispielsweise in der Fig. 3 zum Einsatz.

Die Fig. 2 stellt schematisch die Rückenansicht des Patienten dar. Der beispielhafte projizierte Lichtstreifen 80, welcher mittels einer NIR-Streifenlichtquelle auf dem Rücken 1 des Patienten erzeugt wird, ist im Wesentlichen deutlich länger als das Messfeld 81 der Optik-Einheit 50 des mobilen Messkopfes 30 ausgebildet. Somit kann auch bei moderaten Abstandsänderungen desselben eine vollständige Ausleuchtung des Messfeldes gegeben sein. Beim Messen kann die Bewegung des mobilen Messkopfes 30 insbesondere aus der Hand und lateral über einen Bereich des Rückens erfolgen. Dabei werden zumindest zeitweise mittels des Interferometers 601 in der Optik-Einheit 50 ständig, also ohne wesentliche Unterbrechung und/oder in zeitlichen Abständen mehrmals räumliche Interferogramme rl im Single-Shot-Modus aufgenommen, wobei von der Lichtquelle 40 synchronisiert geblitztes Licht zwecks Beleuchtung des Messfeldes 81 in Form eines Streifens 80 erzeugt wird.

Das Spektrometersystem kann alternativ oder zusätzlich auch zur Untersuchung eines Gewebegebietes bei einer chirurgischen Operation zwecks Gewebedifferenzierung eingesetzt werden.

Die Fig. 3 stellt das Prinzip des Ansatzes für ein Single-Shot-Linien-Spektrometer mit einer Beleuchtung des Rückens 1 mit einem Streifen 80 dar. Mittels eines Transportschlittens 90 mit einem hier nicht dargestellten Schrittmotorantrieb kann hier ein y-Scan erfolgen. Dieser y-Scan erfolgt insbesondere mittels des mobilen Messkopfes 30 im Wesentlichen quer zum Streifen 80 über den Rücken 1 eines bevorzugt ruhig sitzenden Patienten. Der Rücken 1 kann dabei mittels einer gepulsten Streifenlichtquelle 43 in Form eines Streifens 80 intensiv beleuchtet werden. Zur koaxialen Einkopplung des Lichts in die Optik-Einheit 50 des mobilen Messkopfes 30 dient insbesondere ein Einkoppelstrahlteilerwürfel 57. Derart wird das Messfeld 81 insbesondere mittels eines Streifens 80 ausgeleuchtet. Der so beleuchtete Bereich auf dem Rücken 1 wird insbesondere mittels eines vorgeordneten Objektivs 70 im Wesentlichen scharf in das Zweistrahl-Interferometer 601 durch viele einzelne Strahlenbündel abgebildet. Jedem Objektpunkt wird näherungsweise ein Strahlenbündel zugeordnet. Repräsentativ ist ein Strahlenbündel mit durchgezogenen Linien dargestellt, welches zu einem Objektpunkt O auf der optischen Achse gehört. In gepunkteten Linien sind in Fig. 3, repräsentativ für mehrere Strahlenbündel, drei weitere Strahlenbündel eingezeichnet.

Hierbei besteht für die Objektabbildung zumindest näherungsweise Telezentrie auf der dem Zweistrahl-Interferometer 601 zugewandten Seite des vorgeordneten Objektivs 70 mit der optischen Achse OAI, jedoch hier ohne Darstellung einer Telezentrieblende. Weiterhin umfasst das vorgeordnete bzw. davor angeordnete Objektiv 70 eine Autofokus-Funktion. Die mittels dieser ermittelten Objektentfernung wird bei der Aufnahme insbesondere zumindest zeitweise ständig bzw. permanent und/oder in zeitlichen Abständen dem Auswerteprogramm als Information übergeben, da eine Abstandsänderung den Abbildungsmaßstab bei der Abbildung verändert, was bei der Auswertung und Darstellung des hyperspektralen Bildes berücksichtigt werden kann.

Das Zweistrahl-Interferometer 601 erzeugt eine Lateral-Shear s und weist in jedem der beiden Interferometerarme einen jeweils im Wesentlichen baugleichen Bildfeld-Diskriminator BFD1 und BFD2 auf, der in der Detailabbildung 3.1 der Fig. 3 als Bildfeld-Diskriminator BFD1 mit der Breite b und der Länge L dargestellt ist. In Fig. 3 sind in symbolischer bzw. schematischer Darstellung - und perspektivisch zur Sichtbarmachung herausgedreht - die scheinbaren Bilder BFD1's und BFD2's der Bildfeld-Diskriminatoren BFD1 und BFD, siehe Detailabbildung 3.1, im ebenfalls symbolisch bzw. schematisch dargestellten Zweistrahl-Interferometer 601 in der scheinbaren Bildebene SBE12 eingezeichnet.

Mindestens ein Punkt jeweils eines dieser scheinbaren Bilder BFD1's und BFD2's der Bildfeld-Diskriminatoren ist mit jeweils einem scheinbaren Bildpunkt O'1s und O'2s des Objektpunktes O optisch konjugiert, wobei die Bildpunkte O'1s und O'2s durch Strahlteilung im Zweistrahl-Interferometer 601 optisch konjugiert sind und im Fall der präzisen Justierung desselben im Wesentlichen gemeinsam in der scheinbaren Bildebene SBE12 liegen und auch kohärente Bildpunkte darstellen. Die scheinbare Bildebene SBE12 stellt hier für das nachgeordnete anamorphotische Objektiv 51 wiederum die Objektebene dar.

Bevorzugt entstehen deshalb am Ausgang des Zweistrahl-Interferometers 601 paarweise im Wesentlichen kohärente Teilstrahlenbündel, die beispielhaft hier in den Figuren durch zwei Teilstrahlenbündel TB1 und TB2 dargestellt sind, welche jedoch in der Regel repräsentativ für viele Teilstrahlenbündel sind.

Die beiden Bildfeld-Diskriminatoren BFD1 und BFD2 sind im Zweistrahl-Interferometer 601 bevorzugt so angeordnet, dass eine optische Konjugation für diese besteht. Die Bildfeld-Diskriminatoren BFD1 und BFD2 sind dabei im Wesentlichen lateral so ausgedehnt, dass trotz Diskriminierung immer noch mehrere Strahlenbündel von mehreren Objektpunkten diese passieren können. Im Verständnis gehört immer ein Punkt zu einem Strahlenbündel. Auf dem Bildfeld-Diskriminator BFD1 entsteht durch Abbildung ein Bild 80'1 und auf dem Bildfeld-Diskriminator BFD2 entsteht durch Abbildung ein Bild 80'2 vom Streifen 80. Von jedem dieser beiden Bilder 80'1 und 80'2 kann in jedem Interferometerarm ein Teil des Lichts ein Feld der Breite b und der Länge L die Diskriminatoren BFD1 und BFD2 passieren. Licht außerhalb dieses Feldes wird bevorzugt von der weiteren Abbildung ausgeschlossen, geht also bevorzugt verloren. Durch die Rückabbildung der beiden Bildfeld-Diskriminatoren BFD1 und BFD2 sind auf dem Rücken 1 über den Abbildungsmaßstab des vorgeordneten Objektivs 70 die Breite des Messfeldes b' sowie deren Länge L' festgelegt, dargestellt in der Detailabbildung 3.2.

Das erfasste Messfeld 81 auf dem Rücken 1 ergibt sich allein mittels einer Rückabbildung der beiden Bildfeld-Diskriminatoren BFD1 und BFD2 durch das vorgeordnete Objektiv 70. In der Detailabbildung 3.2 sind stellvertretend für viele jedoch nur vier angemessene kleinflächige Objektelemente, nämlich OE1 bis OE4, mit zur besseren Sichtbarmachung vergrößerter Höhe dargestellt. Die reale Anzahl der Objektelemente liegt dagegen typischerweise in der Größenordnung von etwa 500 im NIR-Spektralbereich, entsprechend des Einsatzes einer MIR-Kamera mit näherungsweise etwa 500 Pixeln in x-Richtung. Für jedes Objektelement OE wird im Messverfahren genau ein Spektrum Sp ermittelt, insbesondere wenn dieses Objektelement OE für eine Messung hinreichend kooperativ ist, also ein erfassbares räumliches Interferogramm rI liefern kann. Die Breite des Messfeldes b' beträgt auf dem Rücken 1 hier etwa 0,2mm. Außerdem sind in der Detailabbildung 3.2 die beiden zusammenfallenden Bilder BFD1'r und BFD2'r der beiden Bildfeld-Diskriminatoren BFD1 und BFD2 andeutungsweise bzw. schematisch dargestellt, die sich durch eine gedachte bzw. imaginäre Rückabbildung derselben auf den Rücken 1 ergeben.

Die beleuchteten Bildfeld-Diskriminatoren BFD1 und BFD2, welche zwei kohärente Lichtquellen darstellen, werden von einem dem Zweistrahl-Interferometer 601 nachgeordneten anamorphotischen Objektiv 51, welches auch chromatisch korrigiert ist, am Ausgang desselben abgebildet. Durch die Ausbildung des Objektivs 51, welches neben einer rotatorischen Komponente 511 auch mit einer Zylinderkomponente 512 ausgebildet ist, entstehen aus einem Objektpunkt jeweils zwei im Wesentlichen zueinander geneigte Zylinderwellen, die hier als gerasterten Detektor auf eine Kamera, insbesondere eine InGaAs-Kamera 54 treffen, wobei jedes Paar von Zylinderwellen jeweils ein räumliches Interferogramm rI bildet, welches einem Objektelement OE zugeordnet ist. Ein Objektelement OE kann dabei so klein ausgebildet sein, dass es als Punkt aufgefasst werden kann, da es von der nachfolgenden Optik nicht mehr lateral aufgelöst wird, beziehungsweise bei der Abbildung auf den gerasterten Detektor mit seiner Bildausdehnung in x-Richtung in die Größenordnung eines Pixels des gerasterten Detektors, hier als InGaAs-Kamera 54 ausgebildet, kommt.

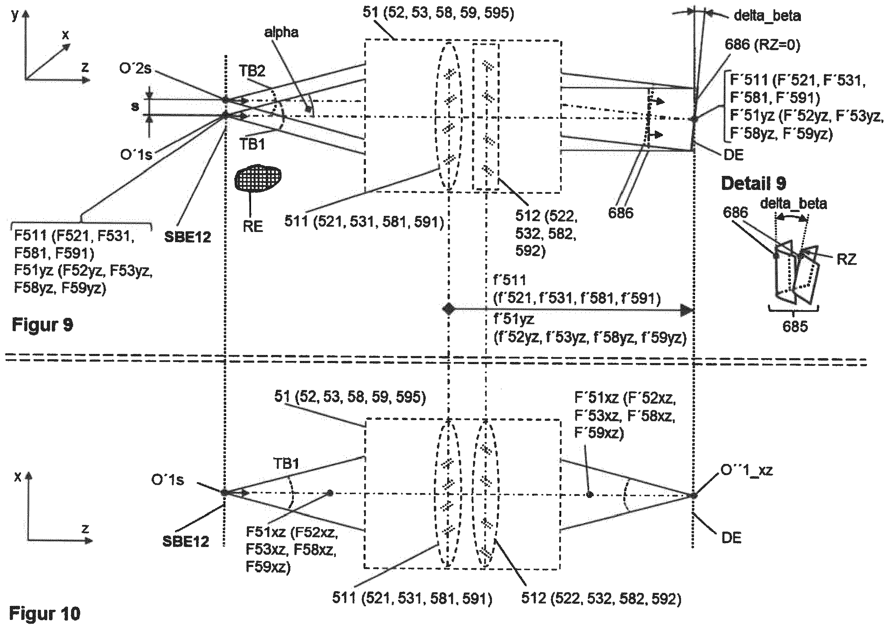

Die zueinander geneigten Zylinderwellenfronten 385 sind in der Detailabbildung 3.3 mit den im Wesentlichen zueinander um delta_beta geneigten Scheitellinien 386 dargestellt. Die spezielle optische Funktion des anamorphotischen Objektivs 51 ist in Fig. 9 und Fig. 10 anschaulich bzw. schematisch sichtbar gemacht. Eine präzise Justierung des Interferometers einschließlich des Abgleichs der optischen Weglängen der Arme des Zweistrahl-Interferometers 601 liegt vor. Dabei ist zu bemerken, dass aufgrund des vergleichsweise geringen Wertes der Breite b von 0,2mm die Anforderungen an die Justierung eines Endspiegels und/oder einer Zweifach-Winkelspiegelgruppe im Zweistrahl-Interferometer 601 vorliegend nicht sehr hoch sind, was einen wesentlichen Vorteil darstellt, beispielsweise bei rauen Umgebungsbedingungen und mit vergleichsweise schnellen Temperaturwechseln. Je geringer der Wert b gewählt ist, desto unkritischer ist in der Regel die Justierung des Interferometers 601. Auch eine Kippung eines Interferometerendspiegels um eine Kippachse im Zweistrahl-Interferometer 601, bei welcher zur Referenzebene RE Parallelität besteht, ist vergleichsweise unkritisch. Diese Unempfindlichkeit einer Verkippung, jedoch innerhalb gewisser Grenzen und stets unter Beachtung des den Fachleuten gut bekannten Abtast-Theorems, ergibt sich insbesondere aus der Ortsauflösung der räumlichen Interferogramme rI mittels gerasterten Empfängers. In der Detailabbildung 3.4 sind einige räumliche Interferogramme rI beispielhaft dargestellt.

Die bei einer dennoch nicht ganz perfekten Justierung des Zweistrahl-Interferometers 601 etwas im Feld variierenden optischen Gangunterschiede in den räumlichen Interferogrammen rI stellen für die numerische Auswertung derselben beim Stand der Technik typischerweise kein wesentliches Problem dar.

Nur wenn der Kontrast der räumlichen Interferogramme rI hinreichend gut bzw. groß ist, kann ein hohes Signal/Rauschverhältnis im Spektrum erreicht werden. Es ist deshalb wichtig, durch die Interferometer-Hardware einen möglichst bzw. relativ hohen Kontrast der räumlichen Interferogramme rI zu sichern bzw. zu erzielen, da die gesuchten spektralen Signaturen, insbesondere in biologischen Messobjekten, oft nicht wesentlich ausgeprägt sind bzw. keine wesentlich hohe Kennzeichnung aufweisen.

In dem Ausführungsbeispiel der Fig. 3 können die Bildfeld-Diskriminatoren BFD1 und BFD2 auch schmale Planspiegel der Breite b und der Länge L darstellen und/oder umfassen. Licht, das außerhalb eines solchen Planspiegels fällt, kann in Lichtfallen und/oder auf eine mattschwarze Maskierung des schmalen Planspiegels mit der Breite b treffen.

Die Detailabbildung 3.5 stellt beispielhafte errechnete Spektren Sp1 bis Sp4 aus einer beispielhaften Bildaufnahme dar, aus denen die räumlichen Interferogramme rI in der Detailabbildung 3.4 gewonnen werden, die gemäß der Detailabbildung 3.5 vorliegend der x-Koordinate entsprechend der Detailabbildung 3.6 zugeordnet bzw. gegen die x-Koordinate aufgetragen werden. Mit delta_sigma ist in der Detailabbildung 3.6 die spektrale Auflösung angedeutet. Mittels eines y-Scans kann der bekannte Datenwürfel bzw. das mehrdimensionale Datenensemble in der Darstellung (x, y, Wellenzahl) und/oder (x, y, Wellenlänge) für einen Bereich des Rückens 1 bestimmt werden bzw. einem Bereich des Rückens 1 zugeordnet werden.

In einem weiteren Ausführungsbeispiel 2 (nicht schematisch anhand einer Zeichnung dargestellt) wird ein Mehrachsen-Roboterarm zur Bewegung des mobilen Messkopfes 30 eingesetzt. Dieser erlaubt, ein besonders hohes Maß an Flexibilität zu erreichen, insbesondere im Vergleich mit einem linear arbeitenden Transportschlitten 90. Bei einer chirurgischen Operation ist ein solches Ausführungsbeispiel 2 aufgrund seiner Flexibilität der Positionierung vor Ort besonders vorteilhaft.

Die Fig. 4 stellt schematisch ein relativ kompaktes Michelsontyp-Interferometer 602 dar, das insbesondere dazu ausgelegt ist, im NIR-Bereich betrieben zu werden. Eine gepulste Lichtquelle 44 dient im Wesentlichen zur Projektion eines Lichtstreifens 80 auf das (biologische) Messobjekt 10 und umfasst dazu eine in die Lichtquelle 44 integrierte Strahlformungsoptik 45 in Spiegelform. Das von der Lichtquelle 44 emittierte Licht wird mittels einer Öffnung in dieser im Wesentlichen koaxial in den Beleuchtungsstrahlengang über eine telezentrische Blende 72 und ein Pentaprisma 46 eingekoppelt und gelangt als fokussiertes Einfallsbündel EB über ein vorgeordnetes Objektiv 71 in das Michelsontyp-Interferometer 602. Dieses Michelsontyp-Interferometer 602 ist vorliegend beispielhaft mit einem Strahlteilerwürfel 622 beispielsweise mit einer Kantenlänge von etwa 10mm und einer Strahlteilerschicht 62 ausgebildet. Der Strahlteilerwürfel 622 umfasst bevorzugt ein Glas und der halbe Öffnungswinkel alpha in dem Glas des Strahlteilerwürfels 622 beträgt beispielsweise bevorzugt etwa 12 Altgrad. Dieser beispielhafte Winkel stellt einen vergleichsweise großen Winkel dar und führt somit insbesondere zur Erfassung eines großen Anteils des vom Messobjekt 10 ausgehenden Lichts. Der Strahlteilerwürfels 622 umfasst beispielsweise Borosilikat-Kronglas (BK7). Alternativ oder zusätzlich kann ein Strahlteiler, insbesondere ein Strahlteilerwürfel andere Materialien umfassen, die für optische Elemente üblich sind, wie etwa Quarzglas, Kronglas, Flintglas und ähnliche Materialien. An der Strahlteilerschicht 62 erfolgt insbesondere die Aufspaltung eines hier beispielhaft für viele dargestellten Eingangsbündels EB in die beiden Arme IA1 und IA2 des Michelsontyp-Interferometers 602. Das hindurchgehende bzw. transmittierte Teilstrahlenbündel TB1 trifft im Arm IA1 des Michelsontyp-Interferometers 602 bevorzugt auf einen Zweispiegel-Winkelreflektor, insbesondere auf einen 90-Altgrad-Dachkantreflektor 641 für eine 180° Umlenkung, umfassend Metall, insbesondere hergestellt aus Metall mittels einer Single-point-Diamantbearbeitung. Dieser Dachkantreflektor 641 ist insbesondere in einem Metallgrundkörper 643 angeordnet. Im 90-Altgrad-Dachkantreflektor 641 ist bevorzugt eine Spaltblende 645 mit der Funktion eines Bildfeld-Diskriminators angeordnet, wie beispielsweise in der Detailabbildung 4.1 angedeutet. Durch eine entsprechende Justierung des vorgeordneten Objektivs 70 in der Tiefe fällt die Position der Bildebene BEI1 mit dem Bildpunkt O'1 mit dem Spalt der Spaltblende 645 zusammen. Der 90-Altgrad-Dachkantreflektor 641 ist in der Figur nach links verschoben, also im Wesentlichen senkrecht zur optischen Achse OAI des vorgeordneten Objektivs 71 von dieser um eine Distanz verschoben, sodass sich für das am 90-Altgrad-Dachkantreflektor 641 reflektierte Teilstrahlenbündel TB1 eine Lateral-Shear s1, die insbesondere und im Wesentlichen der Distanz der Verschiebung senkrecht zur optischen Achse OAI entspricht, ergibt. Die hier genannte Verschiebung des 90-Altgrad-Dachkantreflektor 641 erfolgt bzw. besteht in der vorliegenden Darstellung bzw. Ausführungsform in einer horizontalen lateralen Richtung "nach links". Die Detailabbildung 4.1 deutet den 90-Altgrad-Dachkantreflektor 641 mit der Spaltblende 645 vergrößert an, welche mit der Bildebene BEI1 zusammenfällt. Da der 90-Altgrad-Dachkantreflektor 641 baugleich mit dem 90-Altgrad-Dachkantreflektor 643 ist, sind auch für diesen die Bezugszeichen in Figur 4 angegeben.

Im Allgemeinen kann ein in dieser Beschreibung genannter Strahlteilerwürfel auch durch einem anderen Strahlteiler, beispielsweise einen Strahlteiler in Folienform oder mit einer Membran (Pellicle-Strahlteiler) ausgebildet, ersetzt werden.

Das an der Strahlteilerschicht 62 reflektierte Teilstrahlenbündel TB2 gelangt im Arm IA2 des Michelsontyp-Interferometers 602 ebenfalls auf einen Zweispiegel-Winkelreflektor, insbesondere auf einen 90-Altgrad-Dachkantreflektor 642, der bevorzugt und im Wesentlichen mit dem Dachkantreflektor 641 baugleich ist. Der 90-Altgrad-Dachkantreflektor 642 ist ebenfalls lateral verschoben, hier nach oben, also im Wesentlichen parallel zur optischen Achse OAI des vorgeordneten Objektivs 71 und im Wesentlichen senkrecht zur optischen Achse OA2 der InGaAs-Kamera 54 von dieser optischen Achse OA2 um eine Distanz. Die hier genannte Verschiebung des 90-Altgrad-Dachkantreflektor 642 erfolgt bzw. besteht in anderen Worten in der vorliegenden Darstellung bzw. Ausführungsform in einer vertikalen lateralen Richtung "nach oben". Das sich daraus ergebende Lateral-Shear s2 entspricht insbesondere und im Wesentlichen der Distanz der Verschiebung senkrecht zur optischen Achse OA2 bzw. parallel zur optischen Achse OAI. Das ist jedoch entgegen bezüglich der Symmetrie zur Strahlteilerschicht 62, sodass sich die im Arm IA2 ergebende Lateral-Shear s2 zur Summe von s1 und s2 gleich s addiert. Diese Addition infolge der geometrischen Anordnung stellt eine Möglichkeit dar, eine vergleichsweise große resultierende Lateral-Shear s zu erreichen.

Nach Reflexion an der Strahlteilerschicht 62 im Arm IA1 beziehungsweise nach Passieren derselben im Arm IA2 gelangen die beiden Teilstrahlenbündel TB1 und TB2 am Ausgang des Michelsontyp-Interferometers 602 zumindest teilweise in das anamorphotische und weitgehend achromatische Objektiv 51, das aus einer im Wesentlichen rotationssymmetrischen Komponente 511 und einer Zylinder-Komponente 512 besteht. Zum detaillierten Verständnis des anamorphotischen Objektivs 51 wird auf die Fig. 9 und Fig. 10 und die dazu gegebenen Erläuterungen verwiesen.

Nach Verlassen des anamorphotischen Objektivs 51 werden Zylinderwellen gebildet, welche in der Detailabbildung 4.2 als zylindrische Wellenfronten 685 angedeutet sind. Aus Gründen der besseren Darstellbarkeit sind diese in der Tiefe versetzt gezeichnet und insbesondere perspektivisch dargestellt, um die räumliche Ausdehnung anzudeuten. Durch Interferenz der Zylinderwellen 685 entstehen bevorzugt räumliche Interferogramme rI auf der Kamera, die insbesondere eine InGaAs-Kamera 54 umfasst. Der Winkel delta_beta zwischen den miteinander interferierenden zylindrischen Wellenfronten 685, speziell der Winkel delta_beta zwischen den beiden Scheitellinien 686, ist hier stark vergrößert angedeutet. Das Licht der kohärenten scheinbaren Lichtquellenpunkte O'1s und O'2s führt jeweils zur Generierung eines räumlichen Interferogramms rI als Interferenz von Zylinderwellen, wobei die Scheitellinien 686 der Wellenfronten derselben sich auf der Detektorfläche der InGaAs-Kamera 54 oder in unmittelbarer Umgebung derselben befinden. Dieses räumliche Interferogramm rI ist hier im Wesentlichen mittig auf dem Chip der Kamera, die eine InGaAs-Kamera 54 sein kann, ausgebildet, da hier in der Mitte der Ort mit der optischen Weglängendifferenz OPD durch Abgleich des Michelsontyp-Interferometers 602 bevorzugt gleich null ist. Ein Messobjekt-Scan-Mechanismus für die y-Richtung kann bevorzugt vorhanden sein, was hier jedoch nicht angedeutet ist in den Zeichnungen.

Die maximal erreichbare optische Weglängendifferenz OPDr im Zweistrahl-Interferometer bei einer im Wesentlichen symmetrischen Lage des räumlichen Interferogramms rI kann mittels der Darstellung in der Detailabbildung 4.4 angenähert errechnet werden. Wird hier im Rechenbeispiel von einer numerischen Apertur A=sin(alpha) von etwa 0,1 ausgegangen, was in Luft einem Aperturwinkel von etwa 5,7 Altgrad entspricht und einen vergleichsweise geringen Wert darstellt, ergibt sich bei einer Lateral-Shear von etwa s=1,04mm eine daraus folgende maximale Weglängendifferenz OPDr am Rand des Chips der InGaAs-Kamera 54 in der Detektionsebene DE des Objektiv 51, welches das Objektiv 511 umfasst, mit OPDr=A* s näherungsweise zu 0,104mm. Dabei liegt bzw. entsteht das räumliche Interferogramm rI bevorzugt mittig auf dem Chip. Mit einer angenommenen Dreieck-Apodisation der Intensitätswerte des räumlichen Interferogramms rI bei Berechnung des Spektrums mittels einer schnellen Fourier-Transformation kann mit dem reziproken Wert der optischen Weglängendifferenz OPDr, hier beispielsweise etwa 1/0,102mm, eine spektrale Auflösung delta_sigma von näherungsweise 96 cm$^{-1}$ erreicht werden. Das entspricht bei einer Wellenlänge von etwa 1000nm einer spektralen Auflösung im Wellenlängenbereich von etwa 9,6nm. Für eine Brennweite f'511 von etwa 60mm der rotationssymmetrischen Komponente 511 ergibt sich mit a=2A* f'511 die benötigte minimale Kantenlänge der InGaAs-Kamera 54 zu etwa 15,0mm. Die Kamera ist beispielsweise im vorliegenden Fall, ohne darauf beschränkt zu sein, eine InGaAs-Kamera vom Typ Goldeye P-032 SWIR von Allied Vision und umfasst eine Breite bk von etwa 15,9mm und einen Pixel-Pitch von etwa 25$\mu$m und etwa 636 x 508 Pixel. So kann insbesondere ein symmetrisches, räumliches Interferogramm rI mit der optischen Weglängendifferenz am Rand des Chips OPDr mit dem Betrag von etwa 0,104mm im Spektralbereich von etwa 900nm bis etwa 1700nm unter Einhaltung des Abtast-Theorems vollständig aufgenommen werden, da die Interferenzstreifen der kürzesten Wellenlänge von etwa 900nm auf dem Kamera-Chip hier bevorzugt mit einer Interferenzstreifenbreite von näherungsweise etwa 52 $\mu$m breiter als der doppelte Pixel-Pitch dieses InGaAs-Kamera-Chips sind. Die Interferenzstreifenbreite ergibt sich näherungsweise aus dem Quotienten f'511 dividiert durch die Lateral-Shear s und multipliziert mit der jeweiligen Wellenlänge.

In einem Ausführungsbeispiel 3 (nicht schematisch anhand einer Zeichnung dargestellt) können mit einer derartigen Anordnung mit entsprechenden Modifikationen darüber hinaus auch das Spektren von Fluoreszenzlicht gemessen werden, insbesondere wenn eine Anregungslichtquelle im ultravioletten Spektralbereich und ein dichroitischer Strahlteiler genutzt werden.

In einem Ausführungsbeispiel 4 mit mindestens einer Abwandlung der Ausführungsform von Fig. 4 (nicht schematisch anhand einer Zeichnung dargestellt) kann der Strahlteilerwürfel 622 auch etwas unsymmetrisch ausgebildet sein, sodass sich die Glaswege bzw. die beiden optischen Weglängen durch das Glasmaterial bzw. das Glas in den beiden Interferometerarmen IA1 und IA2 zumindest jedoch geringfügig unterscheiden, typischerweise in der Größenordnung bis zu maximal einem Millimeter. Beispielsweise können diese sich aus den unterschiedlichen optischen Weglängen ergebenden Gangunterschiede zwischen etwa 3nm und 3mm, insbesondere zwischen etwa 200nm und 2nm und bevorzugt zwischen etwa 500nm und 1,5mm liegen. So können bei der Detektion räumliche Interferogramme erzeugt werden, welche eine optische Weglängendifferenz bzw. eine Differenz der beiden optischen Weglängen bzw. ein Gangunterschied im Wesentlichen ungleich null im Zentrum der Kamera, insbesondere in der Mitte der InGaAs-Kamera aufweisen. Die Scheitellinien der zur Interferenz kommenden Zylinderwellen sind hierbei dann wegen der ungleichen Glaswege in den beiden Interferometerarmen für eine Wellenfront aus einem Interferometerarm nur näherungsweise geradlinig, also sind schwach gekrümmt, da ein Rest-Öffnungsfehler besteht, da das vorgeordnete Objektiv 71 nur für eine bestimmte Glasweglänge korrigiert sein kann. Diese unveränderliche Korrektur des vorgeordneten Objektivs 71 im Optik-Design für eine bestimmte Glasweglänge - damit der Öffnungsfehler nicht auftritt - kann also nur für eine bestimmte Glasweglänge eines Interferometerarms perfekt gegeben sein. Dieser Rest-Öffnungsfehler, der dann noch für den anderen Interferometerarm aufgrund einer hier etwas anderen Glasweglänge besteht, für die keine perfekte Korrektur besteht, kann letztlich zu Nichtlinearitäten im räumlichen Interferogramm führen und erfordert gegebenenfalls eine numerische Korrektur des Interferogramms rI.

Es ist aber auch in einem weiteren Ausführungsbeispiel 5 nach Fig. 4 (nicht schematisch anhand einer Zeichnung dargestellt) insbesondere möglich, einen der beiden Planspiegel als einen vergleichsweise sehr schmalen länglichen Planspiegel in der Nähe der Dachkante auszubilden. Der schmale Planspiegel, beispielhaft mit Abmessungen von 0,5mm x 10mm kann somit selbst als Bildfeld-Diskriminator wirken, wobei mit einer derartigen Anordnung nur ein vergleichsweise sehr grobes Hyperspektralbild gewonnen werden kann, beispielsweise nur mit 10 bis 20 Bildstreifen in der Bildhöhe.

Die Fig. 5 bis Fig. 8 umfassen verschiedene Ausführungsformen, insbesondere für die Ausbildung der Dachkantreflektoren mit integrierten Bildfeld-Diskriminatoren, welche jeweils bevorzugt in der Bildebene BEI1 angeordnet sind, für ein Michelsontyp-Interferometer. Es werden in jedem Arm eines Michelsontyp-Interferometers insbesondere jeweils zwei im Wesentlichen baugleiche Bildfeld-Diskriminatoren eingesetzt. Deshalb sind hier jeweils die Bezugszeichen für die Dachkantreflektoren in den beiden Interferometerarmen angegeben. Alternativ können sich die Bildfeld-Diskriminatoren in Beschaffenheit, in optischen Eigenschaften, Dimensionierung, Material etc. im Wesentlichen unterscheiden. In Fig. 5 bis Fig. 8 wie auch in der Detailabbildung 4.1 sind auch die sich durch Entfaltung der Dachkantreflektoren 641 und 642 ergebenden scheinbaren Endspiegelflächen SEF1 und SEF2 dargestellt, welche zur vereinfachten Darstellung genutzt werden können.

Die Fig. 5 stellt einen Single-point-diamantbearbeiteten 90-Altgrad-Dachkantreflektor 641, insbesondere aus Metall für eine 180° Strahlumlenkung dar. Dieser 90-Altgrad-Dachkantreflektor 641 kann insbesondere im ersten Arm IA1 des Michelsontyp-Interferometers 602 gemäß Fig. 4 verwendet werden bzw. abgeordnet sein. Der Bildfeld-Diskriminator ist hierbei insbesondere mit einer Spaltblende 645 mit der Spaltbreite b ausgebildet bzw. ausgestattet, welche im Wesentlichen baugleich mit der Spaltblende 646 im zweiten Arm IA2 des Michelsontyp-Interferometers 602 ausgebildet ist.

Die Fig. 6 stellt einen bevorzugt rechnersteuerbaren Piezosteller 647 dar, welcher bevorzugt der Spaltblende 645 im Dachkantreflektor 641 zugeordnet sein kann und eine Einstellung der Spaltbreite b ermöglichen kann und somit die laterale Auflösung beeinflussen kann. Die beiden Anordnungen nach Fig. 5 und Fig. 6 ermöglichen insbesondere einen vergleichsweise großen Spektralbereich, da es sich insbesondere um Anordnungen zumindest teilweise in Luft handelt.

In einem Ausführungsbeispiel 6 (nicht schematisch anhand einer Zeichnung dargestellt) kann bei Nutzung eines Plattenstrahlteilers für den optischen MIR-Wellenlängenbereich insbesondere mit Substraten, die CaF2 und/oder KBr aufweisen bzw. sein können, in einem Michelsontyp-Interferometer ebenfalls ein Dachkantreflektor 641 eingesetzt bzw. angeordnet werden.

Fig. 7 stellt eine Anordnung mit einem länglichen Pinhole-Array 648 zur feinen Bildfeld-Diskriminierung für ein Michelsontyp-Interferometer dar. In Ergänzung zu diesem Ausführungsbeispiel nach Fig. 7 gibt es ein weiteres Ausführungsbeispiel 7, bei dem das Messobjekt in Durchlicht und/oder in Auflicht mit einem zum Pinhole-Array 648 angepassten Lichtfleckmuster beleuchtet ist, dessen Licht somit im Michelsontyp-Interferometers 602 konfokal diskriminiert wird. Dies kann auch im MIR-Bereich bei Nutzung eines entsprechenden Plattenstrahlteilers mit Substraten umfassend CaF2 und/oder KBr und einer Strahlteilerschicht erfolgen, welche für den MIR-Spektralbereich geeignet ist.

Die Fig. 8 stellt schematisch die Möglichkeit dar, im sichtbaren Spektralbereich mit einem transmissiven Flüssigkristall-Display (LCD) 650 einen im Wesentlichen länglichen, lichtdurchlässigen Bereich 650-1 der Breite b unter Nutzung hier nicht dargestellter, jedoch dem Fachmann gut bekannter, weiterer polarisationsoptischer Komponenten einzuschreiben.

Die Fig. 9 und Fig. 10 stellen die Abbildungseigenschaften eines dem Michelsontyp-Interferometer nachgeordneten, anamorphotischen und weitgehend achromatischen Objektivs 51 zur Detektion räumlicher Interferogramme rI dar. Hier wird die Funktion dieses anamorphotischen Objektivs 51 insbesondere im Zusammenhang bzw. im Einsatz mit der Messanordnung gemäß der Fig. 4 erläutert. Die Zeichenebene in Fig. 9 ist die yz-Ebene, die hier beispielhaft die Referenzebene RE darstellt und wobei diese yz-Ebene die scheinbaren Bildpunkte O'1s und O'2s beinhaltet, die sich nach einer Entfaltung der Strahlengänge ergeben. Diese Bildpunkte O'1s und O'2s stellen durch Amplitudenteilung im Zweistrahl-Interferometer optisch kohärente Bildpunkte von einem angemessenen Objektpunkt O dar. In der yz-Ebene ist für den im Wesentlichen gesamten genutzten mindestens jedoch teilweise für den Spektralbereich des Fourier-Spektrometers eine besonders gute bzw. eine effektive Korrektur der Aberrationen vorteilhaft und zwar insbesondere, ausgehend von dem Objekt bis zum gerasterten Detektor, sodass die räumlichen Interferogramme weitgehend ungestört bleiben.

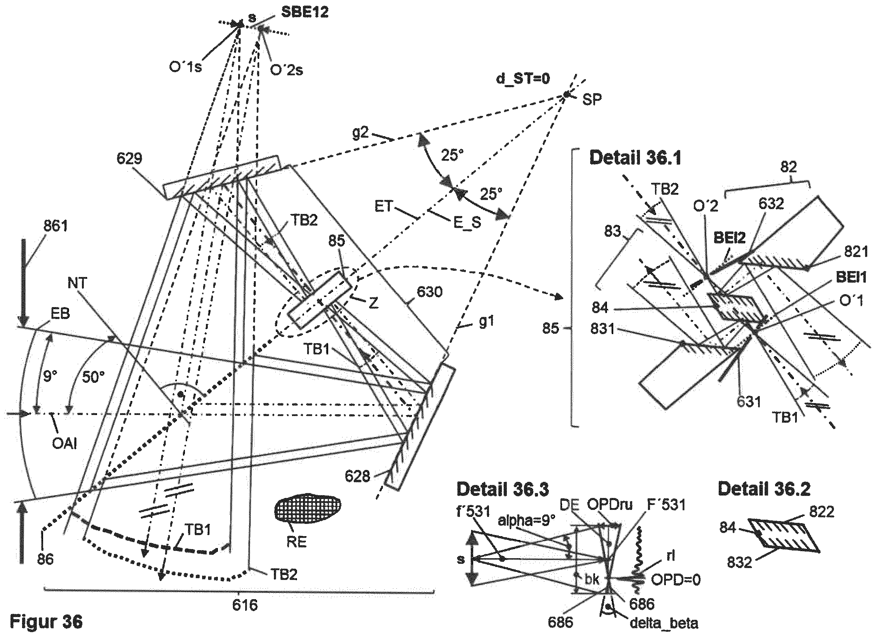

Die Zeichenebene in Fig. 10 entspricht der xz-Ebene, die darin angedeutet ist und welche im Wesentlichen senkrecht zur Referenzebene RE steht. Diese xz-Ebene umfasst ebenfalls den scheinbaren Bildpunkt O'1s. In der xz-Ebene ist durch die sich hier addierende Brechkraft der Zylinder-Komponente 512 die Gesamtbrechkraft des anamorphotischen Objektivs 51 zumindest näherungsweise doppelt so groß verglichen mit der Gesamtbrechkraft der yz-Ebene. In der Detektionsebene DE entstehen, insbesondere zylindrische Wellenfronten 685, deren Scheitellinien 686 im Wesentlichen zueinander geneigt sind und welche im Wesentlichen parallel zur yz-Ebene ausgerichtet sind. Der Punkt O"1_xz in der Detektorebene DE ist insbesondere der Durchstoßpunkt der Zylinderwelle, resultierend insbesondere aus dem scheinbaren Bildpunkt O'1s, durch die xz-Ebene. Der gerasterte Detektor, hier beispielhaft eine InGaAs-Kamera 54, steht im Wesentlichen in der Detektionsebene DE. Diese Zusammenhänge gelten insbesondere auch für die weiteren nachgeordneten, anamorphotischen und weitgehend achromatischen Objektive 52, 53, 58 und 59 sowie auch das anamorphotische Spiegelobjektiv in Fig. 36, welches mit den Freiformflächen 875, 876 und 877 ausgebildet ist.

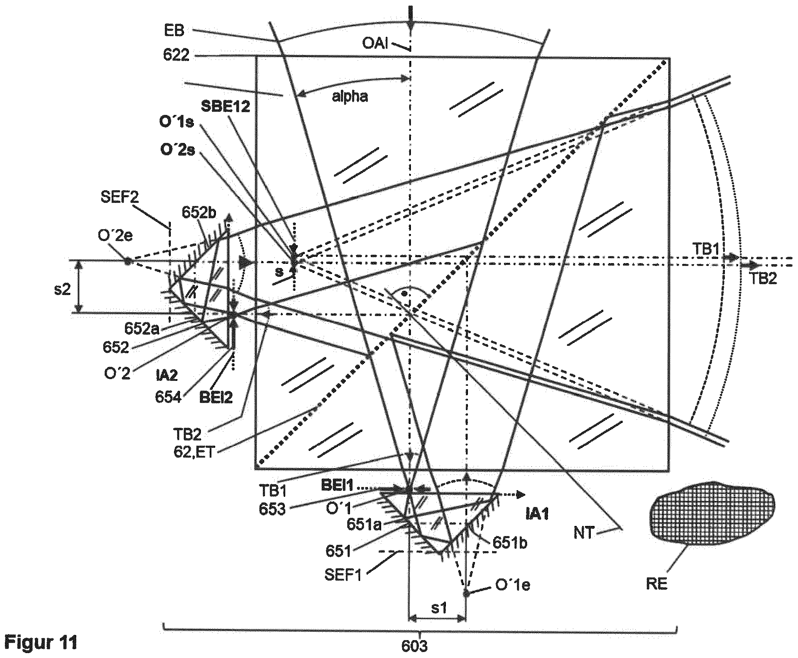

In Fig. 11 ist das Michelsontyp-Interferometer 603 in einer weiteren beispielhaften Ausführungsform dargestellt. Diese ist insbesondere für den Betrieb zumindest teilweise im VIS-Spektralbereich und/oder zumindest teilweise im NIR-Bereich ausgelegt. Das von einem vorgeordneten Objektiv, welches hier nicht dargestellt ist, fokussierte Eingangsbündel EB mit einem halben Öffnungswinkel alpha von beispielsweise etwa 16 Altgrad kann insbesondere in den Strahlteilerwürfel 622 mit der Strahlteilerschicht 62 eintreten. An der Strahlteilerschicht 62 können die beiden Teilstrahlenbündel TB1 und TB2 entstehen, welche jeweils durch ein Prisma 651 und 652, das beispielsweise als etwa 90°-Dachkantprisma ausgebildet sein kann, um etwa 180 Altgrad umgelenkt werden. Die 90°-Dachkantprismen 651 und 652 umfassen insbesondere jeweils eine im Wesentlichen lange, spaltförmige Spaltblende 653 und 654 bzw. werden jeweils mit der Spaltblende 653 und 654 kombiniert. Schamtisch ist hier dargestellt, dass die entfalteten Bildpunkte O'1e und O'2e vom Messobjekt in den beiden Armen des Michelsontyp-Interferometers 603 im Wesentlichen jeweils außerhalb der scheinbaren Endspiegelflächen SEF1 und SEF2 lieg. Die 90°-Dachkantprismen 651 und 652 sind hier gleichsinnig zur Strahlteilerschicht 62 um etwas ungleiche Beträge s1 und s2 quer verschoben. Somit ergibt sich die resultierende Lateral-Shear s der durch das Michelsontyp-Interferometers 603 nach der Strahlzusammenführung gebildeten scheinbaren Bildpunkte O'1s und O'2s in Differenz der Beträge zu s=s2-s1. Diese resultierende Lateral-Shear s hat für alle scheinbaren Bildpunkte vom Messobjekt zumindest näherungsweise den gleichen Betrag. Durch das gleichsinnige Verschieben der 90°-Dachkantprismen 651 und 652 kann bei Bedarf insbesondere auch eine sehr kleine Lateral-Shear s erzeugt werden. Dies kann insbesondere auch rechnergesteuert mit in der Fig. 11 nicht dargestellten Bewegungs- und Steuermitteln durchgeführt bzw. verwirklicht werden. Die Fig. 11 stellt schematisch dar, dass auch bei einer Lage der Bildfeld-Diskriminatoren, hier jeweils dargestellt durch die spaltförmigen Spaltblenden 653 und 654, die sich im Wesentlichen nicht in der scheinbaren Endspiegelfläche SEF1 und SEF2 befinden, noch ein recht großer halben Öffnungswinkel alpha genutzt werden kann, um vergleichsweise viel Licht vom Messobjekt zur Detektion bringen zu können, indem ein Eingangsstrahlenbündel EB mit einem Öffnungswinkel von beispielhaft bis zu 15 Altgrad bestehen kann, was aber maßgeblich durch den Brechungsinex der eingesetzten optischen Materialien für den Strahlteilerwürfel mitbestimmt wird.

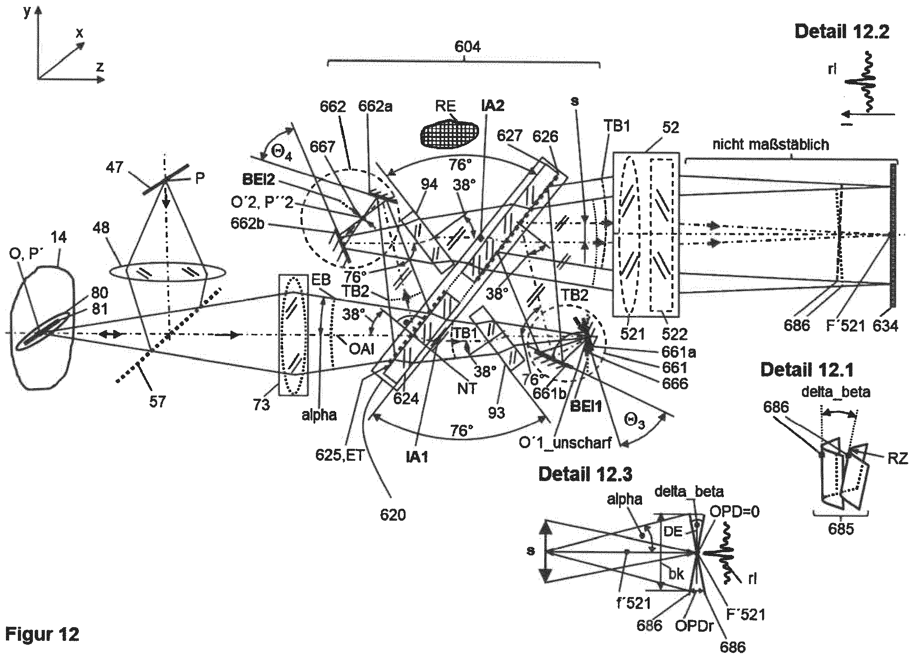

Die Fig. 12 stellt schematisch ein Mach-Zehnder-Interferometer 604, insbesondere und zumindest teilweise für den MIR-Bereich und insbesondere für ein Messobjekt 14 zur Auflichtmessung dar. Das Mach-Zehnder-Interferometer 604 ist beispielsweise als eine Anordnung in der Form des Buchstabens X, kurz X-Anordnung genannt, insbesondere mit einem Strahlteiler, der CaF2-Substrate aufweisen kann, ausgebildet. Der Einfallswinkel für den Hauptstrahl auf die Strahlteilerschicht 625, an welcher die beiden Teilstrahlenbündel TB1 und TB2 entstehen beträgt hier beispielsweise etwa 38°, insbesondere kann auch ein Winkelbereich von 35° bis 50° bestehen. Zu jedem Hauptstrahl in einem Arm des Mach-Zehnder-Interferometers 604 gibt es insbesondere einen dazu im Wesentlichen parallelen Hauptstrahl im anderen Arm. So beträgt der Einfallswinkel für den Hauptstrahl auf die Strahlteilerschicht 626 zur Strahlenbündelzusammenführung hier entsprechend beispielsweise ebenfalls etwa 38°. Im Arm IA1 des Mach-Zehnder-Interferometers 604 befindet sich ein Zweispiegel-Winkelreflektor 661 bevorzugt mit zwei Winkelspiegeln 661a und 661b und im Arm IA2 befindet sich ebenfalls ein weiterer Zweispiegel-Winkelreflektor bevorzugt mit zwei Winkelspiegeln 662. Beide Anordnungen der Winkelspiegel 661 und 662 weisen eine Strahlablenkung von beispielsweise etwa 76° auf. Insbesondere kann auch ein Winkelbereich von 70° bis 100° bestehen. Hier sind die Spaltblenden 666 und 667 jeweils zumindest teilweise zwischen den jeweiligen beiden Spiegeln der Winkelspiegel 661 und 662 angeordnet.

In der Fig. 12 bilden die Planparallelplatten 624 und 627 und die Strahlteilerschichten 625 und 626 eine Einheit, die wir als eine Strahlteilereinheit 620 bezeichnen. Diese Strahlteilereinheit 620 hat also zwei Elemente und zwar die Planparallelplatte 624 mit der Strahlteilerschicht 625 und die Planparallelplatte 627 mit der Strahlteilerschicht 626. Beide Elemente können ein Lichtbündel bzw. einen Strahlengang dessen jeweils aufspalten bzw. teilen und zusammenführen. In der Anordnung ist die Planparallelplatte 624 mit der Strahlteilerschicht 625 dazu ausgelegt, das Eingangsstrahlenbündel EB in die Teilstrahlenbündel TB1 und TB2 zu spalten und die Planparallelplatte 627 mit der Strahlteilerschicht 626 ist dazu ausgelegt die Teilstrahlenbündel TB1 und TB2 zumindest teilweise wieder zu vereinen.

Bei dieser Anordnung kann insbesondere ein relativ scharfes Bild nach der Strahlteilung an der Strahlteilerschicht 625 im Winkelspiegel 662 im zweiten Interferometerarm IA2 entstehen. Das vorgeordnete Objektiv 73 muss nicht notwendigerweise eine Öffnungsfehler-Korrektur aufweisen, da sich bevorzugt keine geneigte Platte im Strahlengang vor dem Winkelspiegel 662 mit der Spaltblende 667 befindet. Im ersten Interferometerarm IA1 entsteht dagegen ein relativ unscharfes Bild des Messobjekts 14, da hier ein Öffnungsfehler aufgrund der im Strahlengang im Wesentlichen geneigten Platten 624 und 93 vor der Spaltblende 666 erzeugt wird. Am Ort der kleinsten Bündeleinschnürung befindet sich deshalb bevorzugt eine im Vergleich zur Spaltblende 667 etwas gröbere Spaltblende 666, die beispielsweis das Vierfache der Spaltweite der Spaltblende 667 beträgt, beispielhaft hier 0,5mm für den mittel-infraroten Bereich. Deshalb ist diese Interferometeranordnung insbesondere für eine vergleichsweise grobe Bildauflösung geeignet, die dann letztlich zu einem Hyperspektralbild führt, welches in x-Richtung, siehe Figur 10, nur etwa 50 auflösbare Bildinkremente aufweist.

Die Zusammenführung der Teilstrahlenbündel TB1 und TB2 erfolgt an der Strahlteilerschicht 626. Zwischen den Teilstrahlenbündeln TB1 und TB2 besteht eine Lateral-Shear s, in anderen Worten sind die Teilstrahlenbündeln TB1 und TB2 um den Betrag der Lateral-Shear s gegeneinander verschoben. Der Öffnungsfehler, der durch die Planparallelplatten 625, 627, 93, 94 im Mach-Zehnder-Interferometer 604 verursacht wird, ist insbesondere im anamorphotischen Objektiv 52 zumindest teilweise einkorrigiert, sodass am Ausgang des Objektivs 52 von jedem Bildpunkt weitestgehend ungestörte Zylinderwellen gebildet werden, die in der Fourier-Ebene zur Interferenz gelangen. In Fig. 12 wurde aus Gründen der besseren Darstellbarkeit auf die Darstellung der entfalteten scheinbaren Bildpunkte O'1s und O'2s verzichtet. Diese ergeben sich insbesondere durch die Rückverlängerung der Randstrahlen der Teilstrahlenbündel TB1 und TB2. Auch sind die scheinbaren Endspiegelflächen SEF1 und SEF1 sowie auch die scheinbare Bildebene SBEI12 hier in Figur 12 aus zeichentechnischen Gründen nicht dargestellt.

In der Detailabbildung 12.1 sind beispielhaft zueinander geneigte Zylinderwellen mit ihren zylindrischen Wellenfronten 685 dargestellt. Die Detailabbildung 12.2 stellt beispielhaft ein räumliches Interferogramm rI dar, welches auf dem Mikrobolometer-Array 634 ausgebildet ist und die Detailabbildung 12.3 stellt schematisch den Zusammenhang zwischen der Lateral-Shear s und der sich am Rand eines Displays bzw. eines Detektors, insbesondere eines Mikrobolometer-Displays 634 in der Detektionsebene DE ergebenden optischen Weglängendifferenz OPDr, aus welchem die spektrale Auflösung abgeleitet, insbesondere errechnet werden kann, dar. Siehe dazu auch die Beschreibung zur Detailabbildung 4.4, die auf die Verhältnisse in Fig. 12 übertragen werden kann.

In einem Ausführungsbeispiel 8 (nicht schematisch anhand einer Zeichnung dargestellt) auf der Basis der Anordnung in Fig. 12 kann ein Spiegel in einem der Zweispiegel-Winkelreflektoren durch ein rechnersteuerbares Mikrospiegel-Array ersetzt werden.

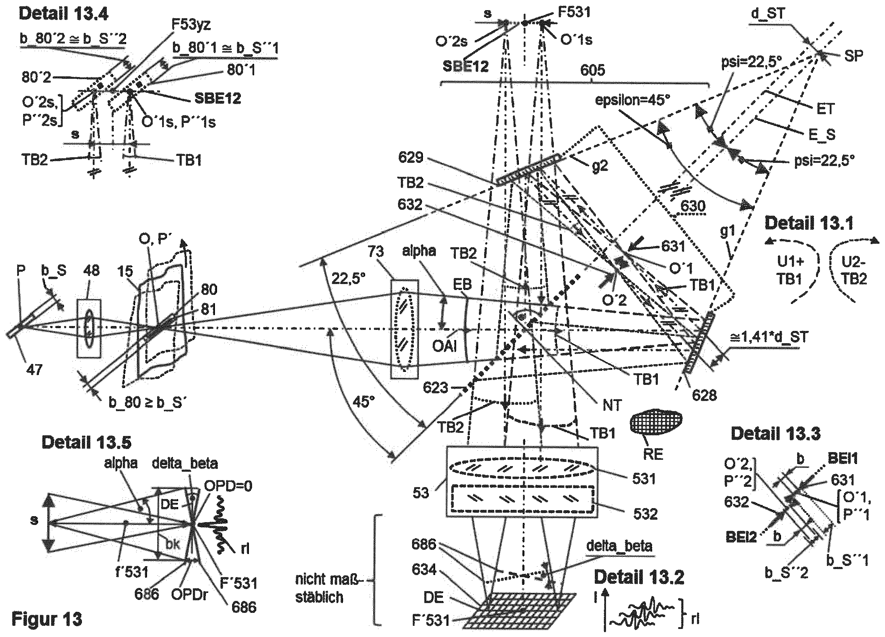

Die Fig. 13 stellt schematisch ein zyklisches Zweistrahl-Interferometer 605 insbesondere für den Betrieb zumindest teilweise im FIR-Spektralbereich dar. Das zyklische Zweistrahl-Interferometer 605 weist einen Strahlteiler 623 insbesondere mit einer dünnen Mylarfolie und zwei Planspiegel 628 und 629 auf. Die Planspiegel 628 und 629 bilden paarweise zumindest einen Teil einer Winkelspiegelanordnung, insbesondere eines Zweispiegel-Winkelreflektors 630.

Das zyklische Zweistrahl-Interferometer 605 ist insbesondere auch als Sagnac-Interferometer und/oder als zyklisches Dreieck-Interferometer bekannt. Die Detailabbildung 13.1 deutet den Richtungssinn des Lichts der Teilstrahlenbündel TB1 und TB2 mittels eingezeichneter Pfeile an. Es erfolgt eine im Wesentlichen aktive, streifenförmige Beleuchtung mit vergleichsweise kurzen Lichtimpulsen - mit Pulslängen im Bereich von einigen Millisekungen im unteren einstelligen Bereich - eines dünnen, bewegten und zumindest teilweise transparenten Messobjekts 15 für eine Durchlichtmessung mittels einer schmalen IR-Lichtquelle 47, welche die beispielhafte Breite b_S von 1mm und eine beispielhafte Länge von 20mm aufweist. Auf dem Messobjekt 15 entsteht ein beleuchteter Streifen 80, welcher der Breite b_S' des Bildes der schmalen IR-Lichtquelle 47 entspricht, da es hier im Idealfall keine nennenswerte Lichtstreuung gibt, die eine erhebliche Verbreiterung des Streifens 80 bewirken könnte.

Das zyklisches Zweistrahl-Interferometer 605 weist eine Strahlteilerebene ET auf, welche schematisch dargestellt ist durch den Strahlteiler bevorzugt mit der Mylarfolie 623, an der insbesondere auch die zumindest teilweise Strahlwiedervereinigung unter Einfluss der Lateral-Shear s stattfindet. Die von den Planspiegeln 628 und 629 verlängerten Geraden g1 und g2 schneiden sich im Schnittpunkt SP in der Referenzebene RE, welche hier im Wesentlichen der Zeichenebene entspricht. Dieser Schnittpunkt SP, welcher im Wesentlichen in der Symmetrieebene E_S liegt, weist zur Strahlteilerebene ET einen Abstand d_ST auf, der hier beispielsweise zwischen etwa 0,1mm und 7mm und insbesondere zwischen etwa 1,5mm und 3mm liegen kann und bevorzugt 2,5mm beträgt. Der Wert von 2,5mm entspricht hier in etwa 100 Wellenlängen der größten Wellenlänge im beispielhaften Spektrum von 25 $\mu$m, was einer Wellenzahl von 400 cm$^{-1}$ entspricht.

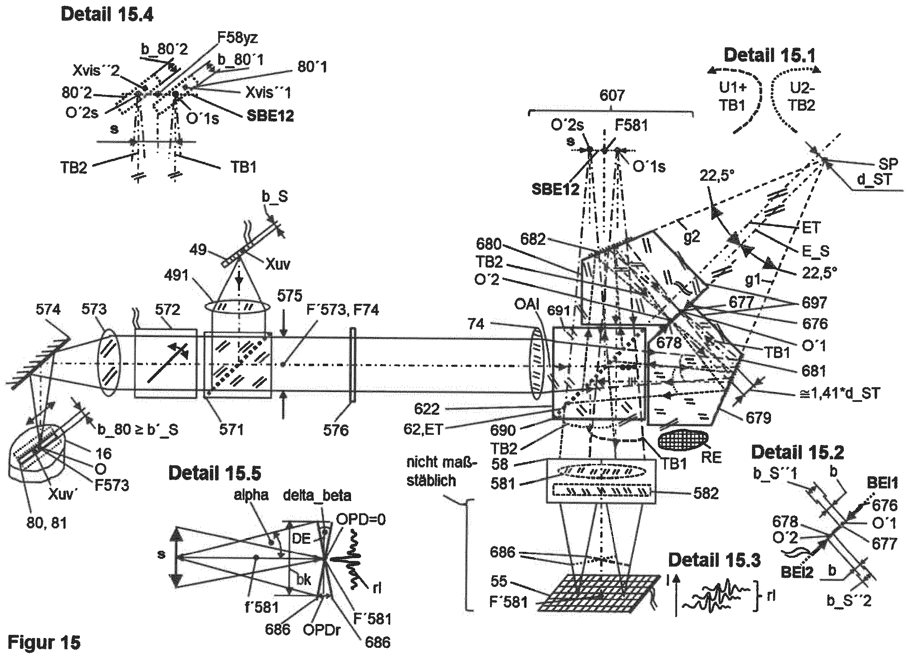

Zumindest teilweise zwischen den Planspiegeln 628 und 629 können wie hier dargestellt zwei vergleichsweise grobe Bildfeld-Diskriminatoren angeordnet sein, welche hier beispielhaft durch die Spaltblenden 631 und 632 dargestellt sind und welche die beispielhafte Breite von etwa b=1 mm aufweisen. Die Breite kann beispielsweise auch zwischen etwa 0,1mm und 10mm und insbesondere zwischen etwa 0,5mm und 3mm liegen. Die Spaltblenden 631 und 632 enthalten jeweils ein Bild O'1 beziehungsweise O'2 des Messpunkts O und sind hier etwas - wie in der Zeichnung durch perspektivischen Versatz angedeutet - in der Tiefe des Bildes versetzt. Dieser Tiefenversatz ergibt sich hier insbesondere aus der Lage des vorgeordneten Objektivs 73, welches in der Tiefe insbesondere eine gewisse Fehllage aufweist. Diese Fehllage ist in gewissen Grenzen tolerierbar. Insbesondere ist es wünschenswert, dass die jeweiligen Bilder O'1 beziehungsweise O'2 des Messpunkts O in einer gemeinsamen Ebene liegen und somit auch die Spaltblenden 631 und 632 bevorzugt in einer gemeinsamen Ebene liegen. Dargestellt ist dies in der Fig. 15, wo die beiden Spaltöffnungen 677 und 678 durch eine Doppelspaltblende 676 dargestellt sind. Das in Fig. 13 an den Spaltblenden 631 und 632 diskriminierte Licht im FIR-Spektralbereich gelangt zumindest teilweise über den Strahlteiler 623, der insbesondere Mylarfolie umfasst, als im Wesentlichen parallel mit einer Lateral-Shear s sich ausbreitende Teilstrahlenbündel TB1 und TB2 über das anamorphotische Objektiv 53 auf einen Detektor, insbesondere auf ein Mikrobolometer-Array 634 zur Interferenz in Form zueinander geneigter Zylinderwellen mit den Scheitellinien 686. Dieses anamorphotische Objektiv 53 umfasst beispielsweise auch eine Zylinderkomponente 532 für zumindest einen Teil des FIR-Spektralbereichs, welches in der optischen Gesamtfunktion prinzipiell dem Objektiv 51 entspricht. Diese Gesamtfunktion ist in den Beschreibungen zu den Fig. 9 und Fig. 10 zu finden. Die Intensitätsverläufe der räumlichen Interferogramme rI sind in der Detailabbildung 13.2 dargestellt.

Die rückwärtigen Verlängerungen der Strahlen der Teilstrahlenbündel TB1 und TB2 bilden im Wesentlichen in der scheinbaren Bildebene SBE12, welche insbesondere auch im Wesentlichen der Brennebene des Objektivs 531 mit seinem Brennpunkt F531 entspricht, die weitestgehend entfalteten und optisch kohärenten, scheinbaren Bildpunkte O'1s und O'2s.

Da es hier aufgrund des Strahlteilers 623, der bevorzugt eine dünne Mylarfolie aufweist, keinen Öffnungsfehler gibt, kann der halbe Öffnungswinkel alpha hier vergleichsweise groß gemacht werden, also insbesondere auch größer als hier in der Fig. 13 dargestellt, beispielhaft um 8 Altgrad. Das Maximum für den halben Öffnungswinkel alpha ergibt sich hier im zyklischen Zweistrahl-Interferometer - je nach Konstruktion - beispielsweise zu etwa 10 Altgrad. Der halbe Öffnungswinkel alpha kann insbesondere zwischen etwa 2 und 12 Altgrad, bevorzugt zwischen etwa 5 und 10 Altgrad liegen.

Die Detailabbildung 13.3 stellt schematisch die geometrischen Verhältnisse an den Spaltblenden 631 und 632 dar. Das beispielhafte Bild der vergleichsweise schmalen IR-Lichtquelle 47 weist insbesondere für die Breiten des Lichtstreifens b_S"1 und b_S"1 jeweils einen Wert von etwa 2mm auf. Dieser Wert kann insbesondere zwischen etwa 0,5mm und 5mm, bevorzugt zwischen etwa 1mm und 3mm liegen. Somit sind diese Bildbreite b_S"1 und b_S"2 kleiner als der Abstand von etwa d_ST=2,5mm und es gibt im Wesentlichen kein optisches Übersprechen zwischen den umlaufenden Strahlengängen, auch, wenn durch Lichtstreuung am Messobjekt 15 der Lichtstreifen 80 noch etwas verbreitert ist. Optisches Übersprechen bedeutet hier, dass in unerwünschter Weise Lichtanteile aus dem in einer Richtung umlaufenden Teilstrahlenbündel in das Teilstrahlenbündel der anderen Umlaufrichtung gelangen und auch zur Detektion kommen.

Die Detailabbildung 13.4 stellt den Sachverhalt in der scheinbaren Bildebene SBE12 dar, die jedem anamorphotischen Objektiv 53 vorgeordnet ist und die Objektebene für dieses Objektiv 53 ist. Gezeigt werden die beiden Bilder 80'1 und 80'2, die sich im Wesentlichen in der scheinbaren Bildebene SBE12 vom Lichtstreifen 80 befinden. Die Breiten derselben b_80'1 und b_80'2 entsprechen hier zumindest näherungsweise den Breiten b_S"1 und b_S"2, die sich aus der geometrischen Abbildung der Lichtquelle 47 ergeben. Wenn vom Messobjekt 15 eine feinere Ortsauflösung erreicht werden soll, können die Spaltbreiten b auf beispielhaft 50 Mikrometer bis 200 Mikrometer verringert werden und damit auch die Breite der Lichtquelle 47.

Wird hier in einem Rechenbeispiel von einer beispielhaften numerischen Apertur A=sin(alpha) von etwa 0,1 ausgegangen, siehe Detailabbildung 13.5, ergibt sich bei einer beispielhaften Lateral-Shear von etwa s=2,82*2,5mm=7,05mm eine daraus folgende maximale optische Weglängendifferenz im Interferogramm in der Detektionsebene DE des Objektivs 53 mit A* s näherungsweise zu beispielhaft etwa 0,705mm. Mit einer angenommenen Dreieck-Apodisation der Intensitätswerte des räumlichen Interferogramms rI bei Berechnung des Spektrums mittels einer schnellen Fourier-Transformation (FFT= fast Fourier transform) kann mit dem reziproken Wert (1/OPD) der optischen Weglängendifferenz, hier beispielhaft etwa 1/0,705mm, eine spektrale Auflösung von näherungsweise beispielhaft etwa 14 cm$^{-1}$ erreicht werden. Die schnelle Fourier-Transformation (englisch fast Fourier transform, daher meist FFT abgekürzt) ist ein Algorithmus zur effizienten Berechnung der diskreten Fourier-Transformation (DFT).

Bei einem vergleichsweise kleinen Öffnungswinkel alpha, beispielsweise von unter etwa 5 Altgrad, insbesondere beispielhaft von 2 Altgrad bis 4 Altgrad und einer kompakten Bauweise eines entsprechend im Wesentlichen asymmetrisch aufgebauten zyklischen Interferometers 606 ist es in einem Ausführungsbeispiel 9 (nicht schematisch anhand einer Zeichnung dargestellt) auf der Basis einer Anordnung nach der Fig. 13 jedoch auch möglich, dass sich die Teilstrahlenbündel TB1 und TB2 zumindest auf einem der beiden Planspiegel des zyklischen Interferometers überhaupt nicht oder zumindest teilweise nicht überdecken. Es werden also unterschiedliche, nebeneinander liegende Bereiche eines Planspiegels zumindest teilweise zur Reflexion genutzt. In diesem Fall kann einer dieser Planspiegel 628 oder 629 insbesondere auch geteilt ausgebildet sein. So ist jedem Teilstrahlenbündel TB 1 und TB2 dann zumindest teilweise ein eigener, nun vergleichsweise kleiner Planspiegel zugeordnet, der insbesondere zur Reflexion des jeweiligen Teilstrahlenbündels dient und beispielhaft eine Größe von 5mm x 20mm aufweist. Dabei ist bevorzugt einer der beiden kleineren Spiegel in der Tiefe etwas versetzt angeordnet, sodass ein Absatz in der Tiefe zwischen den beiden kleineren Planspiegeln besteht. So ist es insbesondere möglich, eine zusätzliche optische Weglängendifferenz im zyklischen Interferometer einzuführen. Dies dient dem Ziel, auf dem gerasterten Detektor im Wesentlichen lateral verschobene räumliche Interferogramme rI mit einer größeren optischen Weglängendifferenz zu erhalten, die dann zu einer verbesserten spektralen Auflösung des Spektrometers führt. In einem weiteren Ausführungsbeispiel 10 (nicht schematisch anhand einer Zeichnung dargestellt) ist mindestens einer der beiden kleineren Spiegel vergleichsweise sehr schwach gekrümmt ausgebildet, stellt also im Wesentlichen keinen Planspiegel dar, um die Bildverschiebung in der Tiefe, welche durch den Absatz gegeben ist, wieder auszugleichen zu können. Von Vorteil ist es, wenn in einem weiteren Ausführungsbeispiel 11 (nicht schematisch anhand einer Zeichnung dargestellt) beide kleineren Spiegel schwach gekrümmt ausgebildet sind und der Betrag der Krümmung zumindest näherungsweise identisch ist, wobei jedoch ein Spiegel im Wesentlichen konvex und ein Spiegel im Wesentlichen konkav ausgestaltet ist. Dies kann insbesondere die Aberrationen verringern.

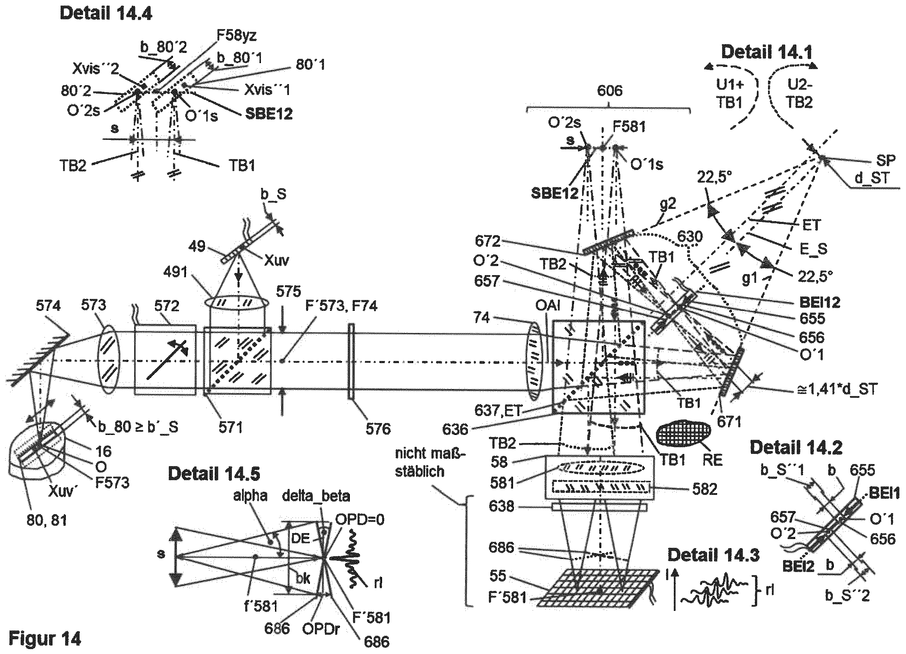

Die Fig. 14 stellt schematisch ein zyklisches Zweistrahl-Interferometer 606 dar, insbesondere für den Betrieb zumindest teilweise im VIS-Spektralbereich. Bevorzugt ist das zyklische Zweistrahl-Interferometer 606 zur Tumor-Erkennung im Gewebe ausgelegt und weist zwei Planspiegel 671 und 672 auf, die paarweise eine Winkelspiegelanordnung bilden, insbesondere einen Zweispiegel-Winkelreflektor 630. Bei diesem zyklischen Zweistrahl-Interferometer 606 weist die Symmetrieebene E_S zumindest teilweise zwischen den Planspiegeln 671 und 672, die auch die Winkelhalbierende umfasst, einen Parallelversatz d_ST zur Strahlteilerebene ET auf, der hier beispielsweise zwischen etwa 0,1mm und 5mm, bevorzugt etwa 1mm ausmacht und dabei etwa 1429 Wellenlängen der größten Wellenlänge von beispielhaft etwa 700nm im Spektrum entspricht.

Ein beispielhaftes biologische Messobjekt 16 umfasst insbesondere Fluoreszenzmarker und wird durch einen projizierten Lichtstreifen 80 zumindest teilweise im UV-Spektrum, der mittels einer rechnersteuerbaren, gerasterten UV-Lichtquelle 49 erzeugt wird, zumindest teilweise beleuchtet. Ein dichroitischer Einkoppelstrahlteilerwürfel 571 mit einer Reflexionsschicht für zumindest einen Teil des UV-Lichts koppelt das entsprechende UV-Licht zumindest teilweise in den Beleuchtungsstrahlengang ein. Die Abbildung der gerasterten UV-Lichtquelle 49 erfolgt über das Kollimatorobjektiv 491 und das Mikroskopobjektiv 573 auf das beispielhafte biologische Messobjekt 16. So wird ein leuchtendes Pixel Xuv zumindest näherungsweise scharf und hier verkleinert als Bild Xuv' des leuchtenden Pixels Xuv auf das biologische Messobjekt 16 abgebildet. Die Breite eines Bildes Xuv' beträgt beispielsweise etwa zwischen 6 $\mu$m und 600 $\mu$m, insbesondere zwischen etwa 20 $\mu$m und 100 $\mu$m, bevorzugt etwa 60 $\mu$m und somit auch die Breite eines Lichtstreifens 80 bei vollständig oder zumindest teilweise eingeschalteten und somit leuchtenden Pixeln. Um ein Übersprechen durch Streulicht zwischen den einzelnen Messpunkten zu verringern, können insbesondere bei jeder Bildaufnahme durch eine Kamera, insbesondere eine CMOS-Kamera 55 jedoch beispielsweise die geradzahligen und die ungeradzahligen Pixel alternierend leuchten.

Zur flächigen Abtastung des Messobjekts 16 kann ein Scanner dienen, insbesondere ein eindimensionaler, rechnersteuerbarer Galvano-Spiegel-Scanner 572. Am Messobjekts 16 entsteht durch eine UV-Fluoreszenz Licht im VIS. Dieses Fluoreszenzlicht im VIS kann den Einkoppelstrahlteilerwürfel 571 mit Transmissionseigenschaften der Teilerschicht für Fluoreszenzlicht im VIS passieren. Zusätzlich wird das verbleibende UV-Licht, welches den Einkoppelstrahlteilerwürfel 571 zumindest teilweise noch passieren konnte, mittels eines UV-Sperrfilters 576 beispielsweise oberhalb von etwa 700nm blockiert. So tritt im Wesentlichen sichtbares Licht über das vorgeordnete Objektiv 74 in das zyklische Interferometer 606 ein. Das Mikroskopobjektiv 573 und das vorgeordnete Objektiv 74 bilden insbesondere einen mikroskopischen Strahlengang mit etwa 2facher Vergrößerung, wobei das vorgeordnete Objektiv 74 insbesondere mit einer Öffnungsfehlerkorrektur für den Polarisationsstrahlteilerwürfel 636 ausgebildet ist.

Das in das zyklische Zweistrahl-Interferometer 606 eintretende Licht bildet dort zumindest teilweise in den beiden umlaufenden Strahlengängen gemäß der Detailabbildung 14.1 das zumindest teilweise in Streifenform beleuchtete Messobjekt 16 auf ein Display, insbesondere ein transmissives Flüssigkristall-Display 655 mit den vergleichsweise scharfen, reellen Bildern O'1 und O'2 des Objektpunkts O im Wesentlichen scharf ab. In das Flüssigkristall-Display 655, welches von beiden Seiten genutzt werden kann, sind zwei schmale Durchlassbereiche 656 und 657 von etwa b=100 $\mu$m Breite angeordnet, die mit etwa 1,41*d_ST um den Mittenabstand bzw. den Abstand zweier Zentren von etwa 1,41mm separiert sind und einen Doppelspalt bilden. Dazu wird in den beiden für den Doppelspalt adressierten Durchlassbereichen 656 und 657 des Flüssigkristall-Displays 655 nicht notwendigerweise eine bzw. keine Drehung der Polarisationsrichtung des Lichts erzeugt. Die Polarisationsrichtung bleibt insbesondere erhalten. Dagegen erfolgt insbesondere in den blockierenden Bereichen des Flüssigkristall-Displays bzw. des LCDs 655 eine Drehung der Polarisationsrichtung um etwa 90° durch eine Phasendifferenz von etwa 180 Altgrad. Im Zusammenwirken mit dem Polarisationsanalysator 638 bei der Auskopplung des Lichts aus dem zyklischen Interferometer 606 ergeben sich so zwei relativ effektive schmale Durchlassbereiche in der Bildebene E_BF. Dabei wird vom Bild des Lichtstreifens 80 im Interferometer 606 jeweils ein Durchlassbereich der beiden Durchlassbereiche 656, 657 zumindest teilweise überdeckt, da insbesondere beispielhaft ein relativ schmaler Lichtstreifen von etwa 60 $\mu$m Breite auf dem biologischen Messobjekt 16 erzeugt wird, dessen Bild eine beispielhafte Breite von b_S"1 beziehungsweise b_S"1 gleich etwa 120 $\mu$m auf dem Flüssigkristall-Display 655 aufweisen kann. Dies ist in der Detailabbildung 14.2 dargestellt.

Die rechnersteuerbare, gerasterte UV-Lichtquelle 49 ist bevorzugt mit dem Display, insbesondere dem transmissiven Flüssigkristall-Display 655 im Pulsbetrieb im Wesentlichen zeitlich synchronisiert und mit Ansteuerung von leuchtenden Pixeln ausgebildet, die - wie schon ausgeführt - zu einem transmissiven Flüssigkristall-Display 655 optisch konjugiert sind.

Das an den Durchlassbereichen 656 und 657 diskriminierte Licht gelangt zumindest teilweise über den Polarisationsstrahlteilerwürfel 636 als im Wesentlichen parallel sich ausbreitende Teilstrahlenbündel TB1 und TB2 über das anamorphotische Objektiv 58 auf die Kamera, insbesondere eine CMOS-Kamera 55 zur Interferenz in Form zueinander im Wesentlichen geneigter Zylinderwellen mit den angedeuteten Scheitellinien 686. Dieses anamorphotische Objektiv 58 ist auch mit Zylinderkomponente ausgebildet, welches in der optischen Gesamtfunktion prinzipiell im Wesentlichen dem Objektiv 51 entspricht. Die Intensitätsverläufe der räumlichen Interferogramme rI sind in der Detailabbildung 14.3 dargestellt. Die rückwärtigen Verlängerungen der Strahlen der Teilstrahlenbündel TB1 und TB2 bilden in der scheinbaren Bildebene SBE12, welche insbesondere auch der Brennebene des Objektivs 581 mit seinem Brennpunkt F581 entspricht, die im Wesentlichen entfalteten und optisch kohärenten, scheinbaren Bildpunkte O'1s und O'2s.

Die Detailabbildung 14.4 stellt den Sachverhalt in der scheinbaren Bildebene SBE12 dar, die jedem anamorphotischen Objektiv 58 vorgeordnet ist und die Objektebene für dieses Objektiv 58 umfasst. Gezeigt werden die beiden Bilder 80'1 und 80'2 in der scheinbaren Bildebene SBE12 vom Lichtstreifen 80. Die Breiten derselben b_80'1 und b_80'2 entsprechen hier durch Lichtstreuung nicht den Breiten b_S"1 und b_S"2, die sich aus der geometrischen Abbildung der Lichtquelle 47 ergeben. Die Breiten b_80'1 und b_80'2 der Bilder vom Lichtstreifen 80 sind durch die Lichtstreuung um bis zu etwa 500 $\mu$m verbreitert. Dennoch gibt es auch in diesem Fall im Wesentlichen kein optisches Übersprechen zwischen den Teilstrahlenbündeln TB1 und TB2.

Die Detailabbildung 14.5 stellt schematisch den Zusammenhang zwischen der Lateral-Shear s und die am Rand des Mikrobolometer-Arrays 634 in der Detektionsebene DE, hier dargestellt durch eine Kamera, insbesondere durch eine CMOS-Kamera 55 für den sichtbaren Spektralbereich, sich ergebende optische Weglängendifferenz OPDr, aus welchem in besondere die spektrale Auflösung errechnet werden kann, dar. Siehe dazu auch die Beschreibung zur Detailabbildung 4.4, die auf die Verhältnisse in Figur übertragen werden kann.

Der Abstand d_ST beträgt hier beispielsweise etwa 1mm und entspricht dabei im Wesentlichen etwa 1429 Wellenlängen der größten Wellenlänge von etwa 700nm im Spektrum.

Die Fig. 15 stellt schematisch ebenfalls ein zyklisches Zweistrahl-Interferometer 607 für den sichtbaren Spektralbereich dar, jedoch hier mit zwei baugleichen Spiegelprismen 679 und 680, die an allen fünf Flächen auspoliert sind. Die Spiegelprismen 679 und 680 werden insbesondere in einem gemeinsamen Bearbeitungsgang hergestellt, wodurch die Abmessungen insbesondere im einstelligen Mikrometerbereich übereinstimmen können und auch die Abweichung der Prismenwinkel voneinander weit unterhalb einer Winkelminute liegen können. Zumindest teilweise zwischen diesen Spiegelprismen 679 und 680 ist eine Doppelspaltblende 676 mit den Spaltöffnungen 677 und 678 angeordnet, insbesondere eingekittet, welche in der Detailabbildung 15.2 dargestellt sind. Die Spaltbreiten b dieser Spaltöffnungen 677 und 678 sind hier vergleichsweise groß und betragen beispielsweise zwischen etwa 0,05mm und etwa 1 cm, insbesondere zwischen etwa 0,1mm und 0,3mm und bevorzugt etwa 0,2mm. Dadurch verringern sich insbesondere die Anforderungen an die Präzision der Justierung, jedoch verringert sich auch die laterale Auflösung, welche durch die Spaltbreite b zumindest teilweise mitbestimmt ist. Die baugleichen Spiegelprismen 679 und 680 wurden vorab unter optischer Beobachtung angeordnet, insbesondere gekittet. Dies erfolgt beispielsweise durch die Verwendung optischer Hilfsmittel insbesondere mit einem Autokollimationsfernrohr und/oder einem Autofokussensor. Die optischen Weglängen im Glas und in Luft sind nach Justierung in beiden Armen IA1 und IA2 zumindest näherungsweise gleich. Abweichungen in der Größenordnung von etwa 0,02mm sind im Wesentlichen noch tolerierbar. Hierbei ist das zyklische Zweistrahl-Interferometer 607 als Baugruppe zum vorgeordneten Objektiv 74 bevorzugt lateral und in der Tiefe ausgerichtet, damit die fokussierten Teilstrahlenbündel TB1 und TB2 die Spaltöffnungen 677 und 678 im Wesentlichen mittig bzw. zentral treffen.

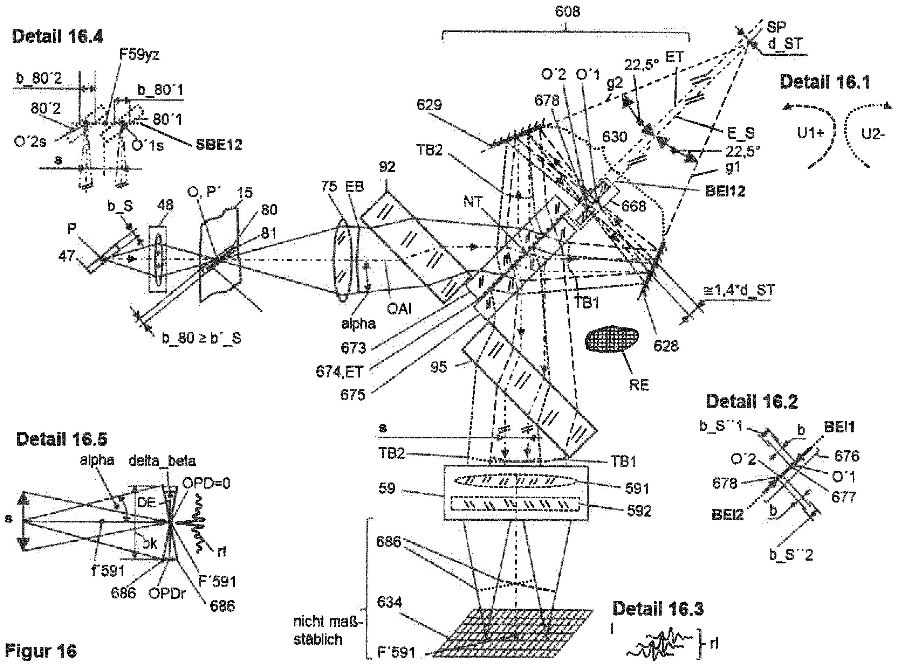

Die Fig. 16 stellt schematisch ein zyklisches Zweistrahl-Interferometer 608 dar, insbesondere für den Betrieb zumindest teilweise im MIR-Spektralbereich mit einer aktiven Beleuchtung mittels einer schmalen IR-Lichtquelle 47, insbesondere für eine streifenförmige Beleuchtung des Messobjekts 15 zur Messung in Transmission im Nahbereich. Das zyklische Zweistrahl-Interferometer 608 umfasst insbesondere zwei Planspiegel 628 und 629. Die Planspiegel 628 und 629 bilden paarweise eine Winkelspiegelanordnung, insbesondere einen Zweispiegel-Winkelreflektor 630. Insbesondere kann in diesem Beispiel ein Detektor verwendet werden, der einen gerasterten Detektor, bevorzugt einen Mikrobolometer-Array 59 umfasst.

Ein Strahlteiler, der hier bevorzugt einen Plattenstrahlteiler mit den Platten 673 und 675 und der Strahlteilerschicht 674 umfasst, kann einen Astigmatismus und Koma erzeugen. Diese Aberrationen werden insbesondere durch eine Platte 92 bevorzugt mit einer Dicke, die der Summe der Dicken der (gleich dicken) Platten 673 und 674 entspricht, kompensiert hinsichtlich eines Öffnungsfehlers, welcher bevorzugt bereits in das vorgeordnete Objektiv 74 einkorrigiert werden kann. So kann ein im Wesentlichen scharfes Bild vom Messobjekt 15 in der Bildebene E_BF entstehen, wo sich die Doppelspaltblende 676 befindet und jede Spaltöffnung 677 und 678 einen Bildfeld-Diskriminator bildet.

Abbildungsfehler, insbesondere wie Astigmatismus und Koma der Platten 673 und 675 können bevorzugt am Ausgang des Interferometers 608, insbesondere durch eine Platte 95 mit einer Dicke, die der Summe der Dicken der gleich dicken Platten 673 und 675 entspricht, kompensiert hinsichtlich eines Öffnungsfehlers, welcher bevorzugt bereits in das nachgeordnete anamorphotische Objektiv 59 einkorrigiert werden kann, wobei das nachgeordnete anamorphotische Objektiv bevorzugt neben der Öffnungsfehler-Korrektur auch eine Feld-Korrektur aufweisen kann. In der Fig. 16 wurde auf die Darstellung der entfalteten scheinbaren Bildpunkte O'1s und O'2s verzichtet.

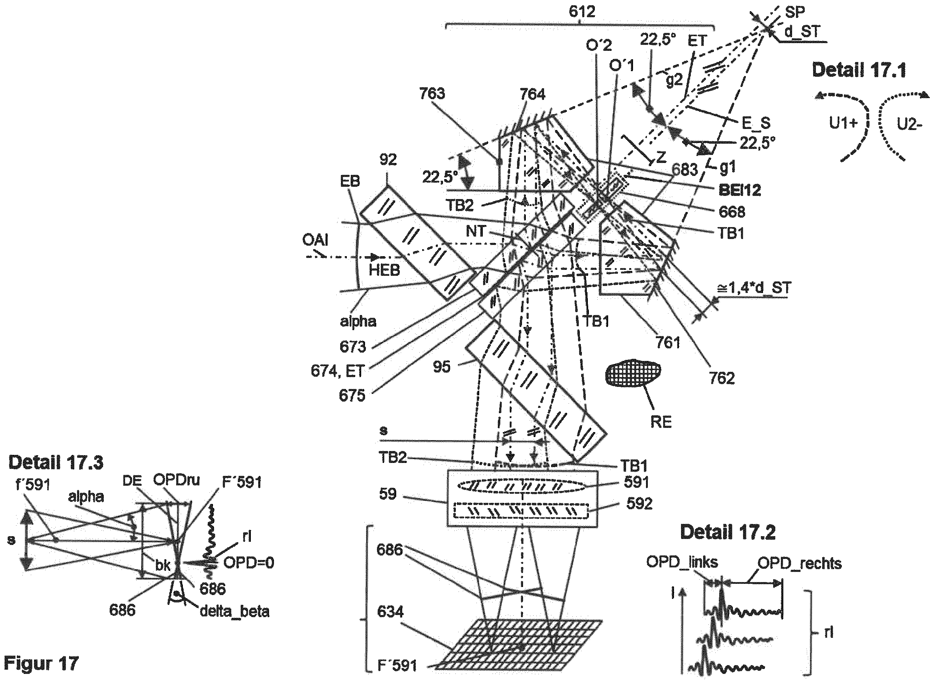

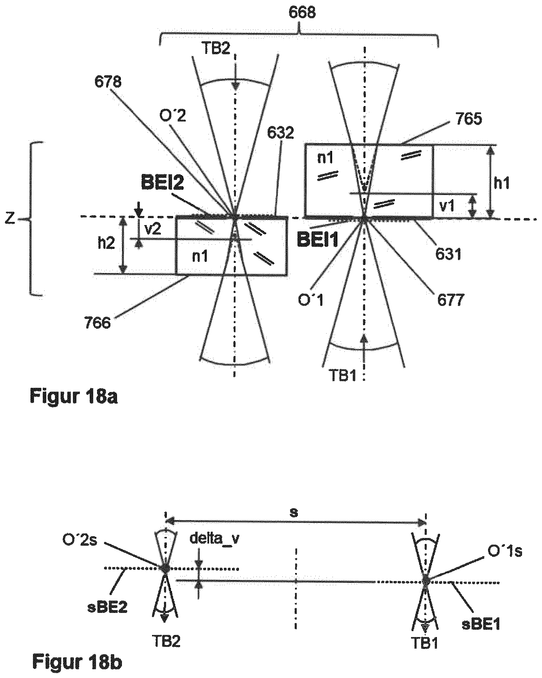

Die Anordnung in Fig. 17, welche für den mittelinfraroten Spektralbereich ausgelegt ist, entspricht zumindest teilweise den Elementen der in der Fig. 16 dargestellten Ausführungsform. Jedoch ist hier eine Doppel-Spiegelprismen-Anordnung 683 im zyklischen Zweistrahl-Interferometer 612 mit zwei Spiegelprismen 761 und 763 insbesondere aus CaF2, angeordnet, welche somit für den mittelinfraroten Spektralbereich ausgelegt ist. Des Weiteren ist bei der Anordnung bzw. Ausführungsform der Fig. 17 z.B. eine Planplattengruppe 668 insbesondere zur Einstellung und/oder Beeinflussung der optischen Weglängendifferenz OPD_zykaP in den Strahlengang des zyklischen Zweistrahl-Interferometers 612 im Bereich Z eingefügt, um die optische Weglängendifferenz vergrößern zu können. Diese Planplattengruppe 668 ist beispielsweise auch in Fig. 18a dargestellt, wobei die Planplattengruppe 668 insbesondere zwei Planparallelplatten 765 und 766 umfasst, die beide jeweils ZnSe umfassen.

Die Planplattengruppe 668 zur Einstellung und/oder Beeinflussung der optischen Weglängendifferenz OPD_zykaP kann der Erzeugung asymmetrischer räumlicher Interferogramme rI auf einem Detektor, insbesondere einem Mikrobolometer-Array 634 dienen. Dies zeigt z.B. auch die Detailabbildung 17.2 mit der maximalen optischen Weglängendifferenz OPD_links auf der linken Seite des räumlichen Interferogramms rI und der maximalen optischen Weglängendifferenz OPD_rechts auf der rechten Seite des räumlichen Interferogramms rI. Etwas einseitig auf dem Mikrobolometer-Array 634 ausgebildete räumliche Interferogramme rI (siehe dazu Detailabbildung 17.3) ermöglichen zumindest teilweise eine etwas höhere spektrale Auflösung aufgrund der nun größeren optischen Weglängendifferenz OPDru am Rand des Detektors, der ein Mikrobolometer-Array 634 umfassen kann. Dabei ist jedoch zu beachten, dass aufgrund der Wirkung der Planplattengruppe 668 zur Einstellung und/oder Beeinflussung der optischen Weglängendifferenz OPD_zykaP die scheinbaren Bildpunkte O'1s und O'2s nicht exakt in einer gemeinsamen Ebene liegen, es Abweichungen von mehreren Zehntelmillimetern gibt und es deswegen also insbesondere keine gemeinsame scheinbare Bildebene gibt. Dies ist unter Umständen in Grenzen tolerabel, wenn die Bildlagendifferenz in der Tiefe delta v deutlich unterhalb der wellenoptischen Schärfentiefe für die kürzeste Wellenlänge in zumindest einem Teil des genutzten Spektrums ist. Bei größeren Bildlagendifferenzen, also beispielsweise deutlich oberhalb von 100 Mikrometern, entstehen merkliche und störende Nichtlinearitäten in den räumlichen Interferogrammen rl.

OPD_zykaP stellt insbesondere eine zusätzliche optische Weglängendifferenz dar, die sich insbesondere durch Einfügen von mindestens je einer Planparallelplatte in jedes Teilstrahlbündel TB1 und TB2 im Bereich Z in einem zyklischen Zweistrahl-Interferometer ergibt, wo sich die Räume der Teilstrahlbündel im Wesentlichen nicht überdecken.

Fig. 18a ist eine schematische Darstellung der optischen Wirkung der Planplattengruppe 668 zur Einstellung und/oder Beeinflussung der optischen Weglängendifferenz OPD_zykaP. Diese Planplattengruppe 668 ist hier insbesondere für den Betrieb in zumindest einem Teilbereich des MIR-Spektralbereichs ausgebildet und hat zwei im Wesentlichen unterschiedlich dicke, freiliegende und für den MIR-Spektralbereich zumindest teilweise transparente Planparallelplatten 765 und 766 mit den beispielhaften Dicken zwischen jeweils etwa 0,2mm und 5mm, insbesondere zwischen etwa 0,7mm und 2mm, bevorzugt von etwa h1=1mm und h2=0,9mm. Die Planparallelplatten 765 und 766 weisen bevorzugt zumindest teilweise das jeweils gleiche optische Material auf, und zwar insbesondere Zinkselenid (ZnSe), mit dem Brechungsindex von etwa n1=2,43 bei einer Wellenlänge von etwa 5 $\mu$m. Jeder Planparallelplatte 765 und 766 umfasst eine Spaltblende 631 und 632 und/oder ist einer eine Spaltblende 631 und 632 zugeordnet. Die Planparallelplatten 765 und 766 liegen im Wesentlichen in unterschiedlichen Tiefen im Strahlengang. Diese Planplattengruppe 668 dient der gezielten Erzeugung einer zusätzlichen optischen Weglängendifferenz OPD_zykaP im zyklischen Zweistrahl-Interferometer.

Für die optische Weglängendifferenz OPD_zykaP gilt insbesondere die Gleichung (9):

$$OPD\_zykaP = Betrag[n1*(h1-h2)]. \qquad\qquad Gleichung\ (9)$$

Die jeweiligen Dicken der Planparallelplatten 765 und 766 betragen hier insbesondere etwa 0,2mm bis 5mm, insbesondere etwa 0,7mm bis 2mm, bevorzugt beträgt die Dicke einer Planparallelplatte 765 etwa h1=1mm und die Dicke der Planparallelplatte 766 etwa h2=0,9mm und der Brechungsindex n1 von ZnSe ist mit etwa nl=2,43 bei einer Wellenlänge von etwa 5 $\mu$m gegeben, woraus mit Gleichung (9) hier ein Betrag für OPD_zykaP mit etwa 243 $\mu$m folgt. Dies ergibt eine bevorzugte laterale Verschiebung der räumlichen Interferogramme rl auf dem Chip eines räumlichen Detektors, beispielsweise auf dem Mikrobolometer-Array 634. Die Bildlagendifferenz in der Tiefe delta_v ergibt sich hier insbesondere aus der Gleichung (10)

$$delta\_v= (h1-h2)*(n1-1)/n1 \qquad\qquad Gleichung\ (10)$$

zu näherungsweise etwa 59 $\mu$m. Dieser Betrag erzeugt bei einem Spektrum oberhalb von etwa 5 $\mu$m und einer numerischen Apertur einer nachgeordneten anamorphotischen Abbildungsstufe, die insbesondere kleiner ist als etwa 1, bevorzugt kleiner als etwa 0,5 und besonders bevorzugt etwa 0,2 und noch keine merklichen Nichtlinearitäten in den räumlichen Interferogrammen rl aufweist. Die Anordnung in Fig. 18a bewirkt insbesondere eine an sich unerwünschte Bildverschiebung in der Tiefe delta_v. Diese ist jedoch grundsätzlich nicht mit zwei Planparallelplatten mit einem gleichen Brechungsindex n1 vollständig zu kompensieren.

Mit den beiden Planparallelplatten 765 und 766 in Fig. 18a kann keine vollständige Kompensation der Bildverschiebungen in der Tiefe erreicht werden. Fig. 18b zeigt, dass eine Bildlagendifferenz in der Tiefe delta_v stets bestehen bleibt. Somit fallen die scheinbare Bildebene SBE1 und SBE2 nicht zusammen und es gibt es keine gemeinsame scheinbare Bildebene SBE12.

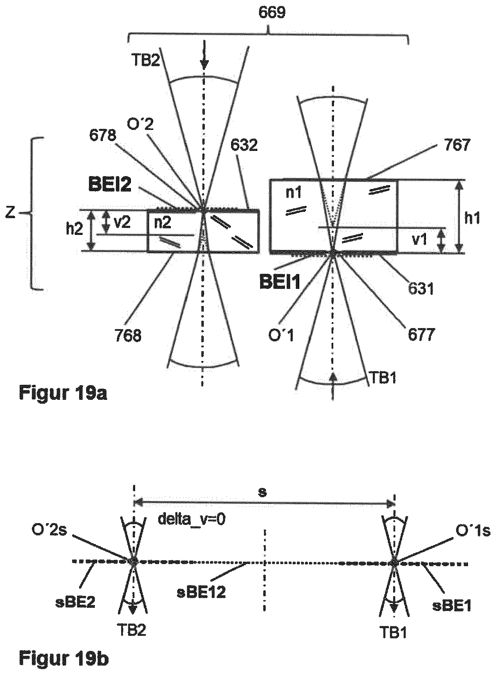

Die Fig. 19a stellt schematisch dagegen eine Planplattengruppe 669 dar, insbesondere zur gezielten Beeinflussung der optischen Weglängendifferenz OPD zykaP, die eine im Wesentlichen vollständige Kompensation der Bildverschiebungen in der Tiefe bewirken kann. Auch hier ist zumindest ein Teil des MIR-Bereichs adressiert. Die Planplattengruppe 669 umfasst hier zwei im Wesentlichen unterschiedlich dicke, freiliegende transparente Planparallelplatten 767 und 768 mit jeweils verschiedenen optischen Materialien mit jeweils einer Dicke h1 und dem Brechungsindex n1 für die Planparallelplatte 767 und der Dicke h2 und dem Brechungsindex n2 für die Planparallelplatten 768. Jeder Planparallelplatte 767 und 768 ist je eine Spaltblende 631 und 632 mit den Spaltöffnungen 677 und 678 zugeordnet bzw. jede Planparallelplatte 767 und 768 hat je eine Spaltblende 631 und 632 mit den Spaltöffnungen 677 und 678. Diese Spaltblenden 631 und 632 liegen insbesondere im Strahlengang im Wesentlichen in gleicher Tiefe und fallen mit den Bildebenen BEI1 beziehungsweise BEI2 zusammen. Die erste Planparallelplatte 767 umfasst hier Kalziumfluorid (CaF2) mit dem Brechungsindex von etwa n1=1,399 bei einer Wellenlänge von etwa 5$\mu$m und die zweite Planparallel-

platte 768 weist Zinkselenid (ZnSe) auf mit dem Brechungsindex von etwa n2=2,43 im Wesentlichen bei derselben Wellenlänge. Die Darstellung ist hier nicht (zwingend) maßstabsgetreu. Die Differenzhöhe hl-h2 der beiden Planparallelplatten 767 und 768 und die genannten Brechungsindizes n1 und n2 der optischen Materialien der Planparallelplatten 767 und 768 bestimmen über die sich daraus ergebende OPD_zykaP die laterale Verschiebung des räumlichen Interferogramms rI auf dem Mikrobolometer-Array 634, sodass sich dort bevorzugt eine asymmetrische Lage der räumlichen Interferogramme rI ergeben kann.

Mit der Planplattengruppe 669 in Fig. 19a kann eine im Wesentlichen vollständige Kompensation der Bildverschiebungen in der Tiefe erreicht werden, insbesondere wenn die Dicke h2 bei gegebener Dicke h1 und gegebenen Brechungsindizes n1 und n2 aus der bereits genannten Gleichung (2) nach Umstellung derselben zur Gleichung (11)

$$h2 = h1 * [(n1 - 1) * n2] / [(n2 - 1) * n1] \qquad \text{Gleichung (11)}$$

bestimmt wird.

Mit einer Plattendicke von etwa h1=1mm und den genannten Brechungsindizes n1 und n2 folgt aus dieser Gleichung (11) eine Plattendicke von etwa h2= 0,4846mm und nach Gleichung (3) ein Wert für OPD_zykaP von etwa 220 $\mu$m. So können die von einem Messpunkt O erzeugten kohärenten Bildpunkte O'1s und O'2s durch eine entsprechende Fokuslagen-Variation eines vorgeordneten Objektivs, welches hier nicht dargestellt ist, schließlich in eine gemeinsame scheinbare Bildebene SBE12 gebracht werden. Dies ist in Fig. 19b dargestellt.

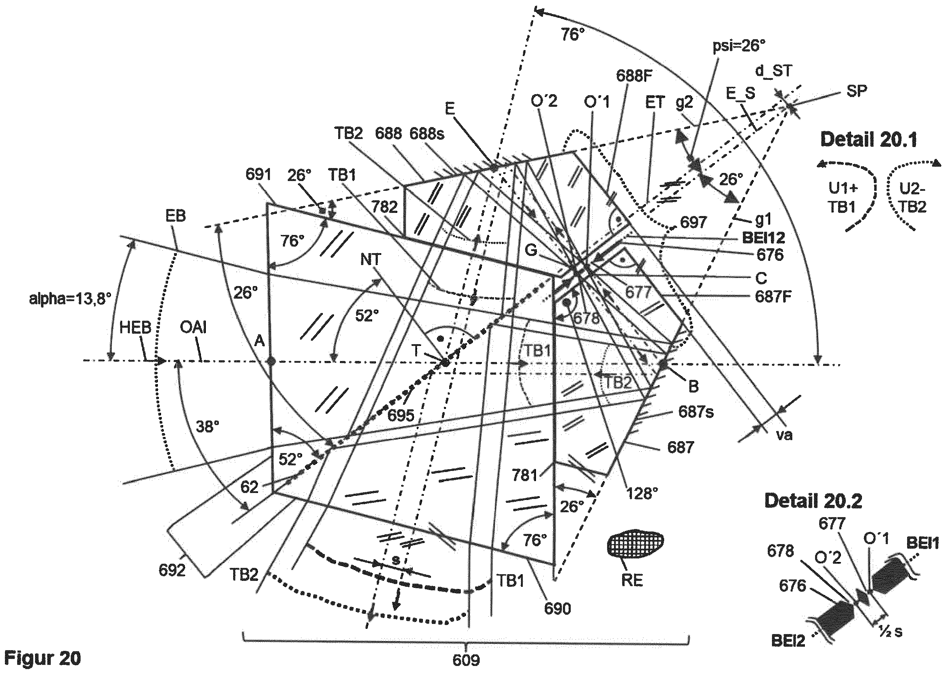

In den Fig. 20 bis Fig. 27 sind beispielhafte zyklische Zweistrahl-Interferometer 609, 610, 611, 613 und 614 schematisch dargestellt, bei denen die Hauptstrahlen der Teilstrahlenbündel TB1 und TB2 nach der Strahlteilung jeweils einen kleineren Winkel als etwa 90 Altgrad, hier etwa 76 Altgrad, zueinander aufweisen. Dies dient insbesondere einer bevorzugten Verkürzung der optischen Weglänge OPL im zyklischen Zweistrahl-Interferometer im Vergleich zu einer Anordnung mit etwa 90 Altgrad zwischen den Hauptstrahlen der Teilstrahlenbündel TB1 und TB2. So kann grundsätzlich ein etwas größerer Aperturwinkel im refraktiven Material erreicht werden. In allen Darstellungen der Fig. 20 bis Fig. 27 wurde hierbei auf die Darstellung der scheinbaren Bildebene SBE12 aus Gründen der Übersichtlichkeit verzichtet.

Die Fig. 20 stellt schematisch ein zyklisches Zweistrahl-Interferometer 609 dar, welches vier verbundene bzw. aneinander angeordnete, insbesondere verkittete Prismen 690, 691 sowie 687 und 688 aufweist. Die beiden Dreieckprismen 690 und 691 sowie die beiden Spiegelprismen 687 und 688 sind im Wesentlichen jeweils baugleich, da diese bevorzugt jeweils paarweise in einem Fertigungsgang hergestellt sein können. Die Spiegelprismen 687 und 688 weisen jeweils den Winkel psi zwischen der Spiegelfläche 687s beziehungsweise der Spiegelfläche 688s und einer unverspiegelten Fläche auf. Es ist zwischen den beiden Spiegelprismen 687 und 688 ein Luftspalt in Rachenform gebildet, in welchem die Doppelspaltblende 676 bevorzugt fest in der gemeinsamen Bildebene BEI12 angeordnet ist. Bevorzugt ist bei jedem der vier Prismen 687, 688, 690 und 691 ist der Strahl-Eintritt für den Hauptstrahl zumindest näherungsweise senkrecht. Die Anordnung basiert hier insbesondere auf einem relativ spitzen Halbwinkel psi von etwa 26 Altgrad, aus dem sich alle relevanten Winkel der vier Prismen 690, 691 sowie 687 und 688 ergeben, wenn an allen Flächen ein im Wesentlichen senkrechter Strahl-Ein- und Strahl-Austritt der Hauptstrahlen erfolgen soll.

Das Spiegelprisma 687 kann bevorzugt bei der Montage unter optischer Beobachtung als letztes Spiegelprisma angeordnet bzw. angekittet werden. Die Lateral-Shear s ist somit insbesondere durch feines Verschieben des Spiegelprisma 687 einmalig einstellbar und kann auf die Doppelspaltblende 676 abgestimmt werden, die dann ebenfalls einmalig fixiert wird, siehe dazu auch Fig. 22. Der Sollwert für den Abstand a der Spaltöffnungen der Doppelspaltblende 676 beträgt etwa die halbe Lateral-Shear s. Die tiefschwarze Doppelspaltblende 676 ist in der Detailabbildung 20.2 dargestellt und umfasst hier insbesondere nanostrukturierte Oberflächen zur Reflexunterdrückung.

In einer bevorzugten Präzisionsfertigung kann nach der Kittung der vier Prismen 687, 688, 690 und 691 des zyklischen Zweistrahl-Interferometers 609 mit dem Winkel psi von 26 Altgrad aus der Ortsfrequenz des Interferenzmusters bei einer Referenzwellenlänge, welche auf einem Chip in der Brennebene eines nachgeordneten Messobjektivs gemessen werden kann, der bevorzugte bzw. geeignete und/oder benötigte Mittenabstand der Spaltöffnungen einer Doppelspaltblende 676 von einem Fachmann errechnet werden. Die Präzisionsfertigung derselben kann rechnersteuert mittels eines 3D-Drucks erfolgen.

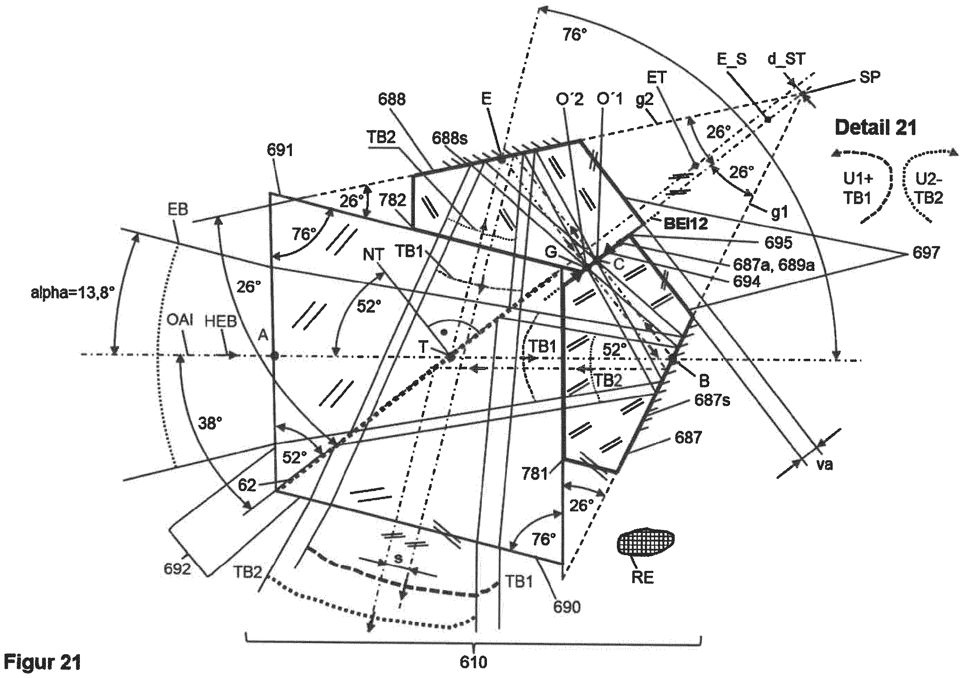

Die Fig. 21 stellt schematisch ein weiteres zyklisches Zweistrahl-Interferometer 610 dar, welches vier Prismen 687, 688, 690 und 691 aufweist, von denen die beiden Dreieckprismen 690 und 691 und die beiden Spiegelprismen 687 und 688 bevorzugt und im Wesentlichen baugleich sind. Es besteht hier insbesondere kein Luftspalt. Auf dem Spiegelprisma 688 kann bevorzugt eine dünne, tiefschwarze Doppelspaltblende 694 mit nur wenigen Mikrometern Dicke in der gemeinsamen Bildebene BEI12 aufgebracht und eingekittet sein. Diese Doppelspaltblende 694 ist hier mit vergleichsweise breiten Spaltöffnungen in der Größe zwischen etwa 0,05mm und 0,8mm, insbesondere zwischen etwa 0,1 und 0,3 und bevorzugt von etwa b=0,2mm ausgebildet.

In der Fig. 22 sind die Winkelbeziehungen für das zyklische Zweistrahl-Interferometer 609 und 610 allgemein dargestellt, die sich aus einem vorgegebenen Halbwinkel psi ableiten lassen, um für die Hauptstrahlen HTB1 und HTB2 der beiden Teilstrahlenbündel TB1 und TB2 in der Regel einen im Wesentlichen senkrechten Strahl-Ein- und Strahl-Austritt zu erreichen. Der Winkel psi an den beiden Spiegelprismen 687 und 688 besteht hier jeweils zwischen einer im Wesentlichen unverspiegelten und der im Wesentlichen verspiegelten Fläche 687s beziehungsweise der im Wesentlichen verspiegelten Fläche 688s. Der Winkel psi liegt hier also zumindest näherungsweise zweimal an jedem der beiden Spiegelprismen 687 und 688. Der Winkel 2rho mit etwa rho=90°-psi ist der stumpfe Winkel jeweils zwischen den beiden im interferometrischen Strahlengang genutzten und im Wesentlichen unverspiegelten Flächen der Spiegelprismen 687 und 688. Der an der Strahlteilerfläche anliegende Winkel der beiden Dreieckprismen 690 und 691 mit dreieckförmiger Grundfläche weist jeweils den Winkel 2psi auf.

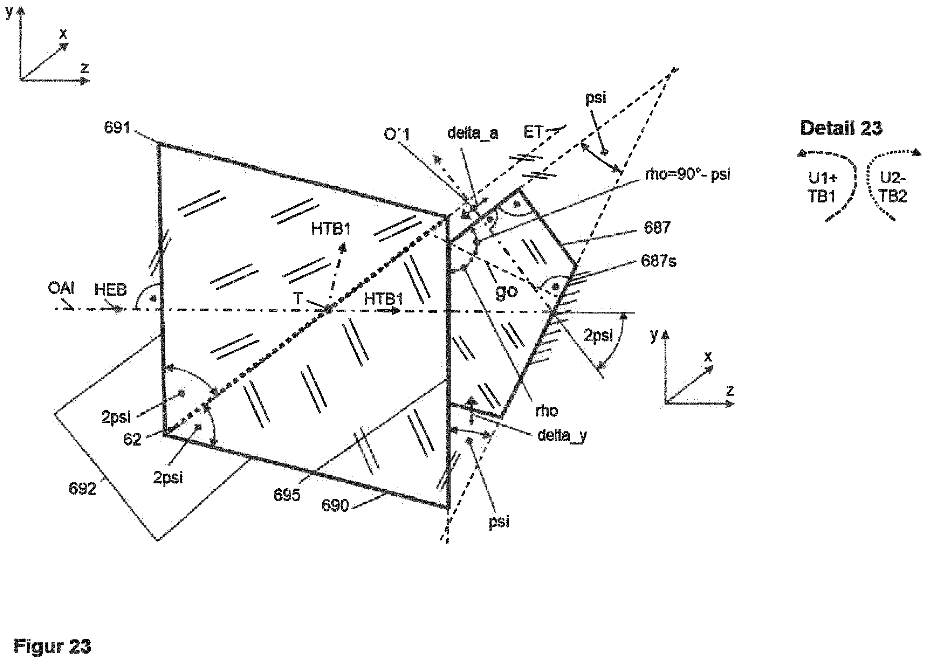

Die Fig. 23 stellt schematisch den Effekt einer parallelen Verschiebung des Spiegelprismas 687 dar, insbesondere bei einer einmaligen Justierung z.B. beim Kitten unter Beobachtung. Bei der parallelen Verschiebung ändern sich die optischen Weglängen aufgrund des senkrechten Strahl-Eintritts und Strahl-Austritts nicht. Jedoch gibt es eine Verschiebung delta_a des Hauptstrahls im Wesentlichen parallel zur Strahlteilerebene ET. Zum einen garantiert dies den im Wesentlichen senkrechten Strahl-Eintritt und -austritt der Hauptstrahlen HTB1 und HTB2 der Teilstrahlenbündel TB1 und TB2. Andererseits kann dies das Justieren hinsichtlich der Lage der Bildfeld-Diskriminatoren in Bezug auf den Bildpunkt O'1 des Messobjekts vereinfachen, da nicht am vorgeordneten Objektiv bei jedem Justierschritt im Kitt nachfokussiert werden muss. Die Winkelhalbierende des stumpfen Winkels 2rho ist im Wesentlichen parallel zur Lotgeraden go der gegenüberliegenden, im Wesentlichen verspiegelten Fläche des Spiegelprismas 687. Der Winkel der Strahlablenkung weist am Spiegelprisma 687 den Betrag 2psi auf, was auch für das baugleiche Spiegelprisma 688 gilt.

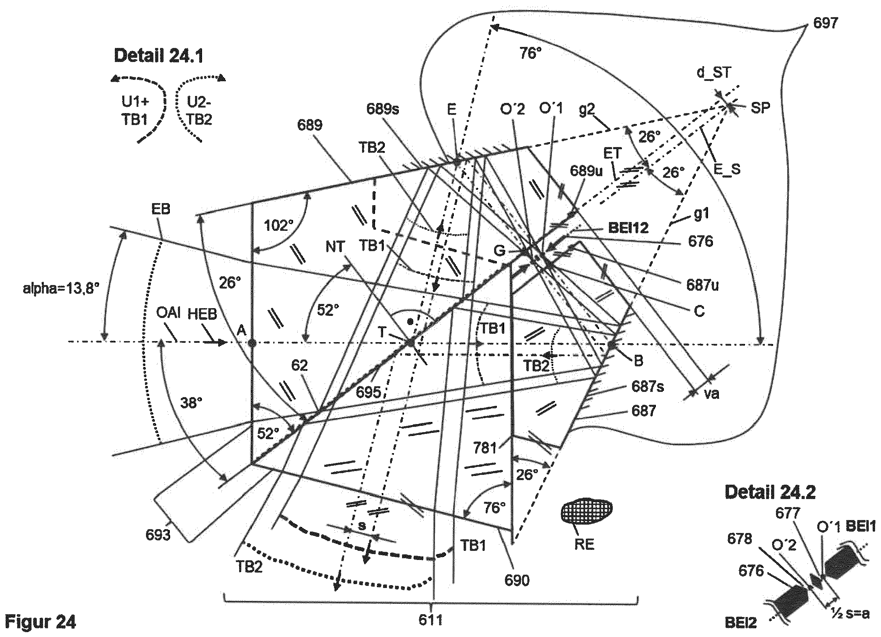

Die Fig. 24 stellt schematisch ein zyklisches Zweistrahl-Interferometer 611 dar, welches im Wesentlichen drei aneinander angeordnete, insbesondere zusammengekittete Komponenten umfasst, und zwar ein Dreieckprisma 690 und zwei Spiegelprismen 689 und 687. Davon weisen die beiden Spiegelprismen 689 und 687 den insbesondere im Wesentlichen den gleichen Winkel psi von etwa 10° bis etwa 50°, insbesondere von etwa 20° bis etwa 30° und bevorzugt von etwa 26° auf. Die Lateral-Shear s ist nicht variabel einstellbar, was einen Vorteil hinsichtlich der Robustheit der Anordnung darstellen kann. Es sind hier die sich ergebenden Winkel am zyklisches Zweistrahl-Interferometer 611 angegeben, wenn beispielsweise psi=26 Altgrad in etwa beträgt. Der Winkel der Strahlablenkung weist an den beiden Spiegelprismen 689 und 687 insbesondere zum Beispiel jeweils den Betrag 2psi =52 Altgrad in etwa auf.

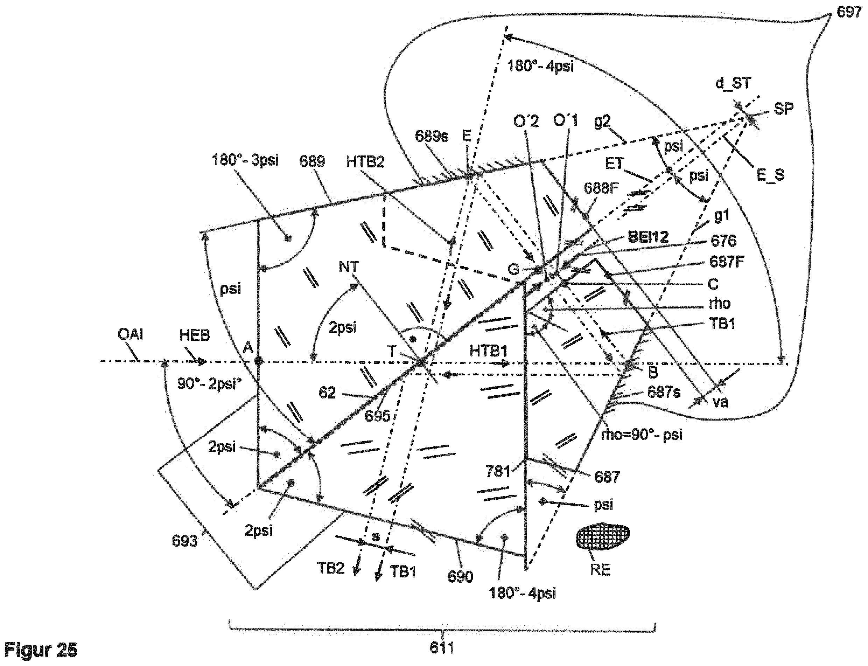

In der Fig. 25 sind die Winkelbeziehungen, die sich aus dem im Wesentlichen spitzen Halbwinkel psi ergeben, für eine Anordnung mit drei zusammengekitteten Komponenten, nämlich einem Dreieckprisma 690 und den beiden Spiegelprismen 689 und 687 allgemein dargestellt, um auch hier für die Hauptstrahlen HTB1 und HTB2 der beiden Teilstrahlenbündel TB1 und TB2 in der Regel einen senkrechten Strahl-Ein- und Strahl-Austritt zu erreichen.

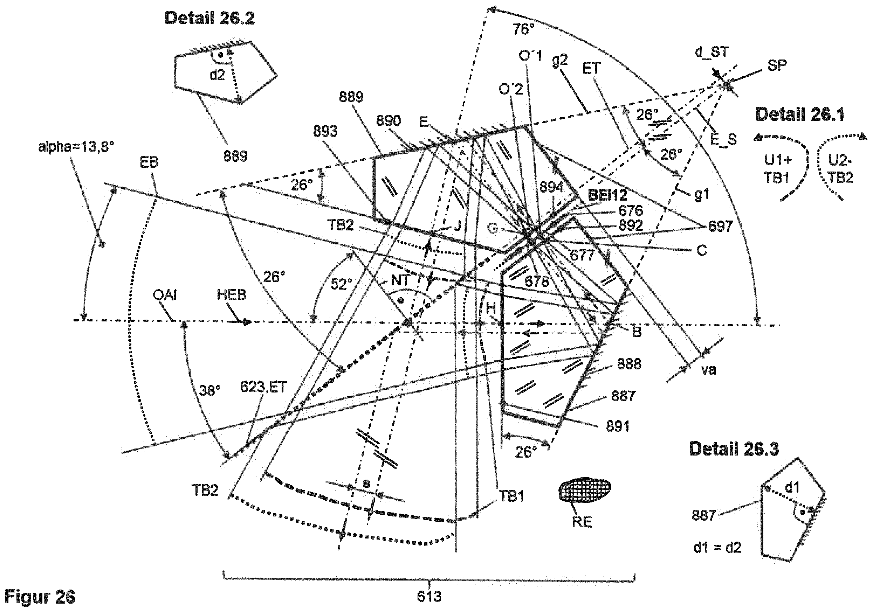

Die Fig. 26 stellt schematisch ein zyklisches Zweistrahl-Interferometer 613 dar, welches Spiegelprismen 887 und 889 aufweist, die jeweils Zinkselenid aufweisen können. Die Transmissionsflächen sind entspiegelt. Zur Strahlteilung wird ein Strahlteiler 623 aus Mylarfolie eingesetzt. Die Detailabbildungen 26.2 und 26.3 zeigen die Spiegelprismen 887 und 889, deren Höhen d1 und d2 möglichst gleichgemacht sind. Im Beispiel der Fig. 26 wird die benötigte Doppelspaltblende 676 in der gemeinsamen Bildebene BEI12 nach der Montage der Anordnung hinsichtlich des benötigten Mittenabstands der Spaltöffnungen a der Spaltöffnungen maßgefertigt.

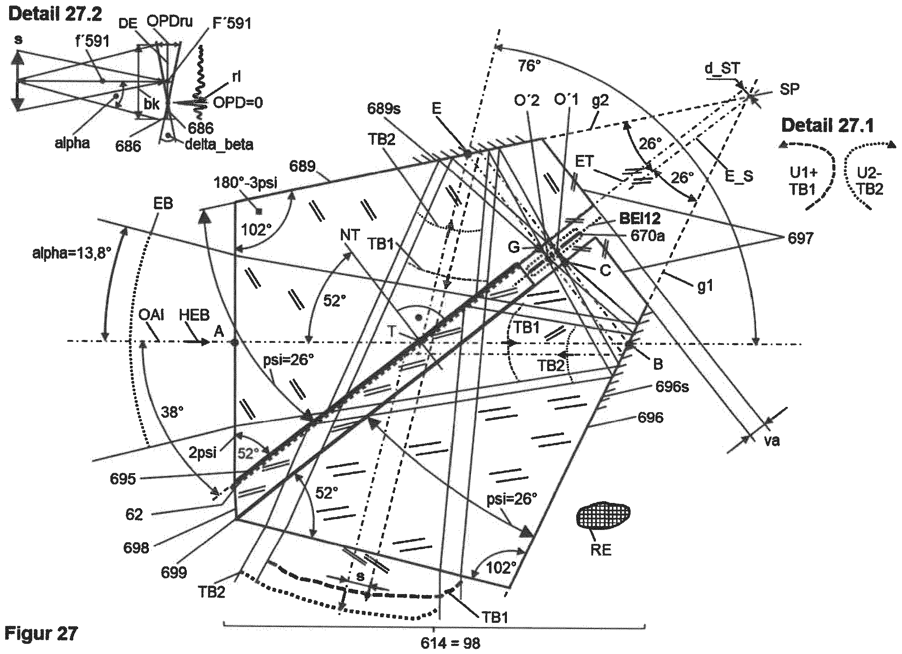

Die Fig. 27 stellt schematisch ein zyklisches Zweistrahl-Interferometer 614 dar, insbesondere für den Betrieb zumindest teilweise im VIS- und/oder im NIR-Bereich. Auf die Darstellung des vorgeordneten Objektivs und eines nachgeordneten anamorphotischen Objektivs wurde hier verzichtet. Dieses zyklische Zweistrahl-Interferometer 614 umfasst zwei winkel- und baugleiche Spiegelprismen 689 und 696, welche die 697 bilden, sowie eine in dem Zweistrahl-Interferometer 614 angeordnete, insbesondere darin eingekittete Planparallelplatte 698 beispielsweise umfassend die Dicke von etwa 2mm, auf bzw. an welcher die Strahlteilerschicht 62 angeordnet ist. Diese Spiegelprismen 689 und 696 umfassen jeweils einen ersten spitzen Winkel psi, einen zweiten spitzen Winkel 2psi und einen dritten Winkel mit dem Betrag 180Altgrad minus 3psi auf und so stimmen insbesondere mindestens drei Winkel an den beiden Spiegelprismen jeweils im Wesentlichen überein. Die Spiegelprismen 689 und 696 weisen bevorzugt einen spitzen Winkel von etwa psi=26 Altgrad auf.

Der Abstand d_ST zwischen dem Schnittpunkt SP von der Strahlteilerebene ET erzeugt eine Lateral-Shear s und beträgt beispielsweise in etwa 2mm. Der Abstand d_ST kann im Allgemeinen zwischen etwa 0,1 mm und etwa 5 mm, insbesondere zwischen etwa 1 mm und etwa 3 mm und bevorzugt zwischen etwa 1,5 mm und etwa 2,5 mm liegen. In dem Spalt, der Luft im Zwischenraum umfasst, insbesondere einen Luftspalt darstellend, ist eine Planplattengruppe 670a zur Einstellung der optischen Weglängendifferenz OPD_zykaP mit einer im Wesentlichen vollständigen Kompensation der Bildverschiebung in der Tiefe angeordnet bzw. eingebracht. Dies dient insbesondere der Gewinnung von in der Detektionsebene DE verschobenen räumlichen Interferogrammen rl (siehe Detailabbildung 27.2), insbesondere um eine möglichst hohe spektrale Auflösung zu erhalten bzw. zu erzielen. Die Planplattengruppe 670a kann nach der Anordnung, beispielsweise nach dem erfolgten Kitten des zyklischen Zweistrahl-Interferometers 614 nach optischem Ausmessen desselben maßgefertigt werden.

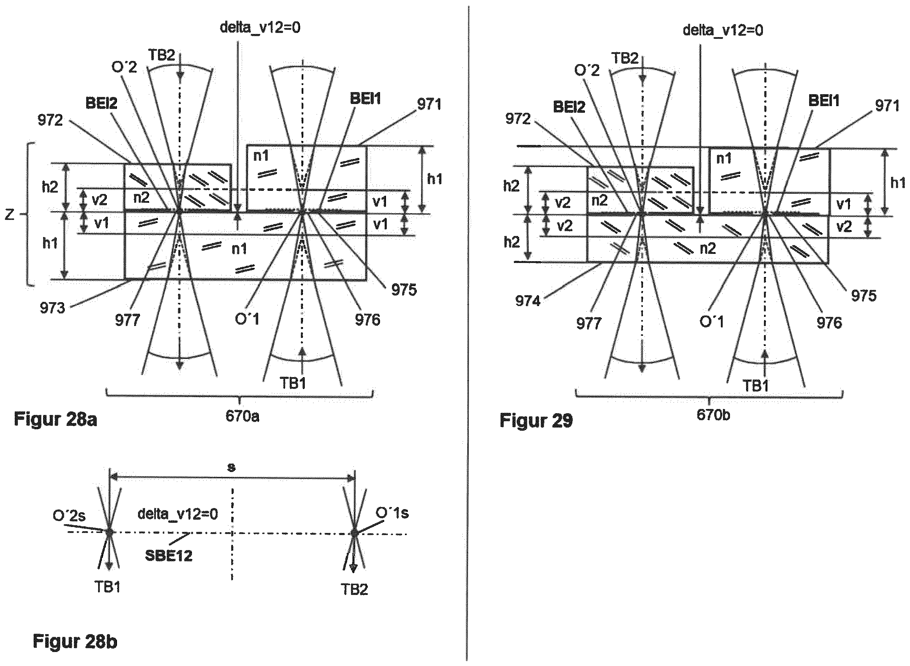

Die Fig. 28a stellt schematisch eine Planplattengruppe 670a dar, insbesondere zur Einstellung der optischen Weglängendifferenz OPD_zykaP mit einer im Wesentlichen vollständigen Kompensation der Bildverschiebung in der Tiefe zwischen den beiden Teilstrahlenbündeln TB1 und TB2. Die Planplattengruppe 670a weist insbesondere für den Betrieb in zumindest einem Teilbereich des VIS- und/oder NIR-Bereichs zumindest teilweise transparente Schichten, insbesondere transparente Kittschichten auf und umfasst zwei im Wesentlichen unterschiedlich dicke zumindest teilweise transparenten Planparallelplatten 971 und 972 umfassend verschiedene Dicken h1 und h2. Die Dicke h1 beträgt z.B. etwa 2mm. Die Dicke h1 kann im Allgemeinen zwischen etwa 0,1mm und etwa 5mm, insbesondere zwischen etwa 1mm und etwa 3mm und bevorzugt zwischen etwa 1,5mm und etwa 2,5mm liegen. Die beiden Planparallelplatten 971 und 972 umfassen bevorzugt zwei unterschiedliche Glassorten, beispielsweise Borkronglas BK7 und Schwerkrongla N-SK4 umfassend jeweils unterschiedliche Brechungsindizes von etwa n1=1,507 für BK7 und von etwa n2=1,601 für Schwerkronglas N-SK4 bei jeweils der Wellenlänge von etwa 1000nm. Diese Planparallelplatten 971 und 972 sind auf eine gemeinsame planparallele Trägerplatte 973 optisch angeordnet, insbesondere aufgekittet. Die Trägerplatte 973 weist ebenfalls den Brechungsindex n1 und die Dicke h1 auf. Es ist eine Doppelspaltblende 975 angeordnet, deren erste Spaltöffnung 976 der ersten Planparallelplatte 971 und deren zweite Spaltöffnung 977 der zweiten Planparallelplatte 972 zugeordnet ist. Diese Anordnung dient der gezielten Erzeugung einer optischen Weglängendifferenz OPD zykaP im zyklischen Zweistrahl-Interferometer. Die Höhen h1 und h2 beider Planparallelplatten 971 und 972 umfassend jeweils unterschiedliche Brechungsindizes n1 und n2 sind wesentlich für bzw. bestimmen die sich daraus ergebende Weglängendifferenz OPD_zykaP und letztlich für die laterale Verschiebung des räumlichen Interferogramms in der Detektionsebene DE, also auf der Sensorfläche eines Detektors, insbesondere eines gerasterten Detektors, sodass sich dort insbesondere eine im Wesentlichen asymmetrische Lage der räumlichen Interferogramme rI ergibt.

Die jeweilige Anordnung in den Fig. 28a und Fig. 29 bewirkt keine wesentlich unterschiedliche Bildverschiebung in der Tiefe, sodass die Differenz der Bildverschiebungen delta v im Wesentlichen null entspricht, also eine etwa vollständige Kompensation der einzelnen Bildverschiebungen erzielt werden kann. In diesem Fall ergibt sich h2 bei gegebener Dicke h1 von beispielsweise etwa 2mm und den gegebenen Brechungsindizes von etwa n1=1,507 und von etwa n2=1,601 aus der Gleichung (11) zu etwa h2=1,792mm. So können die von einem Messpunkt O erzeugten kohärenten Bildpunkte O'1 und O'2 durch eine Fokuslagen-Variation eines vorgeordneten Objektivs schließlich in eine gemeinsame scheinbare Bildebene SBE12 gebracht werden. Die optische Weglängendifferenz OPD_zykaP beträgt mit der Gleichung (3) hier beispielsweise etwa OPD_zykaP=144$\mu$m. Der bekannte und eher unerwünschte Chirping-Effekt in den räumlichen Interferogrammen rI aufgrund der etwas unterschiedlichen Dispersion in den beiden, hier kombinierten optischen Gläsern lässt sich von Fachleuten durch eine Phasenkorrektur bei der Berechnung der Spektren vergleichsweise einfach einflusslos auf das errechnete Spektrum machen.

Mit den drei Planparallelplatten 971, 972 und 973 aus Figur 28a kann eine vollständige Kompensation der Bildverschiebungen delta v in der Tiefe erreicht werden, siehe Fig. 28b. Dort ist dargestellt, dass eine Bildlagendifferenz delta_v=0 erreicht werden kann. Somit besteht eine gemeinsame scheinbare Bildebene SBE12.

Die Fig. 29 stellt schematisch eine Planplattengruppe 670b dar umfassend eine Trägerplatte 974. Diese weist beispielsweise die Dicke h2 und den oben genannten Brechungsindex n2 auf, wobei dieser bevorzugt höher als der Brechungsindex n1 ist. Dadurch kann die Planplattengruppe 670b im Vergleich zur Planplattengruppe 670a in Fig. 28a bevorzugt mit einer etwas geringeren Dicke ausgestaltet werden.

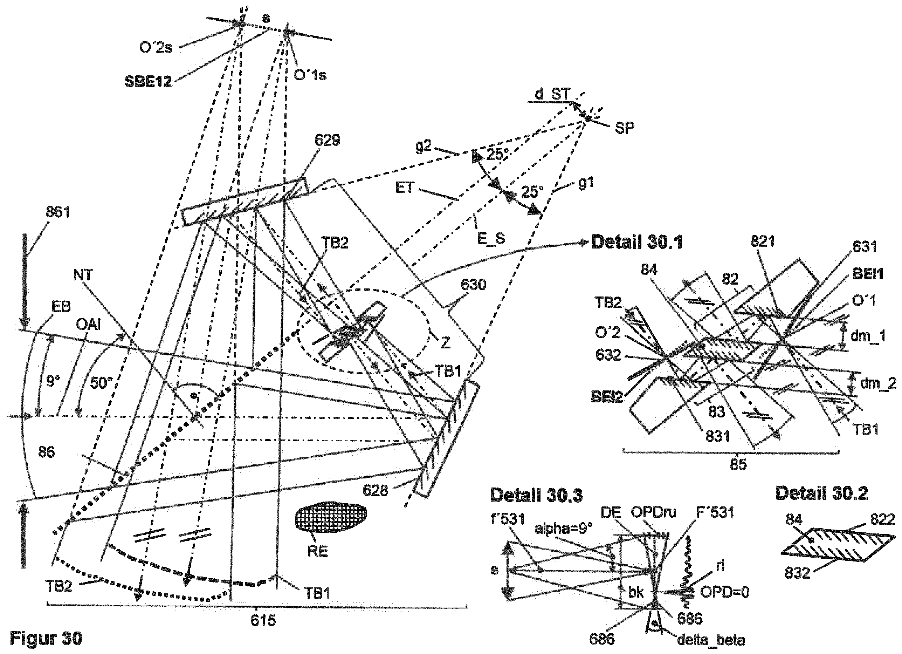

Die Fig. 30 stellt schematisch ein zyklisches Interferometer 615 dar, welches für den Betrieb zumindest teilweise im fern-infraroten Spektralbereich ausgelegt ist und welches insbesondere mit einem spitzen Halbwinkel von beispielsweise etwa 25 Altgrad ausgebildet ist. Zwischen den beiden Spaltblenden 631 und 632, dargestellt in der Detailabbildung 30.1, welche jeweils einen Bildfeld-Diskriminator BFD darstellen, ist im Bereich Z, wo sich die Teilstrahlenbündel TB1 und TB2 im Wesentlichen nicht überdecken, eine Doppelspiegeltreppe 85 angeordnet. Die Doppelspiegeltreppe 85 ist in der Detailabbildung 30.1 dargestellt und umfasst eine erste Spiegeltreppe 82 und eine zweite Spiegeltreppe 83. Diese Doppelspiegeltreppe 85 umfasst auch einen Mittelsteg 84 insbesondere umfassend ein Metall (siehe Detailabbildung 30.2), mit den beiden Planspiegeln 822 und 832, die jeweils insbesondere mittels einer Single-point-Diamantbearbeitung auf dem Mittelsteg 84 hergestellt bzw. verarbeitet sind. Der Mittelsteg 84 ist insbesondere ein Bestandteil eines Metallelementes, insbesondere eines Monolithen umfassend Kupfer. Die vier Planspiegel 821, 822, 831 und 832 der Doppelspiegeltreppe 85 sind insbesondere für den Betrieb zumindest teilweise im fern-infraroten Spektralbereich mit einer Schicht umfassend Gold, insbesondere mit einer reinen Goldschicht ausgebildet. Die Spiegelabstände dm_1 und dm_2 in der Doppelspiegeltreppe 85 sind bevorzugt mit einer Differenz von etwa 0, 02mm bis etwa 0,5mm, insbesondere von etwa 0,1mm bis etwa 0,2mm, bevorzugt von etwa 0,15mm ausgebildet bzw. gefertigt. Dadurch ergibt sich insbesondere eine zusätzliche optische Weglängendifferenz OPD _zykaS, die sich also durch Einfügen von je einer Spiegeltreppe 82 und 83 in jedes Teilstrahlenbündel TB1 und TB2 in einem zyklischen Zweistrahl-Interferometer ergibt, wo sich die Räume der Teilstrahlenbündel im Bereich Z nicht überdecken. Diese zusätzliche optische Weglängendifferenz OPD_zykS ist vorzugsweise kleiner als die wellenoptische Schärfentiefe der Teilstrahlenbündel TB1 und TB2 für die kleinste Wellenlänge im gemessenen Spektrum. Die zusätzliche optische Weglängendifferenz OPD_zykaS ergibt sich insbesondere durch einen Einfallswinkel von etwa 45 Altgrad auf die Planspiegel 821, 822, 831 und 832 und insbesondere Spiegelabständen dm_1 und dm_2 in der Doppelspiegeltreppe 85 mit einer Differenz von 0,15mm insbesondere zu etwa OPD_zykaS=0,42mm im räumlichen Interferogramm rI.

Ein räumliches Interferogramm rl ist somit in der Detektionsebene DE jeweils im Wesentlichen lateral verschoben ausgebildet und umfasst eine im Wesentlichen unsymmetrische Lage auf dem gerasterten Detektor, der hier nicht dargestellt ist. Dies führt in diesem Fall zu einer maximal erreichbaren optischen Weglängendifferenz OPDru am Rand des gerasterten Detektors in der Größenordnung von etwa 1mm, beispielsweise zwischen etwa 0,1mm und 5mm, insbesondere zwischen etwa 0,8mm und etwa 1,2mm. Mit einer derartigen Anordnung kann insbesondere auch ein im Wesentlichen ausgedehntes, selbstleuchtendes Objekt, beispielsweise eine fluoreszierende Lichtquelle untersucht werden, da hierbei durch die geometrische Ausbildung, insbesondere auch durch die Anordnung einer Vorblende 861 in Kombination mit den Spaltblenden 631 und 632, ein optisches Übersprechen zwischen den beiden Teilstrahlengängen im Interferometer 615 reduziert oder gar vermieden wird. Ein optisches Übersprechen kann durch ein ausgedehntes leuchtendes Objekt verursacht werden, dessen kohärente Bilder nach der Strahlteilung ebenfalls lateral so ausgedehnt sind, dass Bildpunkte vom ersten Teilstrahlenbündel TB1 im Bereich der Doppel-Spiegeltreppe 85 in den Strahlengang des Teilstrahlenbündels TB2 gelangen und umgekehrt. Die Vorblende 861 reduziert die Querausdehnung des einfallenden Strahlenbündels EB.

Ein zyklisches Interferometer 615 mit einem im Wesentlichen spitzen Halbwinkel von beispielsweise etwa 25 Altgrad und mit einer Doppelspiegeltreppe 85 umfassend einen Mittelsteg 84 lässt im Vergleich mit zwei separaten Spiegeltreppen einen besonders großen Aperturwinkel alpha von typischerweise bis zu etwa 5 Altgrad, insbesondere bis zu etwa 7 Altgrad und bevorzugt bis zu etwa 10 Altgrad zu, da die beiden Planspiegel 822 und 832 auf dem Mittelsteg 84 räumlich besonders dicht beieinander angeordnet sein können. Die Detailabbildung 30.2 stellt schematisch den vergleichsweise dünnen Mittelsteg 84 dar umfassend die beiden Planspiegelflächen 822 und 832. Außerdem ermöglicht die präzise Fertigung der Doppelspiegeltreppe 85, welche in einem Arbeitsgang durch Single-point-Diamantbearbeitung auf einer Ultrapräzisionsmaschine hergestellt sein kann, dass die Abweichung von der Rechtwinkligkeit zu einer gemeinsamen Ebene der vier Planspiegel 821, 822, 831 und 832 der Doppelspiegeltreppe 85 im Wesentlichen gegen null geht, also beispielhaft unter 10 Winkelsekunden liegt. Eine Winkelsekunde stellt ein 1/3600 eines Altgrads dar, welches wiederum ein 1/360 des Vollkreises darstellt. Diese geringe Abweichung von der Rechtwinkligkeit kann insbesondere die Funktion des zyklischen Interferometers 615 verbessern, indem die gemeinsame Ebene der vier Planspiegel 821, 822, 831 und 832 parallel zur Referenzebene RE justiert werden kann, ebenfalls mit maximalen Abweichungen von der Parallelität von beispielhaft 10 Winkelsekunden. Damit werden unerwünschte Kontrastverluste in den räumlichen Interferogrammen rl minimiert. Die Detailabbildung 30.3 stellt die laterale Verschiebung des räumlichen Interferogramms in der Detektionsebene DE auf Basis der Wirkung der Doppelspiegeltreppe 85 dar.

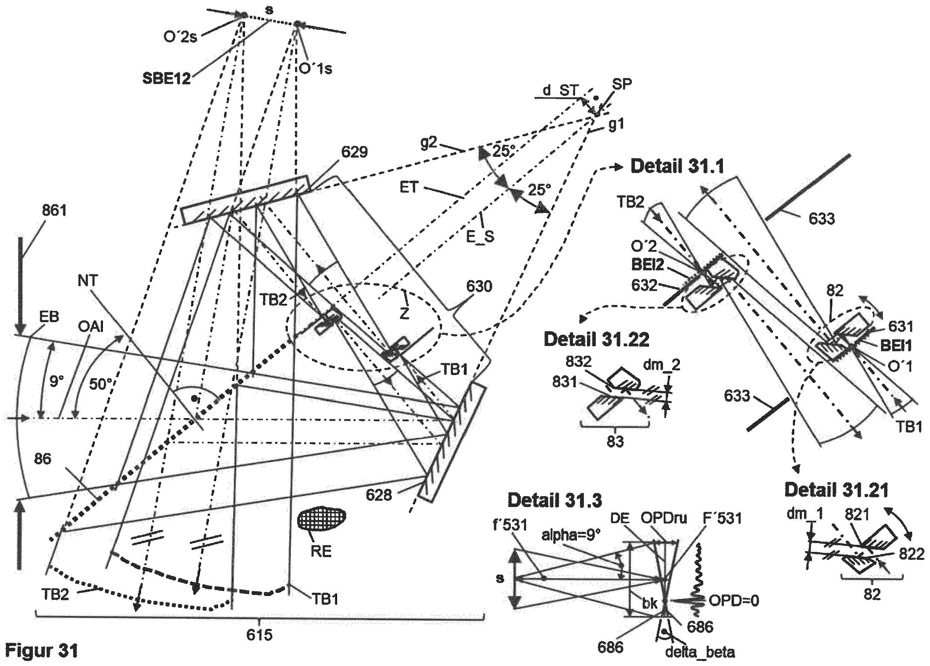

In der Fig. 31 sind zwei im Wesentlichen getrennte und in sich starre Spiegeltreppen 82 und 83 angeordnet, die zumindest teilweise in der Tiefe versetzt sind. Durch die Separierung der beiden Spiegeltreppen 82 und 83 ist auch eine Drehung einer Spiegeltreppe, hier der Spiegeltreppe 82, möglich, um eine feine Einstellung der optischen Weglängendifferenz OPD_zykaS insbesondere durch Drehen derselben zu ermöglichen. Mittels einer Anordnung nach Fig. 31 kann auch ein ausgedehntes, selbstleuchtendes Objekt untersucht werden, da praktisch kein optisches Übersprechen zwischen den Teilstrahlengängen möglich ist.

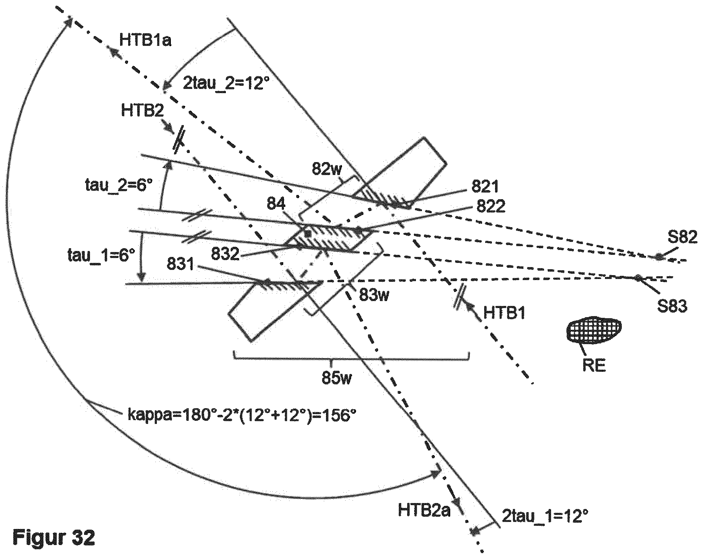

Die Fig. 32 stellt eine Doppelspiegeltreppe 85w mit einem abgeknickten Strahlengang dar. Zur besseren Anschauung wurde hier die Doppelspiegeltreppe 85w mit den Winkeln tau_1 und tau_2 von beispielsweise jeweils etwa 6 Altgrad gezeichnet. In einer üblichen Anordnung betragen diese Winkel üblicherweise jedoch relativ geringe Winkel, wie etwa 3 Altgrad. Der Winkel kappa zur Beschreibung der Strahlabknickung mit der Gleichung (1) beträgt somit etwa 180Altgrad -24°=156 Altgrad.

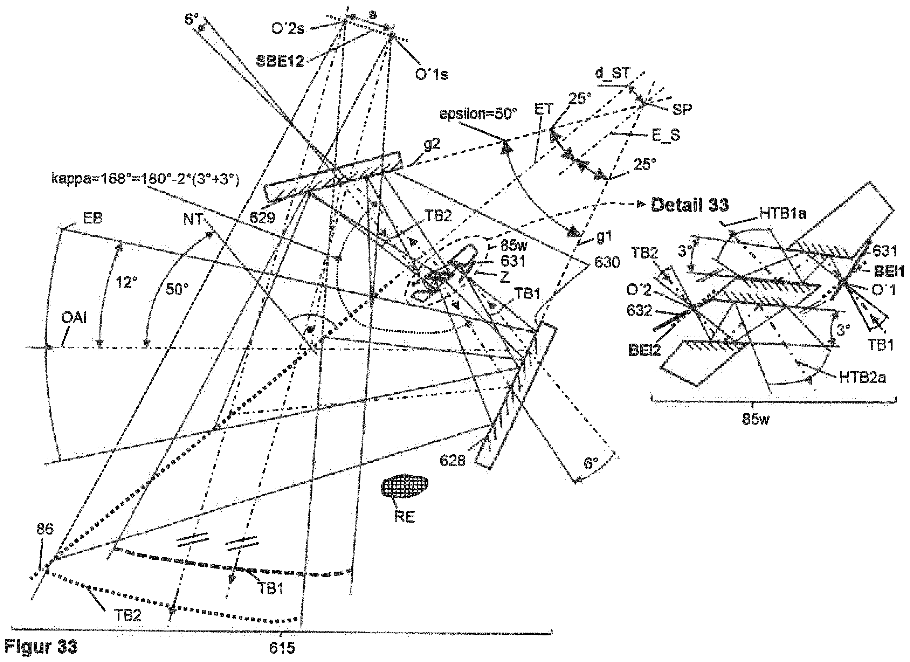

Fig. 33 stellt ein zyklisches Zweistrahl-Interferometer 615 mit einer Doppelspiegeltreppe 85w dar, die einen abgeknickten Strahlengang mit einem Ablenkwinkel von etwa 6 Altgrad aufweist. Es ist hier tau_1=tau_2=3 Altgrad dargestellt. Der Winkel kappa beträgt mit der Gleichung (1) hier etwa 168 Altgrad. Diese Abknickung der beiden Strahlengänge ermöglicht einen größeren Aperturwinkel alpha, wie beispielsweise etwa 12 Altgrad, insbesondere wenn ein Folienstrahlteiler 86 mit einer vernachlässigbaren Dicke eingesetzt wird, durch welche auch Aberrationen am Strahlteiler vermieden werden. Dieser Aperturwinkel alpha von etwa 12 Altgrad stellt einen vergleichsweise relativ großen Wert für ein zyklisches Zweistrahl-Interferometer dar und ermöglicht einen entsprechend hohen Lichtdurchsatz durch das zyklische Zweistrahl-Interferometer 615.

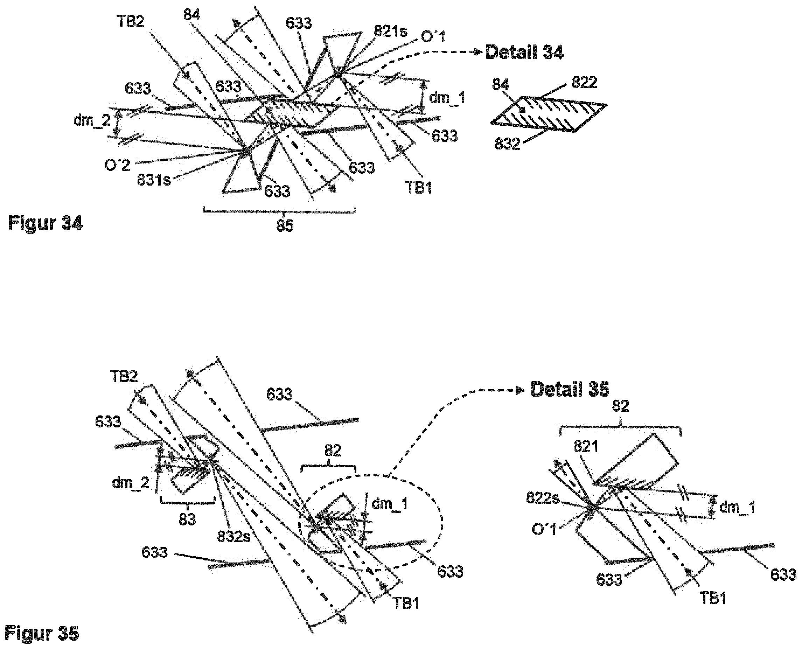

Die Fig. 34 stellt eine Doppelspiegeltreppe 85 dar, bei welchem die beiden äußeren Planspiegel 821s und 831s im Wesentlichen so schmal ausgebildet sind, dass diese selbst als Bildfeld-Diskriminatoren wirken können. Die Hilfsblenden 633 können ein optisches Übersprechen verhindern. So kann insbesondere auf Spaltblenden als Bildfeld-Diskriminatoren verzichtet werden. Die Detailabbildung 34 stellt schematisch den Mittelsteg 84 mit den beiden Planspiegeln 822 und 832 dar.

Die Fig. 35 stellt schematisch eine Anordnung mit zwei separierten Spiegeltreppen, und zwar einer ersten Spiegeltreppe 82 und einer zweiten Spiegeltreppe 83, dar. Bei dieser Anordnung sind sowohl der zweite Planspiegel 822s der ersten Spiegeltreppe 82, siehe Detailabbildung 34, als auch der zweite Planspiegel 832s der zweiten Spiegeltreppe 83 schmal ausgebildet und somit können diese jeweils selbst als Bildfeld-Diskriminatoren dienen. Die Hilfsblenden 633 können ferner ein optisches Übersprechen reduzieren und insbesondere verhindern. Die Detailabbildung 35 stellt schematisch die erste Spiegeltreppe 82 mit dem relativ schmal ausgebildeten Planspiegel 822s, der hier als Bildfeld-Diskriminator wirkt, dar. Aufgrund der großen Wellenlängen, insbesondere zwischen etwa $50\mu m$ und $1000\mu m$ im fern-infraroten Spektralbereich ist die Neigung des relativ schmal, also beispielhaft im Bereich einiger Zehntelmillimeter bis zu einigen Millimetern im unteren FIR-Bereich, ausgebildeten Planspiegels 822s im Strahlengang hier gut tolerierbar, da hier die wellenoptische Schärfentiefe vergleichsweise groß ist.

Die Fig. 36 stellt ein symmetrisch aufgebautes zyklisches Zweistrahl-Interferometers 616 dar, bei dem der Abstand d_ST des Schnittpunkts SP von der Strahlteilerebene ET etwa gleich null ist. Die Lateral-Shear s wird insbesondere durch die Doppel-Spiegeltreppe 85 erzeugt, welche in der Detailabbildung 36.1 gezeigt ist. Durch hier nicht dargestellte im Wesentlichen ungleiche Spiegelabstände der Planspiegel 821 und 822 und 831 und 832 ist die Lage der räumlichen Interferogramme rI auf dem gerasterten Detektor etwas verschoben, was in der Detailabbildung 36.3 dargestellt ist.

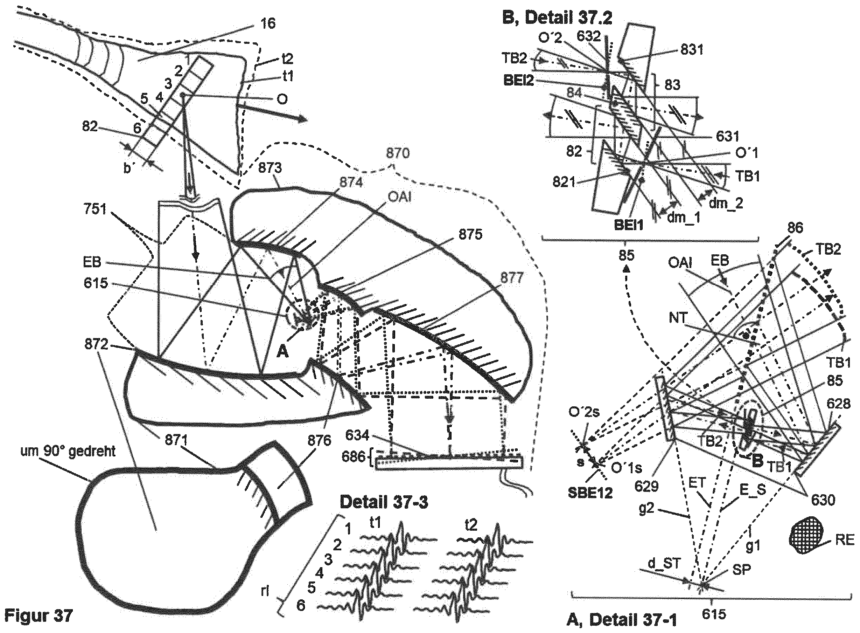

Die Fig. 37 stellt schematisch eine Abgaswolke 16, insbesondere eine strömende heiße Abgaswolke 16 oberhalb einer Fabrikanlage dar, insbesondere in einer größeren Entfernung zur Fabrikanlage, zum Zeitpunkt t1, welche sich hier von links nach rechts bewegt und dabei sowohl spektral im fern-infraroten Bereich (FIR) als auch ortsaufgelöst untersucht werden soll. Dazu wird im Spektrometersystem, das sich beispielsweise am Boden befinden kann, eine Anordnung 870 mit einer Spiegeloptik und mit einem zyklischen Zweistrahl-Interferometer 615 für die Analyse der heißen Abgaswolke 16 eingesetzt. Diese Anordnung 870 weist ein vorgeordnetes Objektiv 751 zur Fokussierung der ankommenden Strahlung auf, welches mit der spiegelnden Freiformfläche 872 am Spiegelblock 871 und der ersten spiegelnden Freiformfläche 874 am Spiegelblock 873 ausgebildet ist. Der Spiegelblock 871 ist hier verkleinert dargestellt.

Nach dem Passieren der Freiformfläche 874 tritt die Strahlung in das zyklische Zweistrahl-Interferometer 615 ein. Die interferierenden Teilstrahlenbündel treten in das nachgeordnete anamorphotische Objektiv ein, welches hier mit den spiegelnden Freiformflächen 875, 876 und 877 ausgestaltet ist. Die spiegelnden Freiformflächen 875, 876 und 877 bilden ein anamorphotisches Objektiv 595, welches dem zyklischen Zweistrahl-Interferometer 615 nachgeordnet und dem Mikrobolometer-Array 634 vorgeordnet ist. Die räumlichen Interferogramme rI entstehen also nach der Strahlformung von Teilstrahlenbündeln TB1 und TB2 mittels der zweiten Freiformfläche 875, ausgebildet auf dem Spiegelblock 871, sowie mittels der vierten Freiformfläche 876, welche als Sattelfläche auf dem Spiegelblock 871 gestaltet ist, und mittels der dritten Freiformfläche 877 des Spiegelblocks 871, welche die insgesamt fünfte Freiformfläche darstellt, schließlich auf dem Mikrobolometer-Array 634. Diese räumlichen Interferogramme rI sind in der Detailabbildung 36.3 für die Zeitpunkte t1 und t2 dargestellt.

Hierbei spielt bei der Messung und bei der Auswertung die Bildinformation über die Abgaswolke 16 selbst eine eher untergeordnete Rolle, da die Objektform derselben bei der Analyse von Luftschadstoffen von geringerem Interesse ist. Vielmehr soll hier nur näherungsweise ortsaufgelöst die spektrale Zusammensetzung ermittelt werden, die insbesondere auf für Mensch, Tier und Umwelt schädliche Komponenten schließen lässt. Dabei soll jedoch in keinem Fall ein Nichterfassen oder Übersehen von wesentlicher spektraler Information vorkommen, die beispielsweise giftige Komponenten kenntlich macht. Wo örtlich ganz genau diese giftige Komponente sich in der Abgaswolke befindet, ist zunächst nicht von allererstem Interesse. Es genügt schon, wenn man die spektrale Information, welche auf Schadstoffe hinweist, wenigstens, beispielsweise unter Kenntnis der aktuellen Windrichtung, einem einzelnen Schornstein zuordnen kann.

Die Detailabbildung A, welche dieses zyklische Zweistrahl-Interferometer 615 hier in der Detailabbildung 37.1 darstellt, entspricht dem in der Fig. 30. Die hier angeordnete Doppel-Spiegeltreppe 85 ist in der Detailabbildung 37.2 dargestellt und entspricht der Detailabbildung 30.1 in der Fig. 30. Die Beschreibung der Doppel-Spiegeltreppe 85 entspricht der zu der Detailabbildung 30.1.

Im Folgenden werden verwendete Begriffe näher erläutert, beschrieben und/oder definiert.

Der Begriff Lateral-Shear leitet sich aus dem Phänomen der Interferenz zweier reflektierter Lichtstrahlenbündel mit lateralem Versatz, also einem Querversatz ab. Dabei werden in konventionellen Anordnungen typischerweise ein zu testendes Lichtstrahlenbündel an einer äußeren Fläche und ein Lichtstrahlenbündel an einer inneren Fläche einer sogenannten Scherplatte (shear plate) derart reflektiert, dass sie räumlich (und zeitlich) zueinander verschoben reflektiert werden. Insbesondere kann eine Lateral-Shear üblicherweise mit einem Scher-Interferometer erzeugt werden, wobei das Scher-Interferometer ein optisch vergleichsweise einfaches Mittel in Form einer Platte darstellt, um eine Wellenfront-Analyse durchzuführen. Damit kann die Kollimation von Lichtstrahlenbündeln getestet werden, insbesondere von Laserquellen, deren Kohärenzlänge in der Regel deutlich größer ist als die zweifache optische Dicke der Scherplatte. Das Scher-Interferometer, ausgebildet als Platte, umfasst üblicherweise ein im Wesentlichen hochwertiges optisches Glas wie beispielsweise N-BK7 oder auch Quarzglas mit besonders ebenen und glatten optischen Oberflächen, die normalerweise in einem sehr kleinen Winkel zueinander stehen, also im Wesentlichen nicht parallel zueinander angeordnet sind und damit einen sehr schwach keilförmigen Charakter aufweisen. Dabei fällt im Test ein gut kollimiertes Strahlenbündel, beispielsweise in einem Winkel von etwa 45° auf das Scher-Interferometer in Form der Platte ein und wird zweimal reflektiert. Die beiden reflektierten Lichtstrahlenbündel sind aufgrund des schwach keilförmigen Charakters nach Passieren der Platte zueinander leicht verkippt und zeigen bei perfekter Kollimation des Eingangsstrahlenbündels (ebene Wellenfront) Interferenzstreifen nach der Scherplatte, welche üblicherweise bei perfekter Kollimation parallel zur Lateral-Shear orientiert sind. Diese durch die Scherplatte erzeugte Trennung bzw. laterale Verschiebung der Strahlenbündel wird als Scherung bzw. Shear, insbesondere als Lateral-Shear bezeichnet. Die Scherung bzw. Lateral-Shear kann auch durch Gitter erzeugt werden oder, wie im vorliegenden Fall, durch ein geeignetes Spiegelelement, insbesondere durch einen Zweispiegel-Winkelreflektor mit erfindungsgemäßer Bildfeld-Diskriminatoreinheit. Die Lateral-Shear ist in den jeweiligen Zeichnungen mit dem Bezugszeichen "s" angedeutet.

Der Begriff "Zweistrahl-Interferometer" umfasst insbesondere Zweistrahl-Interferometer-Typen, wie beispielsweise ein Michelsontyp-Interferometer, ein Mach-Zehnder-Interferometer oder ein zyklisches Zweistrahl-Interferometer.

Der Begriff Licht wird in dieser Schrift als Synonym für elektromagnetische Strahlung, und zwar insbesondere vom UV- bis zum Terahertz-Bereich verwendet.

Mit dem Akronym FIR ist insbesondere der ferninfrarote Spektralbereich gemeint, wobei dieser insbesondere in etwa zwischen 50 $\mu$m und 1000 $\mu$m liegt.

Bei einem Michelsontyp-Interferometer ist es insbesondere so, dass das hinlaufende und das rücklaufende Bündel in jedem Interferometerarm zumindest näherungsweise parallel zueinander verlaufen und die Strahlteilung und Strahlvereinigung an derselben Strahlteilerfläche erfolgen. Es wird vorliegend hauptsächlich der Begriff "Michelsontyp-Interferometer" anstelle von "Michelson-Interferometer" verwendet, da insbesondere hierin Anordnungen beschrieben werden, in denen mehr als ein Planspiegel in den Interferometerarmen IA1 und IA2 vorliegen und somit kein reines "Michelson-Interferometer" im engeren Sinne zu verstehen ist.

Das Akronym MIR bezieht sich insbesondere auf den mittleren Infrarotbereich, der insbesondere zwischen etwa 3 $\mu$m und 50 $\mu$m liegt.

Das Akronym NIR bezieht sich insbesondere auf den nahen Infrarotbereich, der insbesondere zwischen etwa 0,78 $\mu$m und 3 $\mu$m liegt und sich in den IR-A zwischen etwa 0,78 $\mu$m und 1,4 $\mu$m und den IR-B Bereich zwischen etwa 1,4 $\mu$m und 3 $\mu$m unterteilt.

Das Akronym SEF bezieht sich insbesondere auf eine scheinbare Endspiegelfläche, die sich nach der Entfaltung von Spiegelflächen ergibt.

Das Akronym VEIS bezieht sich insbesondere auf den visuellen bzw. für das menschliche Auge sichtbaren Spektralbereich zwischen etwa 380nm und 780nm, insbesondere zwischen etwa 400nm bis 700nm.

Ein Zweispiegel-Winkelreflektor kann insbesondere als 90-Altgrad-Dachkantreflektor ausgebildet sein, aber auch als Winkelreflektor mit 45-Altgrad- Strahlablenkung. Es sind vorzugsweise Winkelwerte der Strahlablenkung zwischen etwa 40 Altgrad und etwa 150 Altgrad 60, insbesondere zwischen etwa 60 Altgrad und etwa 120 Altgrad möglich.

Ein Bildfeld-Diskriminator ist insbesondere eine Öffnung in einer Blende, wobei die Öffnung bevorzugt eine Spaltblende 645, ein Pinhole, ein Pinhole-Array, welches eine Mehrzahl von Pinholes aufweist, und/oder ein Durchlassbereich 656, 657 eines Flüssigkristall-Displays 655 sein.

Ein nichtdiskriminierender Bereich eines Bildfeld-Diskriminators umfasst beispielsweise den Durchlassbereich bei einem spaltförmigen Bildfeld-Diskriminators, also im einfachsten Fall die Spaltöffnung und/oder den reflektierenden Bereich beispielsweise bei einem sehr schmalen Planspiegel als Bildfeld-Diskriminator. Der nichtdiskriminierende Bereich kann auch durch einen räumlichen Lichtmodulator in Transmission (Flüssigkristall-Display) oder in Reflexion (Digitales Mikrospiegel-Array) dargestellt sein. Der nichtdiskriminierende Bereich kann in sich auch noch eine feine Strukturierung aufweisen.

Ein Bildfeld-Diskriminator kann insbesondere auch einen schmalen reflektierenden Bereich auf einem Planspiegel und/oder auf einem Mikrospiegel-Array und/oder auch einen schmalen Planspiegel aufweisen.

Die genannten Formen eines Bildfeld-Diskriminators sind insbesondere dazu ausgelegt, zumindest einen Teil eines Lichtbündels auf einem wohldefinierten Strahlengang in den weiteren Abschnitt des Strahlengangs passieren zu lassen, ganz besonders bevorzugt in Form eines schmalen Streifens, der bevorzugt ein Zehntel bis zu einem Tausendstel der Ausdehnung des Bildes entsprechen kann, beispielsweise ein Zehntel bis zu einem Tausendstel der Höhe des Bildes vom Messobjekt im Zweistrahl-Interferometer. Die faktische "Eindimensionalität" des schmalen Streifens - mit Bildelementen bevorzugt nur in einer einzigen Reihe - ermöglicht es letztlich, aus jedem nach der Selektion noch verbliebenen Bildelement jeweils genau ein räumliches Interferogramm zu erzeugen. In anderen Worten werden insbesondere solche Anteile eines Lichtbündels mittels des Bildfeld-Diskriminators ausgeblendet bzw. blockiert, die nicht dem vorbestimmten Strahlengang folgen sollen. Beispielsweise kann auch Streulicht mittels des Bildfeld-Diskriminators ausgeblendet bzw. blockiert werden. Das bedeutet, dass Streulicht am Passieren des Bildfeld-Diskriminators in den weiteren Abschnitt des Strahlengangs gehindert wird. Mit dem Begriff "räumliches Selektieren" ist die Auswahl bzw. das Passieren des Lichtes gemeint, das durch den Bildfeld-Diskriminator, beispielsweise den Spalt treten bzw. fallen kann und letztlich zur Detektion kommt. Es wird in anderen Worten nicht nur das Bildfeld selektiert, sondern auch das hier unerwünschte Streulicht minimiert.

Ein Spiegelprisma, das hier alternativ auch Prisma genannt wird, weist insbesondere ein refraktives Material, wie z.B. CaF$_2$, Si, BK7, Quarzglas, kristalliner Quarz und/oder andere gängige optische Materialien für die gewünschten Spektralbereiche auf. Das Spiegelprisma kann zumindest teilweise beschichtet oder auch vollständig unbeschichtet sein. Insbesondere weist das Spiegelprisma zumindest eine für den Ein- und/oder Austritt eines Lichtbündels geeignete Ein- und/oder Austrittsfläche. Insbesondere weist das Spiegelprisma auch zumindest eine Reflexionsfläche bzw. eine Spiegelfläche auf, die unter geeigneten Bedingungen dazu ausgelegt ist, zumindest einen Teil des in das Prisma eingetretenen Lichtbündels zu reflektieren bzw. zu spiegeln. Eine im Wesentlichen vollständige Reflexion kann insbesondere unter Winkeln der Totalreflexion erfolgen. Insbesondere kann das Spiegelprisma auch eine zweite Reflexionsfläche bzw. Spiegelfläche, die sich dazu eignet, zumindest einen Teil des in das Prisma eingetretenen Lichtbündels ein zweites Mal zu reflektieren bzw. zu spiegeln, aufweisen.

Das Spiegelprisma kann eine Spiegelschicht auf zumindest einem Abschnitt einer Reflexionsfläche aufweisen. Beispielsweise kann die Oberfläche einer Reflexionsfläche zumindest teilweise mit Gold und/oder Silber beschichtet sein.

Im Allgemeinen entsprechen sich die Begriffe Reflexionsfläche und Spiegelfläche. Ein Spiegeln bzw. eine Reflexion kann an einer zumindest teilweisen verspiegelten Fläche erfolgen oder insbesondere auch unter bestimmten Winkeln an einem Übergang zwischen Medien unterschiedlicher Brechungsindizes, beispielsweise beim Auftreffen eines Lichtbündels, das ein optisch dichtes Medium durchläuft auf eine Grenzfläche zu einem optisch dünnen Medium. Unter besonderen Winkeln kann eine Totalreflexion erfolgen, bei der der gesamte Anteil des Lichtbündels vollständig reflektiert wird.

Ein Spiegelprisma, bei dem zwei Reflexionsflächen genutzt werden sollen, kann insbesondere ein Prisma sein, bei dem die beiden Reflexionsflächen einen rechten Winkel einschließen und die Ein- und Austrittsfläche des Prismas diesem Winkel gegenüber liegt.

Ein Strahlteiler einer Strahlteilereinheit entspricht insbesondere einem Strahlteilerwürfel oder zwei einzelnen Planparallelplatten, die bevorzugt jeweils CaF$_2$ und eine Strahlteilerschicht aufweisen oder einem Polarisationsstrahlteilerwürfel oder einem Plattenstrahlteiler mit zwei Platten und einer Strahlteilerschicht. Ein Strahlteiler ist insbesondere dazu ausgelegt, zumindest einen Anteil des einfallenden Lichtbündels an einer Auftrittsoberfläche zu transmittieren, um das erste Teilstrahlenbündel zu erzeugen und zumindest einen weiteren Anteil des einfallenden Lichtbündels zu reflektieren, um das zweite Teilstrahlenbündel zu erzeugen. Ein Strahlteiler ist ferner insbesondere dazu ausgelegt, zumindest einen Anteil des ersten Teilstrahlenbündels an einer Auftrittsoberfläche zu reflektieren und zumindest einen Anteil des zweiten Teilstrahlenbündels zu transmittieren.

Eine Strahlteilereinheit weist insbesondere in einem Strahlteilerwürfel oder in einem System aus Planparallelplatten zumindest eine plane Strahlteilerschicht auf. Die Strahlteilerschicht hat eine erste dem einfallenden Lichtbündel zugewandten Seite, die eine Strahlteilerfläche darstellt und eine dem einfallenden Lichtbündel abgewandte Seite, die eine Überlagerungsfläche darstellt. Die Strahlteilerfläche ist dazu ausgelegt, einfallendes Licht in Teilen zu transmittieren, um einen ersten Teilstrahlbündel zu erzeugen und in Teilen zu reflektieren, um einen zweiten Teilstrahlbündel zu erzeugen. Insbesondere ergeben sich somit zwei im Wesentlichen zueinander senkrecht verlaufende Teilstrahlbündel.

Ein Spiegelprisma, bei dem lediglich eine Reflexionsfläche genutzt werden soll kann z.B. ein 90°-Umlenkprisma darstellen. Abweichungen vom 90°-Winkel sind in Bereichen +/- 30° möglich. 1 ° ist der 360ste Teil eines Vollkreises.

Eine optische Konjugation zwischen der ersten Bildfeld-Diskriminatoreinheit (BFD1; 631; 645; 653; 656; 666) und der zweiten Bildfeld-Diskriminatoreinheit (BFD2; 632; 646; 654; 657; 667) liegt insbesondere vor, wenn diese - vom Eingang des Zweistrahl-Interferometers aus in Richtung Zweistrahl-Interferometer gesehen - optisch zusammenfallen und somit optisch nicht mehr unterscheidbar sind.

Eine schmale IR-Lichtquelle kann ein dunkelrot glühender, langgestreckter Siliziumkarbid-Stab sein, der beispielhaft 20 mm lang ist und 1 mm im Durchmesser aufweist.

Ein scharfes Bild ist insbesondere dadurch ausgezeichnet, dass es nahezu beugungsbegrenzt ist.

Ein unscharfes Bild ist insbesondere dadurch ausgezeichnet, dass dessen von einem Objektpunkt gebildete Bildflecken die Größe eines Airy-Scheibchens um ein Mehrfaches übertreffen.

Eine Koma umfasst insbesondere Asymmetriefehler, insbesondere solche, die bei schräg zur optischen Achse einfallendem Strahlenbündel durch eine Überlagerung zweier Abbildungsfehler entstehen. Anstelle eines scharfen Beugungsscheibchens kann insbesondere ein Bildpunkt mit zum Rand der Optik gerichtetem "Schweif entstehen. Koma kann insbesondere sowohl bei Linsen- als auch bei Spiegeloptiken auftreten.

Astigmatismus umfasst insbesondere das Phänomen, dass in zwei senkrecht zueinander angeordneten Schnittebenen zwei Bildpunkte, die jeweils von Strahlenbüscheln der jeweiligen Schnittebene gebildet sind, erheblich in der Tiefenlage, also in Ausbreitungsrichtung des Lichts, signifikant separiert sind. In der Erfindung liegt so bei der Detektion einer der Bildpunkte im Wesentlichen bevorzugt im Unendlichen und der andere im Nahbereich, bevorzugt auf dem gerasterten Detektor.

Der Begriff "Tiefe" ist die Dimension in Ausbreitungsrichtung des Lichts.

Der Begriff "wellenoptische Schärfentiefe" ist durch die Relation Lichtwellenlänge dividiert durch das Quadrat des Sinus des Aperturwinkels des zugehörigen Lichtbündels gegeben.

Eine verspiegelte Fläche ist insbesondere dazu ausgelegt mindestens etwa 80 %, insbesondere mindestens etwa 93 % und bevorzugt mindestens etwa 97 % bis etwa 99,9 % des einfallenden Lichtes in zumindest einem Teil des Wellenlängenspektrums elektromagnetischer Strahlung zu reflektieren.

Eine unverspiegelte bzw. nicht verspiegelte Fläche ist insbesondere dazu ausgelegt weniger als etwa 60 %, insbesondere weniger als etwa 30 % und bevorzugt weniger als etwa 10 % des einfallenden Lichtes in zumindest einem Teil des Wellenlängenspektrums elektromagnetischer Strahlung zu reflektieren.

Der Begriff "spitzer Halbwinkel" umfasst folgende Werte: Im Betrag größer als 15 Altgrad und mit einem Betrag kleiner als 35 Altgrad.

Ein optisches Übersprechen kann bei der Messung von biologischem Gewebe durch Streulicht auftreten, welches sich auf mehrere Detektorelemente eines gerasterten Detektors verteilt. Dieses Streulicht wird durch eine konfokale Diskriminierung weitgehend blockiert, wodurch sich das optische Übersprechen reduziert. Weiterhin kann ein optisches Übersprechen auch so verstanden werden, dass Strahlen eines nichtperfekten und/oder auch etwas defokussierten Lichtstrahlenbündels, welche einen Bildpunkt, beispielsweise auf einem Detektorelement eines gerasterten Detektors erzeugen, auch ein oder mehrere benachbarte Detektorelemente des gerasterten Detektors treffen.

Zylinderwellen sind insbesondere Lichtwellen, welche zumindest näherungs- und teilweiseweise die Form einer Zylinderfläche aufweisen, also einen Ausschnitt derselben.

Ein Zweispiegel-Winkelreflektor kann als entweder ein Dachkantreflektor in Metall und/oder auch als ein Dachkantprisma, als ein Pentaprisma und/oder als ein Winkelreflektor in Luft und/oder Vakuum mit bevorzugter Strahlablenkung zwischen 60 Altgrad und 120 Altgrad ausgebildet sein. Beide Planspiegel des Zweispiegel-Winkelreflektors sind im Zweistrahl-Interferometer senkrecht zur Referenzebene RE angeordnet, zu der auch die Strahlteilerfläche senkrecht steht.

In einem Mach-Zehnder-Interferometer sind in der Regel jeweils zwei Zweispiegel-Winkelreflektoren angeordnet.

In einem Michelsontyp-Interferometer sind vorzugsweise ebenfalls jeweils zwei Zweispiegel-Winkelreflektoren angeordnet. Jedoch können dort auch zwei Tripelspiegel-Winkelreflektoren angeordnet sein, welche dann die Strahlumlenkungseinheit darstellen.

In einem zyklischen Zweistrahl-Interferometer ist in der Regel nur ein einziger Zweispiegel-Winkelreflektor angeordnet. Jedoch kann dem Zweispiegel-Winkelreflektor im zyklischen Zweistrahl-Interferometer bevorzugt eine vergleichsweise kleine Doppelspiegeltreppe (85, 85w) oder es können bevorzugt zwei einzelne Spiegeltreppen (82, 82w, 83, 83w) diesem Zweispiegel-Winkelreflektor zugeordnet sein.

Im Folgenden werden besondere Ausführungsformen und Beispiele beschrieben, die miteinander und insbesondere mit den Aspekten der Erfindung kombinierbar sind, sofern sie sich nicht ausschließen:

Fourier-Transformations-Spektrometer mit zumindest teilweiser hyperspektraler Single-Shot-Bildgebung von einem Messobjekt als Ergebnis einer Berechnung mit einem Rechnersystem mit einem Rechenprogramm zur Gewinnung von Spektren mittels einer Fourier-Transformation

und mit einem vorgeordneten Objektiv als Abbildungssystem für das Messobjekt und mit einem Zweistrahl-Interferometer, dem das vorgeordnete Objektiv vorangestellt ist und welches aufweist:

entweder einerseits einen Strahlteiler mit ebener Strahlteilerfläche und der Strahlteiler sowohl zur Strahlenbündelteilung in der Strahlteilerebene genutzt wird, wodurch zwei Teilstrahlenbündel TB1, TB2 gebildet werden, als auch zur zumindest teilweisen Strahlenbündelvereinigung in der Strahlteilerebene mit einer Lateral-Shear s zwischen den beiden Teilstrahlenbündeln genutzt wird

oder andererseits zwei Strahlteiler mit jeweils ebener Strahlteilerfläche, ein erster zur Strahlenbündelteilung in der Strahlteilerebene, wodurch zwei Teilstrahlenbündel TB1, TB2 gebildet werden, und ein zweiter Strahlteiler zur zumindest teilweisen Strahlenbündelvereinigung mit einer Lateral-Shear s zwischen den beiden Teilstrahlenbündeln und am Zweistrahl-Interferometer eine Referenzebene besteht, welche durch die Normale der ebenen Strahlteilerfläche und durch die optische Achse des vorgeordneten Objektivs am Eingang des Zweistrahl-Interferometers aufgespannt ist,

und einen gerasterten Detektor am Ausgang des Zweistrahl-Interferometers

und das vorgeordnete Objektiv vor dem Zweistrahl-Interferometer - unter Berücksichtigung der Position des Messobjekts - ein Bild oder mindestens ein Teilbild des Messobjekts in Lichtrichtung in der Regel nach dem Strahlteiler, jedoch in der Regel vor dem gerasterten Detektor, erzeugt

und unter Mitwirkung einer dem Zweistrahl-Interferometer nachgeordneten anamorphotischen Abbildungsstufe eine Vielzahl, mindestens jedoch zwei, von räumlichen Interferogrammen gebildet wird und diese räumlichen Interferogramme auf dem gerasterten flächigen Empfänger ausgebildet sind und jedem räumlichen Interferogramm ein Objektpunkt auf dem Messobjekt zugeordnet ist,

wobei

entweder das Zweistrahl-Interferometer 601 mit zwei Interferometerarmen IA1, IA2 als nichtzyklisches Interferometer 602, 603, 604 ausgebildet ist und in jedem Interferometerarm IA1, IA2 jeweils ein Zweispiegel-Winkelreflektor oder ein Prisma 640, 641, 642, 651, 652, 661, 662 oder in jedem Interferometerarm jeweils ein Tripelspiegel-Reflektor oder jeweils ein Tripelspiegelprisma zumindest näherungsweise in Raumeckenform angeordnet ist

und die Differenz der Spiegel bzw. Spiegelflächen in den beiden Interferometerarmen null ist und in einer Position zwischen einem Drittel und zwei Dritteln der optischen Weglänge OPD im Strahlverlauf zwischen Strahlenbündelteilung und Strahlenbündelwiedervereinigung in jedem Interferometerarm ein Bildfeld-Diskriminator BFD1, BFD2 angeordnet ist und diese beiden Bildfeld-Diskriminatoren BFD1, BFD2 in der Regel zumindest näherungsweise zueinander optisch konjugiert sind

oder das Zweistrahl-Interferometer 601 als zyklisches Zweistrahl-Interferometer 605, 606, 608, 615 entweder mit zwei Planspiegeln 628, 629, 671, 672 ausgebildet ist, die eine Winkelspiegelanordnung 630 bilden, bei welcher die beiden Planspiegel 628, 629, 671, 672 von der Strahlteilerebene ET, welche die ebene Strahlteilerfläche enthält, ungleich beabstandet und zumindest näherungsweise senkrecht zur Referenzebene RE ausgerichtet sind oder das Zweistrahl-Interferometer 601 als zyklisches Zweistrahl- Interferometer 607, 609, 610, 611,612,613,614

mit zwei planen Spiegelflächen 681, 682, 762, 674, 687s, 688s, 689s, 888, 890, 696s ausgebildet ist, von denen jeweils genau eine an je einem Spiegelprisma 679, 680, 687, 688, 689, 696, 761, 763, 887, 889 aus refraktivem Material ausgebildet ist und so eine Doppel-Spiegelprismen-Anordnung 697 besteht, die mit jeweils genau zwei Spiegelprismen 679, 680, 687, 688, 689, 689, 696, 761, 763, 887 ausgebildet ist,

und entweder die beiden Planspiegel 628, 629, 671, 672, welche als Winkelspiegelanordnung 630 ausgestaltet sind, oder die beiden planen Spiegelflächen 681, 682, 762, 674, 687s, 688s, 689s, 888, 890, 689s, 696s der Doppel-Spiegelprismen-Anordnung 697 von der Strahlteilerebene ET, welche die Strahlteilerfläche enthält, vorbestimmt ungleich beabstandet und zumindest näherungsweise senkrecht zur Referenzebene RE ausgerichtet sind

und zwischen den beiden Planspiegeln 628, 629, 671, 672 oder planen Spiegelflächen 681, 682, 687s, 688s, 696s, 689s, 696s, 762, 764, 888, 890 ein Winkel epsilon mit dem doppelten Betrag des Halbwinkels psi besteht und dieser Winkel epsilon durch zwei verlängerte Geraden g1, g2 aus den Planspiegeln 628, 629, 671, 672 bzw. aus den planen Spiegelflächen 681, 682, 687s, 688s, 689s, 696s, 762, 764 und 888, 890 in der Referenzebene RE dargestellt ist.

und der Halbwinkel psi mit einem Betrag größer als 20 Altgrad und mit einem Betrag kleiner als 30 Altgrad ausgebildet ist

und im Zweistrahl-Interferometer 601, 602, 603, 604, 605, 605, 607, 608, 609, 610, 611, 612, 613, 614, 615 eine erste Bildebene BEI1 sowie eine zweite Bildebene BEI2 bestehen, welche jeweils durch das vorgeordnete Objektiv 70, 71, 73, 74, 75, die Lage des Messobjekts 1, 10, 14, 15,16 und das Zweistrahl-Interferometer 601, 602,603, 604, 605, 605, 607, 608, 609, 610, 611, 612, 613, 614, 615 gegeben sind,

und der ersten Bildebene BEI1 ein erster Bildfeld-Diskriminator BFD1 sowie der zweiten Bildebene BEI2 ein zweiter Bildfeld-Diskriminator BFD2 im Zweistrahl-Interferometer 601, 602, 603, 604, 605, 605, 607, 608, 609, 610, 611, 612, 613, 614, 615 zugeordnet ist und diese Bildfeld-Diskriminatoren BFD1, BFD2 mit jeweils mindestens einem nichtdiskriminierenden Bereich in Transmission oder Reflexion ausgebildet sind

und diese beiden Bildfeld-Diskriminatoren BFD1, BFD2 so im Zweistrahl-Interferometer angeordnet sind, dass zumindest näherungsweise zwischen diesen Bildfeld-Diskriminatoren eine optische Konjugation besteht.

Fourier-Transformations-Spektrometer,
wobei in einem Zweistrahl-Interferometer 601, 602, 603, 604, 605, 605, 607, 608, 609, 610, 611, 612, 613, 614. 615 jeder Bildfeld-Diskriminator BFD1, BFD2 einem Zweispiegel-Winkelreflektor oder einem Prisma 630, 640, 641, 642, 651, 652, 661, 662 oder einem Tripelspiegel-Reflektor in Form einer Raumecke zugeordnet ist.

Fourier-Transformations-Spektrometer,
wobei mindestens eine Bildebene BEI1, BEI2 bei der Abbildung des Messobjekts 1, 10, 14, 15, 16 in einem Zweistrahl-Interferometer 601, 602, 603, 604, 605, 605, 607, 608, 609, 610, 611, 612, 613, 614. 615 zumindest näherungsweise jeweils mit einem Bildfeld-Diskriminator BFD1, BFD2 zumindest teilweise zur Koinzidenz gebracht ist.

Fourier-Transformations-Spektrometer,
wobei die zwei Bildfeld-Diskriminatoren BFD1, BFD2 in einem Zweistrahl-Interferometer 601, 602, 603, 604, 605, 605, 607, 608, 609, 610, 611, 612, 613, 614, 615 spaltförmig ausgebildet sind.

Fourier-Transformations-Spektrometer,
wobei mindestens ein Bildfeld-Diskriminator BFD in einem Zweistrahl-Interferometer 601, 602, 606 als rechnersteuerbarer, räumlicher Lichtmodulator 649, 656 ausgebildet ist.

Fourier-Transformations-Spektrometer,
wobei die beiden Bildfeld-Diskriminatoren BFD1, BFD2 in einem Michelsontyp-Interferometer 602, 603 als Spaltblende 645, 646, 653, 654 ausgebildet und angeordnet sind und jeweils genau eine Spaltblende 645, 646, 653, 654 in jedem Arm IA1, IA2 des Michelsontyp-Interferometers 602, 603 positioniert ist.

Fourier-Transformations-Spektrometer, wobei

die erste Bildfeld-Diskriminatoreinheit 653 eine Öffnung aufweist, die dazu ausgelegt ist, das erste Teilstrahlenbündel TB1 nach dem Aufspalten des einfallenden Lichtbündels EB mittels der Strahlteilereinheit 622 und nach dem zweiten Reflektieren des ersten Teilstrahlenbündels TB1 mittels der zweiten Reflexionsfläche 651b des ersten Prismas 651 räumlich zu selektieren; und/oder

die zweite Bildfeld-Diskriminatoreinheit 654 eine Öffnung aufweist, die dazu ausgelegt ist, das zweite Teilstrahlenbündel TB2 nach dem Aufspalten des einfallenden Lichtbündels EB mittels der Strahlteilereinheit 622 und nach dem zweiten Reflektieren des zweiten Teilstrahlenbündels TB2 mittels der ersten Reflexionsfläche 652b des zweiten Prismas 652 räumlich zu selektieren.

Fourier-Transformations-Spektrometer, wobei

die erste Bildfeld-Diskriminatoreinheit 653 eine Öffnung aufweist, die dazu ausgelegt ist, das erste Teilstrahlenbündel TB1 nach dem Aufspalten des einfallenden Lichtbündels EB mittels der Strahlteilereinheit 622 und vor dem zweiten Reflektieren des ersten Teilstrahlenbündels TB1 mittels der zweiten Reflexionsfläche 651b des ersten Prismas 651 räumlich zu selektieren; und/oder
die zweite Bildfeld-Diskriminatoreinheit 654 eine Öffnung aufweist, die dazu ausgelegt ist, das zweite Teilstrahlenbündel TB2 nach dem Aufspalten des einfallenden Lichtbündels EB mittels der Strahlteilereinheit 622 und vor dem zweiten Reflektieren des zweiten Teilstrahlenbündels TB2 mittels der ersten Reflexionsfläche 652b des zweiten Prismas 652 räumlich zu selektieren.

Fourier-Transformations-Spektrometer,
wobei in einem Michelsontyp- Interferometer 602 jede Spaltblende 645, 646 zur scheinbaren Endspiegelfläche SEF1, SEF2 des jeweiligen Interferometerarms IA1, IA2 in optischer Konjugation angeordnet ist.
Fourier-Transformations-Spektrometer,
wobei die Bildfeld-Diskriminatoren BFD1, BFD2 in einem Michelsontyp-Interferometer 602, 603 jeweils in einer Position zumindest näherungsweise bei der Hälfte der optischen Weglänge im Strahlverlauf zwischen Strahlenbündelteilung und Strahlenbündelwiedervereinigung angeordnet ist.
Fourier-Transformations-Spektrometer,
wobei jeder Zweispiegel-Winkelreflektor oder ein Prisma 640, 641, 642, 651, 652 in einem Michelsontyp-Interferometer 602, 603 mit einer Strahlablenkung von zumindest näherungsweise 180 Altgrad ausgebildet ist.
Fourier-Transformations- Spektrometer,
wobei mindestens ein Bildfeld-Diskriminator BFD1, BFD2 in einem Michelsontyp-Interferometer 602, 603 mit einem Flüssigkristall-Display 649-1, 649-2 gebildet ist.
Fourier-Transformations-Spektrometer,
wobei die Bildfeld-Diskriminatoren BFD1, BFD2 in einem Mach-Zehnder-Interferometer 604 angeordnet sind und dabei jeweils genau eine Spaltblende 666, 667 als Bildfeld-Diskriminator in jedem Arm IA1, IA2 des Mach-Zehnder-Interferometers 604 positioniert ist.
Fourier-Transformations-Spektrometer,
wobei die Bildfeld-Diskriminatoren BFD1, BFD2 in einem Zweistrahl-Interferometer 601, 602, 603, 604, 605, 605, 607, 608, 609, 610, 611, 612, 613, 614, 615 jeweils in einer Position zumindest näherungsweise bei der Hälfte der optischen Weglänge im Strahlverlauf zwischen Strahlenbündelteilung und Strahlenbündelwiedervereinigung angeordnet sind.
Fourier-Transformations-Spektrometer,
wobei die Zweispiegel-Winkelreflektoren 661, 662 in einem Mach-Zehnder-Interferometer 604 mit einer Strahlablenkung zwischen 45 Altgrad und 135 Altgrad ausgebildet sind.
Fourier-Transformations-Spektrometer,
wobei mindestens ein Bildfeld-Diskriminator BFD1, BFD2 in einem Mach-Zehnder-Interferometer als ein digitales Mikrospiegel-Array ausgebildet ist.
Fourier-Transformations-Spektrometer,
wobei in einem zyklischen Zweistrahl-Interferometer 605, 605, 607, 608, 609, 610, 611, 612, 613, 614, 615 mit zwei Planspiegeln 628, 629, 671, 672 bzw. zwei planen Spiegelflächen 681, 682, 687s, 688s, 689s, 696s, 762, 764, 888, 890 in dessen beiden entgegengesetzt umlaufenden Teilstrahlenbündeln TB1, TB2 je eine Spaltöffnung 677, 678, 976, 977 in jeweils einer Spaltblende 631, 632, 976, 977 als Bildfeld-Diskriminator BFD1, BFD2 in einer Bildebene des Messobjekts 1, 10, 14, 15, 16 angeordnet ist.
Fourier-Transformations-Spektrometer,
wobei in einem zyklischen Zweistrahl-Interferometer 607, 613, 614 mit zwei Planspiegeln 628, 629, 671, 672 bzw. zwei planen Spiegelflächen 681, 682, 687s, 688s, 689s, 696s, 762, 764, 888, 890 in dessen beiden entgegengesetzt umlaufenden Teilstrahlenbündeln TB1, TB2 eine Doppelspaltblende 676, 694, 975 mit jeweils zwei Spaltöffnungen 677, 678, 977, 977 in einer Ebene senkrecht zu den Hauptstrahlen der Teilstrahlenbündel TB1, TB2 angeordnet ist, welche die gemeinsame Bildebene BEI12 darstellt.

Fourier-Transformations-Spektrometer,

wobei vorzugsweise in einem zyklischen Zweistrahl-Interferometer 606 mit zwei Planspiegeln 671, 672 ein rechnersteuerbares, transmissives Flüssigkristall-Display 655 zwischen diesen Planspiegeln 628, 629, 671, 672 bzw. planen Spiegelflächen mit mindestens zwei Durchlassbereichen 656, 657 angeordnet ist.

Fourier-Transformations-Spektrometer,

wobei im transmissiven Flüssigkristall-Display 655 zwei spaltförmige Durchlassbereiche 656 und 657 eingeschriebenen sind.

Fourier-Transformations-Spektrometer,

wobei in einem zyklischen Zweistrahl-Interferometer 605, 605, 607, 608, 609, 610, 611, 612, 613, 614, die Strahlteilerebene ET und der Schnittpunkt SP der Geraden g1 und g2 um den Abstand d_ST voneinander separiert sind und dieser Abstand d_ST mindestens zehn Wellenlängen der größten Wellenlänge im Spektrum des zur Detektion kommenden Lichts beträgt.

Fourier-Transformations-Spektrometer,

wobei in einem zyklischen Zweistrahl-Interferometer 615 im Bereich Z, wo es keine räumliche Überdeckung der beiden Teilstrahlenbündel TB1, TB2 gibt, mindestens eine Spiegeltreppe mit je zwei Spiegeln angeordnet ist.

Fourier-Transformations-Spektrometer,

wobei in einem zyklischen Zweistrahl-Interferometer 615 im Bereich Z, wo es keine räumliche Überdeckung der beiden Teilstrahlenbündel TB1, TB2 gibt, zwei Spiegeltreppen 82, 83 mit je zwei Spiegeln angeordnet sind.

Fourier-Transformations-Spektrometer,

wobei in einem zyklischen Zweistrahl-Interferometer 615 die beiden Spiegel einer ersten Spiegeltreppe 82 als Planspiegel 821, 822 und die beiden Spiegel einer zweiten Spiegeltreppe 83 als Planspiegel 831, 832 ausgebildet sind.

Fourier-Transformations-Spektrometer,

wobei in einem zyklischen Zweistrahl-Interferometer 615 im Bereich Z, wo es keine räumliche Überdeckung der beiden Teilstrahlenbündel TB1, TB2 gibt, sowohl bei der ersten miniaturisierten Spiegeltreppe 82 die beiden Planspiegel 821, 822 als auch bei der zweiten miniaturisierten Spiegeltreppe 83 die beiden Planspiegel 831, 832 jeweils parallel zueinander angeordnet sind.

Fourier-Transformations-Spektrometer,

wobei die beiden parallel gestellten Planspiegel 821, 822 einer ersten Spiegeltreppe 82 sowie die parallel gestellten Planspiegel 831, 832 einer zweiten Spiegeltreppe 83 in einem zyklischen Zweistrahl-Interferometer 615 bevorzugt einen etwas unterschiedlichen Abstand dm_1 und dm_2 aufweisen.

Fourier-Transformations-Spektrometer,

wobei in einem zyklischen Zweistrahl-Interferometer 615 im Bereich Z, wo es keine räumliche Überdeckung der beiden Teilstrahlenbündel TB1, TB2 gibt, die erste Spiegeltreppe 82 und die zweite Spiegeltreppe 83 zu einer Doppelspiegeltreppe 85 mit einem Mittelsteg 84 kombiniert sind und die beiden inneren Planspiegel 822, 832 jeweils auf einer Seite des Mittelsteges 84 der Doppelspiegeltreppe 85 parallel zueinander angeordnet sind.

Fourier-Transformations-Spektrometer,

wobei in einem zyklischen Zweistrahl-Interferometer 615 mindestens einer Spiegeltreppe 82, 83 ein Bildfeld-Diskriminator BFD zugeordnet ist.

Fourier-Transformations-Spektrometer,

wobei der Bildfeld-Diskriminator BFD entweder als eine Spaltblende 631, 632, als ein schmales Pinhole-Array und/oder als ein gerastertes, rechnersteuerbares mikromechanisches Pinhole-Array ausgebildet ist.

Fourier-Transformations-Spektrometer,

wobei ein Bildfeld-Diskriminator BFD als ein schmaler Planspiegel 821s, 831s ausgebildet ist, welcher einen der beiden Planspiegel einer Spiegeltreppe 82, 83 bildet.

Fourier-Transformations-Spektrometer,

wobei ein Bildfeld-Diskriminator BFD als ein rechnersteuerbares Digitales Mikrospiegel-Array DMD in mindestens einer Spiegeltreppe 82, 83 ausgebildet ist.

Fourier-Transformations-Spektrometer

wobei in einem zyklischen Zweistrahl-Interferometer 615 sowohl ein vergleichsweise kleiner Winkel tau_1 von bis zu 10 Altgrad zwischen den beiden Planspiegeln 821, 822 einer ersten Spiegeltreppe 82w als auch ein vergleichsweise kleiner Winkel tau_2 von bis zu 10 Altgrad zwischen den beiden Planspiegeln 831, 832 einer zweiten Spiegeltreppe 82w besteht und die Beträge der Winkel tau_1, tau_2 in beiden Spiegeltreppen 82w, 83w zumindest näherungsweise gleich gemacht sind und der Winkel kappa zwischen dem Hauptstrahl HTB1a, der von der ersten Spiegeltreppe 82 abgeht und dem Hauptstrahl HTB2a, der von der zweiten Spiegeltreppe 83 abgeht, auf der dem Interferometer 615 zugekehrten Seite kleiner als 180 Altgrad, jedoch nicht kleiner als 140 Altgrad ausgebildet ist.

Fourier-Transformations-Spektrometer,

wobei in einem zyklischen Zweistrahl-Interferometer 615 mindestens eine miniaturisierte Spiegeltreppe im Bereich Z angeordnet ist, bei welcher mindestens ein Spiegel mit einer schwachen Krümmung ausgebildet ist.

Fourier-Transformations-Spektrometer,

wobei in einem zyklischen Zweistrahl-Interferometer 607, 609, 610, 612, 613, 614 für die Fourier-Transformations-Spektroskopie im umlaufenden Strahlengang zwei baugleiche Spiegelprismen 679, 680, 687, 688, 761, 763, 887, 889, 696, 689 aus jeweils gleichem refraktiven Material angeordnet sind

und die Spiegelprismen 679, 680, 687, 688, 761, 763, 887, 889 jeweils mit einer einzigen Spiegelfläche ausgebildet sind und diese Spiegelprismen 679, 680, 687, 688, 761, 763, 887, 889 jeweils den gleichen spitzen Winkel psi zwischen der Spiegelfläche und jeweils einer nichtverspiegelten Fläche aufweisen

und zwischen diesen beiden baugleichen Spiegelprismen zwei Bildfeld-Diskriminatoren BFD1, BFD2 positioniert sind.

Fourier-Transformations-Spektrometer,

wobei in einem zyklischen Zweistrahl-Interferometer 611, 614 für die Fourier-Transfonnations-Spektroskopie im umlaufenden Strahlengang zwei winkelgleiche Spiegelprismen 687, 696, 689 aus jeweils gleichem refraktiven Material angeordnet sind

und die Spiegelprismen 687, 696, 689 jeweils mit einer einzigen Spiegelfläche 687s, 689s, 696s, ausgebildet sind und diese Spiegelprismen 687, 689, 696, 689 jeweils den gleichen spitzen Winkel psi zwischen der Spiegelfläche 687s, 696s, 689s und jeweils einer nichtverspiegelten Fläche aufweisen

und zwischen diesen beiden winkelgleichen Spiegelprismen 687, 696, 689 zwei Bildfeld-Diskriminatoren BFD1, BFD2 positioniert sind.

Fourier-Transformations-Spektrometer,

wobei in einem zyklischen Zweistrahl-Interferometer 614 zwei Spiegelprismen 696, 689 aus gleichem refraktiven Material, welche nur jeweils eine einzige Spiegelfläche 696s, 689s aufweisen, angeordnet sind

und die beiden Spiegelprismen 696, 689 jeweils einen ersten spitzen Winkel psi, einen zweiten spitzen Winkel 2psi und einen dritten Winkel mit dem Betrag 180Altgrad minus 3psi aufweisen und so mindestens drei Winkel an den beiden Spiegelprismen 696, 689 jeweils übereinstimmen und außerdem eine Planparallelplatte 698 aus refraktivem Material zwischen diesen beiden Spiegelprismen 696, 689 angeordnet ist und die Planparallelplatte 698 durch zwei optisch transparente Kittschichten 695, 699 zwischen diesen beiden Spiegelprismen 696, 689 fixiert ist und entweder einerseits eine Strahlteilerschicht 62 auf der Seite der Planparallelplatte 698 aufgebracht ist, welche dem Spiegelprisma 689 zugekehrt ist,

oder andererseits die Strahlteilerschicht 62 auf dem Spiegelprisma 689 auf der Seite der Planparallelplatte 698 aufgebracht ist, welche der Planparallelplatte 698 zugekehrt ist.

Fourier-Transformations-Spektrometer mit zumindest teilweiser hyperspektraler Single-Shot-Bildgebung von einem Messobjekt als Ergebnis einer Berechnung mit einem Rechnersystem zur Gewinnung von Spektren mittels einer Fourier-Transformation

und mit einem vorgeordneten Objektiv als Abbildungssystem für das Messobjekt und mit einem zyklischen Zweistrahl-Interferometer, dem das vorgeordnete Objektiv vorangestellt ist und welches aufweist:

einen Strahlteiler mit ebener Strahlteilerfläche und der Strahlteiler sowohl zur Strahlenbündelteilung in der Strahlteilerebene genutzt wird, wodurch zwei Teilstrahlenbündel TB1, TB2 gebildet werden, als auch zur zumindest teilweisen Strahlenbündelvereinigung in der Strahlteilerebene mit einer Lateral-Shear s zwischen den beiden Teilstrahlenbündeln genutzt wird

und am zyklischen Zweistrahl-Interferometer eine Referenzebene besteht, welche durch die Normale der ebenen Strahlteilerfläche und durch die optische Achse des vorgeordneten Objektivs am Eingang des Interferometers aufgespannt ist,

und einen gerasterten Detektor am Ausgang des zyklischen Zweistrahl-Interferometers und das vorgeordnete Objektiv vor dem zyklischen Zweistrahl-Interferometer - unter Berücksichtigung der Position des Messobjekts - ein Bild oder mindestens ein Teilbild des Messobjekts in Lichtrichtung in der Regel nach dem Strahlteiler, jedoch in der Regel vor dem gerasterten Detektor, erzeugt

und unter Mitwirkung einer dem zyklischen Zweistrahl-Interferometer nachgeordneten anamorphotischen Abbildungsstufe eine Vielzahl, mindestens jedoch zwei, von räumlichen Interferogrammen gebildet wird und diese räumlichen Interferogramme auf dem gerasterten flächigen Empfänger ausgebildet sind und jedem räumlichen Interferogramm ein Objektpunkt auf dem Messobjekt zugeordnet ist,

wobei das zyklische Zweistrahl-Interferometer 616

mit zwei Planspiegeln 628, 629 ausgebildet ist, die eine Winkelspiegelanordnung 630 bilden, bei welcher die beiden Planspiegel 628, 629 von der Strahlteilerebene ET, welche die ebene Strahlteilerfläche enthält, gleich im Abstand sind und zumindest näherungsweise senkrecht zur Referenzebene RE ausgerichtet sind

und zwischen den beiden Planspiegeln, welche die Winkelspiegelanordnung 630 bilden, ein Winkel epsilon mit dem doppelten Betrag des Halbwinkels psi besteht und dieser Winkel epsilon durch zwei verlängerte Geraden g1, g2 aus den Planspiegeln oder aus den planen Spiegelflächen in der Referenzebene RE dargestellt ist und die Winkelhalbierende des Winkels epsilon in der Strahlteilerebene ET liegt

und der Halbwinkel psi mit einem Betrag größer als 20 Altgrad und mit einem Betrag kleiner als 30 Altgrad ausgebildet ist

und im symmetrisch aufgebauten zyklischen Zweistrahl-Interferometer 616 eine erste Bildebene BEI1 sowie eine zweite Bildebene BEI2 bestehen, welche jeweils durch das vorgeordnete Objektiv, die Lage des Messobjekts und das Zweistrahl-Interferometer 616 gegeben sind,

und der ersten Bildebene BEI1 ein erster Bildfeld-Diskriminator BFD1 sowie der zweiten Bildebene BEI2 ein zweiter Bildfeld-Diskriminator BFD2 im Zweistrahl-Interferometer 616 zugeordnet ist.

und diese beiden Bildfeld-Diskriminatoren BFD1, BFD2 im symmetrisch aufgebauten zyklischen Zweistrahl-Interferometer 616 so angeordnet sind, dass zumindest näherungsweise zwischen diesen Bildfeld-Diskriminatoren BFD1, BFD2 eine optische Konjugation besteht,

und im zyklischen Zweistrahl-Interferometer 616 zwischen den Planspiegeln 628, 629 im umlaufenden Strahlengang, die eine Winkelspiegelanordnung 630 bilden, je eine optische Baugruppe zur Erzeugung eines Strahlversatzes je einem Bildfeld-Diskriminator BFD zugeordnet ist und die optische Baugruppe einen Strahlversatz in jeweils entgegengesetzter Richtung erzeugen und so am Ausgang des zyklischen Zweistrahl-Interferometers eine Lateral-Shear s besteht.

Fourier-Transformations-Spektrometer, wobei die optischen Baugruppen zur Erzeugung eines Strahlversatzes zyklischen Zweistrahl-Interferometer als zwei Spiegeltreppen 82, 83 in Luft und/oder als zwei zumindest Rhomboidähnliche Spiegelprismen ausgebildet sind.

Fourier-Transformations-Spektrometer, wobei die Bildfeld-Diskriminatoren BFD1, BFD2 als Spaltblenden 831, 832 ausgebildet sind.

Es werden unter dem Begriff "Zweistrahl-Interferometer" hier drei Zweistrahl-Interferometer-Typen (Michelsontyp-Interferometer, Mach-Zehnder-Interferometer, zyklisches Zweistrahl-Interferometer) beschrieben. Die logische Klammer besteht darin, dass die Bildfeld-Diskriminierung in der Regel mittels zweier Bildfeld-Diskriminatoren bei allen drei Zweistrahl-Interferometer-Typen und in der Regel direkt im Zweistrahl-Interferometer an den beiden dort entstehenden kohärenten Bildern vom Messobjekt stattfindet. Dabei bleibt von je einem Bild nach der Bildfeld-Diskriminierung (z.B. durch zwei Spalt-blenden, (645-646, 652-653, 666-667) in der Regel nur jeweils ein eher schmales Teilbild für die Detektion übrig. Aus diesen beiden kohärenten Teilbildern kann jedoch aus allen Messflecken der-selben ein räumliches Interferogramm (rI) im Single-Shot-Modus gewonnen werden, so dass viele räumliche Interferogramme (rI) gleichzeitig detektiert werden können. So kann ein hyperspektrales Teilbild erzeugt werden. Aus zeitseriell gewonnenen Teilbildern kann sich das hyperspektrale Gesamtbild ergeben.

Ferner besteht eine logische Klammer, darin dass sowohl im Michelsontyp-Interferometer (Fig. 4 und Fig. 11) als auch im Mach-Zehnder-Interferometer (Fig. 12) ein Paar von versetzten Winkelspiegeln (Zweispiegel-Winkelreflektoren) jeweils als Luft-Typ (641-642) oder (661-662) oder Prismen-Typ (651-652) angeordnet ist. Demzufolge ist jeweils genau ein Winkelspiegel in einem Interferometerarm (IA1, IA2) der eben genannten Interferometer angeordnet.

Erfindungsgemäß ist jeweils ein Bildfeld-Diskriminator (meist eine Spaltblende 645, 646) einem Zweispiegel-Winkelreflektor (640) zugeordnet (vorgeordnet, nachgeordnet oder zwischen den beiden Spiegeln eines Zweispiegel-Winkelreflektors 640 angeordnet). Der QuerVersatz eines Zweispiegel-Winkelreflektors 640 im Zweistrahl-Interferometer bewirkt die Lateral-Shear s. Erfindungsgemäß erfolgt die Diskriminierung im oder am Zweispiegel-Winkelreflektor 640.

Alternativ zu jedem Zweispiegel-Winkelreflektor oder zu jedem Doppelspiegelprisma kann ein Tripelspiegel verwendet werden.

Im zyklischen Zweistrahl-Interferometer (Fig. 13 bis zu Fig. 37) liegt insbesondere nur genau ein Zweispiegel-Winkelreflektor (630) oder eine Doppel-Spiegelprismen-Anordnung (683) im umlaufenden Strahlengang als Luft-Typ, (also dann 630) oder als Spiegelprismen-Typ (also dann 683) vor. Eine Doppel-Spiegelprismen-Anordnung (683) hat insbesondere und im Wesentlichen dieselbe Funktion eines Zweispiegel-Winkelreflektors. Diesem Zweispiegel-Winkelreflektor (630) oder der Doppel-Spiegelprismen-Anordnung (683) sind in den beiden entgegengesetzt umlaufenden Teilstrahlengängen erfindungsgemäß jeweils genau ein Bildfeld-Diskriminator (631, 632) oder (656, 567) oder (677, 678) zugeordnet. So liegen in diesem Fall ebenfalls zwei Bildfeld-Diskriminatoren vor, die aber insbesondere nur dem Zweispiegel-Winkelreflektor (630) oder der Doppel-Spiegelprismen-Anordnung (683) zugeordnet sind, indem diese beiden Bildfeld-Diskriminatoren sich in der Regel innerhalb des Zweispiegel-Winkelreflektors (630) oder der Doppel-Spiegelprismen-Anordnung (683) befinden.

Für sein zyklisches Zweistrahl-Interferometer bestehen insbesondere zwei Möglichkeiten:

1. Die Lateral-Shear kann durch eine Asymmetrie im zyklischen Zweistrahl-Interferometer durch einen Versatz des Zweispiegel-Winkelreflektors (630) oder der Doppel-Spiegelprismen-Anordnung (683) zur Strahlteiler-Ebene ET erzeugt werden. Dieser Versatz wird durch d_ST, den Abstand des Schnittpunkts SP von der Strahlteilerebene ET, beschrieben. Dieser Abstand d_ST ist erstens ungleich null. Der Schnittpunkt SP liegt in der Symmetrie-Ebene E_S eines Zweispiegel-Winkelreilektors (630) oder einer Doppel-Spiegelprismen-Anordnung (683).

2. Die Lateral-Shear s entsteht im zweiten Fall d_ST gleich null, was eine Symmetrie in einem zyklischen Zweistrahl-Interferometer bewirkt, durch die Zuordnung von zwei Spiegeltreppen als Luft-Typ (Fig. 31 bis 36) oder als Prismen-Typ (ohne Figur) zwischen den Planspiegeln (628, 629) (des Zweispiegel-Winkelreflektors (630) oder den Spiegelprismen der Doppel-Spiegelprismen-Anordnung (683). Dabei ist es so, dass die Symmetrie - also der Fall d_ST gleich null - in einem zyklischen Zweistrahl-Interferometer nicht zwingend gegeben sein muss, um zwei Spiegeltreppen zuzuordnen. Dann basiert die entstehende Lateral-Shear s lediglich auf dem Vorhandensein der Spiegeltreppen und deren Anordnung.

3. Aber auch im Fall d_ST ungleich null können zwei Spiegeltreppen zugeordnet sein. Dann ergibt sich die resultierende Lateral-Shear s durch beides, und zwar durch die Asymmetrie (d_ST ungleich null) und das Vorhandensein von zwei Spiegeltreppen. Der Fall der Anordnung nur einer Spiegeltreppe ist nicht keine bevorzugte Variante, da in dem Fall i.d.R. eine relativ große optische Wegdifferenz zwischen den interferierenden Teilstrahlenbündeln entsteht.

Das erfindungsgemäße FT-Spektrometer kommt insbesondere dann vorteilhaft zum Einsatz, wenn bei lateral ausgedehnten Messobjekten eine Bildfeld-Diskriminierung notwendig ist sowie die Messflecken im hyperspektralen Bild vorbestimmt - um ein hohes Signal-RauschVerhältnis zu erreichen - eher recht grob gewählt sind und so die Tilt-invarianz der Zweispiegel-Winkelreflektoren oder das zyklische Interferometer unter Standardbedingungen keine Dejustierung des Interferometers zulassen.

Zusammenfassend wird bei einem Verfahren und einem Zweistrahl-Interferometer zur Single-Shot-Imaging-Fourier-Spektroskopie, insbesondere für das Messen von chaotisch bewegten Messobjekten und turbulenten Szenen, mit zumindest teilweiser hyperspektraler Bildgebung vom Messobjekt und/oder der Szene im Spektrometer ein Zweistrahl-Interferometer mit einem gerasterten Detektor eingesetzt. Das Zweistrahl-Interferometer kann dabei als Michelsontyp-Interferometer oder als Mach-Zehnder-Interferometer oder als zyklisches Zweistrahl-Interferometer ausgebildet sein. Am Ausgang des Interferometers werden jeweils zwei Teilstrahlenbündel TB1 und TB2 mit einer Lateral-Shear s gebildet. Dazu sind die im Interferometer paarweise angeordneten Planspiegel mindestens als ein Zweispiegel-Winkelreflektor ausgebildet, der lateral gegenüber dem ersten Lichtstrahl bzw. dem zweiten Lichtstrahl versetzt angeordnet ist oder diesem Zweispiegel-Winkelreflektor ist zusätzlich eine Spiegeltreppe zugeordnet. Es entsteht mittels vorgeordneten Objektivs und Interferometers ein Paar kohärenter Teilbilder des Messobjekts im Interferometer. Diese Teilbilder erfahren dort mittels zweier Spaltblenden eine Bildfeld-Diskriminierung. Nach Abbildung dieser diskriminierten Bilder und/oder Teilbilder mit Lateral-Shear s durch ein anamorphotisches Objektiv werden aus den Bildelementen der Teilbilder des Messobjekts räumliche Interferogramme auf dem Detektor gebildet, aus denen Spektren für ein hyperspektrales Bild errechnet werden.

Bezugszeichenliste mit Erläuterungen

| Bezugszeichen | Bezeichnung |
|---|---|
| 1 | Rücken eines älteren Patienten unter Diagnose als Messobjekt |
| 10 | biologisches Messobjekt |
| 11 | Hautmerkmal, welches aus medizinischer Sicht sorgfältig untersucht werden muss, da es sich um ein Muttermal und/oder ein Melanom handeln kann. |
| 12 | Hochrisikobereich, farblich auf dem Monitor hervorgehoben Die Risikohöhe kann nach Auswertung der Spektraldaten mittels künstlicher Intelligenz ermittelt werden. |
| 13 | Darstellung des Bereiches auf dem Rücken 1, der mittels eines Spektrometer-Scans erfasst wird |
| 14 | Messobjekt zur Auflichtmessung |
| 15 | bewegtes, teiltransparentes Messobjekt für eine Durchlichtmessung |

(fortgesetzt)

| Bezugszeichen | Bezeichnung |
|---|---|
| 16 | biologisches Messobjekt mit Fluoreszenzmarkern |
| 20 | Fourier-Transformations-Spektrometer-System |
| 21 | Rechnersystem für die Steuerung der Komponenten wie Lichtquelle und gerasterter Detektor und mit Rechenprogramm zur Auswertung der räumlichen Interferogramme rI und Berechnung von Spektren |
| 22 | Auswerteprogramm für die errechneten Spektren SP, auch um Hochrisikobereiche für eine Hautkrebserkrankung zu lokalisieren mit farblicher Kenntlichmachung für das ermittelte Risiko auf einem Monitor 23 |
| 23 | Monitor zur Darstellung der ausgewerteten Daten |
| 25 | Steuer- und Synchronisierungsmittel |
| 28 | Datenverbindung zu Datenbanken |
| 30 | mobiler Messkopf |
| 31 | Handgriff des mobilen Messkopfes 30 |
| 32 | Startknopf für die Datenaufnahme |
| 40 | gepulste Streifenlichtquelle im NIR-Bereich |
| 41 | Lichtquellentreiber, durch den Rechner 21 steuerbar |
| 42 | Datenverbindung |
| 43 | gepulste Streifenlichtquelle, durch den Rechner 21 steuerbar, deren Licht mittels eines Einkoppelstrahlteilerwürfels 57 koaxial in den Beleuchtungsstrahlengang eingekoppelt wird |
| 44 | gepulste NIR-Lichtquelle, durch den Rechner 21 steuerbar, mit integrierter Strahlformungsoptik 45 zur Projektion eines Lichtstreifens 80 auf ein biologisches Messobjekt 10 in koaxialer Anordnung |
| 45 | Strahlformungsoptik in Spiegelform 45 zur Projektion eines Lichtstreifens 80 auf ein biologisches Messobjekt 10 |
| 46 | Pentaprisma |
| 47 | schmale IR-Lichtquelle für eine streifenförmige Kurzzeit-Beleuchtung eines teiltransparenten, bewegten Messobjekts 15 |
| 48 | Objektiv für die Abbildung einer IR-Lichtquelle auf ein teiltransparentes Messobjekt 15 |
| 49 | schmale, gerasterte UV-Lichtquelle, durch den Rechner 21 steuerbar, welche mit dem transmissiven Flüssigkristall-Display 655 zeitlich synchronisiert ist und mit Ansteuerung von leuchtenden Pixeln ausgebildet ist, die zu einem transmissiven Flüssigkristall-Display 655 optisch konjugiert sind. |
| 491 | Kollimatorobjektiv für eine UV-Lichtquelle 49 |
| 50 | Optik-Einheit des mobilen Messkopfes 30 mit einem Zweistrahl-Interferometer 601 mit einem diesem nachgeordneten Objektiv 51 am Ausgang desselben und einem gerasterten Detektor 54, welcher der Detektion räumlicher Interferogramme rI dient. |
| 51 | dem Zweistrahl-Interferometer nachgeordnetes, anamorphotisches und weitgehend achromatisches Objektiv am Ausgang desselben, um von kohärenten Bildpunkten von einem Messobjekt 1 zueinander geneigte und interferierende Zylinderwellen auf einem gerasterten Detektor 54 zu erzeugen. |
| 511 | rotationssymmetrische Komponente des anamorphotischen Objektivs 51 |
| 512 | Zylinder-Komponente des anamorphotischen Objektivs 51 |

(fortgesetzt)

| Bezugszeichen | Bezeichnung |
|---|---|
| 52 | anamorphotisches Objektiv auch mit Zylinderkomponente 522 für den MIR-CaF2-Spektralbereich ausgebildet, welches in der optischen Gesamtfunktion prinzipiell dem Objektiv 51 entspricht und für einen chromatischen Öffnungsfehler im Interferometer korrigiert ist. |
| 521 | rotationssymmetrische Komponente des anamorphotischen Objektivs 52 |
| 522 | Zylinder-Komponente des anamorphotischen Objektivs 52 |
| 53 | anamorphotisches Spiegel-Objektiv auch mit Zylinderkomponente 532 für den MIR-Spektralbereich, welches in der optischen Gesamtfunktion prinzipiell dem Objektiv 51 entspricht und ohne Öffnungsfehlerkorrektur ausgebildet ist. |
| 531 | rotationssymmetrische Komponente des anamorphotischen Objektivs 53 |
| 532 | Zylinder-Komponente des anamorphotischen Objektivs 53 |
| 54 | InGaAs-Kamera für den nahinfraroten Spektralbereich, welche durch den Rechner 21 steuerbar ausgebildet ist |
| 55 | CMOS-Kamera für den sichtbaren Spektralbereich, welche durch den Rechner 21 steuerbar ausgebildet ist |
| 56 | Steuer- und Datenverbindung |
| 57 | Einkoppelstrahlteilerwürfel für Licht zur Beleuchtung eines Messobjekts |
| 571 | dichroitischer Einkoppelstrahlteilerwürfel mit einer reflektierenden Teilerschicht für UV-Licht und mit Transmissionseigenschaften der Teilerschicht für das Fluoreszenzlicht im VIS-Spektralbereich |
| 572 | eindimensionaler Galvano-Spiegel-Scanner, durch den Rechner 21 steuerbar |
| 573 | Mikroskopobjektiv |
| 574 | Umlenkspiegel |
| 575 | telezentrische Blende |
| 576 | UV-Sperrfilter |
| 58 | anamorphotisches Objektiv mit chromatischer Öffnungsfehler-Korrektur für den Polarisationsstrahlteilerwürfel 636 |
| 581 | rotationssymmetrische Komponente des anamorphotischen Objektivs 58 |
| 582 | Zylinder-Komponente des anamorphotischen Objektivs 58 |
| 59 | anamorphotisches Objektiv für den MIR-Bereich, auch mit Spiegeloptik |
| 591 | rotationssymmetrische Komponente des anamorphotischen Objektivs 59 |
| 592 | Zylinder-Komponente des anamorphotischen Objektivs 59 |
| 595 | anamorphotisches Objektiv für den MIR-Bereich mit drei spiegelnden Freiformflächen 875, 876 und 877 |
| 601 | Zweistrahl-Interferometer, welches eine Lateral-Shear s erzeugt |
| 602 | Michelsontyp-Interferometer mit Lateral-Shear s, welches hier mit einem Teilerwürfel und einem Dachkantreflektor insbesondere mit Metall ausgebildet ist |
| 603 | Michelsontyp-Interferometer mit einer Lateral-Shear s, welches hier mit einem Teilerwürfel und einem 90°-Dachkantprisma ausgebildet ist |
| 604 | Mach-Zehnder-Interferometer mit einer Lateral-Shear s |
| 605 | zyklisches Zweistrahl-Interferometer mit Lateral-Shear s mit zwei Planspiegeln 628 und 629 und einem Folienstrahlteiler 623 |

| Bezugszeichen | Bezeichnung |
|---|---|
| 606 | zyklisches Zweistrahl-Interferometer für den sichtbaren Spektralbereich mit zwei Planspiegeln 671 und 672 und einem Polarisationsstrahlteilerwürfel 636 |
| 607 | zyklisches Zweistrahl-Interferometer für den sichtbaren Spektralbereich mit zwei Spiegelprismen 679 und 680 und einem Strahlteilerwürfel 622 |
| 608 | zyklisches Zweistrahl-Interferometer mit zwei Planspiegeln 628 und 629 und einem Plattenstrahlteiler |
| 609 | zyklisches Zweistrahl-Interferometer mit zwei baugleichen Prismen 690 und 691 und zwei baugleichen Spiegelprismen 687 und 688 und mit einem Luftspalt im Rachen |
| 610 | zyklisches Zweistrahl-Interferometer mit zwei baugleichen Prismen 690 und 691 und zwei baugleichen Spiegelprismen 687 und 688 und ohne Luftspalt |
| 611 | zyklisches Zweistrahl-Interferometer mit einem Dreieckprisma 690 und zwei Spiegelprismen 687 und 689 und ohne Luftspalt |
| 612 | zyklisches Zweistrahl-Interferometer mit zwei Spiegelprismen 761 und 763 insbesondere aus CaF2, welche die Doppel-Spiegelprismen-Anordnung 683 bilden, und den geneigten Planparallelplatten 92 und 95 insbesondere aus CaF2 |
| 613 | zyklisches Zweistrahl-Interferometer für den infraroten Spektralbereich mit einem Strahlteiler 623 insbesondere mit Mylarfolie und zwei Spiegelprismen 687 und 689 insbesondere mit ZnSe |
| 614 | zyklisches Zweistrahl-Interferometer für den VIS- und NIR-Spektralbereich als gekitteter Block mit eingekitteter Planparallelplatte 698 |
| 615 | zyklisches Zweistrahl-Interferometer für den fern-infraroten Spektralbereich insbesondere mit einer Mylarfolie 86 zur Strahlteilung und zwei Planspiegeln 628 und 629 |
| 616 | symmetrisch aufgebautes zyklisches Zweistrahl-Interferometer Es ist d_ST=0. |
| 62 | Strahlteilerschicht Diese stellt die ebene Strahlteilerfläche dar. |
| 620 | Strahlteilereinheit, die die Planparallelplatten 624 und 627 und die Strahlteilerschichten 625 und 626 aufweist. |
| 622 | Strahlteilerwürfel |
| 623 | Strahlteiler insbesondere mit Mylarfolie |
| 624 | Planparallelplatte insbesondere mit CaF2 mit einer Strahlteilerschicht 625 |
| 625 | Strahlteilerschicht für den Wellenzahlbereich $4000cm^{-1}$ bis $1200cm^{-1}$ Diese stellt die ebene Strahlteilerfläche dar. |
| 626 | Strahlteilerschicht zur Strahlenbündelzusammenführung für den Wellenzahlbereich $4000cm^{-1}$ bis $1200cm^{-1}$ Diese stellt die ebene Strahlteilerfläche dar. |
| 627 | Planparallelplatte insbesondere mit CaF2 als Substrat für die Strahlteilerschicht 626 |
| 628 | erster Planspiegel in einem zyklischen Interferometer für den MIR- und FIR-Spektralbereich, welcher Bestandteil des Zweispiegel-Winkelreflektors 630 ist |
| 629 | zweiter Planspiegel in einem zyklischen Interferometer für den MIR- und FIR-Spektralbereich, welcher Bestandteil des Zweispiegel-Winkelreflektors 630 ist |
| 630 | Winkelspiegelanordnung, insbesondere Winkelreflektor, bevorzugt Zweispiegel-Winkelreflektor (630), der mittels der Planspiegel 628 und 629 für den infraroten und/oder mittels der Planspiegel 671 und 672 für den sichtbaren Spektralbereich gebildet ist. Die Strahlumlenkungseinheit ist hier als Zweispiegel-Winkelreflektor ausgebildet 630. |

(fortgesetzt)

| Bezugszeichen | Bezeichnung |
|---|---|
| 631 | Spaltblende für das Teilstrahlenbündel TB 1 mit einer Spaltöffnung im Sinne einer Abschattblende, die einen Bildfeld-Diskriminator in der Bildebene BEI1 darstellt. Die Breite einer Spaltöffnung sollte den Wert d_ST, also den Abstand zwischen der Symmetrieebene E _S und der Strahlteilerebene ET und Strahlvereinigung, nicht überschreiten. |
| 632 | Spaltblende für das Teilstrahlenbündel TB2 mit einer Spaltöffnung im Sinne einer Abschattblende, die einen Bildfeld-Diskriminator in der Bildebene BEI2 darstellt. |
| 633 | Hilfs-Abschattblende, um optisches Übersprechen zu vermeiden |
| 634 | Mikrobolometer-Array, durch den Rechner 21 steuerbar |
| 636 | Polarisationsstrahlteilerwürfel |
| 637 | polarisierende Strahlteilerschicht Diese stellt die ebene Strahlteilerfläche dar. |
| 638 | Polarisationsanalysator |
| 641 | Zweispiegel-Winkelreflektor, insbesondere ausgebildet als 90-Altgrad-Dachkantreflektor insbesondere mit Metall, also ein Hohlkörper, hergestellt durch eine Single-point-Diamantbearbeitung für eine 180 Altgrad-Umlenkung im ersten Arm IA1 des Michelsontyp-Interferometers 602, dargestellt in den Figuren 4 bis 8 Eine Strahlumlenkungseinheit ist hier als Zweispiegel-Winkelreflektor ausgebildet 641. |
| 641a | erster Spiegel, dargestellt in den Figuren 4 bis 8 |
| 641b | zweiter Spiegel, dargestellt in den Figuren 4 bis 8 |
| 642 | Zweispiegel-Winkelreflektor, insbesondere ausgebildet als 90-Altgrad-Dachkantreflektor und insbesondere mit Metall, also ein Hohlkörper, hergestellt durch eine Single-point-Diamantbearbeitung für eine 180 Altgrad-Umlenkung im zweiten Arm IA2 des Michelsontyp-Interferometers 602, dargestellt in den Figuren 4 bis 8 Eine Strahlumlenkungseinheit ist hier als Zweispiegel-Winkelreflektor ausgebildet 641. |
| 642a | erster Spiegel, dargestellt in den Figuren 4 bis 8 |
| 642b | zweiter Spiegel, dargestellt in den Figuren 4 bis 8 |
| 643 | Metallgrundkörper im ersten Arm IA1 des Michelsontyp-Interferometers 602, dargestellt in den Figuren 5 bis 8 |
| 644 | Metallgrundkörper im zweiten Arm IA2 des Michelsontyp-Interferometers 602, dargestellt in den Figuren 5 bis 8 |
| 645 | Spaltblende mit der Breite b in den Figuren 5 und 6 im ersten Arm IA1 eines Michelsontyp-Interferometers 602, welche einen Bildfeld-Diskriminator darstellt |
| 646 | Spaltblende mit der Breite b, dargestellt in den Figuren 5 und 6, im zweiten Arm IA2 eines Michelsontyp-Interferometers 602, welche einen Bildfeld-Diskriminator darstellt |
| 647-1 | Piezosteller, dargestellt in Figur 6, im ersten Interferometerarm IA1 eines Michelsontyp-Interferometers 602, durch den Rechner 21 steuerbar, welcher der Spaltblende 645 zugeordnet ist |
| 647-2 | Piezosteller, dargestellt in Figur 6, im zweiten Interferometerarm IA2 eines Michelsontyp-Interferometers 602, durch den Rechner 21 steuerbar, welcher der Spaltblende 646 zugeordnet ist |
| 648-1 | längliches Pinhole-Array mit der Breite b, dargestellt in Figur 7, im ersten Interferometerarm IA1, welches eine Vielzahl von Bildfeld-Diskriminatoren darstellt |
| 648-2 | längliches Pinhole-Array mit der Breite b, dargestellt in Figur 7, im zweiten Interferometerarm IA2, welches eine Vielzahl von Bildfeld-Diskriminatoren darstellt |
| 649-1 | erstes transmissives Flüssigkristall-Display (LCD), dargestellt in Figur 8, im ersten Interferometerarm IA1, durch den Rechner 21 steuerbar |

(fortgesetzt)

| Bezugszeichen | Bezeichnung |
|---|---|
| 649-2 | zweites transmissives Flüssigkristall-Display (LCD), dargestellt in Figur 8, im zweiten Interferometerarm IA2, durch den Rechner 21 steuerbar |
| 650-1 | länglicher lichtdurchlässiger Bereich der Breite b, dargestellt in Figur 8, eingeschrieben in transmissives Flüssigkristall-Display im ersten Interferometerarm IA1 |
| 650-2 | länglicher lichtdurchlässiger Bereich der Breite b, dargestellt in Figur 8, eingeschrieben in transmissives Flüssigkristall-Display im zweiten Interferometerarm IA2 |
| 651 | Prisma, bevorzugt ausgebildet als 90°-Dachkantprisma insbesondere für 180° Umlenkung im ersten Interferometerarm IA1 mit zwei Reflexionsflächen bzw. Spiegelflächen, dargestellt in Figur 11 Das Prisma 651 stellt einen Zweispiegel-Winkelreflektor dar und ist eine Strahlumlenkungseinheit. |
| 651a | erste Spiegelfläche am Prisma 651, dargestellt in Figur 11 |
| 651b | zweite Spiegelfläche am Prisma 651, dargestellt in Figur 11 |
| 652 | Prisma, bevorzugt ausgebildet als 90°-Daclikantprisma insbesondere für eine 180° Strahlumlenkung im zweiten Interferometerarm IA2 mit zwei Reflexionsflächen bzw. Spiegelflächen, dargestellt in Figur 11 Das Prisma 652 stellt einen Zweispiegel-Winkelreflektor dar und ist eine Strahlumlenkungseinheit. |
| 652a | erste Spiegelfläche am Prisma 652, dargestellt in Figur 11 |
| 652b | zweite Spiegelfläche am Prisma 652, dargestellt in Figur 11 |
| 653 | lange, spaltförmige Spaltblende mit der Breite b, dargestellt in Figur 11, im ersten Interferometerarm IA1 Die Spaltblende 653 stellt einen Bildfeld-Diskriminator dar. |
| 654 | lange, spaltförmige Spaltblende mit der Breite b, dargestellt in Figur 11, im zweiten Interferometerarm IA2 Die Spaltblende 654 stellt einen Bildfeld-Diskriminator dar. |
| 655 | transmissives Flüssigkristall-Display in einem zyklischen Interferometer, durch den Rechner 21 steuerbar |
| 656 | erster Durchlassbereich in einem transmissiven Flüssigkristall-Display 655 in einem zyklischen Interferometer, stellt den Bildfeld-Diskriminator BFD1 dar |
| 657 | zweiter Durchlassbereich in einem transmissiven Flüssigkristall-Display 655 in einem zyklischen Interferometer, stellt den Bildfeld-Diskriminator BFD2 dar |
| 661 | Zweispiegel-Winkelreflektor im ersten Interferometerarm IA1 eines Mach-Zehnder-Interferometers 604 Eine Strahlumlenkungseinheit ist hier als Zweispiegel-Winkelreflektor ausgebildet 661. |
| 661a | Planspiegel |
| 661b | Planspiegel |
| 662 | Zweispiegel-Winkelreflektor im zweiten Interferometerarm IA2 eines Mach-Zehnder-Interferometers 604 Eine Strahlumlenkungseinheit ist hier als Zweispiegel-Winkelreflektor ausgebildet 661. |
| 662a | Planspiegel |
| 662b | Planspiegel |
| 666 | Spaltblende im Mach-Zehnder-Interferometers 604, angeordnet bei der kleinsten Bündeleinschnürung und ausgebildet mit einer mindestens doppelt so breiten Spaltöffnung wie bei der feinen Spaltblende 667, stellt einen Bildfeld-Diskriminator dar |
| 667 | feine Spaltblende im Mach-Zehnder-Interferometers 604 in der Bildebene, stellt einen Bildfeld-Diskriminator dar |

| Bezugszeichen | Bezeichnung |
|---|---|
| 668 | Planplattengruppe zur Einstellung und/oder Beeinflussung der optischen Weglängendifferenz OPD _zykaP, dargestellt in Fig. 18a, die bevorzugt für den MIR-Spektralbereich ausgebildet ist |
| 669 | Planplattengruppe zur Einstellung und/oder Beeinflussung der optischen Weglängendifferenz OPD_zykaP zur vollständigen Kompensation der Bildverschiebung in der Tiefe, dargestellt in Fig. 19a, die bevorzugt für den MIR- Bereich ausgebildet ist |
| 670a, 670b | Planplattengruppe zur Einstellung und/oder Beeinflussung der optischen Weglängendifferenz OPD_zykaP zur vollständigen Kompensation der Bildverschiebung in der Tiefe, bevorzugt für den VIS- und NIR-Bereich ausgebildet und dargestellt in den Fig. 28a und Fig. 29 |
| 671 | erster Planspiegel in einem zyklischen Interferometer für den VIS-Spektralbereich, welcher Bestandteil des Zweispiegel-Winkelreflektors 630 ist |
| 672 | zweiter Planspiegel in einem zyklischen Interferometer für den VIS-Spektralbereich, welcher Bestandteil des Zweispiegel-Winkelreflektors 630 ist |
| 673 | CaF2-Strahlteilerplatte |
| 674 | Strahlteilerschicht |
| 675 | CaF2-Kompensationsplatte |
| 676 | Doppelspaltblende in einem zyklischen Zweistrahl-Interferometer mit den Spaltöffnungen 677 und 678 |
| 677 | erste Spaltöffnung, stellt einen Bildfeld-Diskriminator BFD1 dar |
| 678 | zweite Spaltöffnung, stellt einen Bildfeld-Diskriminator BFD2 dar |
| 679 | Spiegelprisma im Arm IA1 eines zyklischen Zweistrahl-Interferometers 607 |
| 680 | Spiegelprisma im Arm IA2 eines zyklischen Zweistrahl-Interferometers 607 |
| 681 | Spiegelfläche im Arm IA1 eines zyklischen Zweistrahl-Interferometers an einem Spiegelprisma |
| 682 | Spiegelfläche im Arm IA2 eines zyklischen Zweistrahl-Interferometers an einem Spiegelprisma |
| 683 | Doppel-Spiegelprismen-Anordnung für den mittelinfraroten Spektralbereich mit den beiden Spiegelprismen 761 und 763 Die Doppel-Spiegelprismen-Anordnung 683 stellt einen Zweispiegel-Winkelreflektor dar. |
| 685 | zylindrische Wellenfronten |
| 686 | Scheitellinien zueinander geneigter zylindrischer Wellenfronten |
| 687 | Spiegelprisma im Arm IA1 eines zyklischen Zweistrahl-Interferometers, in einer Aufspannung mit dem Spiegelprisma 688 hergestellt und somit baugleich und auch insbesondere mit gleichem Material |
| 687F | Frontfläche des Spiegelprismas 687, hier nur Zweck der Schaffung einer optischen Justiermöglichkeit auspoliert |
| 687s | Spiegelfläche des Spiegelprismas 687 |
| 687u | unverspiegelte Fläche des Spiegelprismas 687, genutzt sowohl zum Strahl-Eintritt als auch zum Strahl-Austritt |
| 688 | Spiegelprisma im Arm IA2 eines zyklischen Zweistrahl-Interferometers Dieses ist in einer Aufspannung mit dem Spiegelprisma 687 hergestellt und somit baugleich und auch aus gleichem Material. |
| 688F | Frontfläche des Spiegelprismas 688, hier nur Zweck der Schaffung einer optischen Justiermöglichkeit auspoliert |
| 688s | Spiegelfläche des Spiegelprismas 688 |
| 689 | Spiegelprisma |

(fortgesetzt)

| Bezugszeichen | Bezeichnung |
|---|---|
| 689s | Spiegelfläche des Spiegelprismas 689 |
| 689u | unverspiegelte Fläche des Spiegelprismas 689 sowohl zum Strahl-Eintritt als auch zum Strahl-Austritt |
| 690 | spitzwinkliges und auch gleichschenkliges Dreieckprisma mit einem Winkel von 2psi in einem gekitteten Strahlteiler, baugleich mit Dreieckprisma 691 |
| 691 | spitzwinkliges und auch gleichschenkliges Dreieckprisma mit einem Winkel von 2psi in einem gekitteten Strahlteiler, baugleich mit Dreieckprisma 690 |
| 692 | gekitteter Strahlteiler mit zwei baugleichen Dreieckprismen 690 und 691 |
| 693 | gekitteter Strahlteiler insbesondere mit dem Spiegelprisma 689 und dem Dreieckprisma 690, die nicht baugleich sind |
| 694 | gedruckte Doppelspaltblende in einer Kittgruppe in einem zyklischen Zweistrahl-Interferometer 610 mit zwei Spaltöffnungen |
| 695 | dünne, transparente Kittschicht zwischen dem Spiegelprisma 689 und der Strahlteilerschicht 62, die sich auf der Planparallelplatte 698 befindet |
| 696 | baugleiches Spiegelprisma wie 689, gespiegelt angeordnet |
| 696s | Spiegelschicht auf dem Spiegelprisma wie 689 |
| 697 | Doppel-Spiegelprismen- Anordnung Diese Doppel-Spiegelprismen-Anordnung 697 ist jeweils mit den beiden Spiegelprismen: 679 und 680 (VIS-Bereich) oder 687 und 688 oder 687 und 689 oder 689 und 696 oder 887 und 889 gebildet. Die Doppel-Spiegelprismen-Anordnung 697 stellt auch einen Zweispiegel-Winkelreflektor dar. Der Zweispiegel-Winkelreflektor ist eine Strahlumlenkungseinheit. |
| 698 | Planparallelplatte mit Strahlteilerschicht 62 zwischen den Spiegelprismen 689 und 696 |
| 699 | dünne, transparente Kittschicht zwischen dem Spiegelprisma 696 und der Planparallelplatte 698 |
| 70 | vorgeordnetes Objektiv Es ist dem zyklischen Zweistrahl-Interferometer 601 vorgeordnet und stellt ein Abbildungssystem dar. |
| 71 | vorgeordnetes Objektiv mit vorgeordneter telezentrischer Blende 72 Es ist dem zyklischen Zweistrahl-Interferometer 602 vorgeordnet und stellt ein Abbildungssystem dar. |
| 72 | telezentrische Blende |
| 73 | vorgeordnetes Objektiv für den MIR-Spektralbereich ohne Öffiiungsfehlerkorrektur Es ist jeweils den zyklischen Zweistrahl-Interferometern 604 und 605 vorgeordnet und stellt ein Abbildungssystem dar. |
| 74 | vorgeordnetes Objektiv für den VIS-Spektralbereich mit Korrektur der sphäro-chromatischen Aberration für refraktive Komponenten, welche entfaltet Planparallelplatten darstellen Es ist jeweils den zyklischen Zweistrahl-Interferometern 606 und 607 vorgeordnet und stellt ein Abbildungssystem dar. |
| 75 | vorgeordnetes Objektiv für den MIR-Spektralbereich, insbesondere mit Korrektur der sphäro-chromatischen Aberration für die CaF2-Platten 673, 675 und 92 Es ist dem zyklischen Zweistrahl-Interferometer 608 vorgeordnet und stellt ein Abbildungssystem dar. |
| 751 | vorgeordnetes Spiegelobjektiv für den MIR-Spektralbereich, ausgebildet mit der Freiformfläche 872 am Spiegelblock 871 und der ersten Freiformfläche 874 am Spiegelblock 873 Es ist dem zyklischen Zweistrahl-Interferometer 615 vorgeordnet und stellt ein Abbildungssystem dar. |
| 761 | Spiegelprisma insbesondere aus Kalziumfluorid |
| 762 | Spiegelschicht auf Spiegelprisma 761 |
| 763 | Spiegelprisma insbesondere aus Kalziumfluorid |

(fortgesetzt)

| Bezugszeichen | Bezeichnung |
|---|---|
| 764 | Spiegelschicht auf Spiegelprisma 763 |
| 765 | erste Planparallelplatte insbesondere mit ZnSe der geometrischen Dicke h1 für den MIR-Spektralbereich, mit dem Brechungsindex n1 und mit Spaltblende |
| 766 | zweite Planparallelplatte insbesondere mit ZnSe der geometrischen Dicke h2 für den MIR-Spektralbereich, mit dem Brechungsindex n1 und mit Spaltblende |
| 767 | erste Planparallelplatte insbesondere mit CaF2 der geometrischen Dicke h1 für den MIR-Spektralbereich mit dem Brechungsindex n1 und mit Spaltblende |
| 768 | zweite Planparallelplatte insbesondere mit ZnSe der geometrischen Dicke h2 für den MIR-Spektralbereich mit dem Brechungsindex n2 und mit Spaltblende |
| 781 | dünne, transparente Kittschicht |
| 782 | dünne, transparente Kittschicht |
| 80 | Lichtstreifen, welcher projiziert wird, und in der Höhe und Länge etwas überdimensioniert ist, damit das Messfeld 81 in der Regel voll ausgeleuchtet ist. Das Messfeld 81 ist mittels einer Rückabbildung der Bildfeld-Diskriminatoren BFD auf das Messobjekt bestimmt. |
| 81 | Messfeld, welches zu einem Zeitpunkt t1 erfasst wird |
| 82 | erste Spiegeltreppe für das Teilstrahlenbündel TB1 mit zwei Planspiegeln 821 und 822 in Parallelanordnung mit dem Spiegelabstand dm_1 |
| 82w | erste Spiegeltreppe in einer Winkelanordnung für das Teilstrahlenbündel TB1 mit zwei Planspiegeln 821 und 822 |
| 821 | erster Planspiegel der ersten Spiegeltreppe 82 für das Teilstrahlenbündel TB1, hergestellt in Single-point-Diamantbearbeitung und goldbeschichtet |
| 821s | erster Planspiegel der ersten Spiegeltreppe 82 für das Teilstrahlenbündel TB1 Dieser Planspiegel ist schmal ausgebildet sowie goldbeschichtet und wirkt hier als Bildfeld-Diskriminator. Die Herstellung erfolgt mittels einer Single-point-Diamantbearbeitung. |
| 822 | zweiter Planspiegel der ersten Spiegeltreppe 82 für das Teilstrahlenbündel TB 1 Dieser Planspiegel ist mittels einer Single-point-Diamantbearbeitung hergestellt und goldbeschichtet. |
| 822s | zweiter Planspiegel der ersten Spiegeltreppe 82 für das erste Teilstrahlenbündel TB1 Dieser Planspiegel ist schmal ausgebildet sowie goldbeschichtet und wirkt hier als Bildfeld-Diskriminator. Die Herstellung erfolgt mittels einer Single-point-Diamantbearbeitung. |
| 83 | zweite Spiegeltreppe für das zweite Teilstrahlenbündel TB2 mit zwei Planspiegeln 831 und 832 in Parallelanordnung mit dem Spiegelabstand dm_2 und es gilt: dm_2 ist kleiner als dm_1. |
| 83w | zweite Spiegeltreppe in einer Winkelanordnung für das Teilstrahlenbündel TB2 mit zwei Planspiegeln 831 und 832 |
| 831 | erster Planspiegel der ersten Spiegeltreppe 83 für das Teilstrahlenbündel TB2, hergestellt mittels einer Single-point-Diamantbearbeitung und goldbeschichtet |
| 831s | erster Planspiegel der ersten Spiegeltreppe 83 für das Teilstrahlenbündel TB2 Dieser Planspiegel ist schmal ausgebildet sowie goldbeschichtet und wirkt hier als Bildfeld-Diskriminator. Die Herstellung erfolgt mittels einer Single-point-Diamantbearbeitung. |
| 832 | zweiter Planspiegel der zweiten Spiegeltreppe 83 für das Teilstrahlenbündel TB2, hergestellt in Single-point-Diamantbearbeitung und goldbeschichtet |
| 832s | zweiter Planspiegel der zweiten Spiegeltreppe 83 für das Teilstrahlenbündel TB2 Dieser Planspiegel ist schmal ausgebildet sowie goldbeschichtet und wirkt hier als Bildfeld-Diskriminator. Die Herstellung erfolgt mittels einer Single-point-Diamantbearbeitung. |
| 84 | Mittelsteg insbesondere mit Metall mit den beiden Planspiegeln 822 und 832, hergestellt in Single-point-Diamantbearbeitung |

(fortgesetzt)

| Bezugszeichen | Bezeichnung |
|---|---|
| 85 | Doppel-Spiegeltreppe mit vier Planspiegeln 821, 822, 831, 832, die parallel zueinander ausgerichtet sind, ausgebildet als metallischer Monolith und hergestellt in Single-point-Diamantbearbeitung |
| 85w | Doppel-Spiegeltreppe mit vier Planspiegeln 821, 822, 831, 832, die paarweise zueinander geneigt sind, ausgebildet als metallischer Monolith und hergestellt in Single-point-Diamantbearbeitung |
| 86 | Mylarfolie zur Strahlteilung für den ferninfraroten Spektralbereich, beispielsweise für den Wellenzahlbereich um 200cm$^{-1}$ |
| 861 | Vorblende zur Bündelbegrenzung, die jedoch keine Bildfeldblende darstellt. |
| 870 | Anordnung mit einer Spiegeloptik und mit einem zyklischem Zweistrahl-Interferometer 615 für die Messung im Fernbereich im FIR |
| 871 | erster Spiegelblock zur Fokussierung der ankommenden elektromagnetischen Strahlung im FIR mittels der Freiformfläche 872 |
| 872 | erste spiegelnde Freiformfläche am Spiegelblock 871 |
| 873 | zweiter Spiegelblock |
| 874 | erste spiegelnde Freiformfläche am Spiegelblock 873 und dabei insgesamt die zweite Freiformfläche |
| 875 | zweite spiegelnde Freiformfläche am Spiegelblock 873 und dabei insgesamt die dritte spiegelnde Freiformfläche |
| 876 | zweite spiegelnde Freiformfläche am Spiegelblock 871, welche in Sattelform ausgebildet ist, und dabei insgesamt die vierte spiegelnde Freiformfläche |
| 877 | dritte spiegelnde Freiformfläche am Spiegelblock 873 und dabei insgesamt die fünfte spiegelnde Freiformfläche Diese dient der Auskopplung der Teilstrahlenbündel TB1 und TB2 zwecks Detektion von räumlichen Interferogrammen rI. Die spiegelnden Freiformflächen 875, 876 und 877 bilden ein nachgeordnetes anamorphotisches Objektiv. |
| 887 | Spiegelprisma insbesondere mit ZnSe im Arm IA1 eines zyklischen Zweistrahl- Interferometers |
| 888 | Spiegelfläche auf dem Spiegelprisma 887 |
| 889 | Spiegelprisma insbesondere mit ZnSe im Arm IA2 eines zyklischen Zweistrahl- Interferometers |
| 890 | Spiegelfläche auf dem Spiegelprisma 889 |
| 891 | unverspiegelte Eintrittsfläche auf dem Spiegelprisma 887 |
| 892 | unverspiegelte Austrittsfläche auf dem Spiegelprisma 887 |
| 893 | unverspiegelte Eintrittsfläche auf dem Spiegelprisma 889 |
| 894 | unverspiegelte Austrittsfläche auf dem Spiegelprisma 889 |
| 92 | geneigte Planparallelplatte insbesondere mit CaF2 vor einem zyklischen Zweistrahl-Interferometer zur Kompensation von Astigmatismus und Koma |
| 93 | geneigte Planparallelplatte insbesondere mit CaF2 im ersten Arm eines Mach-Zehnder-Interferometers zur Kompensation von Astigmatismus und Koma |
| 94 | geneigte Planparallelplatte insbesondere mit CaF2 im zweiten Arm eines Mach-Zehnder-Interferometers zur Kompensation von Astigmatismus und Koma |
| 95 | geneigte Planparallelplatte insbesondere mit CaF2 nach einem zyklischen Zweistrahl-Interferometer zur Kompensation von Astigmatismus |
| 971 | erste Planparallelplatte insbesondere mit Glas der Höhe h1 und insbesondere mit optischem Material mit dem Brechungsindex n1 und vorzugsweise mit n1 < n2 |

| Bezugszeichen | Bezeichnung |
|---|---|
| 972 | zweite Planparallelplatte insbesondere mit Glas der Höhe h2 und insbesondere mit optischem Material mit dem Brechungsindex n2 und vorzugsweise mit n1 < n2 |
| 973 | Trägerplatte insbesondere mit Glas der Höhe h1 und insbesondere mit optischem Material mit dem Brechungsindex n1 und vorzugsweise mit n1 < n2 |
| 974 | Trägerplatte insbesondere mit Glas der Höhe h2 und insbesondere mit optischem Material mit dem Brechungsindex n2 und vorzugsweise mit n1 < n2 |
| 975 | Doppelspaltblende auf Trägerplatte 973 Oder 974 |
| 976 | erste Spaltöffnung in der Doppelspaltblende 975, stellt einen Bildfeld-Diskriminator dar. |
| 977 | zweite Spaltöffnung in der Doppelspaltblende 975, stellt einen Bildfeld-Diskriminator dar. |
| A | Punkt |
| a | Mittenabstand der Spaltöffnungen, entspricht vorzugsweise der halben Lateral-Shear s |
| alpha | Aperturwinkel, gleich dem halben Öffnungswinkel |
| b | Breite der beiden Bildfeld-Diskriminatoren BFD1 und BFD2, die vorzugsweise zumindest näherungsweise gleich ist. Im zyklischen Zweistrahl-Interferometer (605, 606, 607) ist b die Gesamtbreite des nichtdiskriminierenden Bereichs, also beispielsweise der Öffnung desselben, eines Bildfeld-Diskriminators im umlaufenden Strahlengang. Die Breite b kann die Spaltbreite einer einfachen spaltförmigen Abschattblende 631 beziehungsweise 632 und/oder die Gesamtbreite einer feinstrukturierten Abschattblende sein. Die Breite b kann aber auch die maximale Breite des Durchlassbereiches eines Flüssigkristall-Displays sein, auch, wenn dieses einen feinstrukturierten Durchlassbereich aufweist. Die Breite b kann aber auch die Breite eines schmalen Planspiegels darstellen, der als Bildfeld-Diskriminators genutzt wird und/oder die Gesamtbreite von reflektierenden Mikrospiegeln eines Digitalen Mikrospiegel- Arrays. Die Breite b kann auch die Spaltbreite der Spaltblende 645 (646) im Dachkantreflektor 641a (641b) darstellen, der bevorzugt ein rechnersteuerbarer Piezostellers 647 zugeordnet sein kann. |
| b' | Breite des lateralen Messobjekt-Inkrements durch eine gedachte Rückabbildung einer spaltförmigen Öffnung der beiden baugleichen und exakt einjustierten Bildfeld-Diskriminatoren, welche optisch zueinander konjugiert sind |
| bk | Breite des Kamera-Chips, beziehungsweise Breite der Fläche des gerasterten Detektors |
| b_S | Breite der Lichtquelle |
| b_S' | Breite des Lichtstreifens in einer Ebene des Messobjekts senkrecht zur optischen Achse im geometrisch-optischen Bild der Lichtquelle, Bei nichtstreuenden Objekten ist die Breite des Lichtstreifens b_S' aus dem Bild der Lichtquelle zumindest näherungsweise gleich der Breite b_80 des Lichtstreifens am Messobjekt. |
| b_S" | Breite des Lichtstreifens in der Ebene der Spaltblende |
| b_80'1 | Breite des Bildes vom Lichtstreifen 80, gebildet vom Teilstrahlenbündel mit mathematisch positivem Richtungssinn im zyklischen Zweistrahl-Interferometer 605, 606, 607 |
| b_80'2 | Breite des Bildes vom Lichtstreifen 80, gebildet vom Teilstrahlenbündel TB2 mit mathematisch negativem Richtungssinn im zyklischen Zweistrahl-Interferometer 605, 606, 607 |
| B | Punkt |
| BEI12 | gemeinsame Bildebene, in welcher die Bildebenen BEI1 und BEI2 der beiden umlaufenden Strahlengänge im zyklischen Zweistrahl-Interferometer bei perfekter Justierung desselben zusammenfallen und somit optisch konjugiert sind In der gemeinsamen Bildebene BEI12 befinden sich zumindest näherungsweise auch körperlich die Bildfeld-Diskriminatoren BFD1 und BFD2. |

(fortgesetzt)

| Bezugszeichen | Bezeichnung |
|---|---|
| BEI1 | Bildebene im Interferometerarm IA1, in welcher sich auch der Bildfeld-Diskriminator BFD1 befindet Die Bildebene BEI1 und die Bildebene BEI2 sind vorzugsweise zumindest näherungsweise optisch konjugiert. Die Differenz der Positionen der beiden Bildebene BEI1 und BEI2 in der Tiefe beträgt vorzugsweise weniger als die wellenoptische Schärfentiefe, welche sich für den Fachmann aus der numerischen Apertur der Teilstrahlenbündel TB1 und TB2, welche zumindest näherungsweise gleich ist, und der zugehörigen Wellenlänge ergibt. Vorzugsweise sind beide Bildebenen BEI1 und BEI2 mit der scheinbaren Endspiegelfläche SEF in einem Michelsontyp-Interferometer optisch konjugiert. Das kann einen Vorteil für die Größe des Aperturwinkel alpha ergeben. |
| BEI2 | Bildebene im Interferometerarm IA2, in welcher sich auch der Bildfeld-Diskriminator BFD2 befindet |
| BFD1's | scheinbares Bild des ersten Bildfeld-Diskriminators BFD1 in der scheinbaren Bildebene SBE12 |
| BFD1 | erster Bildfeld-Diskriminator Der Bildfeld-Diskriminator BFD1 ist der Bildebene BEI1 zugeordnet. Ein Bildfeld-Diskriminator kann beispielsweise eine Spaltblende 645, ein Pinhole-Array, ein Durchlassbereich 656, 657 eines Flüssigkristall-Displays 655 und/oder ein schmaler reflektierender Bereich auf einem Planspiegel und/oder auf einem Mikrospiegel-Array und/oder auch ein schmaler Planspiegel sein. Ein Bildfeld-Diskriminator weist also einen nichtdiskriminierenden Bereich auf, ausgebildet beispielsweise als relativ feine Öffnung zum Durchlassen von Licht und/oder einen relativ schmalen reflektierenden Bereich. |
| BFD2's | scheinbares Bild des zweiten Bildfeld-Diskriminators BFD2 in der scheinbaren Bildebene SBE12 |
| BFD2 | zweiter Bildfeld-Diskriminator, s. a. zu BFD1 Der Bildfeld-Diskriminator BFD2 ist der Bildebene BEI2 zugeordnet. |
| BFD1'r | reelles rückabgebildetes Bild des ersten Bildfeld-Diskriminators BFD1 auf dem Messobjekt Im justierten Zustand des Zweistrahl-Interferometers fallen die Bilder BFD1'r und BFD2'r zusammen, sind also optisch konjugiert. |
| BFD2'r | reelles rückabgebildetes Bild des zweiten Bildfeld-Diskriminators BFD2 auf dem Messobjekt |
| c | Punkt |
| d1, d2 | Dicken und/oder Höhen der Spiegelprismen 887 und 889 Von Vorteil für den Abgleich des Zweistrahl-Interferometers ist, wenn d1=d2 angenähert wird, was durch eine gemeinsame Fertigung ermöglicht wird |
| d_ST | Abstand des Schnittpunkts SP von der Strahlteilerebene ET Der Abstand d_ST beschreibt die Asymmetrie in einem zyklischen Interferometer. |
| delta_a | Verschiebung des Bildpunkts O'1 bei der Justierbewegung delta_y |
| delta_beta | Winkel zwischen den interferierenden Wellenfronten |
| DE | Detektionsebene der Interferogramme |
| delta_sigma | spektrale Auflösung im mittels einer FFT errechneten Spektrum, welche sich näherungsweise aus dem reziproken Wert der maximalen optischen Weglängendifferenz OPD bei einer Dreieck-Apodisation der Interferogrammwerte ergibt und in der Maßeinheit 1/cm (hier: $cm^{-1}$) angegeben wird |
| delta_v | Bildlagendifferenz in der Tiefe in einem zyklischen Zweistrahl-Interferometer |
| dm_1 | Spiegelabstand der Planspiegel 821 und 822 in der Spiegeltreppe 82 Es gilt dm_1 > dm_2. |
| dm_2 | Spiegelabstand der Planspiegel 831 und 832 in der Spiegeltreppe 83 Es gilt dm_1 > dm_2. |
| E | Punkt |
| EB | Eingangs strahlenbündel |

| Bezugszeichen | Bezeichnung |
|---|---|
| epsilon | Winkel zwischen den Geraden g1 und g2 in der Referenzebene RE in einem zyklischen Zweistrahl-Interferometer 605 bis 615 Die verlängerten Geraden g1 und g2 liegen in den Planspiegeln (628, 629) beziehungsweise (671, 672) und/oder in den planen Spiegelflächen (681, 682), (687s, 688s), (689s, 696s) beziehungsweise (762, 764) und in der Referenzebene RE. Es gilt epsilon=2psi. |
| E_S | Symmetrieebene zwischen den beiden Planspiegeln und/oder planen Spiegelflächen eines zyklischen Zweistrahl-Interferometers, die auch die Winkelhalbierende zwischen den beiden Planspiegeln und/oder planen Spiegelflächen und den Schnittpunkt SP enthält |
| ET | Strahlteilerebene |
| F511 (F521, F531, F581, F591) | vorderer Brennpunkt des Objektivs 511 im nachgeordneten anamorphotischen Objektiv 51 (52, 53, 58, 59) in der yz-Ebene, welche die Referenzebene RE darstellt |
| F'511 (F'521, F'531, F'581, F'591) | hinterer Brennpunkt des Objektivs 511 im nachgeordneten anamorphotischen Objektiv 51 (52, 53, 58, 59) in der yz-Ebene, welche die Referenzebene RE darstellt |
| f'511 (f'521, f'531, f'581, f'591) | hintere Brennweite des Objektivs 511 (521, 531, 581, 591) im nachgeordneten anamorphotischen Objektiv 51 (52, 53, 58, 59) Es ist f'511= f'51yz (f'521=f'52yz, f'531=f'53yz, f'581=f'58yz, f'591=f'59yz) |
| F51xz (F52xz, F53xz, F58xz, F59xz) | vorderer Brennpunkt des anamorphotischen Objektivs 51 (52, 53, 58, 59) in der in der xz-Ebene |
| F'51xz (F'52xz, F'53xz, F'58xz, F'59xz) | hinterer Brennpunkt des anamorphotischen Objektivs 51 (52, 53, 58, 59) in der in der xz-Ebene Es ist F'511= F'51yz (F'521=F'52yz, F'531=F'53yz, F'581=F'58yz, F'591=F'59yz) |
| F51yz (F52yz, F53yz, F58yz, F59yz) | vorderer Brennpunkt des anamorphotischen Objektivs 51 (52, 53, 58, 59) in der yz-Ebene, welche die Referenzebene RE darstellt Es ist F511= F51yz (F521=F52yz, F531=F53yz, F581=F58yz, F591=F59yz) |
| F'51yz (F'52yz, F'53yz, F'58yz, F'59yz) | hinterer Brennpunkt des anamorphotischen Objektivs 51 (52, 53, 58, 59) in der yz- Ebene, welche die Referenzebene RE darstellt |
| FFT | (schnelle) Fourier-Transformation, die insbesondere zur Berechnung eines Spektrums aus einem Interferogramm genutzt wird |
| G | Punkt |
| go | Lotgerade auf der Spiegelfläche eines Spiegelprismas 687 und/oder 688 |
| g1 | verlängerte Gerade in der Referenzebene RE in einem zyklischen Interferometer 605 bis 615 in der Fläche des Planspiegels 628 und/oder des Planspiegels 671 oder der Spiegelfläche 681 und/oder der Spiegelfläche 762 oder der Spiegelfläche 687s und/oder der Spiegelfläche 689s oder der Spiegelfläche 888 |
| g2 | verlängerte Gerade in einem zyklischen Interferometer 605 bis 615 in der Fläche des Planspiegels 629 und/oder des Planspiegels 672 oder der Spiegelfläche 682 und/oder der Spiegelfläche 764 oder der Spiegelfläche 688s und/oder der Spiegelfläche 696s oder der Spiegelfläche 890 |
| h1 | Dicke einer ersten Planparallelplatte mit dem Brechungsindex n1 |
| h2 | Dicke einer zweiten Planparallelplatte mit dem Brechungsindex n1 und/oder n2 |
| HTB1, HTB2 | Hauptstrahlen der Teilstrahlenbündel TB1 und TB2 |
| HEB | Hauptstrahl des Eingangsstrahlenbündels |
| I | Intensität |

(fortgesetzt)

| Bezugszeichen | Bezeichnung |
|---|---|
| IA1 | erster Arm eines Zweistrahl-Interferometers |
| IA2 | zweiter Arm eines Zweistrahl-Interferometers |
| kappa | Winkel in einem zyklischen Zweistrahl-Interferometer zwischen dem Hauptstrahl HTB1a, der von der ersten Spiegeltreppe (82) abgeht, und dem Hauptstrahl HTB2a, der von der zweiten Spiegeltreppe (83) abgeht. |
| L | Länge eines Bildfeld-Diskriminators, beispielsweise einer Spaltöffnung |
| L' | Länge des Bildes eines Bildfeld-Diskriminators, beispielsweise einer Spaltöffnung |
| n1 | Brechungsindex, z. B. nd=1,517 für das Glas N-BK7 |
| n2 | Brechungsindex, z. B. nd=1,613 für das Glas N-SK4 |
| O | beleuchteter und/oder selbstleuchtender Objektpunkt auf dem Messobjekt (1, 10, 14, 15, 16) auf der Achse eines vorgeordneten Objektivs 70, 71, 73, 74, 75, 751 Zu diesem Objektpunkt O sind die Bildpunkte O'1, O'1e, O'1s, O'2, O'2e, O'2s optisch konjugiert. |
| O'1 | scharfes, reelles Bild des Objektpunkts O im Interferometer |
| O'1e | scharfes, entfaltetes Bild des Objektpunkts O nach teilweiser geometrischer Entfaltung des Strahlenganges des ersten Interferometerarms IA1 |
| O'1s | vollständig entfalteter, scheinbarer Bildpunkt vom Objektpunkt O vor dem anamorphotischen Objektiv, auch unter Berücksichtigung refraktiver Materialien im Strahlengang Der vollständig entfaltete, scheinbare Bildpunkt O'1s stammt aus dem Arm des Zweistrahl-Interferometers IA1. |
| O'1_unscharf | unscharfer Bildfleck des Objektpunkts O im ersten Interferometerarm IA1, verursacht durch sphärische Aberration von Planparallelplatten im Strahlengang |
| O'2 | scharfes, reelles Bild des Objektpunkts O im Interferometer |
| O'2e | scharfes, entfaltetes Bild des Objektpunkts O nach teilweiser geometrischer Entfaltung des Strahlenganges des zweiten Interferometerarms IA2 |
| O'2s | vollständig entfalteter, scheinbarer Bildpunkt vom Objektpunkt O vor dem anamorphotischen Objektiv, auch unter Berücksichtigung refraktiver Materialien im Strahlengang Der vollständig entfaltete, scheinbare Bildpunkt O'2s stammt aus dem Arm des Zweistrahl-Interferometers IA2. |
| O"1_xz | scharfes, scheinbares Bild des Objektpunkts O am Ausgang des anamorphotischen Objektivs 51, gebildet durch ein Strahlenbüschel |
| OAI | optische Achse des vorgeordneten Objektivs 70, 71, 73, 74, 75 und/oder auch 751, welche dem Zweistrahl-Interferometer in der Regel zugewandt ist Diese optische Achse fällt mit der z-Achse zusammen. |
| OA2 | Optische Achse der Kamera, insbesondere InGaAs-Kamera |
| OE_1, OE_2, OE_3, OE_4 | Die Objektelemente OE_1, OE_2 OE_3, OE_4 sind in x-Richtung angeordnet, die so ein streifenförmiges Messfeld bilden. |
| OE | Objektelement |
| OPD | optische Weglängendifferenz, auch optische Weglängendifferenz im Zweistrahl-Interferometer (engl. optical path difference, OPD) |
| OPDr | optische Weglängendifferenz, auch optische Weglängendifferenz im Zweistrahl-Interferometer (engl. optical path difference, OPD), hier die maximal erreichbare optische Weglängendifferenz im räumlichen Interferogramm am Rand des gerasterten Detektors bei symmetrischer Lage des räumlichen Interferogramms auf dem gerasterten Detektor |

(fortgesetzt)

| Bezugzeichen | Bezeichnung |
|---|---|
| OPDru | maximal erreichbare optische Weglängendifferenz am Rand des gerasterten Detektors bei unsymmetrischer Lage des räumlichen Interferogramms Es kann im Grenzfall gelten: OPDru =2*OPDr. Jedoch ist es in vielen Fällen optimal, mit OPDru =1,5*OPDr zu rechnen, da im Standardfall ein Stück eines linksseitigen Interferogramms für die Phasenkorrektur bei der Berechnung des Spektrums mittels einer FFT benötigt wird. |
| OPD_links | maximale optische Weglängendifferenz auf der linken Seite des räumlichen Interferogramms rI, bei in der Fachwelt üblicher Darstellung eines Interferogramms rI über der optischen Weglängendifferenz OPD |
| OPD_rechts | maximale optische Weglängendifferenz auf der rechten Seite des räumlichen Interferogramms rI, bei in der Fachwelt üblicher Darstellung eines Interferogramms rI über der optischen Weglängendifferenz OPD Die maximale optische Weglängendifferenz OPD rechts kann dem Wert der maximal erreichbaren optischen Weglängendifferenz am Rand des gerasterten Detektors OPDru bei unsymmetrischer Lage des räumlichen Interferogramms entsprechen. |
| P | Punkt an einer Lichtquelle, der mit dem Objektpunkt O optisch konjugiert ist |
| P' | Bild des Punktes P nach Abbildung desselben durch das Objektiv 48 auf das Messobjekt 14 und/ oder 15 |
| P"1 | Bild des Punktes P' nach Abbildung desselben mittels eines vorgeordneten Objektivs 73 in das Zweistrahl-Interferometer Der Punkt P " 1 fällt mit dem Punkt O'1 in Figur 13 zusammen. |
| P"2 | Bild des Punktes P' nach Abbildung desselben mittels eines vorgeordneten Objektivs 73 in das Zweistrahl-Interferometer Der Punkt P"2 fällt mit dem Punkt O'2 zusammen, was in Fig. 12 dargestellt ist und auch in Figur 13. |
| P"ls | Bild des Punktes P' in der scheinbaren Bildebene SBE12, dem ersten Interferometerarm IA1 zugeordnet |
| P"2s | Bild des Punktes P' in der scheinbaren Bildebene SBE12, dem zweiten Interferometerarm IA2 zugeordnet |
| psi | der Halbwinkel des zyklischen Zweistrahl-Interferometers und in der Regel ein spitzer Winkel und psi beträgt die Hälfte des Winkels epsilon Der Winkel psi ist in einem zyklischen Zweistrahl-Interferometer 605 bis 615 der halbe Winkel zwischen den Geraden g1 und g2 in der Referenzebene RE. |
| RE | Referenzebene Die Referenzebene RE ist durch die Normale der ebenen Strahlteilerfläche und durch die optische Achse OAI des vorgeordneten Objektivs am Eingang eines Zweistrahl-Interferometers aufgespannt. Damit steht die Referenzebene in der Regel senkrecht zur Strahlteilerfläche. In einem Mach-Zehnder-Interferometer bestimmt die Normale der ersten Strahlteilerfläche die Referenzebene RE. Der gerasterte Detektor steht im Standardfall senkrecht auf der Referenzebene RE. |
| rho | Winkel |
| rI | räumliches Interferogramm |
| RZ | Radius einer Zylinderwelle, der auf dem gerasterten Detektor sehr klein wird, bzw. gegen null geht |
| s | Lateral-Shear, auch Querversatz, der kohärenten Bildpunkte O'1s, O'2s vor dem anamorphotischen Objektiv 51, 52, 53, 58, 59 und somit auch der Teilstrahlbündel TB1 und TB2 Die Lateral-Shear kann je nach Anordnung als Summe und/oder als Differenz von s1 und s2 gebildet sein. |
| s1 | Lateral-Shear nach Passieren eines ersten, zum Teilstrahlenbündel TB 1 querversetzten Dachkantreflektors |
| s2 | Lateral-Shear nach Passieren eines zweiten, zum Teilstrahlenbündel TB2 querversetzten Dachkantreflektors |

| Bezugszeichen | Bezeichnung |
|---|---|
| S82 | Durchstoßpunkt der Schnittlinie durch die Referenzebene RE der verlängerten Spiegelflächen 821 und 822 der Spiegeltreppe 82 |
| S83 | Durchstoßpunkt der Schnittlinie durch die Referenzebene RE der verlängerten Spiegelflächen 831 und 832 der Spiegeltreppe 83 |
| SP | Schnittpunkt der Geraden g1 und g2 in einem zyklischen Zweistrahl-Interferometer 605 bis 615 in der Referenzebene RE, welche hier die Zeichenebene darstellt |
| sigma | Wellenzahl in der Maßeinheit 1/cm (cm$^{-1}$) mit 1 dividiert durch die Wellenlänge in cm |
| Sp1, Sp2, SP3, SP4 | Spektren Jedes Spektrum Sp1, Sp2, SP3, SP4 wird jeweils aus einem räumlichen Interferogramm rI1, rI2, rI3, rI4 mittels einer FFT errechnet. |
| t | Zeit |
| t1, t2, t3, t4, t5, t6 | Zeitpunkte |
| tau_1 | Keilwinkel zwischen den beiden Planspiegeln der ersten Spiegeltreppe Zumindest näherungsweise ist tau_1= tau_2 anzustreben. |
| tau_2 | Keilwinkel zwischen den beiden Planspiegeln der zweiten Spiegeltreppe |
| T | Punkt |
| TB1 | erstes Teilstrahlenbündel nach Strahlteilung Es gibt jedoch gleichzeitig viele Teilstrahlenbündel, die von jeweils einem Objektpunkt stammen. Das in den Figuren dargestellte Teilstrahlenbündel TB1 steht also nur stellvertretend für eine Vielzahl derselben. Die Teilstrahlenbündel TB1 und TB2 stellen kohärente Bündel dar. |
| TB2 | zweites Teilstrahlenbündel nach Strahlteilung |
| va | Absatz zwischen den Flächen von zwei Spiegelprismen |
| v1 | Bildverschiebung in der Tiefe an einer Planparallelplatte mit der Dicke h1 und dem Brechungsindex n1 |
| v2 | Bildverschiebung in der Tiefe an einer Planparallelplatte mit der Dicke h2 und dem Brechungsindex n1 und/oder dem Brechungsindex n2 |
| SBE12 | scheinbare Bildebene, die jedem anamorphotischen Objektiv 51, 52, 53, 58, 59 vorgeordnet ist und welche dann wiederum die Objektebene für dieses Objektiv 51, 52, 53, 58, 59 darstellt. Diese scheinbare Bildebene SBE12 ergibt sich aus der geometrischen Entfaltung der optischen Anordnung mit all deren optischen Komponenten und auch unter Berücksichtigung der Refraktion. Die scheinbare Bildebene SBE12 besteht nur für das justierte Interferometer, bei dem auch die Bildebenen optisch konjugiert sind. In diesem Fall fallen die einzelnen Bildebenen SBE1 und SBE2 zu einer gemeinsamen scheinbaren Bildebene SBE12 innerhalb der wellenoptischen Schärfentiefe zusammen, was in dieser Schrift der Standardfall ist. |
| SEF1 | scheinbare Endspiegelfläche im ersten Arm IA1 des Zweistrahl-Interferometers |
| SEF2 | scheinbare Endspiegelfläche im zweiten Arm IA2 des Zweistrahl-Interferometers |
| Xuv | leuchtendes Pixel im UV-Bereich durch Ansteuerung mittels eines Steuerrechners |
| Xuv' | Bild des leuchtenden Pixels Xuv im UV-Bereich |
| Xvis"1 | Bild des leuchtenden Pixels Xvis"1 im VIS-Bereich im ersten Arm IA1 des Zweistrahl-Interferometers |
| Xvis"2 | Bild des leuchtenden Pixels Xvis"2 im VIS-Bereich im zweiten Arm IA2 des Zweistrahl-Interferometers |
| x (-Richtung) | Streifenlängsrichtung, bzw. Längsrichtung einer Streifenlichtquelle und/oder auch einer Spaltöffnung, in der Regel senkrecht zur Referenzebene RE |
| y (-Richtung) | Richtung der Lateral-Shear, bzw. die Querrichtung zu einem Streifen im entfalteten Zustand |

(fortgesetzt)

| Bezugszeichen | Bezeichnung |
|---|---|
| Z | Bereich in einem zyklischen Zweistrahl-Interferometer (605 bis 615), wo es überhaupt keine räumliche Überdeckung der Teilstrahlenbündel TB1 und TB2 gibt |
| z (-Richtung) | Ausbreitungsrichtung des Interferenzlichts in Richtung Detektion Die z-Achse steht im entfalteten Zustand der optischen Anordnung senkrecht auf dem gerasterten Detektor. Die z-Achse fällt mit der optischen Achse OAI des vorgeordneten Objektivs zusammen. |
| $\Theta_1, \Theta_2 \Theta_3, \Theta_4$ | Winkel zwischen erstem 661a, 662a und zweiten Spiegel 661b, 662b der ersten und der zweiten Strahlumlenkungseinheit im Mach-Zehnder Interferometer |

**Patentansprüche**

1.  Fourier-Transformations-Spektrometer, FT-Spektrometer, aufweisend:

    ein Zweistrahl-Interferometer aufweisend:

    zumindest eine Strahlteilereinheit (622; 623; 620; 636; 673, 674, 675) zum Aufspalten eines einfallenden Lichtbündels (EB) eines räumlich ausgedehnten Objekts (1; 10; 11; 14; 15; 16) in ein erstes Teilstrahlenbündel (TB1) und ein zweites Teilstrahlenbündel (TB2);
    zumindest eine erste Strahlumlenkungseinheit (630; 643; 651; 661; 697), die dazu ausgelegt ist, das erste Teilstrahlenbündel (TB1) zumindest ein erstes und ein zweites Mal abzulenken, wobei

    die erste Strahlumlenkungseinheit (630, 697) dazu ausgelegt ist, auch das zweite Teilstrahlenbündel (TB2) zumindest ein erstes und ein zweites Mal abzulenken; oder
    das Zweistrahl-Interferometer eine zweite Strahlumlenkungseinheit (644; 652; 662) umfasst, die dazu ausgelegt ist, das zweite Teilstrahlenbündel (TB2) zumindest ein erstes und ein zweites Mal abzulenken,

    wobei die Strahlteilereinheit (622; 623; 620; 636; 673, 674, 675) auch dazu ausgelegt ist, das erste Teilstrahlenbündel (TB1) und das zweite Teilstrahlenbündel (TB2) zumindest teilweise räumlich zu überlagern, und wobei das jeweils erste und zweite Ablenken des ersten Teilstrahlenbündel (TB1) und des zweiten Teilstrahlenbündel (TB2) eine Lateral-Shear (s) erzeugt;
    zumindest eine erste Bildfeld-Diskriminatoreinheit (BFD1; 631; 645; 653; 656; 666; 677; 976), die so angeordnet ist, dass das erste Teilstrahlenbündel (TB1) nach dem Aufspalten und vor dem zweiten Ablenken räumlich selektiert wird;
    zumindest eine zweite Bildfeld-Diskriminatoreinheit (BFD2; 632; 646; 654; 657; 667; 678; 977), die so angeordnet ist, dass das zweite Teilstrahlenbündel (TB2) nach dem Aufspalten und vor dem zweiten Ablenken räumlich selektiert wird wobei zumindest eine der beiden Bildfeld-Diskriminatoreinheiten spaltförmig ausgebildet ist;

    wobei das FT-Spektrometer ferner umfasst:

    zumindest ein vorgeordnetes Objektiv (70; 71; 73; 74; 75), das so gegenüber der Strahlteilereinheit angeordnet ist, dass das einfallende Lichtbündel (EB) das Objektiv zumindest teilweise vor dessen Aufspaltung an der Strahlteilereinheit passiert und das erste Teilstrahlenbündel (TB1) und das zweite Teilstrahlenbündel (TB2) jeweils eine Vielzahl von kohärenten Bildpunkten (O'1, O'2) des räumlich ausgedehnten Objekts (1; 10; 11; 14; 15; 16) in einer Bildebene zwischen der Strahlteilereinheit und einem Detektor (54) erzeugt;
    den Detektor (54, 55, 634) zur Erfassung einer Vielzahl von räumlichen Interferogrammen (rI1, rI2, rI3, rI4) auf Basis der räumlichen Überlagerung des ersten Teilstrahlenbündels (TB1) und des zweiten Teilstrahlenbündels (TB2), die der zumindest teilweisen Abbildung der Vielzahl von kohärenten Bildpunkten (O'1, O'2) entspricht; und
    zumindest eine Recheneinheit zur Fourier-Transformation der Vielzahl von räumlichen Interferogrammen (rI1, rI2, rI3, rI4), zur Erzeugung einer Vielzahl von Spektren (Sp1, Sp2, Sp3, Sp4) und darauf basierend zur Erzeugung eines hyperspektralen Bildes des räumlich ausgedehnten Objekts,

wobei die erste Bildfeld-Diskriminatoreinheit (BFD1; 631; 645; 653; 656; 666; 677; 976) und die zweite Bildfeld-Diskriminatoreinheit (BFD2; 632; 646; 654; 657; 667; 678; 977) von einem dem Zweistrahl-Interferometer nachgeordneten anamorphotischen Objektiv (51; 53; 58; 59; 875, 876, 877) abgebildet werden, und

wobei das zumindest eine vorgeordnete Objektiv (70; 71; 73; 74; 75) dazu ausgelegt ist, so justiert zu werden, dass jeweils eine Bildebene (BEI1, BEI2) des zumindest einen vorgeordneten Objektivs (70; 71; 73; 74; 75) in der ersten Bildfeld-Diskriminatoreinheit (BFD1; 631; 645; 653; 656; 666; 677; 976) und in der zweiten Bildfeld-Diskriminatoreinheit (BFD2; 632; 646; 654; 657; 667; 678; 977) liegt.

2. FT-Spektrometer nach Anspruch 1, wobei die Strahlteilereinheit einen einzelnen Strahlteiler aufweist (622; 623; 636; 673, 674), um

das einfallende Lichtbündel (EB) eines räumlich ausgedehnten Objekts (1; 10; 11; 14; 15; 16), in das erste Teilstrahlenbündel (TB1) und das zweite Teilstrahlenbündel (TB2) aufzuspalten; und

das erste Teilstrahlenbündel (TB1) und das zweite Teilstrahlenbündel (TB2) zumindest teilweise räumlich zu überlagern.

3. FT-Spektrometer nach Anspruch 1 oder 2, wobei die erste Bildfeld-Diskriminatoreinheit (BFD1; 631; 645; 653; 656; 666; 677; 976) und die zweite Bildfeld-Diskriminatoreinheit (BFD2; 632; 646; 654; 657; 667; 678; 977) so zueinander angeordnet sind, dass zumindest näherungsweise eine optische Konjugation zwischen der ersten Bildfeld-Diskriminatoreinheit (BFD1; 631; 645; 653; 656; 666; 677; 976) und der zweiten Bildfeld-Diskriminatoreinheit (BFD2; 632; 646; 654; 657; 667; 678; 977) besteht.

4. FT-Spektrometer nach einem der vorangehenden Ansprüche, wobei das Zweistrahl-Interferometer einem Michelsontyp-Interferometer entspricht, umfassend die zweite Strahlumlenkungseinheit (642), wobei

die erste Strahlumlenkungseinheit (641) einen Zweispiegel-Winkelreflektor aufweist, der im Folgenden umfasst

einen ersten Spiegel (641a), der dazu ausgelegt ist, das erste Teilstrahlenbündel zumindest ein erstes Mal zu reflektieren; und
einen zweiten Spiegel (641b), der in einen Winkel $\Theta_1$ gegenüber dem ersten Spiegel angeordnet ist und dazu ausgelegt ist, das erste Teilstrahlenbündel (TB1) zumindest ein zweites Mal zu reflektieren; und/oder

die zweite Strahlumlenkungseinheit (642) einen weiteren Zweispiegel-Winkelreflektor aufweist, der im Folgenden umfasst

einen ersten Spiegel (624a), der dazu ausgelegt ist, das zweite Teilstrahlenbündel (TB2) zumindest ein erstes Mal zu reflektieren; und
einen zweiten Spiegel (642b), der in einem Winkel $\Theta_2$ gegenüber dem ersten Spiegel angeordnet ist und dazu ausgelegt ist, das zweite Teilstrahlenbündel (TB2) zumindest ein zweites Mal zu reflektieren.

5. FT-Spektrometer nach einem der vorangehenden Ansprüche, wobei das Zweistrahl-Interferometer einem Michelsontyp-Interferometer entspricht, umfassend die zweite Strahlumlenkungseinheit (642), wobei die erste Strahlumlenkungseinheit (641) einen Tripelspiegel-Winkelreflektor aufweist, der im Folgenden umfasst

einen ersten Spiegel (641a), der dazu ausgelegt ist, das erste Teilstrahlenbündel zumindest ein erstes Mal zu reflektieren; und
einen zweiten Spiegel (641b), der in einen Winkel $\Theta_1$ gegenüber dem ersten Spiegel angeordnet ist und dazu ausgelegt ist, das erste Teilstrahlenbündel (TB1) zumindest ein zweites Mal zu reflektieren; und
einen dritten Spiegel, der in einem Winkel $\Theta_{a1}$ gegenüber dem ersten Spiegel (641a) und in einem Winkel $\Theta_{b1}$ gegenüber dem zweiten Spiegel (641b) angeordnet ist und dazu ausgelegt ist, das erste Teilstrahlenbündel (TB1) zumindest ein drittes Mal zu reflektieren; und/oder
die zweite Strahlumlenkungseinheit (642) einen weiteren Tripelspiegel-Winkelreflektor aufweist, der im Folgenden umfasst

einen ersten Spiegel (642a), der dazu ausgelegt ist, das zweite Teilstrahlenbündel (TB2) zumindest ein erstes Mal zu reflektieren; und
einen zweiten Spiegel (642b), der in einem Winkel $\Theta_2$ gegenüber dem ersten Spiegel angeordnet ist und dazu ausgelegt ist, das zweite Teilstrahlenbündel (TB2) zumindest ein zweites Mal zu reflektieren; und einen dritten Spiegel, der in einem Winkel $\Theta_{a2}$ gegenüber dem ersten Spiegel (642a) und in einem Winkel $\Theta_{b2}$ gegenüber dem zweiten Spiegel (642b) angeordnet ist und dazu ausgelegt ist, das zweite Teilstrahlenbündel (TB2) zumindest ein drittes Mal zu reflektieren,

wobei optional

die erste Bildfeld-Diskriminatoreinheit (645) eine Öffnung aufweist, die dazu ausgelegt ist, das erste Teilstrahlenbündel (TB1) nach dem ersten Reflektieren mittels des ersten Spiegels (641a) der ersten Strahlumlenkungseinheit (641) und vor dem zweiten Reflektieren mittels des zweiten Spiegels (641b) der ersten Strahlumlenkungseinheit (641) räumlich zu selektieren; und/oder die zweite Bildfeld-Diskriminatoreinheit (646) eine weitere Öffnung aufweist, die dazu ausgelegt ist, das zweite Teilstrahlenbündel (TB2) nach dem ersten Reflektieren mittels des ersten Spiegels (642a) der zweiten Strahlumlenkungseinheit (642) und vor dem zweiten Reflektieren mittels des zweiten Spiegels (642b) der zweiten Strahlumlenkungseinheit (642) räumlich zu selektieren.

6. FT-Spektrometer nach einem der vorangehenden Ansprüche, wobei das Zweistrahl-Interferometer einem Michelsontyp-Interferometer entspricht, umfassend die zweite Strahlumlenkungseinheit (652), wobei

die erste Strahlumlenkungseinheit (651) ferner umfasst
ein erstes Prisma mit
einer ersten Reflexionsfläche (651a), die dazu ausgelegt ist, das erste Teilstrahlenbündel (TB1) zumindest ein erstes Mal zu reflektieren; und einer zweiten Reflexionsfläche (651b), die dazu ausgelegt ist, das erste Teilstrahlenbündel (TB1) zumindest ein zweites Mal zu reflektieren; und/oder die zweite Strahlumlenkungseinheit (652) ferner umfasst
ein zweites Prisma mit
einer ersten Reflexionsfläche (652a), die dazu ausgelegt ist, das zweite Teilstrahlenbündel (TB2) zumindest ein erstes Mal zu reflektieren; und einer zweiten Reflexionsfläche (652b), die dazu ausgelegt ist, das zweite Teilstrahlenbündel (TB2) zumindest ein zweites Mal zu reflektieren.

7. FT-Spektrometer nach Anspruch 6, wobei

die erste Bildfeld-Diskriminatoreinheit (653) eine Öffnung aufweist, die dazu ausgelegt ist, das erste Teilstrahlenbündel (TB1) nach dem Aufspalten des einfallenden Lichtbündels (EB) mittels der Strahlteilereinheit (622) und vor dem ersten Reflektieren des ersten Teilstrahlenbündels (TB1) mittels der ersten Reflexionsfläche (651a) des ersten Prismas (651) räumlich zu selektieren; und/oder die zweite Bildfeld-Diskriminatoreinheit (654) eine Öffnung aufweist, die dazu ausgelegt ist, das zweite Teilstrahlenbündel (TB2) nach dem Aufspalten des einfallenden Lichtbündels (EB) mittels der Strahlteilereinheit (622) und vor dem ersten Reflektieren des zweiten Teilstrahlenbündels (TB2) mittels der ersten Reflexionsfläche (652a) des zweiten Prismas (652) räumlich zu selektieren.

8. FT-Spektrometer nach einem der Ansprüche 1 bis 3, wobei das Zweistrahl-Interferometer einem Mach-Zehnder-Interferometer entspricht, umfassend die zweite Strahlumlenkungseinheit (662) und wobei die Strahlteilereinheit (624, 625, 626, 627) ferner umfasst:

einen ersten Einzel-Strahlteiler (624, 625) zum Aufspalten des einfallenden Lichtbündels (EB) in das erste Teilstrahlenbündel (TB1) und das zweite Teilstrahlenbündel (TB2); und einen zweiten Einzel-Strahlteiler (626, 627) zum zumindest teilweise räumlichen Überlagern des ersten Teilstrahlenbündels (TB1) und des zweiten Teilstrahlenbündels (TB2), wobei optional die erste Strahlumlenkungseinheit (661) ferner umfasst

einen ersten Spiegel (661a), der dazu ausgelegt ist, das erste Teilstrahlenbündel (TB1) zumindest ein erstes Mal zu reflektieren; und einen zweiten Spiegel (661b), der in einem Winkel $\Theta_3$ gegenüber dem ersten Spiegel (661a) angeordnet ist und dazu ausgelegt ist, das erste Teilstrahlenbündel (TB1) zumindest ein zweites Mal zu reflektieren; und/oder

die zweite Strahlumlenkungseinheit (662) ferner umfasst

einen ersten Spiegel (662a), der dazu ausgelegt ist, das zweite Teilstrahlenbündel (TB2) zumindest ein erstes Mal zu reflektieren; und
einen zweiten Spiegel (662b), der in einem Winkel $\Theta_4$ gegenüber dem ersten Spiegel (662a) angeordnet ist und dazu ausgelegt ist, das zweite Teilstrahlenbündel (TB2) zumindest ein zweites Mal zu reflektieren.

9. FT-Spektrometer nach Anspruch 8, wobei

die erste Bildfeld-Diskriminatoreinheit (666) eine Öffnung aufweist, die dazu ausgelegt ist, das erste Teilstrahlenbündel (TB1) nach dem ersten Reflektieren mittels des ersten Spiegels (661a) der ersten Strahlumlenkungseinheit (661) und vor dem zweiten Reflektieren mittels des zweiten Spiegels (661b) der ersten Strahlumlenkungseinheit (661) räumlich zu selektieren; und/oder
die zweite Bildfeld-Diskriminatoreinheit (667) eine weitere Öffnung aufweist, die dazu ausgelegt ist, das zweite Teilstrahlenbündel (TB2) nach dem ersten Reflektieren mittels des ersten Spiegels (662a) der zweiten Strahlumlenkungseinheit (662) und vor dem zweiten Reflektieren mittels des zweiten Spiegels (662b) der zweiten Strahlumlenkungseinheit (662) räumlich zu selektieren.

10. FT-Spektrometer nach einem der Ansprüche 1 bis 3, wobei das Zweistrahl-Interferometer einem zyklischen Zweistrahl-Interferometer entspricht, wobei

die erste Strahlumlenkungseinheit (630; 697) dazu ausgelegt ist, auch das zweite Teilstrahlenbündel (TB2) zumindest ein erstes und ein zweites Mal abzulenken,
die erste Bildfeld-Diskriminatoreinheit (631; 656; 677; 976) eine Öffnung aufweist, die dazu ausgelegt ist, das erste Teilstrahlenbündel (TB1) nach dem ersten Ablenken und vor dem zweiten Ablenken räumlich zu selektieren; und
die zweite Bildfeld-Diskriminatoreinheit (632; 657; 678; 977) eine weitere Öffnung aufweist, die dazu ausgelegt ist, das zweite Teilstrahlenbündel (TB2) nach dem ersten Ablenken und vor dem zweiten Ablenken räumlich zu selektieren.

11. FT-Spektrometer nach Anspruch 10, wobei die Strahlumlenkungseinheit einen Zweispiegel-Winkelreflektor (630) aufweist, der im Folgenden umfasst

einen ersten Spiegel (628), der dazu ausgelegt ist, das erste Teilstrahlenbündel (TB1) zumindest ein erstes Mal zu reflektieren und das zweite Teilstrahlenbündel (TB2) zumindest ein zweites Mal zu reflektieren; und
einen zweiten Spiegel (629), der in einem Winkel epsilon gegenüber dem ersten Spiegel angeordnet ist und dazu ausgelegt ist, das zweite Teilstrahlenbündel (TB2) zumindest ein erstes Mal zu reflektieren und das erste Teilstrahlenbündel (TB1) zumindest ein zweites Mal zu reflektieren.

12. FT-Spektrometer nach Anspruch 10, wobei die Strahlumlenkungseinheit (697) eine Doppelprismen-Anordnung aufweist, die im Folgenden umfasst
ein erstes Prisma (679; 687; 689; 887) mit zumindest einer Reflexionsfläche (888), die dazu ausgelegt ist, das erste Teilstrahlenbündel (TB1) zumindest ein erstes Mal zu reflektieren und das zweite Teilstrahlenbündel (TB2) zumindest ein zweites Mal zu reflektieren; und ein zweites Prisma (680; 688; 689; 896) mit zumindest einer Reflexionsfläche (890), die dazu ausgelegt ist, das zweite Teilstrahlenbündel (TB2) zumindest ein erstes Mal zu reflektieren und das erste Teilstrahlenbündel (TB1) zumindest ein zweites Mal zu reflektieren.

13. FT-Spektrometer nach einem der Ansprüche 10 bis 12, wobei

die erste Bildfeld-Diskriminatoreinheit (631) eine Öffnung aufweist, die dazu ausgelegt ist, das erste Teilstrahlenbündel (TB1) nach dem ersten Reflektieren mittels des ersten Spiegels (628) oder der Reflexionsfläche (888) des erstes Prismas (679; 687; 689; 887) und vor dem zweiten Reflektieren mittels des zweiten Spiegels (629) oder der Reflexionsfläche (890) des zweiten Prismas (680; 688; 689; 896) räumlich zu selektieren; und
die zweite Bildfeld-Diskriminatoreinheit (632) eine weitere Öffnung aufweist, die dazu ausgelegt ist, das zweite Teilstrahlenbündel (TB2) nach dem ersten Reflektieren mittels des zweiten Spiegels (629) oder der Reflexionsfläche (890) des zweiten Prismas (680; 688; 689; 896) und vor dem zweiten Reflektieren mittels des ersten Spiegels (628) oder der Reflexionsfläche (888) des erstes Prismas (679; 687; 689; 887) räumlich zu selektieren.

**14.** Verfahren zur interferometrischen Messung mittels eines FT-Spektrometers mit Zweistrahl-Interferometer umfassend:

Aufspalten eines einfallenden Lichtbündels, welcher von einem räumlich ausgedehnten Objekt ausgesendet wird, in ein erstes Teilstrahlenbündel und ein zweites Teilstrahlenbündel mittels einer Strahlteilereinheit; erstes und zweites Ablenken des ersten Teilstrahlenbündels mittels einer ersten Strahlumlenkungseinheit; erstes und zweites Ablenken des zweiten Teilstrahlenbündels mittels der ersten Strahlumlenkungseinheit oder mittels einer zweiten Strahlumlenkungseinheit, wobei das erste und zweite Ablenken des ersten Teilstrahlenbündel und des zweiten Teilstrahlenbündel eine Lateral-Shear (s) erzeugt; räumliches Selektieren zumindest eines Teils des ersten Teilstrahlenbündels, nach dem Aufspalten und vor dem zweiten Ablenken, mittels einer ersten Bildfeld-Diskriminatoreinheit im Zweistrahl-Interferometer; und räumliches Selektieren zumindest eines Teils des zweiten Teilstrahlenbündels, nach dem Aufspalten und vor dem zweiten Ablenken, mittels einer zweiten Bildfeld-Diskriminatoreinheit im Zweistrahl-Interferometer, wobei zumindest eine der beiden Bildfeld-Diskriminatoreinheiten spaltförmig ausgebildet ist; oder räumliches Selektieren zumindest eines Teils des ersten Teilstrahlenbündels - nach dem Aufspalten und nach dem zweiten Ablenken - mittels einer ersten Bildfeld-Diskriminatoreinheit im Zweistrahl-Interferometer; und oder räumliches Selektieren zumindest eines Teils des zweiten Teilstrahlenbündels - nach dem Aufspalten und nach dem zweiten Ablenken - mittels einer zweiten Bildfeld-Diskriminatoreinheit in einer Bildebene im Zweistrahl-Interferometer; Senden des einfallenden Lichtbündels durch ein vorgeordnetes Objektiv vor dem Aufspalten, zur Erzeugung einer Vielzahl von kohärenten Bildpunkten des räumlich ausgedehnten Objekts in einer Bildebene zwischen der Strahlteilereinheit und einem Detektor; zumindest teilweises räumliches Überlagern des ersten Teilstrahlenbündels und des zweiten Teilstrahlenbündels mittels der Strahlteilereinheit; zumindest teilweises Abbilden der Vielzahl von kohärenten Bildpunkten bei gleichzeitigem Erzeugen einer Vielzahl von räumlichen Interferogrammen auf einem Detektorfeld des Detektors auf Basis der räumlichen Überlagerung; Erfassen der Vielzahl von Interferogrammen mittels des Detektors; Fourier-Transformation der Vielzahl von räumlichen Interferogrammen zur Erzeugung einer Vielzahl von Spektren und darauf basierend Erzeugung eines hyperspektralen Bildes zumindest eines Ausschnitts des räumlich ausgedehnten Objekts, Abbilden der ersten Bildfeld-Diskriminatoreinheit (BFD1; 631; 645; 653; 656; 666; 677; 976) und der zweiten Bildfeld-Diskriminatoreinheit (BFD2; 632; 646; 654; 657; 667; 678; 977) mittels eines dem Zweistrahl-Interferometer nachgeordneten anamorphotischen Objektivs (51; 53; 58; 59; 875, 876, 877), und Justieren des zumindest einen vorgeordneten Objektivs (70; 71; 73; 74; 75), sodass jeweils eine Bildebene (BEI1, BEI2) des zumindest einen vorgeordneten Objektivs (70; 71; 73; 74; 75) in der ersten Bildfeld-Diskriminatoreinheit (BFD1; 631; 645; 653; 656; 666; 677; 976) und in der zweiten Bildfeld-Diskriminatoreinheit (BFD2; 632; 646; 654; 657; 667; 678; 977) liegt.

**15.** Verfahren zur interferometrischen Messung nach Anspruch 14, ferner umfassend die Schritte, die ausgelöst werden mittels zumindest einer Recheneinheit:

mehrmaliges gleichzeitiges Erfassen der Vielzahl von räumlichen Interferogrammen zu jeweils unterschiedlichen Zeitpunkten; Fourier-Transformierung der zu jeweils unterschiedlichen Zeitpunkten erfassten Vielzahl von räumlichen Interferogrammen zur Erzeugung einer Vielzahl von Spektren; und Erzeugung eines hyperspektralen Bildes des räumlich ausgedehnten Objekts.

**Claims**

**1.** A Fourier transform spectrometer, FT spectrometer, including:

a two-beam interferometer including:

at least one beam splitter unit (622; 623; 620; 636; 673, 674, 675) for splitting an incident light beam (EB) of a spatially extended object (1; 10; 11; 14; 15; 16) into a first partial beam (TB1) and a second partial beam (TB2);

at least one first beam deflection unit (630; 643; 651; 661; 697) configured to deflect the first partial beam (TB1) at least a first and a second time, wherein

the first beam deflection unit (630, 697) is configured to also deflect the second partial beam (TB2) at least a first and a second time; or
the two-beam interferometer comprises a second beam deflection unit (644; 652; 662) configured to deflect the second partial beam (TB2) at least a first and a second time,

wherein the beam splitter unit (622; 623; 620; 636; 673, 674, 675) is also configured to at least partially spatially superimpose the first partial beam (TB1) and the second partial beam (TB2), and wherein the respective first and second deflection of the first partial beam (TB1) and the second partial beam (TB2) generates a lateral shear(s);
at least a first image field discriminator unit (BFD1; 631; 645; 653; 656; 666; 677; 976) arranged such that the first partial beam (TB1) is spatially selected after splitting and before the second deflection;
at least a second image field discriminator unit (BFD2; 632; 646; 654; 657; 667; 678; 977) arranged such that the second partial beam (TB2) is spatially selected after splitting and before the second deflection, wherein at least one of the two image field discriminator units is formed in a slit shape;

wherein the FT spectrometer further comprises:

at least one upstream objective (70; 71; 73; 74; 75) arranged opposite the beam splitter unit such that the incident light beam (EB) at least partially passes the objective before it is split at the beam splitter unit and the first partial beam (TB1) and the second partial beam (TB2) each generate a plurality of coherent image points (O'1, O'2) of the spatially extended object (1; 10; 11; 14; 15; 16) in an image plane between the beam splitter unit and a detector (54);
the detector (54, 55, 634) for detecting a plurality of spatial interferograms (rl1, rl2, rl3, rl4) on the basis of the spatial superimposition of the first partial beam (TB1) and the second partial beam (TB2), which corresponds to the at least partial imaging of the plurality of coherent image points (O'1, O'2); and
at least one computing unit for Fourier transformation of the plurality of spatial interferograms (rl1, rl2, rl3, rl4), for generating a plurality of spectra (Sp1, Sp2, Sp3, Sp4) and, based thereon, for generating a hyperspectral image of the spatially extended object,
wherein the first image field discriminator unit (BFD1; 631; 645; 653; 656; 666; 677; 976) and the second image field discriminator unit (BFD2; 632; 646; 654; 657; 667; 678; 977) are imaged by an anamorphic objective (51; 53; 58; 59; 875, 876, 877) downstream of the two-beam interferometer, and wherein the at least one upstream objective (70; 71; 73; 74; 75) is configured to be adjusted such that respectively one image plane (BEI1, BEI2) of the at least one upstream objective (70; 71; 73; 74; 75) lies in the first image field discriminator unit (BFD1; 631; 645; 653; 656; 666; 677; 976) and in the second image field discriminator unit (BFD2; 632; 646; 654; 657; 667; 678; 977).

2. The FT spectrometer according to claim 1, wherein the beam splitter unit includes a single beam splitter (622; 623; 636; 673, 674) to split the incident light beam (EB) of a spatially extended object (1; 10; 11; 14; 15; 16) into the first partial beam (TB1) and the second partial beam (TB2); and
spatially superimpose at least partially the first partial beam (TB1) and the second partial beam (TB2).

3. The FT spectrometer according to claim 1 or 2, wherein the first image field discriminator unit (BFD1; 631; 645; 653; 656; 666; 677; 976) and the second image field discriminator unit (BFD2; 632; 646; 654; 657 ; 667; 678; 977) are arranged with respect to each other such that there is at least approximately an optical conjugation between the first image field discriminator unit (BFD1; 631; 645; 653; 656; 666; 677; 976) and the second image field discriminator unit (BFD2; 632; 646; 654; 657; 667; 678; 977).

4. The FT spectrometer according to one of the preceding claims, wherein the two-beam interferometer corresponds to a Michelson-type interferometer, comprising the second beam deflection unit (642), wherein

the first beam deflection unit (641) includes a two-mirror corner reflector, which comprises the following

a first mirror (641a) configured to reflect the first partial beam at least a first time; and
a second mirror (641b) arranged at an angle $\Theta_1$ with respect to the first mirror and configured to reflect the first partial beam (TB1) at least a second time; and/or

the second beam deflection unit (642) includes a further two-mirror corner reflector, which comprises the following

a first mirror (624a) configured to reflect the second partial beam (TB2) at least a first time; and
a second mirror (642b) arranged at an angle $\Theta_2$ with respect to the first mirror and configured to reflect the second partial beam (TB2) at least a second time.

5. The FT spectrometer according to one of the preceding claims, wherein the two-beam interferometer corresponds to a Michelson-type interferometer, comprising the second beam deflection unit (642), wherein the first beam deflection unit (641) includes a triple mirror corner reflector, which comprises the following

a first mirror (641a) configured to reflect the first partial beam at least a first time; and
a second mirror (641b) arranged at an angle $\Theta_1$ with respect to the first mirror and configured to reflect the first partial beam (TB1) at least a second time; and
a third mirror which is arranged at an angle $\Theta_{a1}$ with respect to the first mirror (641a) and at an angle $\Theta_{b1}$ with respect to the second mirror (641b) and is configured to reflect the first partial beam (TB1) at least a third time; and/or

the second beam deflection unit (642) includes a further triple mirror corner reflector, which comprises the following

a first mirror (642a) configured to reflect the second partial beam (TB2) at least a first time; and
a second mirror (642b) arranged at an angle $\Theta_2$ with respect to the first mirror and configured to reflect the second partial beam (TB2) at least a second time; and
a third mirror arranged at an angle $\Theta_{a2}$ with respect to the first mirror (642a) and at an angle $\Theta_{b2}$ with respect to the second mirror (642b) and configured to reflect the second partial beam (TB2) at least a third time,

wherein optionally

the first image field discriminator unit (645) includes an opening configured to spatially select the first partial beam (TB1) after the first reflection by means of the first mirror (641a) of the first beam deflection unit (641) and before the second reflection by means of the second mirror (641b) of the first beam deflection unit (641); and/or
the second image field discriminator unit (646) includes a further opening configured to spatially select the second partial beam (TB2) after the first reflection by means of the first mirror (642a) of the second beam deflection unit (642) and before the second reflection by means of the second mirror (642b) of the second beam deflection unit (642).

6. The FT spectrometer according to one of the preceding claims, wherein the two-beam interferometer corresponds to a Michelson-type interferometer, comprising the second beam deflection unit (652), wherein

the first beam deflection unit (651) further comprises
a first prism with

a first reflection surface (651a) configured to reflect the first partial beam (TB1) at least a first time; and
a second reflection surface (651b) configured to reflect the first partial beam (TB1) at least a second time; and/or

the second beam deflection unit (652) further comprises
a second prism with

a first reflection surface (652a) configured to reflect the second partial beam (TB2) at least a first time; and
a second reflection surface (652b) configured to reflect the second partial beam (TB2) at least a second time.

7. The FT spectrometer according to claim 6, wherein

the first image field discriminator unit (653) includes an opening configured to spatially select the first partial beam (TB1) after splitting of the incident light beam (EB) by means of the beam splitter unit (622) and before the first reflection of the first partial beam (TB1) by means of the first reflection surface (651a) of the first prism (651); and/or

the second image field discriminator unit (654) includes an opening configured to spatially select the second partial beam (TB2) after splitting of the incident light beam (EB) by means of the beam splitter unit (622) and before the first reflection of the second partial beam (TB2) by means of the first reflection surface (652a) of the second prism (652).

8. The FT spectrometer according to one of claims 1 to 3, wherein the two-beam interferometer corresponds to a Mach-Zehnder interferometer, comprising the second beam deflection unit (662), and wherein the beam splitter unit (624, 625, 626, 627) further comprises:

a first single beam splitter (624, 625) for splitting the incident light beam (EB) into the first partial beam (TB1) and the second partial beam (TB2); and
a second single beam splitter (626, 627) for at least partially spatially superimposing the first partial beam (TB1) and the second partial beam (TB2), wherein optionally
the first beam deflection unit (661) further comprises

a first mirror (661a) configured to reflect the first partial beam (TB1) at least a first time; and
a second mirror (661b) arranged at an angle $\Theta_3$ with respect to the first mirror (661a) and configured to reflect the first partial beam (TB1) at least a second time; and/or

the second beam deflection unit (662) further comprises

a first mirror (662a) configured to reflect the second partial beam (TB2) at least a first time; and
a second mirror (662b) arranged at an angle $\Theta_4$ with respect to the first mirror (662a) and configured to reflect the second partial beam (TB2) at least a second time.

9. The FT spectrometer according to claim 8, wherein

the first image field discriminator unit (666) includes an opening configured to spatially select the first partial beam (TB1) after the first reflection by means of the first mirror (661a) of the first beam deflection unit (661) and before the second reflection by means of the second mirror (661b) of the first beam deflection unit (661); and/or
the second image field discriminator unit (667) includes a further opening configured to spatially select the second partial beam (TB2) after the first reflection by means of the first mirror (662a) of the second beam deflection unit (662) and before the second reflection by means of the second mirror (662b) of the second beam deflection unit (662).

10. The FT spectrometer according to one of claims 1 to 3, wherein the two-beam interferometer corresponds to a cyclic two-beam interferometer, wherein

the first beam deflection unit (630; 697) is configured to also deflect the second partial beam (TB2) at least a first and a second time,
the first image field discriminator unit (631; 656; 677; 976) includes an opening configured to spatially select the first partial beam (TB1) after the first deflection and before the second deflection; and
the second image field discriminator unit (632; 657; 678; 977) includes a further opening configured to spatially select the second partial beam (TB2) after the first deflection and before the second deflection.

11. The FT spectrometer according to claim 10, wherein the beam deflection unit comprises a two-mirror corner reflector (630), which comprises the following

a first mirror (628) configured to reflect the first partial beam (TB1) at least a first time and to reflect the second partial beam (TB2) at least a second time; and
a second mirror (629) arranged at an angle epsilon with respect to the first mirror and configured to reflect the second partial beam (TB2) at least a first time and to reflect the first partial beam (TB1) at least a second time.

12. The FT spectrometer according to claim 10, wherein the beam deflection unit (697) comprises a double prism arrangement, which comprises the following

a first prism (679; 687; 689; 887) with at least one reflection surface (888) configured to reflect the first partial beam (TB1) at least a first time and to reflect the second partial beam (TB2) at least a second time; and
a second prism (680; 688; 689; 896) with at least one reflection surface (890) configured to reflect the second partial beam (TB2) at least a first time and to reflect the first partial beam (TB1) at least a second time.

13. The FT spectrometer according to one of claims 10 to 12, wherein

the first image field discriminator unit (631) includes an opening configured to spatially select the first partial beam (TB1) after the first reflection by means of the first mirror (628) or the reflection surface (888) of the first prism (679; 687; 689; 887) and before the second reflection by means of the second mirror (629) or the reflection surface (890) of the second prism (680; 688; 689; 896); and
the second image field discriminator unit (632) includes a further opening configured to spatially select the second partial beam (TB2) after the first reflection by means of the second mirror (629) or the reflection surface (890) of the second prism (680; 688; 689; 896) and before the second reflection by means of the first mirror (628) or the reflection surface (888) of the first prism (679; 687; 689; 887).

14. A method for interferometric measurement using an FT spectrometer with a two-beam interferometer, comprising:

splitting an incident light beam, which is emitted by a spatially extended object, into a first partial beam and a second partial beam by means of a beam splitter unit;
first and second deflection of the first partial beam by means of a first beam deflection unit;
first and second deflection of the second partial beam by means of the first beam deflection unit or by means of a second beam deflection unit, wherein the first and second deflection of the first partial beam and the second partial beam generates a lateral shear(s);
spatially selecting at least part of the first partial beam, after splitting and before the second deflection, by means of a first image field discriminator unit in the two-beam interferometer; and
spatially selecting at least part of the second partial beam, after splitting and before the second deflection, by means of a second image field discriminator unit in the two-beam interferometer, wherein at least one of the two image field discriminator units is formed in a slit shape;
or spatially selecting at least part of the first partial beam - after splitting and after the second deflection - by means of a first image field discriminator unit in the two-beam interferometer; and
or spatially selecting at least part of the second partial beam - after splitting and after the second deflection - by means of a second image field discriminator unit in an image plane in the two-beam interferometer;
sending the incident light beam through an upstream objective before splitting in order to generate a plurality of coherent image points of the spatially extended object in an image plane between the beam splitter unit and a detector;
at least partially spatially superimposing the first partial beam and the second partial beam by means of the beam splitter unit;
at least partially imaging the plurality of coherent image points while simultaneously generating a plurality of spatial interferograms on a detector field of the detector based on the spatial superposition;
detecting the plurality of interferograms with the detector;
Fourier transformation of the plurality of spatial interferograms to generate a plurality of spectra and, based thereon, generate a hyperspectral image of at least a section of the spatially extended object,
imaging the first image field discriminator unit (BFD1; 631; 645; 653; 656; 666; 677; 976) and the second image field discriminator unit (BFD2; 632; 646; 654; 657; 667; 678; 977) by means of an anamorphic objective (51; 53; 58; 59; 875, 876, 877) downstream of the two-beam interferometer, and
adjusting the at least one upstream objective (70; 71; 73; 74; 75) such that such that respectively one image plane (BEI1, BEI2) of the at least one upstream objective (70; 71; 73; 74; 75) lies in the first image field discriminator unit (BFD1; 631; 645; 653; 656; 666; 677; 976) and in the second image field discriminator unit (BFD2; 632; 646; 654; 657; 667; 678; 977).

15. The method for interferometric measurement according to claim 14, further comprising the steps that are triggered by at least one computing unit:

simultaneously acquiring the plurality of spatial interferograms several times at different points in time;
Fourier transforming the plurality of spatial interferograms acquired at respectively different points in time to generate a plurality of spectra; and
generating a hyperspectral image of the spatially extended object.

**Revendications**

1. Spectromètre à transformée de Fourier, spectromètre FT, présentant :

   un interféromètre à deux ondes présentant :

   au moins un module séparateur d'onde (622 ; 623 ; 620 ; 636 ; 673, 674, 675) pour la division d'un faisceau lumineux incident (EB) d'un objet à expansion spatiale (1 ; 10 ; 11 ; 14; 15; 16) en un premier faisceau d'ondes partielles (TB1) et un second faisceau d'ondes partielles (TB2) ;
   au moins un premier module de déviation d'onde (630 ; 643 ; 651 ; 661 ; 697) qui est conçu pour dévier le premier faisceau d'ondes partielles (TB1) au moins une première et une deuxième fois, dans lequel
   le premier module de déviation d'onde (630, 697) est conçu pour dévier également le second faisceau d'ondes partielles (TB2) au moins une première et
   une deuxième fois; ou
   l'interféromètre à deux ondes comprend un second module de déviation d'onde (644 ; 652 ; 662) qui est conçu pour dévier le second faisceau d'ondes partielles (TB2) au moins une première et une deuxième fois, dans lequel le module séparateur d'onde (622 ; 623 ; 620 ; 636 ; 673, 674, 675) est également conçu pour superposer spatialement au moins partiellement le premier faisceau d'ondes partielles (TB1) et le second faisceau d'ondes partielles (TB2), et dans lequel les première et seconde déviations respectives du premier faisceau d'ondes partielles (TB1) et du second faisceau d'ondes partielles (TB2) génèrent un décalage latéral (s) ;
   au moins un premier module discriminateur de champ d'image (BFD1 ; 631 ; 645 ; 653 ; 656 ; 666 ; 677 ; 976) qui est agencé de sorte que le premier faisceau d'ondes partielles (TB1) est sélectionné spatialement après la division et avant la seconde déviation ;
   au moins un second module discriminateur de champ d'image (BFD2 ; 632 ; 646 ; 654 ; 657 ; 667 ; 678 ; 977) qui est agencé de sorte que le second faisceau d'ondes partielles (TB2) est sélectionné spatialement après la division et avant la seconde déviation, dans lequel au moins un des deux modules discriminateurs de champ d'image est réalisé en forme de colonne ;

   dans lequel le spectromètre FT comprend en outre :

   au moins un objectif disposé en amont (70 ; 71 ; 73 ; 74; 75) qui est agencé en face du module séparateur d'onde de sorte que le faisceau lumineux incident (EB) passe l'objectif au moins partiellement avant sa division au niveau du module séparateur d'onde, et le premier faisceau d'ondes partielles (TB1) et le second faisceau d'ondes partielles (TB2) génèrent chacun une pluralité de points d'image cohérents (O'1, O'2) de l'objet à expansion spatiale (1 ; 10; 11 ; 14 ; 15 ; 16) dans un plan d'image entre le module séparateur d'onde et un détecteur (54) ;
   le détecteur (54, 55, 634) pour la détection d'une pluralité d'interférogrammes spatiaux (rI1, rI2, rI3, rI4) sur la base de la superposition spatiale du premier faisceau d'ondes partielles (TB1) et du second faisceau d'ondes partielles (TB2) qui correspond à la représentation au moins partielle de la pluralité de points d'image cohérents (O'1, O'2) ; et
   au moins une unité centrale pour la transformée de Fourier de la pluralité d'interférogrammes spatiaux (rI1, rI2, rI3, rI4), pour la génération d'une pluralité de spectres (Sp1, Sp2, Sp3, Sp4) et en se basant sur ceux-ci pour la génération d'une image hyperspectrale de l'objet à expansion spatiale,
   dans lequel le premier module discriminateur de champ d'image (BFD1 ; 631 ; 645 ; 653 ; 656 ; 666 ; 677 ; 976) et le second module discriminateur de champ d'image (BFD2 ; 632 ; 646 ; 654 ; 657 ; 667 ; 678 ; 977) sont représentés par un objectif anamorphique (51 ; 53 ; 58; 59; 875, 876, 877) disposé en aval de l'interféromètre à deux ondes, et
   dans lequel l'au moins un objectif disposé en amont (70; 71 ; 73 ; 74; 75) est conçu pour être aligné de sorte qu'un plan d'image (BEI1, BEI2) de l'au moins un objectif disposé en amont (70 ; 71 ; 73 ; 74 ; 75) se situe à chaque fois dans le premier module discriminateur de champ d'image (BFD1 ; 631 ; 645; 653 ; 656; 666; 677; 976) et dans le second module discriminateur de champ d'image (BFD2 ; 632 ; 646 ; 654 ; 657 ; 667 ; 678 ; 977).

2. Spectromètre FT selon la revendication 1, dans lequel le module séparateur d'onde présente un séparateur d'onde individuel (622 ; 623 ; 636; 673, 674) pour

   diviser le faisceau lumineux incident (EB) d'un objet à expansion spatiale (1 ; 10; 11; 14; 15; 16) en le premier faisceau d'ondes partielles (TB1) et le second faisceau d'ondes partielles (TB2) ; et
superposer spatialement au moins partiellement le premier faisceau d'ondes partielles (TB1) et le second faisceau d'ondes partielles (TB2).

3. Spectromètre FT selon la revendication 1 ou 2, dans lequel le premier module discriminateur de champ d'image (BFD1 ; 631 ; 645 ; 653 ; 656; 666; 677 ; 976) et le second module discriminateur de champ d'image (BFD2 ; 632 ; 646 ; 654; 657; 667; 678; 977) sont agencés l'un par rapport à l'autre de sorte qu'une conjugaison optique entre le premier module discriminateur de champ d'image (BFD1 ; 631 ; 645 ; 653 ; 656 ; 666 ; 677; 976) et le second module discriminateur de champ d'image (BFD2 ; 632 ; 646; 654; 657; 667; 678 ; 977) existe au moins approximativement.

4. Spectromètre FT selon une des revendications précédentes, dans lequel l'interféromètre à deux ondes correspond à un interféromètre de type Michelson, comprenant le second module de déviation d'onde (642), dans lequel

le premier module de déviation d'onde (641) présente un réflecteur angulaire à deux miroirs qui comprend dans ce qui suit
un premier miroir (641a) qui est conçu pour réfléchir le premier faisceau d'ondes partielles au moins une première fois; et
un deuxième miroir (641b) qui est agencé à un angle $\Xi_1$ par rapport au premier miroir et est conçu pour réfléchir le premier faisceau d'ondes partielles (TB1) au moins une deuxième fois; et/ou
le second module de déviation d'onde (642) présente un autre réflecteur angulaire à deux miroirs qui comprend dans ce qui suit
un premier miroir (624a) qui est conçu pour réfléchir le second faisceau d'ondes partielles (TB2) au moins une première fois; et
un deuxième miroir (642b) qui est agencé à un angle $\Xi_2$ par rapport au premier miroir et est conçu pour réfléchir le second faisceau d'ondes partielles (TB2) au moins une deuxième fois.

5. Spectromètre FT selon une des revendications précédentes, dans lequel l'interféromètre à deux ondes correspond à un interféromètre de type Michelson, comprenant le second module de déviation d'onde (642), dans lequel le premier module de déviation d'onde (641) présente un réflecteur angulaire à triple miroir qui comprend dans ce qui suit

un premier miroir (641a) qui est conçu pour réfléchir le premier faisceau d'ondes partielles au moins une première fois; et
un deuxième miroir (641b) qui est agencé à un angle $\Xi_1$ par rapport au premier miroir et est conçu pour réfléchir le premier faisceau d'ondes partielles (TB1) au moins une deuxième fois; et
un troisième miroir qui est agencé à un angle $\Xi_{a1}$ par rapport au premier miroir (641a) et à un angle $\Xi_{b1}$ par rapport au deuxième miroir (641b), et est conçu pour réfléchir le premier faisceau d'ondes partielles (TB1) au moins une troisième fois; et/ou
le second module de déviation d'onde (642) présente un autre réflecteur angulaire à triple miroir qui comprend dans ce qui suit
un premier miroir (642a) qui est conçu pour réfléchir le second faisceau d'ondes partielles (TB2) au moins une première fois; et
un deuxième miroir (642b) qui est agencé à un angle $\Xi_2$ par rapport au premier miroir et est conçu pour réfléchir le second faisceau d'ondes partielles (TB2) au moins une deuxième fois; et
un troisième miroir qui est agencé à un angle $\Xi_{a2}$ par rapport au premier miroir (642a) et à un angle $\Xi_{b2}$ par rapport au deuxième miroir (642b), et est conçu pour réfléchir le second faisceau d'ondes partielles (TB2) au moins une troisième fois,
dans lequel en option
le premier module discriminateur de champ d'image (645) présente une ouverture qui est conçue pour sélectionner spatialement le premier faisceau d'ondes partielles (TB1) après la première réflexion au moyen du premier miroir (641a) du premier module de déviation d'onde (641) et avant la seconde réflexion au moyen du deuxième miroir (641b) du premier module de déviation d'onde (641) ; et/ou
le second module discriminateur de champ d'image (646) présente une autre ouverture qui est conçue pour sélectionner spatialement le second faisceau d'ondes partielles (TB2) après la première réflexion au moyen du premier miroir (642a) du second module de déviation d'onde (642) et avant la seconde réflexion au moyen du deuxième miroir (642b) du second module de déviation d'onde (642).

6. Spectromètre FT selon une des revendications précédentes, dans lequel l'interféromètre à deux ondes correspond à un interféromètre de type Michelson, comprenant le second module de déviation d'onde (652), dans lequel

le premier module de déviation d'onde (651) comprend en outre
un premier prisme avec
une première face de réflexion (651a) qui est conçue pour réfléchir le premier faisceau d'ondes partielles (TB1) au moins une première fois; et
une seconde face de réflexion (651b) qui est conçue pour réfléchir le premier faisceau d'ondes partielles (TB1) au moins une deuxième fois; et/ou
le second module de déviation d'onde (652) comprend en outre
un second prisme avec
une première face de réflexion (652a) qui est conçue pour réfléchir le second faisceau d'ondes partielles (TB2) au moins une première fois; et
une seconde face de réflexion (652b) qui est conçue pour réfléchir le second faisceau d'ondes partielles (TB2) au moins une deuxième fois.

7. Spectromètre FT selon la revendication 6, dans lequel

le premier module discriminateur de champ d'image (653) présente une ouverture qui est conçue pour sélectionner spatialement le premier faisceau d'ondes partielles (TB1) après la division du faisceau lumineux incident (EB) au moyen du module séparateur d'onde (622) et avant la première réflexion du premier faisceau d'ondes partielles (TB1) au moyen de la première face de réflexion (651a) du premier prisme (651) ; et/ou
le second module discriminateur de champ d'image (654) présente une ouverture qui est conçue pour sélectionner spatialement le second faisceau d'ondes partielles (TB2) après la division du faisceau lumineux incident (EB) au moyen du module séparateur d'onde (622) et avant la première réflexion du second faisceau d'ondes partielles (TB2) au moyen de la première face de réflexion (652a) du second prisme (652).

8. Spectromètre FT selon une des revendications 1 à 3, dans lequel l'interféromètre à deux ondes correspond à un interféromètre de Mach-Zehnder, comprenant le second module de déviation d'onde (662) et dans lequel le module séparateur d'onde (624, 625, 626, 627) comprend en outre :

un premier séparateur d'onde individuel (624, 625) pour la division du faisceau lumineux incident (EB) en le premier faisceau d'ondes partielles (TB1) et le second faisceau d'ondes partielles (TB2) ; et
un second séparateur d'onde individuel (626, 627) pour la superposition spatiale au moins partielle du premier faisceau d'ondes partielles (TB1) et du second faisceau d'ondes partielles (TB2),
dans lequel en option
le premier module de déviation d'onde (661) comprend en outre un premier miroir (661a) qui est conçu pour réfléchir le premier faisceau d'ondes partielles (TB1) au moins une première fois; et
un deuxième miroir (661b) qui est agencé à un angle $\Xi_3$ par rapport au premier miroir (661a) et est conçu pour réfléchir le premier faisceau d'ondes partielles (TB1) au moins une deuxième fois; et/ou
le second module de déviation d'onde (662) comprend en outre un premier miroir (662a) qui est conçu pour réfléchir le second faisceau d'ondes partielles (TB2) au moins une première fois; et
un deuxième miroir (662b) qui est agencé à un angle $\Xi_4$ par rapport au premier miroir (662a) et est conçu pour réfléchir le second faisceau d'ondes partielles (TB2) au moins une deuxième fois.

9. Spectromètre FT selon la revendication 8, dans lequel

le premier module discriminateur de champ d'image (666) présente une ouverture qui est conçue pour sélectionner spatialement le premier faisceau d'ondes partielles (TB1) après la première réflexion au moyen du premier miroir (661a) du premier module de déviation d'onde (661) et avant la seconde réflexion au moyen du deuxième miroir (661b) du premier module de déviation d'onde (661) ; et/ou
le second module discriminateur de champ d'image (667) présente une autre ouverture qui est conçue pour sélectionner spatialement le second faisceau d'ondes partielles (TB2) après la première réflexion au moyen du premier miroir (662a) du second module de déviation d'onde (662) et avant la seconde réflexion au moyen du deuxième miroir (662b) du second module de déviation d'onde (662).

10. Spectromètre FT selon une des revendications 1 à 3, dans lequel l'interféromètre à deux ondes correspond à un interféromètre à deux ondes cyclique, dans lequel

le premier module de déviation d'onde (630 ; 697) est conçu pour dévier également le second faisceau d'ondes partielles (TB2) au moins une première et une deuxième fois,

le premier module discriminateur de champ d'image (631 ; 656; 677; 976) présente une ouverture qui est conçue pour sélectionner spatialement le premier faisceau d'ondes partielles (TB1) après la première déviation et avant la seconde déviation ; et

le second module discriminateur de champ d'image (632; 657; 678; 977) présente une autre ouverture qui est conçue pour sélectionner spatialement le second faisceau d'ondes partielles (TB2) après la première déviation et avant la seconde déviation.

11. Spectromètre FT selon la revendication 10, dans lequel le module de déviation d'onde présente un réflecteur angulaire à deux miroirs (630) qui comprend dans ce qui suit

un premier miroir (628) qui est conçu pour réfléchir le premier faisceau d'ondes partielles (TB1) au moins une première fois et pour réfléchir le second faisceau d'ondes partielles (TB2) au moins une deuxième fois; et

un deuxième miroir (629) qui est agencé à un angle epsilon par rapport au premier miroir et est conçu pour réfléchir le second faisceau d'ondes partielles (TB2) au moins une première fois et pour réfléchir le premier faisceau d'ondes partielles (TB1) au moins une deuxième fois.

12. Spectromètre FT selon la revendication 10, dans lequel le module de déviation d'onde (697) présente un agencement à double prisme qui comprend dans ce qui suit

un premier prisme (679 ; 687 ; 689 ; 887) avec au moins une face de réflexion (888) qui est conçue pour réfléchir le premier faisceau d'ondes partielles (TB1) au moins une première fois et pour réfléchir le second faisceau d'ondes partielles (TB2) au moins une deuxième fois; et

un second prisme (680 ; 688 ; 689 ; 896) avec au moins une face de réflexion (890) qui est conçue pour réfléchir le second faisceau d'ondes partielles (TB2) au moins une première fois et pour réfléchir le premier faisceau d'ondes partielles (TB1) au moins une deuxième fois.

13. Spectromètre FT selon une des revendications 10 à 12, dans lequel

le premier module discriminateur de champ d'image (631) présente une ouverture qui est conçue pour sélectionner spatialement le premier faisceau d'ondes partielles (TB1) après la première réflexion au moyen du premier miroir (628) ou de la face de réflexion (888) du premier prisme (679 ; 687 ; 689 ; 887) et avant la seconde réflexion au moyen du deuxième miroir (629) ou de la face de réflexion (890) du second prisme (680 ; 688 ; 689 ; 896) ; et

le second module discriminateur de champ d'image (632) présente une autre ouverture qui est conçue pour sélectionner spatialement le second faisceau d'ondes partielles (TB2) après la première réflexion au moyen du deuxième miroir (629) ou de la face de réflexion (890) du second prisme (680 ; 688 ; 689 ; 896) et avant la seconde réflexion au moyen du premier miroir (628) ou de la face de réflexion (888) du premier prisme (679 ; 687 ; 689 ; 887).

14. Procédé de mesure interférométrique au moyen d'un spectromètre FT avec un interféromètre à deux ondes comprenant :

division d'un faisceau lumineux incident qui est émis par un objet à expansion spatiale en un premier faisceau d'ondes partielles et un second faisceau d'ondes partielles au moyen d'un module séparateur d'onde ;

première et seconde déviation du premier faisceau d'ondes partielles au moyen d'un premier module de déviation d'onde ;

première et seconde déviation du second faisceau d'ondes partielles au moyen du premier module de déviation d'onde ou au moyen d'un second module de déviation d'onde, dans lequel les première et seconde déviations du premier faisceau d'ondes partielles et du second faisceau d'ondes partielles génèrent un décalage latéral (s) ;

sélection spatiale d'au moins une partie du premier faisceau d'ondes partielles, après la division et avant la seconde déviation, au moyen d'un premier module discriminateur de champ d'image dans l'interféromètre à deux ondes ; et

sélection spatiale d'au moins une partie du second faisceau d'ondes partielles, après la division et avant la seconde déviation, au moyen d'un second module discriminateur de champ d'image dans l'interféromètre à deux ondes, dans lequel au moins un des deux modules discriminateurs de champ d'image est réalisé en forme de colonne ;

ou sélection spatiale d'au moins une partie du premier faisceau d'ondes partielles, après la division et après la seconde déviation, au moyen d'un premier module discriminateur de champ d'image dans l'interféromètre à deux ondes ; et

ou sélection spatiale d'au moins une partie du second faisceau d'ondes partielles, après la division et après la seconde déviation, au moyen d'un second module discriminateur de champ d'image dans un plan d'image dans l'interféromètre à deux ondes ;

émission du faisceau lumineux incident à travers un objectif disposé en amont avant la division, pour la génération d'une pluralité de points d'image cohérents de l'objet à expansion spatiale dans un plan d'image entre le module séparateur d'onde et un détecteur ;

superposition spatiale au moins partielle du premier faisceau d'ondes partielles et du second faisceau d'ondes partielles au moyen du module séparateur d'onde ;

représentation au moins partielle de la pluralité de points d'image cohérents avec génération simultanée d'une pluralité d'interférogrammes spatiaux sur un champ de détecteur du détecteur sur la base de la superposition spatiale ;

détection de la pluralité d'interférogrammes au moyen du détecteur ;

transformée de Fourier de la pluralité d'interférogrammes spatiaux pour la génération d'une pluralité de spectres et en se basant sur ceux-ci génération d'une image hyperspectrale d'au moins une section de l'objet à expansion spatiale,

représentation du premier module discriminateur de champ d'image (BFD1 ; 631 ; 645 ; 653 ; 656 ; 666 ; 677 ; 976) et du second module discriminateur de champ d'image (BFD2 ; 632 ; 646 ; 654 ; 657 ; 667 ; 678 ; 977) au moyen d'un objectif anamorphique (51 ; 53 ; 58 ; 59 ; 875, 876, 877) disposé en aval de l'interféromètre à deux ondes, et

alignement de l'au moins un objectif disposé en amont (70 ; 71 ; 73 ; 74 ; 75) de sorte qu'un plan d'image (BEI1, BEI2) de l'au moins un objectif disposé en amont (70 ; 71 ; 73 ; 74 ; 75) se situe à chaque fois dans le premier module discriminateur de champ d'image (BFD1 ; 631 ; 645 ; 653 ; 656 ; 666 ; 677 ; 976) et dans le second module discriminateur de champ d'image (BFD2 ; 632 ; 646 ; 654 ; 657 ; 667 ; 678 ; 977).

15. Procédé de mesure interférométrique selon la revendication 14, comprenant en outre les étapes qui sont déclenchées au moyen d'au moins une unité centrale :

détection simultanée répétée de la pluralité d'interférogrammes spatiaux à différents instants respectifs ;
transformée de Fourier de la pluralité d'interférogrammes spatiaux détectée à différents instants respectifs pour la génération d'une pluralité de spectres ; et génération d'une image hyperspectrale de l'objet à expansion spatiale.

**Figur 1**

**Figur 2**

EP 3 760 992 B1

**Figur 3**

Detail 3.5
Detail 3.4
Detail 3.2
Detail 3.1
Detail 3.3
Detail 3.6

EP 3 760 992 B1

Figur 4

Detail 4.1

Detail 4.2

Detail 4.3

Detail 4.4

Figur 6

Figur 5

Figur 8

Figur 7

Figur 9

Figur 10

EP 3 760 992 B1

**Figur 11**

Figur 12

Detail 12.2

Detail 12.1

Detail 12.3

Figur 13

Figur 14

Detail 14.1
Detail 14.2
Detail 14.3
Detail 14.4
Detail 14.5

Figur 15

Figur 16

EP 3 760 992 B1

Figur 17

Figur 18a

Figur 18b

EP 3 760 992 B1

**Figur 19a**

**Figur 19b**

Figur 20

Figur 21

Figur 22

Figur 23

Figur 24

Detail 24.1

Detail 24.2

**Figur 25**

**Detail 26.2**

**Detail 26.1**

**Detail 26.3**

**Figur 26**

Detail 27.1

Detail 27.2

Figur 27

**Figur 29**

**Figur 28a**

**Figur 28b**

Figur 30

Figur 31

EP 3 760 992 B1

Figur 32

**Figur 33**

EP 3 760 992 B1

Figur 34

Figur 35

Figur 36

Detail 36.1

Detail 36.2

Detail 36.3

B, Detail 37.2

A, Detail 37-1

Detail 37-3

um 90° gedreht

**Figur 37**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4976542 A
- US 5777736 A
- US 3684379 A
- US 4523846 A
- US 1565533 A
- US 5131747 A
- CN 106338342 A
- US 5539517 A
- US 6930781 B2
- US 5541728 A
- US 2019162520 A1
- WO 2013140396 A1

**In der Beschreibung aufgeführte Nicht-Patentliteratur**

- **MALACARA.** Optical Shop Testing. John Wiley & Sons, Inc, 1992, 140-141
- Steel, Interferometry. Cambridge University Press, 1967, 83
- **CHENGMIAO LIU.** Large field-of-view Fourier transform imaging spectrometer using dualchannel stitching. *OPTICS EXPRESS,* 12. Dezember 2016, vol. 24 (25), 28473-28490
- **T. OKAMOTO ; S. KAWATA ; S. MINAMI.** *Applied Optics,* 15. Januar 1984, vol. 23 (2), 269-273
- **YUNZHAO WU.** Global estimates of lunar iron and titanium contents from the Chang' E-1 IIM Data. *JOURNAL OF GEOPHYSICAL RESEARCH,* 2012, vol. 117
- Parallel two-step phase-shifting point-diffraction interferometry for microscopy based on a pair of cube beamsplitters. *Opt. Express,* 2011, vol. 19, 1930-1935